# EUROPEAN PATENT SPECIFICATION

(11) **EP 2 872 161 B1**
(45) Date of publication and mention of the grant of the patent: **16.12.2020**
(21) Application number: 13809749.8
(22) Date of filing: 24.06.2013
(51) Int. Cl.: A61K 31/047, A61K 31/075, A61K 31/336, A61K 45/06, A61K 38/12, A61P 35/00, A61P 35/02

(54) **DIANHYDROGALACTITOL FOR USE IN TREATING TYROSINE-KINASE-INHIBITOR-RESISTANT MALIGNANCIES IN PATIENTS WITH GENETIC POLYMORPHISMS OR AHI1 DYSREGULATIONS OR MUTATIONS**
DIANHYDROGALACTITOL ZUR VERWENDUNG BEI DER BEHANDLUNG VON TYROSINKINASEINHIBITOR-RESISTENTEN MALIGNOMEN BEI PATIENTEN MIT GENETISCHEN POLYMORPHISMEN ODER AHI1-DYSREGULIERUNGEN ODER MUTATIONEN
DIANHYDROGALACTITOL POUR UTILISATION DANS LE TRAITEMENT DE MALIGNITÉS RÉSISTANTES À UN INHIBITEUR DE TYROSINE KINASE CHEZ DES PATIENTS AYANT DES POLYMORPHISMES GÉNÉTIQUES OU DES DÉRÉGULATIONS OU DES MUTATIONS D'AHI1

(30) Priority: 26.06.2012 US 201261664279 P; 17.05.2013 US 201361824672 P
(43) Date of publication of application: 20.05.2015
(73) Proprietor: Del Mar Pharmaceuticals, Vancouver (CA)
(72) Inventor: BROWN, Dennis M., Menlo Park, California 94025 (US); BACHA, Jeffrey A., Vancouver, British Columbia V5T 4T5 (CA); GARNER, William J., San Francisco, California 94109 (US)
(74) Representative: MacLean, Martin Robert
(86) International application number: PCT/US2013/047320
(87) International publication number: WO 2014/004376

(56) References cited:
- WO-A1-2012/082074
- WO-A2-2013/142817
- US-A1- 2007 154 931
- US-A1- 2007 154 931
- US-A1- 2011 287 003
- US-A1- 2012 053 063
- US-A1- 2012 149 648
- DATABASE WPI Week 200950 Thomson Scientific, London, GB; AN 2009-K59818 XP002754069, & CN 101 444 490 A (TIANJIN JINGUIGU XYLITOL CO LTD) 3 June 2009 (2009-06-03)
- Xiaoyan Jiang ET AL: "Deregulated expression in Ph+ human leukemias of AHI-1, a gene activated by insertional mutagenesis in mouse models of leukemia", Blood, 15 May 2004 (2004-05-15), pages 1-9, XP055246386, Retrieved from the Internet: URL:http://www.bloodjournal.org/content/10 3/10/3897.long?sso-checked=true [retrieved on 2016-02-01]
- DATABASE WPI Week 200948 Thomson Scientific, London, GB; AN 2009-K61061 XP002754070, & CN 101 450 100 A (TIANJIN JINSHI PHARMACY CO LTD) 10 June 2009 (2009-06-10)
- RUBIN J ET AL: "221: Platinum base polychemotherapy versus dianhydrogalactitol (DAG) in advanced non small cell lung cancer (NSCLC)", AMERICAN ASSOCIATION FOR CANCER RESEARCH. PROCEEDINGS; 68TH ANNUAL MEETING OF THE AMERICAN ASSOCIATION FOR CANCER RESEARCH, AMERICAN ASSOCIATION FOR CANCER RESEARCH, US, vol. 18, 1 January 1977 (1977-01-01), page 56, XP008178841, ISSN: 0569-2261
- D. F. Chiuten ET AL: "Clinical Trials with the Hexitol Derivatives in the U S", Cancer, 1 January 1981 (1981-01-01), pages 442-451, XP055246265, Retrieved from the Internet: URL:http://onlinelibrary.wiley.com/store/1 0.1002/1097-0142(19810201)47:3<442::AID-CN CR2820470304>3.0.CO;2-1/asset/2820470304_f tp.pdf?v=1&t=ik3s821c&s=a52eeca81089ba4dc4 9ca930ac19b4e0ef023e13 [retrieved on 2016-02-01]
- JOSS R A ET AL: "New agents in non-small cell lung cancer", CANCER TREATMENT REVIEWS, SAUNDERS, US, vol. 11, no. 3, 1 September 1984 (1984-09-01), pages 205-236, XP023271025, ISSN: 0305-7372, DOI: 10.1016/0305-7372(84)90009-4 [retrieved on 1984-09-01]
- EAGAN R T ET AL: "EVALUATION OF AN INTERMITTENT SCHEDULE OF MITOLACTOL IN ADVANCED NON-SMALL-CELLLUNGCANCER", CANCER TREATMENT REPORTS, US GOV. PRINT. OFF, US, vol. 65, no. 11-12, 1 November 1981 (1981-11-01), pages 1099-1102, XP008178844, ISSN: 0361-5960
- MARSONI S ET AL: "Clinical drug development: An analysis of phase II trials, 1970-1985", CANCER TREATMENT REPORTS, US GOV. PRINT. OFF, US, vol. 71, no. 1, 1 January 1987 (1987-01-01), pages 71-80, XP008178842, ISSN: 0361-5960
- BAKOWSKI M T ET AL: "Chemotherapy of non-small cell lung cancer: a reappraisal and a look to the future", CANCER TREATMENT REVIEWS, SAUNDERS, US, vol. 10, no. 3, 1 September 1983 (1983-09-01), pages 159-172, XP023288583, ISSN: 0305-7372, DOI: 10.1016/0305-7372(83)90030-0 [retrieved on 1983-09-01]
- KING PAN NG ET AL: "A common BIM deletion polymorphism mediates intrinsic resistance and inferior responses to tyrosine kinase inhibitors in cancer", NATURE MEDICINE, vol. 18, no. 4, 18 March 2012 (2012-03-18) , pages 521-528, XP055144177, ISSN: 1078-8956, DOI: 10.1038/nm.2713
- ZHOU ET AL.: 'AHI-1 interacts with BCR-ABL and modulates BCR-ABL transforming activity and imatinib response of CML stemlprogenitor cells' J. EXP. MED. vol. 205, no. 11, 20 October 2008, pages 2657 - 2671, XP055144531
- PONASSI ET AL.: 'A novel Bim-BH3-derived Bcl-XL inhibitor.' CELL CYCLE vol. 7, no. 20, 15 October 2008, pages 3211 - 3224, XP055183941
- PELLICANO ET AL.: 'Assembling défenses against therapy-resistant leukemic stem cells: Bcl6 joins the ranks' J. EXP. MED. vol. 208, no. 11, 24 October 2011, pages 2155 - 2158, XP055144531

### SEQUENCE LISTING

<110> Del Mar Pharmaceuticals Brown, Dennis M. Bacha, Jeffrey A. Garner, William J.
<120> METHODS FOR TREATING TYROSINE-KINASE-INHIBITOR-RESISTANT MALIGNANCIES IN PATIENTS WITH GENETIC POLYMORPHISMS OR AHI1 DYSREGULATIONS OR MUTATIONS EMPLOYING DIANHYDROGALACTITOL, DIACETYLDIANHYDROGALACTITOL, DIBROMODULCITOL, OR ANALOGS OR DERIVATIVES THEREOF
<130> P6886PC00
<140> PCT/US13/47320
   <141> 2013-06-24
<150> US 61/824,672
   <151> 2013-05-17
<150> US 61/664,279
   <151> 2012-06-24
<160> 13
<170> PatentIn version 3.5
<210> 1
   <211> 198
   <212> PRT
   <213> Homo sapiens
<400> 1
<210> 2
   <211> 15
   <212> PRT
   <213> Homo sapiens
<400> 2
   Ile Ala Gln Glu Leu Arg Arg Ile Gly Asp Glu Phe Asn Ala Tyr
   1               5                   10                  15
<210> 3
   <211> 9
   <212> PRT
   <213> Homo sapiens
<400> 3
   Leu Ala Cys Ile Gly Asp Glu Met Asp
   1               5
<210> 4
   <211> 9
   <212> PRT
   <213> Homo sapiens
<400> 4
   Leu Lys Ala Leu Gly Asp Glu Leu Asp
   1               5
<210> 5
   <211> 9
   <212> PRT
   <213> Homo sapiens
<400> 5
   Leu Ala Ile Ile Gly Asp Asp Ile Asn
   1               5
<210> 6
   <211> 9
   <212> PRT
   <213> Homo sapiens
<400> 6
   Leu Ala Gln Val Gly Asp Ser Met Asp
   1               5
<210> 7
   <211> 9
   <212> PRT
   <213> Homo sapiens
<400> 7
   Leu Lys Arg Ile Gly Asp Glu Leu Asp
   1               5
<210> 8
   <211> 9
   <212> PRT
   <213> Homo sapiens
<400> 8
   Leu Lys Lys Asn Ser Asp Trp Ile Trp
   1               5
<210> 9
   <211> 9
   <212> PRT
   <213> Homo sapiens
<400> 9
   Leu Arg Arg Met Ser Asp Glu Phe Glu
   1               5
<210> 10
   <211> 9
   <212> PRT
   <213> Homo sapiens
<400> 10
   Leu Arg Gln Ala Gly Asp Asp Phe Ser
   1               5
<210> 11
   <211> 9
   <212> PRT
   <213> Homo sapiens
<400> 11
   Leu Arg Glu Ala Gly Asp Glu Phe Glu
   1               5
<210> 12
   <211> 60
   <212> DNA
   <213> Homo sapiens
<400> 12
   gatccccgtg atgatcccga cactatttca agagaatagt gtcgggatca tcacttttta 60
<210> 13
   <211> 60
   <212> DNA
   <213> Homo sapiens
<400> 13
   agcttaaaaa gtgatgatcc cgacactatt ctcttgaaat agtgtcggga tcatcacggg 60

## Description

### FIELD OF THE INVENTION

The invention is defined by the claims. The present invention is directed to a medicament comprising a therapeutically effective quantity of an alkylating hexitol derivative for use in the treatment of a malignancy in a patient resistant to tyrosine kinase inhibitor (TKI) chemotherapy wherein the malignancy is characterized by resistance to at least one tyrosine kinase inhibitor (TKI) due to:
(1) at least one mutation in a gene encoding a protein that is a target of at least one TKI; or
(2) the presence of at least one additional gene in either a wild-type or mutated state encoding a protein that confers resistance to the therapeutic effects of at least one TKI,
wherein the additional gene in either a wild-type or mutated state encoding the protein that confers resistance to the therapeutic effects of at least one TKI is AHI-1, or
wherein the resistance to at least one TKI is due to a mutation in the kinase domain of ABL1 protein that is part of a BCR-ABL fusion protein that is a target of TKIs,
wherein the alkylating hexitol derivative is dianhydrogalactitol, and
wherein the malignancy is selected from the group consisting of chronic myelogenous leukemia (CML), non-small cell lung carcinoma (NSCLC) and triple-negative breast cancer.
Described herein are methods for treating tyrosine-kinase-inhibitor resistant malignancies in patients with genetic polymorphisms, methods for treating malignancies where resistance is mediated by expression of the *AHI1* gene, or methods for treating triple-negative breast cancer, employing dianhydrogalactitol, diacetyldianhydrogalactitol, dibromodulcitol, or analogs or derivatives thereof, as well as pharmaceutical compositions for treating tyrosine-kinase-inhibitor resistant malignancies in patients with genetic polymorphisms, malignancies where resistance is mediated by expression of the *AHI1* gene, or triple-negative breast cancer.

### BACKGROUND OF THE INVENTION

The use of tyrosine kinase inhibitors (TKIs) has been responsible for effective therapeutic responses in patients presenting with a range of malignancies believed to be driven by the activity of oncogenic kinases (P.A. Janne et al., "Factors Underlying Sensitivity of Cancers to Small-Molecule Kinase Inhibitors," Nat. Rev. Drug Discov. 8: 709-723 (2009). However, before the use of TKIs, such malignancies were generally regarded as highly chemoresistant, as exemplified by breakpoint cluster region (BCR)-c-abl oncogene 1, non-receptor tyrosine kinase (ABL1) kinase-driven chronic myelocytic leukemia (CML) and EGFR non-small-cell lung carcinoma (NSCLC) (A.M. Carella et al., "New Insights in Biology and Current Therapeutic Options for Patients with Chronic Myelogenous Leukemia," Haematotogica 82: 478-95 (1997) and J.H. Schiller et al., "Comparison of Four Chemotherapy Regimens for Advanced Non-Small-Cell Lung Cancer," New Engl. J. Med. 346: 92-98 (2002). After the advent and clinical use of TKIs, treatment responses in both of these malignancies typically approached 80% (V.L. Keedy et al., "American Society of Clinical Oncology Provisional Clinical Opinion: Epidermal Growth Factor Receptor (EGFR) Mutation Testing for Patients with Advanced Non-Small-Cell Lung Cancer Considering First-Line EGFR Tyrosine Kinase Inhibitor Therapy," J. Clin. Oncol. 29: 2121-2127 (2011) and M. Baccarani et al., "Chronic Myeloid Leukemia: An Update of Concepts and Management Recommendations of European LeukemiaNet," J. Clin. Oncol. 27: 6041-6051 (2009). CN101444490A describes an injection preparation containing anhydrogalactitol and a preparation method thereof. CN1014501 00A describes a medicine composition for treating urgent and chronic myelocytic leukemia. Rubin et. al. (American Association for Cancer Research Proceedings; 68th annual meeting of the American Association for Cancer Research, American Association for Cancer Research US, vol. 18, 1 January 1977; page 5) describes platinum base polychemotherapy versus dianhydrogalactitol (DAG) in advanced non-small cell lung cancer (NSCLC). Chiuten et. al. (Cancer, 1 January 1981, pages 442-45) describes clinical trials with the hexitol derivatives in the US. Joss et. al. (Cancer Treatment Review, Saunders, US, vol. 11, no. 3, 1 September 1984, pages 205-236) describes new agents in non-small cell lung cancer. Eagan et. al. (Cancer Treatment Reports, US Gov. Print. Off, US, vol. 65, no. 11-12, 1 November 1981, pages 1099-1102) describes evaluation of an intermittent schedule of mitolactol in advanced non-small cell lung cancer. Marsoni et. al. (Cancer Treatment Reports, US Gov. Print. Off, US, vol. 71, no. 1, 1 January 1987, pages 71-80) describes clinical drug development: an analysis of phase I trials 1970-1985). Bakowski et. al. (Cancer Treatment Review, Saunders, US, vol. 10, no. 3, 1 September 1983, pages 159-172) describes chemotherapy of non-small cell lung cancer: a reappraisal and a look to the future

However, as effective as TKIs have proven to be in treating a number of types of malignancy that had previously been considered untreatable by chemotherapy, there is a significant proportion of patients that is resistant to TKI chemotherapy. Many of these patients are of East Asian ancestry, suggesting the existence of genetic variations that may cause resistance to TKI chemotherapy.

Therefore, there is an urgent need for therapeutic methods and pharmaceutical compositions that can treat malignancies in patients resistant to TKI chemotherapy.

Additionally, there are other malignancies, particularly, but not limited to, chronic lymphocytic leukemia, that are associated with the *AHI1* gene, particularly with mutation or dysregulation of the *AHI1* gene. The *AHI1* gene is a gene that encodes a modular protein with WD40 repeat and SH3 domains. Insertion of provirus at the genomic location of this gene is associated with the development of malignancy, possibly by the expression of truncated forms of the gene (X. Jiang et al., "Ahi-1, a Novel Gene Encoding a Modular Protein with WD40-Repeat and SH3 Domains, Is Targeted by the Ahi-1 and Mis-2 Provirus Insertions," J. Virol. 76: 9046-9059 (2002). Human leukemias that are Philadelphia chromosome-positive (Ph⁺) also show deregulated expression of the *AHI1* gene (X. Jiang et al., "Deregulated Expression in Ph+ Human Leukemias of AHI-1, a Gene Activated by Insertional Mutagenesis in Mouse Models of Leukemia," Blood 103: 3897-3904 (2004).

Therefore, there is an urgent need for improved methods to treat malignancies associated with mutation or dysregulation of the *AHI1* gene, particularly leukemias.

Additionally, triple-negative breast cancer is a form of breast cancer that is characterized by tumors that do not express *estrogen receptor (ER)*, *progesterone receptor (PR)*, or *HER-2* genes. This form of breast cancer represents an important clinical challenge because these cancers do not respond to endocrine therapy or to a number of targeted agents. Current treatment strategies for triple-negative breast cancer include many chemotherapy agents, such as the anthracyclines, taxanes, ixabepilone, and platinum agents, as well as selected biologic agents and possibly anti-EGFR drugs.

However, there is also an urgent need for improved methods to treat triple-negative breast cancer.

### SUMMARY OF THE INVENTION

The present disclosure is directed to methods and pharmaceutical compositions that provide an alternative treatment route for the treatment of malignancies in patients resistant to TKI chemotherapy.

One aspect of the invention is a medicament comprising a therapeutically effective quantity of an alkylating hexitol derivative for use in the treatment of a malignancy in a patient resistant to tyrosine kinase inhibitor (TKI) chemotherapy wherein the malignancy is characterized by resistance to at least one tyrosine kinase inhibitor (TKI) due to:
(1) at least one mutation in a gene encoding a protein that is a target of at least one TKI; or
(2) the presence of at least one additional gene in either a wild-type or mutated state encoding a protein that confers resistance to the therapeutic effects of at least one TKI,
wherein the additional gene in either a wild-type or mutated state encoding the protein that confers resistance to the therapeutic effects of at least one TKI is *AHI-1*, or
wherein the resistance to at least one TKI is due to a mutation in the kinase domain of ABL1 protein that is part of a BCR-ABL fusion protein that is a target of TKIs,
wherein the alkylating hexitol derivative is dianhydrogalactitol, and
wherein the malignancy is selected from the group consisting of chronic myelogenous leukemia (CML), non-small cell lung carcinoma (NSCLC) and triple-negative breast cancer. In one alternative, wherein the additional gene in either a wild-type or mutated state encoding a product that confers resistance to the therapeutic effects of at least one TKI is *AHI-1.* In another alternative, the resistance to at least one TKI is due to a mutation in the kinase domain of ABL1 protein that is part of a BCR-ABL fusion protein that is a target of TKIs. The method can further comprise the step of administering a therapeutically effective quantity of a BH3 mimetic as claimed to the subject. Alternatively, the method can further comprise the step of administering a therapeutically effective quantity of a STAT5 inhibitor as claimed, a JAK2 inhibitor as claimed, a Src inhibitor as claimed, or a combination of two or more kinase inhibitors as claimed.

Another aspect of the invention is a medicament comprising a therapeutically effective quantity of dianhydrogalactitol for use in the treatment of a malignancy in a subject resistant to TKI chemotherapy suffering from a malignancy who has a germline deletion polymorphism conferring resistance to tyrosine kinase inhibitors (TKIs), wherein the malignancy is selected from the group consisting of chronic myelogenous leukemia (CML), non-small cell lung carcinoma (NSCLC) and triple-negative breast cancer.

The malignancy can be chronic myelogenous leukemia (CML), non-small cell lung carcinoma (NSCLC) or triple-negative breast cancer.

A therapeutic agent for use in a method for the treatment of a malignancy in a subject suffering from a malignancy associated with a mutation or a dysregulation of the *AHI1* gene comprising the step of administering a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy is described herein. The malignancy, in this alternative described herein, can be chronic myelocytic leukemia. The method described herein can further comprise the administration of a therapeutically effective quantity of an agent that modulates the expression or activity of either the *AHI1* gene or the AHI1 protein.

A medicament for use in a method that combines screening for the germline deletion polymorphism and, if the germline deletion polymorphism is found to exist, treatment of a malignancy resistant to TKIs in a subject is described herein.

In general, this method described herein comprises the steps of:
(1) screening for the germline deletion polymorphism in a subject with a malignancy; and
(2) if the germline deletion polymorphism is found to exist in the subject with the malignancy, administering a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy.

A medicament for use as claimed can further comprise a therapeutically effective quantity of: (1) a BH3 mimetic as claimed; or (2) both a BH3 mimetic as claimed and a tyrosine kinase inhibitor therapeutic agent as claimed.

Suitable BH3 mimetics include:
(1) peptides;
(2) modified peptides;
(3) terpyridine-based peptidomimetics;
(4) terephthalamide-based peptidomimetics;
(5) benzoylurea-based peptidomimetics;
(6) obatoclax;
(7) TW37;
(8) an analog of TW37 as claimed;
(9) (-) gossypol;
(10) gossypol derivatives;
(11) A-385358;
(12) an analog or derivative of A-385358 as claimed;
(13) ABT-737;
(14) an analog of ABT-737 as claimed;
(15) ABT-263;
(16) an analog of ABT-263 as claimed;
(17) TM-1206; and
(18) an analog of TM-1206 as claimed.

A particularly preferred BH3 mimetic is ABT-737. An isoxazolidine derivative is described herein.

The tyrosine kinase inhibitor can be imatinib, bosutinib, nilotinib, erlotinib, afatinib, dacomitinib or dasatinib. A particularly preferred tyrosine kinase inhibitor is imatinib.

There is described herein a method to improve the efficacy and/or reduce the side effects of the administration of an alkylating hexitol derivative for treatment of a TKI-resistant malignancy comprising the steps of:
(1) identifying at least one factor or parameter associated with the efficacy and/or occurrence of side effects of the administration of an alkylating hexitol derivative for treatment of a TKI-resistant malignancy; and
(2) modifying the factor or parameter to improve the efficacy and/or reduce the side effects of the administration of the alkylating hexitol derivative for treatment of the TKI-resistant malignancy.

Typically, in this method described herein, the factor or parameter is selected from the group consisting of:
(1) dose modification;
(2) route of administration;
(3) schedule of administration;
(4) indications for use;
(5) selection of disease stage;
(6) other indications;
(7) patient selection;
(8) patient/disease phenotype;
(9) patient/disease genotype;
(10) pre/post-treatment preparation
(11) toxicity management;
(12) pharmacokinetic/pharmacodynamic monitoring;
(13) drug combinations;
(14) chemosensitization;
(15) chemopotentiation;
(16) post-treatment patient management;
(17) alternative medicine/therapeutic support;
(18) bulk drug product improvements;
(19) diluent systems;
(20) solvent systems;
(21) excipients;
(22) dosage forms;
(23) dosage kits and packaging;
(24) drug delivery systems;
(25) drug conjugate forms;
(26) compound analogs;
(27) prodrugs;
(28) multiple drug systems;
(29) biotherapeutic enhancement;
(30) biotherapeutic resistance modulation;
(31) radiation therapy enhancement;
(32) novel mechanisms of action;
(33) selective target cell population therapeutics; and
(34) use with an agent enhancing its activity.

A composition to improve the efficacy and/or reduce the side effects of suboptimally administered drug therapy employing an alkylating hexitol derivative for the treatment of a TKI resistant malignancy is described herein comprising an alternative selected from the group consisting of:
(i) a therapeutically effective quantity of a modified alkylating hexitol derivative or a derivative, analog, or prodrug of an alkylating hexitol derivative or a modified alkylating hexitol derivative, wherein the modified alkylating hexitol derivative or the derivative, analog or prodrug of the modified alkylating hexitol derivative possesses increased therapeutic efficacy or reduced side effects for treatment of a TKI-resistant malignancy or a malignancy associated with mutation or dysregulation of the *AHI1* gene as compared with an unmodified alkylating hexitol derivative;
(ii) a composition comprising:
   (a) a therapeutically effective quantity of an alkylating hexitol derivative, a modified alkylating hexitol derivative, or a derivative, analog, or prodrug of an alkylating hexitol derivative or a modified alkylating hexitol derivative; and
   (b) at least one additional therapeutic agent, therapeutic agent subject to chemosensitization, therapeutic agent subject to chemopotentiation, diluent, excipient, solvent system, or drug delivery system, wherein the composition possesses increased therapeutic efficacy or reduced side effects for treatment of a TKI-resistant malignancy or a malignancy associated with mutation or dysregulation of the *AHI1* gene as compared with an unmodified alkylating hexitol derivative;
(iii) a therapeutically effective quantity of an alkylating hexitol derivative, a modified alkylating hexitol derivative, or a derivative, analog, or prodrug of an alkylating hexitol derivative or a modified alkylating hexitol derivative that is incorporated into a dosage form, wherein an alkylating hexitol derivative, a modified alkylating hexitol derivative, or a derivative, analog, or prodrug of an alkylating hexitol derivative or a modified alkylating hexitol derivative incorporated into the dosage form possesses increased therapeutic efficacy or reduced side effects for treatment of a TKI-resistant malignancy or a malignancy associated with mutation or dysregulation of the *AHI1* gene as compared with an unmodified alkylating hexitol derivative;
(iv) a therapeutically effective quantity of an alkylating hexitol derivative, a modified alkylating hexitol derivative, or a derivative, analog, or prodrug of an alkylating hexitol derivative or a modified alkylating hexitol derivative that is incorporated into a dosage kit and packaging, wherein an alkylating hexitol derivative, a modified alkylating hexitol derivative, or a derivative, analog, or prodrug of an alkylating hexitol derivative or a modified alkylating hexitol derivative incorporated into the dosage kit and packaging possesses increased therapeutic efficacy or reduced side effects for treatment of a TKI-resistant malignancy or a malignancy associated with mutation or dysregulation of the *AHI1* gene as compared with an unmodified alkylating hexitol derivative; and
(v) a therapeutically effective quantity of an alkylating hexitol derivative, a modified alkylating hexitol derivative, or a derivative, analog, or prodrug of an alkylating hexitol derivative or a modified alkylating hexitol derivative that is subjected to a bulk drug product improvement, wherein the an alkylating hexitol derivative, a modified alkylating hexitol derivative, or a derivative, analog, or prodrug of an alkylating hexitol derivative or a modified alkylating hexitol derivative subject to the bulk drug product improvement possesses increased therapeutic efficacy or reduced side effects for treatment of a TKI-resistant malignancy or a malignancy associated with mutation or dysregulation of the *AHI1* gene as compared with an unmodified alkylating hexitol derivative.

The composition described herein can include, for example: a drug combination, including, among other alternatives, a BH3 mimetic; a therapeutic agent subject to chemosensitization; a therapeutic agent subject to chemopotentiation; a bulk drug product improvement; a diluent; a solvent system; an excipient; a dosage form; a dosage kit and packaging; a drug delivery system; a modification of the therapeutic agent; a prodrug system; or a multiple drug system.

### BRIEF DESCRIPTION OF THE DRAWINGS

These and other features, aspects, and advantages of the present invention will become better understood with reference to the following description, appended claims, and accompanying drawings where:
Figure 1 is a graph showing the effect of dianhydrogalactitol on the growth of MDA-MB-231 cancer cells, using concentrations of dianhydrogalactitol from 0.1 to 100 µM, using 3000 cells/well and a period of 72 hours (two repetitions).
Figure 2 is a graph showing the effect of dianhydrogalactitol on the growth of HCC1143 cancer cells, using concentrations of dianhydrogalactitol from 0.1 to 100 µM, using 3000 cells/well and a period of 72 hours (two repetitions).
Figure 3 is a graph showing the effect of dianhydrogalactitol on the growth of K562 cancer cells, using concentrations of dianhydrogalactitol from 0.1 to 100 µM, using 2000 cells/well and a period of 72 hours (two repetitions).
Figure 4 is a graph showing the effect of dianhydrogalactitol on the growth of K562-Ahi-1 cancer cells, using concentrations of dianhydrogalactitol from 0.1 to 100 µM, using 2000 cells/well and a period of 72 hours (two repetitions).

### DETAILED DESCRIPTION OF THE INVENTION

The present invention is defined by the claims. Methods and pharmaceutical compositions that can treat malignancies in patients who are resistant to TKI chemotherapy, particularly in patients whose resistance is due to genetic polymorphisms are described herein. Methods and pharmaceutical compositions that can treat malignancies associated with mutation or dysregulation of the *AHI1* gene, particularly leukemias are described herein. Methods and pharmaceutical compositions that can treat triple-negative breast cancer are described herein.

Recent work has established that resistance to TKI chemotherapy is at least partially due to genetic polymorphisms that affect the apoptotic response to TKI.

Specifically, these polymorphisms include, but are not necessarily limited to, polymorphisms in the gene *BCL2L11* (also known as *BIM*), which encodes a BH3-only protein that is a BCL-2 family member. The BH3-only proteins activate cell death by either opposing the prosurvival members of the BCL2 family (BCL2, BCL2-like 1 (BCL-XL, also known as BCL2L1), myeloid cell leukemia sequence 1 (MCL1) and BCL2-related protein A1 (BCL2A1)) or by binding to the pro-apoptotic BCL2 family members (BCL2-associated X protein (BAX) and BCL2-antagonist/killer 1 (BAK1)) and directly activating their pro-apoptotic functions; the activation of pro-apoptotic functions would result in cell death (R.J. Youle & A. Strasser, "The BCL-2 Protein Family: Opposing Activities that Mediate Cell Death," Nat. Rev. Mol. Cell. Biol. 9: 47-59 (2008).

It also has been previously shown that several kinase-driven cancers, such as CML and EGFR NSCLC, can maintain a survival advantage by suppressing *BIM* transcription and also by targeting BIM protein for proteasomal degradation through mitogen-activated protein kinase 1 (MAPK-1)-dependent phosphorylation. In all of these malignancies, BIM upregulation is required for TKIs to induce apoptosis of cancer cells, and suppression of BIM expression is sufficient to confer *in vitro* resistance to TKIs (J. Kuroda et al., "Bim and Bad Mediate Imatinib-Induced Killing of Bcr/Abl+ Leukemic Cells, and Resistance Due to Their Loss is Overcome by a BH3 Mimetic," Proc. Natl. Acad. Sci. USA 103: 14907-14912 (2006); K.J. Aichberger et al., "Low-Level Expression of Proapoptotic Bcl-2-Interacting Mediator in Leukemic Cells in Patients with Chronic Myeloid Leukemia: Role of BCR/ABL, Characterization of Underlying Signaling Pathways, and Reexpression by Novel Pharmacologic Compounds," Cancer Res. 65: 9436-9444 (2005); R. Kuribara et al., "Roles of Bim in Apoptosis of Normal and Bcl-Abr-Expressing Hematopoietic Progenitors," Mol. Cell. Biol. 24: 6172-6183 (2004); M.S. Cragg et al., "Gefitinib-Induced Killing of NSCLC Cell Lines Expressing Mutant EGFR Requires BIM and Can Be Enhanced by BH3 Mimetics," PLoS Med. 4: 1681-1689 (2007); Y. Gong et al., "Induction of BIM Is Essential for Apoptosis Triggered by EGFR Kinase Inhibitors in Mutant EGFR-Dependent Lung Adenocarcinomas," PLoS Med. 4: e294 (2007); D.B. Costa et al., "BIM Mediates EGFR Tyrosine Kinase Inhibitor-Induced Apoptosis in Lung Cancers with Oncogenic EGFR Mutations," PLoS Med. 4: 1669-1679 (2007).

One recent finding has been the discovery of a deletion polymorphism in the *BIM* gene that results in the generation of alternatively spliced isoforms of BIM that lack the crucial BH3 domain that is involved in the promotion of apoptosis. This polymorphism has a profound effect on the TKI sensitivity of CML and EGFR NSCLC cells, such that one copy of the deleted allele is sufficient to render cells intrinsically TKI resistant. This polymorphism therefore functions in a dominant manner to render such cells resistant to TKI chemotherapy. This finding also includes the result that individuals with the polymorphism have markedly inferior responses to TKI than do individuals without the polymorphism. In particular, the presence of the polymorphism was correlated with a lesser degree of response to imatinib, a TKI, in CML, as well as a shorter progression-free survival (PFS) with EGFR TKI therapy in EGFR NSCLC (K.P. Ng et al., "A Common BIM Deletion Polymorphism Mediates Intrinsic Resistance and Inferior Responses to Tyrosine Kinase Inhibitors in Cancer," Nature Med. doi 10.138/nm.2713 (March 18, 2012).

To identify these new TKI-resistance mechanisms in CML, massively parallel DNA sequencing of paired-end ditags was performed to interrogate the genomes of five CML samples obtained from subjects who were either sensitive to or resistant to treatment with TKIs. The BCR-ABL1 translocation was identified in all CML samples, but not in control samples obtained from patients in complete remission.

Although various structural variations were found that were common to all the TKI-resistant samples, one particular variation was considered to be especially significant. This variation occurred in intron 2 of the *BIM* gene and comprised an identical 2903-bp deletion that was common to all three samples from resistant patients; the fact that this variation was identical in all three patients suggested that it was germline and polymorphic. Screening resulted in the finding that this polymorphism occurred at significant frequency in patients of East Asian ancestry but was absent in individuals of African or European ancestry.

An inspection of the structure of the *BIM* gene suggested that the splicing of exon 3 and the splicing of exon 4 occur in a mutually exclusive manner because of the presence of a stop codon and a polyadenylation signal within exon 3. Sequencing of all identifiable *BIM* transcripts in CML cells has confirmed that exons 3 and 4 never occurred in the same transcript. Because of its close proximity (107 bp) to the intronexon boundary at the 5'-end of exon 3, it was hypothesized that the deletion polymorphism described above would result in preferential splicing of exon 3 over exon 4. When a minigene was constructed to assess whether the presence of the deletion would lead to the preferential inclusion of exon 3 over exon 4; results indicated that there was at least a fivefold preference for inclusion of exon 3 over exon 4 in this model system. The results in this model system were confirmed by studies of primary CML cells; the same preference was shown for polymorphism-containing CML cells, while general *BIM* transcription was unaffected by the polymorphism. Similar results were obtained in lymphoblastoid cell lines obtained from normal healthy HapMap individuals, indicating that the polymorphism has a cell-lineage-independent effect. These results therefore suggest that the 2.9-kb deleted region contains *cis* elements that suppress the splicing of *BIM* exon 3, which, in cells harboring the deletion, results in preferential splicing of exon 3 over exon 4.

The pro-apoptotic BH3 domain is encoded exclusively by exon 4 of *BIM* (M. Adachi et al., "Nomenclature of Dynein Light Chain-Linked BH3-Only Protein Bim Isoforms," Cell Death Different. 12: 192-193 (2005)). This domain is required for the apoptotic function of *BIM* (E.H Cheng et al., "BCL-2, BCL-X(L) Sequester BH3 Domain-Only Molecules Preventing BAX- and BAK-Mediated Apoptosis," Mol. Cell. 8: 705-711 (2001); D.C. Huang & A. Strasser, "BH3-Only Proteins: Essential Initiators of Apoptotic Cell Death," Cell 103: 839-842 (2000) These observations suggest a previously unidentified mechanism for TKI resistance. In this mechanism, after TKI exposure, polymorphism-containing CML cells, and, conceivably, other malignant cells carrying this polymorphism or other polymorphisms altering the splicing of *BIM*, would favor the expression of exon-3-containing over exon-4-containing *BIM* transcripts, resulting in the decreased expression of BH3-containing BIM isoforms, and, consequently, impaired BH3-domain-dependent apoptosis. To confirm this, a Japanese cell line, KCL22 (I. Kubonishi & I. Miyoshi, "Establishment of a Ph1 Chromosome-Positive Cell Line from Chronic Myelogenous Leukemia in Blast Crisis," Int. J. Cell Cloning 1: 105-117 (1983)) that contained the 2.9-kb deletion was tested; it was confirmed that cells from that line expressed an increased ratio of exon 3 to exon 4 transcripts compared to cells without the deletion. These KCL22 cells also showed a decreased induction of exon-4-containing transcripts after TKI exposure as well as decreased concentrations of BIMEL protein, a major BH3-containing BIM isoform (M. Adachi et al. (2005), supra).

Consistent with these findings, KCL22 cells were resistant to imatinib-induced apoptosis and showed impaired apoptotic signaling after imatinib exposure despite effective BCR-ABL1 inhibition, as confirmed by a decrease in BCR-ABL1-dependent signaling. KCL22 cells were also highly sensitive to the induction of apoptosis after increased expression of exon 4-containing and therefore BH3-encoding (but not exon 3 containing) *BIM* isoforms. This, in turn, suggests that the impaired imatinib-induced apoptosis in KCL22 cells could be restored by the addition of a BH3-mimetic drug, which functionally mimic BH3-only proteins by binding and inhibiting pro-survival BCL2 family members (M.S. Cragg et al., "Unleashing the Power of Inhibitors of Oncogenic Kinases Through BH3 Mimetics," Nat. Rev. Cancer 9: 321-326 (2009)).

One of these BH3 mimetics is ABT-737. ABT-737 is 4[4-[(4'-chloro[1,1'-biphenyl]-2-yl)methyl]1-piperazinyl]-N-[[4-[[(1R)-3-dimethylamino)-1-[(phenylthio)methyl]propyl]amino]-3-nitrophenyl]sulfonyl]benzamide. The structural formula of ABT-737 is shown below as Formula (I). The activity of ATB-737 is described in M.F. van Delft et al., "The BH3 Mimetic ABT-737 Targets Selective Bcl-2 Proteins and Efficiently Induces Apoptosis via Bak/Bax if Mcl-1 Is Neutralized," Cancer Cell 10: 398-399 (2006) and in M.F. Bruncko et al., "Studies Leading to Potent, Dual Inhibitors of Bcl-2 and Bcl-xL," J. Med. Chem. 50: 641-662 (2007).

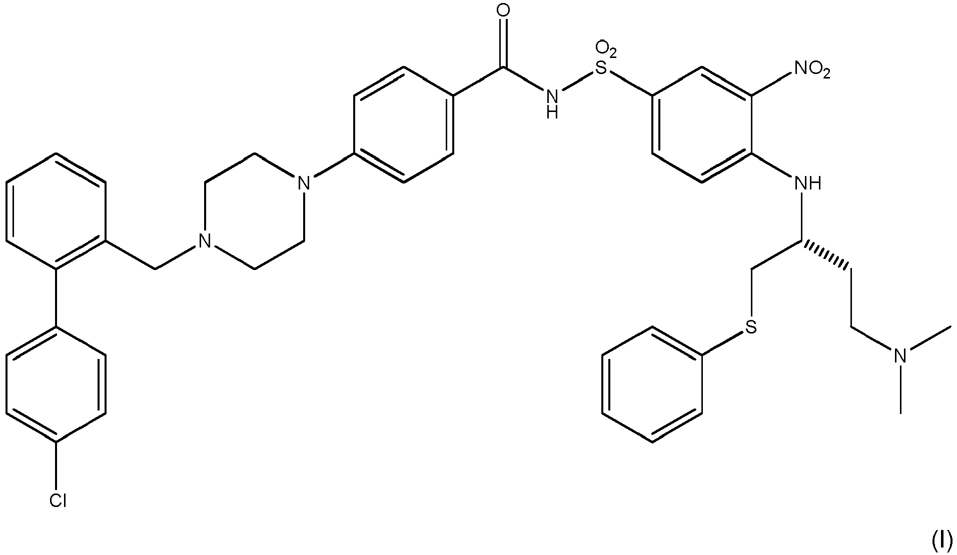

Additional BH3 mimetics described herein include, but are not limited to, analogs or derivatives of ABT-737, including the following: (1) analogs of ABT-737 wherein the chlorine bound to the benzene ring is replaced with fluorine, bromine, or iodine; (2) analogs of ABT-737 wherein one or more of the hydrogens of the benzene rings are replaced with lower alkyl; (3) analogs of ABT-737 wherein one or more of the hydrogens of the piperazinyl moiety are replaced with lower alkyl; and (4) analogs of ABT-737 where the dimethylamino moiety is replaced with another moiety including one or two lower alkyl groups bound to the amino group of the diethylamino moiety.

Other BH3 mimetics are known in the art and are described further below.

The use of a BH3 mimetic did indeed restore imatinib-induced apoptosis in KCL22 cells. The results also confirmed that siRNA-mediated knockdown of exon-3-containing transcripts did not sensitize KCL22 cells to imatinib, indicating that exon-3-containing isoforms probably do not play a significant role in TKI resistance.

Gene targeting facilitated by zinc finger nuclease (ZFN) was used to precisely recreate the deletion polymorphism in the *BIM* gene of originally imatinib-sensitive K562 CML cells. Before this targeting was undertaken, these K562 cells lacked the deletion polymorphism and were sensitive to imatinib; the cells responded to imatinib by initiating apoptosis. Subsequent to gene targeting facilitated by ZFN, the cells were then analyzed for changes in *BIM* splicing and expression, and for TKI-induced apoptosis. Subclones were generated that were heterozygous (K562-*BIM*^{i2+/-}) or homozygous (K562-*BIM*^{i2-/-}) for the deletion polymorphism. An increased ratio of exon 3 to exon 4 transcripts as well as a small but reproducible increase in BIM-γ protein expression in cells from both subclones in a polymorphism-dosage-dependent manner was observed. The low expression of BIM-γprotein, even in the cells homozygous for the deletion polymorphism, was attributed to the relatively short half-life of BIM-γprotein (< 1 hr). Cells containing the deletion polymorphisms also showed decreased induction of exon-4-containing transcripts after imatinib exposure, as well as increased upregulation of BIMEL protein, diminished apoptotic signaling and decreased apoptotic cell death, as measured by DNA fragmentation in an ELISA-based assay. As in KCL22 cells, the combination of the BH3 mimetic ABT-737, described above, with imatinib enhanced the ability of the thymidine kinase inhibitor imatinib to activate apoptosis in polymorphism-containing cells.

The most abundant BIM isoform, BIMEL, was re-expressed in polymorphism-containing cells with or without treatment with imatinib. Analogously to the effects seen with ABT-737, the forced expression of BIMEL also enhanced the ability of imatinib to activate apoptosis in deletion-containing K562 cells. Similarly, primary CML cells obtained from subjects with the deletion polymorphism were less sensitive to imatinib-induced death through apoptosis as compared to cells without the deletion and also that the relative TKI resistance of the deletion polymorphism-containing cells could be overcome by the addition of the BH3 mimetic ABT-737.

These results have established that the *BIM* deletion polymorphism impairs the apoptotic response to imatinib by biasing splicing away from BH3-containing isoforms and that this bias is sufficient to render CML cells intrinsically resistant to imatinib. These results have also established that the apoptotic response to imatinib can be restored in polymorphism-containing cells by treatment with BH3 mimetics, such as, but not limited to, ABT-737.

As indicated above, resistance to TKI therapy is particularly prevalent among subjects of East Asian ancestry. Therefore, a retrospective analysis has been performed on the influence of the deletion polymorphism on TKI responses in East Asian subjects with CML. This analysis involved a group of newly diagnosed patients with chronic phase CML from Singapore, Malaysian, or Japanese cohorts. In these patients, the clinical responses were compared for first-line therapy with a standard dose of imatinib (400 mg/day) in individuals with and without the deletion polymorphism. Clinical responses were classified according to European LeukemiaNet (ELN) criteria; resistant individuals were defined as "suboptimal responders" or "failures" according to the ELN criteria (which includes subjects who never achieve a complete cytogenic response or a 3-log decrease in BCR-ABL1 transcript levels), whereas sensitive individuals corresponded to ELN-defined "optimal responders," In both geographic cohorts, subjects with the deletion polymorphism were more likely to have resistant disease than sensitive disease compared to controls; the overall odds ratio for resistant disease among patients with the deletion polymorphism compared to those without it was 2.94, indicating a significant result. By contrast, no significant differences between the two groups with respect to other potential prognostic or confounding factors, including median time from diagnosis to initiation of imatinib treatment, Sokal score at diagnosis or prior treatment with interferon. It was also noted that the majority of resistant subjects with the deletion polymorphism subsequently did not respond to second-generation TKI therapy with bosutinib, nilotinib, or dasatinib, a finding that is consistent with the intrinsic resistance observed in the cell lines and also a finding that indicates that the resistance conferred by the deletion polymorphism is not limited to imatinib but extends to other TKI therapeutic agents.

TKI resistance in CML is most commonly associated with the acquisition of somatic mutations in the *BCR-ABL1* kinase domain, which can be found in up to 50% of resistant individuals in the chronic phase of disease (P. La Rosée & A. Hochhaus, "Resistance to Imatinib in Chronic Myelogenous Leukemia: Mechanisms and Clinical Implications," Curr. Hematol. Maliq. Rep. 3: 72-79 (2008)). However, the deletion polymorphism described above is germline and is sufficient to cause intrinsic TKI resistance *in vitro*, it was predicted that individuals possessing the germline deletion polymorphism would be resistant to TKI therapy even in the absence of a kinase-domain mutation. In a study to verify this hypothesis, the subjects were divided into the following three clinical groups: (1) resistant without a *BCR-ABL1* mutation; (2) resistant with a *BCR-ABL1* mutation; and (3) sensitive. Individuals with the deletion polymorphism, compared to those without, were more likely to be in group (1) than in groups (2) and (3) combined. This is strong evidence of the *in vivo* effect of the deletion polymorphism in generating resistance to TKI therapy.

The role of the *BIM* biomarker has also been validated in another kinase-driver cancer, namely EGFR NSCLC, in which sensitizing mutations in EGFR predict high response rates in patients treated with EGFR inhibitors (J.G. Paez et al., "EGFR Mutations in Lung Cancer: Correlation with Clinical Response to Gefitinib Therapy," Science 304: 1497-1500 (2004); T.J. Lynch et al., "Activating Mutations in the Epidermal Growth Factor Receptor Underlying Responsiveness of Non-Small-Cell Lung Cancer to Gefitinib," N. Engl. J. Med. 350: 2129-2139 (2004)) and in which BIM expression is required for TKI sensitivity. EGFR inhibitors described herein include, but are not limited to, gefitinib, erlotinib, cetuximab, lapatinib, panitumumab, and vandetanib. An additional and relevant aspect of non-small cell lung carcinoma is that it is particularly common in East Asian countries, where activating *EGFR* mutations can be found in up to 50% of NSCLCs (compared to 15% in western countries) and are enriched for among female East Asian nonsmokers.

Accordingly, a search was undertaken for NSCLC cell lines that harbored TKI-sensitizing *EGFR* mutations but were inexplicably TKI resistant (defined as lacking any of the known secondary-resistance-conferring mutations). One such cell line, HCC2279, was identified, which notably fails to activate apoptosis despite effective EGFR inhibition. The presence of the deletion polymorphism was confirmed in the HCC2279 cells; the effect of the presence of the polymorphism on BIM function was also determined. The deletion resulted in greater expression of exon-3-containing compared to exon-4-containing (and hence BH3-containing) BIM isoforms as compared to cells without the polymorphism. Notably, primary peripheral blood mononuclear cells from subjects with EGFR NSCLC, and with or without the deletion polymorphism, also showed the same results (increased expression of exon-3-containing BIM isoforms in cells with the polymorphism). HCC2279 cells also had decreased induction of exon-4-containing transcripts and BIMEL protein after TKI exposure, as well as impaired activation of apoptotic signaling as measured by poly (ADP-ribose) polymerase (PARP) cleavage. Consistent with the notion that TKI resistance is a result of decreased concentrations of BH3-containing BIM protein, the addition of the BH3-mimetic drug ABT-737 as described above enhanced TKI-induced apoptotic signaling and cell death. To confirm that the deletion polymorphism was sufficient to cause TKI resistance in EGFR NSCLC, the deletion polymorphism was introduced into TKI-sensitive PC9 cells. Analogous to the findings with respect to K562-*BIM*^{i2-/-} cells, it was found that, compared to PC9-*BIM*^{i2+/+} cells, PC9-*BIM*^{i2-/-} cells had decreased expression of exon-4-containing and BH3-containing BIM transcripts and protein, respectively, were intrinsically TKI resistant, and were resensitized to TKIs by the BH3 mimetic ABT-737.

A study was also undertaken to determine whether the presence of the deletion polymorphism correlated with the duration of response to EGFR TKIs in subjects with NSCLC with activating *EGFR* mutations. Individuals with or without the deletion polymorphism did not differ with respect to known prognostic factors, including stage (as more than 85% of the subjects were Stage IV). Nevertheless, the presence of the polymorphism was predictive of a significantly shorter progression-free survival (PFS), with a median PFS of 6.6 months in individuals with the deletion polymorphism compared to 11.9 months for those without it. In multivariate analyses using the Cox regression model, only the deletion polymorphism and the presence of the TKI-resistant exon 20 mutation (J. Wu et al., "Lung Cancer with Epidermal Growth Factor Exon 20 Mutations Is Associated with Poor Gefitinib Treatment Response," Clin. Cancer Res. 14: 4877-4882 (2008); H. Sasaki et al., "EGFR Exon 20 Insertion Mutation in Japanese Lung Cancer," Lung Cancer 58: 324-328 (2007)) emerged as independent prognostic factors for shorter PFS.

These results demonstrate the principle that, although cancers should be classified according to their somatically acquired driver mutations, germline polymorphisms can directly modulate the responses of such cancers to targeted therapies and can strongly influence clinical outcomes. In particular, these results demonstrate that a common *BIM* deletion polymorphism contributes to the heterogeneity of responses seen among molecularly defined patients with a particular type of cancer who are treated with targeted therapies. These results also highlight how a single germline polymorphism can strongly affect clinical outcomes in different cancers that share a common or substantially common biology and probably reflect the central role of BIM in mediating TKI sensitivity in those malignancies. This may well include other malignancies that also depend on BIM expression for TKI sensitivity (P.M. Gordon & D.E. Fisher, "Role for the Proapoptotic Factor BIM in Mediating Imatinib-Induced Apoptosis in a c-KIT-Dependent Gastrointestinal Stromal Tumor Cell Line," J. Biol. Chem. 285: 14109-14114 (2010); B. Will et al., "Apoptosis Induced by JAK2 Inhibition is Mediated by Bim and Enhanced by the BH3 Mimetic ABT-737 in JAK2 Mutant Human Erythroid Cells," Blood 115: 2901-2909 (2010)).

The BIM deletion polymorphism has been found only in individuals of East Asian descent. It is therefore interesting to note that in CML, a higher rate of incomplete cytogenetic responses to imatinib has been reported among individuals in East Asia (-50%) as compared to individuals in Europe and North America (26%). It has been estimated that the deletion polymorphism underlies resistance in ~21% of East Asian patients; this might explain, in part, the difference in complete cytogenetic response rates observed between these two world populations.

As a germline biomarker for TKI resistance, the *BIM* deletion polymorphism also offers several advantages over biomarkers comprising acquired (i.e., somatic) mutations. First, the *BIM* deletion polymorphism can be used at the time of initial presentation to predict which individuals are at an increased risk of developing TKI resistance. Secondly, the assessment of the polymorphism status of an individual does not require an analysis of tumor-specific DNA because the deletion polymorphism is a germline polymorphism that is not associated with the DNA of any particular tumor or class of tumors. The ability to screen for this deletion polymorphism at the time of initial presentation of the patient offers the potential for preventing the emergence of TKI resistance by therapeutic means, such as the administration of a BH3-mimetic drug at the time of initial presentation or at the first sign of resistance to one or more TKI therapeutic drugs. The fact that the deletion polymorphism is a germline polymorphism that is not associated with the DNA of any particular tumor or class of tumors is particularly advantageous in solid tumor situations, such as EGFR NSCLC, where a second biopsy for tumor-specific tissue usually necessitates an invasive procedure, with the risks that such a procedure may entail, including infection. These diagnostic measures can be undertaken together with measurement of *BIM* RNA levels in tumors before treatment to predict TKI responsiveness (A. Faber et al., "BIM Expression in Treatment-naïve Cancers Predicts Responsiveness to Kinase Inhibitors," Cancer Discov. 1: 352-365 (2011)); however, the discovery of the deletion polymorphism described above emphasizes the importance of biomarkers that can also predict the induction of functional isoforms of BIM after TKI exposure.

The results described above, in elucidating the effects of the deletion polymorphism on BIM function, also described a novel splicing mechanism by which the polymorphism contributes to drug resistance in CML and EGFR-driven NSCLC. This suggests that pharmacologic restoration of BIM function could overcome this particular form of TKI resistance in both cancers. These results also support the increasingly recognized role of alterations in the splicing patterns of genes in human disease (L. Cartegni et al., "Listening to Silence and Understanding Nonsense: Exonic Mutations That Affect Splicing," Nat. Rev. Genet. 3: 285-298 (2002); N. Lopez-Bigas et al., "Are Splicing Mutations the Most Frequent Cause of Hereditary Disease?," FEBS Lett. 579: 1900-1903 (2005) and provide a new example of an inherited germline mutation that contributes to resistance against targeted cancer therapies. Although the presence of the deletion polymorphism is strongly associated with clinical TKI resistance and shorter PFS, other genetic factors, both acquired and inherited, will probably dictate the final response to TKI therapy in any individual patient. Several other mechanisms of EGFR-independent resistance have been described, including upregulated hepatocyte growth factor-dependent signaling (S. Yano et al., "Hepatocyte Growth Factor Induces Gefitinib Resistance of Lung Adenocarcinoma with Epidermal Growth Factor Receptor-Activating Mutations," Cancer Res. 68: 9479-9487 (2008)), nuclear factor κ-light-chain-enhancer of activated B cells (NF-κB)-dependent signaling (T.G. Bivona et al., "FAS and NF-κB Signalling Modulate Dependence of Lung Cancers on Mutant EGFR," Nature 471: 523-526 (2011)) and v-Ki-ras2 Kirsten rat sarcoma viral oncogene homolog (KRAS) mutations (M. Takeda et al., "De Novo Resistance to Epidermal Growth Factor Receptor-Tyrosine Kinase Inhibitors in EGFR-Mutation-Positive Patients with Non-Small Cell Lung Cancer," J. Thorac. Oncol. 5: 399-400 (2010)).

Clinical resistance to TKIs is commonly classified as being primary or secondary, with the latter defined as occurring in individuals who experienced an initial response to TKI therapy and then later developed resistance. It is generally assumed that secondary resistance is mediated by acquired somatic mutations that emerge under the selective pressure of TKI therapy, whereas intrinsic mechanisms of resistance, including germline polymorphisms, are more likely to present with primary resistance and a lack of any upfront response. This line of reasoning is based on the assumption that resistance-conferring germline polymorphisms result in absolute as opposed to relative resistance to TKIs. However, the results described above show that, by creating both CML and EGFR NSCLC cells with the deletion polymorphism, it is demonstrated that the *BIM* polymorphism results in relative TKI resistance. This is consistent with cancer cells being sensitive to small changes in BIM protein concentrations (Kuroda et al. (2006), supra: A Egle et al., "Bim Is a Suppressor of Myc-Induced Mouse B Cell Leukemia," Proc. Natl. Acad. Sci. USA 101: 6164-6169 (2004)). This means that cells carrying the deletion polymorphism are not completely resistant to TKIs and may show some response in certain contexts.

These results also suggest that other polymorphisms may account for heterogeneity among other aspects of cancer biology. It is also possible that these polymorphisms may exist or may occur at higher frequencies in particular populations, especially populations that are known to be substantially endogamic. A number of such populations are known, and, in certain cases, these populations have been shown to have increased risk for particular types of cancer. In particular, as described below, therapy with alkylating hexitol derivatives as described herein is useful in patients possessing either germline mutations or somatic mutations.

The BH3 domain is a domain shared among a number of proteins in a family known s the BCL-2 family that have both pro-apoptotic and anti-apoptotic activities. These proteins include BAK, BAX, BIK, BID, and HRK in addition to BIM. The BH3 domain is conserved both in the pro-apoptotic and anti-apoptotic BCL-2 family proteins. The BH3 domain of the pro-apoptotic proteins serves a dual function. It is essential for their cell death activity and for mediating heterodimerization with anti-apoptosis proteins.

The sequence of native human BIMEL, the predominant form of BIM, is as follows:

The BH3 domain within this protein is residues 148-162 (15 amino acids), which has the sequence IAQELRRIGDEFNAY (SEQ ID NO: 2). Sequences analogous to portions of BH3 in BIMEL are found in other proteins, including LACIGDEMD (SEQ ID NO: 3) in BIK, LKALGDELD (SEQ ID NO: 4) in HRK, LAIIGDDIN (SEQ ID NO: 5) in BAK, LAQVGDSMD (SEQ ID NO: 6) in BID, LKRIGDELD (SEQ ID NO: 7) in BAX, LKKNSDWIW (SEQ ID NO: 8) in BNIP3, LRRMSDEFE (SEQ ID NO: 9) in BAD, LRQAGDDFS (SEQ ID NO: 10) in BCL-2, and LREAGDEFE (SEQ ID NO: 11) in BCL-X_{L}. These sequences have several amino acids in common, including an initial leucine (L), corresponding to the fifth amino acid of the BH3 domain in BIMEL, and an aspartic acid (D) corresponding to the tenth amino acid of the BH3 domain in BIMEL. Other identical or conservatively substituted amino acids occur in these sequences. These homologies are described in M. Yasuda et al., "Adenovirus E1B-19K/BCL-2 Interacting Protein BNIP3 Contains a BH3 Domain and a Mitochondrial Targeting Sequence," J. Biol. Chem. 273: 12415-12421 (1998).

As indicated above, a number of BH3 mimetics have been discovered, including ABT-737. BH3 mimetics are described in G. Lessene et al., "BCL-2 Family Antagonists for Cancer Therapy," Nature Rev. Drug Discovery 7: 989-1000 (2007).

In mammalian cells, the occurrence or non-occurrence of apoptosis is governed by interactions between pro-survival and pro-apoptotic proteins, particularly members of the BCL-2 family of proteins. In mammalian cells, five pro-survival proteins, BCL-2, BCL-X_{L}, BCL-w, MCL1, and A1, antagonize the pro-apoptotic function of BAK and BAX. The killing activity of BAK and BAX is localized on the mitochondrial outer membrane, which becomes permeabilized in response to death signals. As a result, cytochrome c is released from the mitochondria into the cytosol, leading to the activation of the caspase cascade and the induction of apoptosis.

The five pro-survival proteins as well as BAK and BAX all share four domains of sequence homology known as BCL-2 homology 1 (BH1), BH2, BH3, and BH4. They also have a carboxy-terminal membrane-anchoring sequence and a similar tertiary structure. However, there also exist other proteins that orchestrate apoptosis. These additional proteins with a pro-apoptotic function are the BH-3 only proteins, designated that because they lack the BH1, BH2, and BH4 domains. Eight BH3-only proteins are known in mammals, namely BIM, BID, PUMA, NOXA, BAD, BMF, HRK, and BIK; these proteins are upregulated by transcription or post-translational processing in response to stress signals.

The BH3 domain of pro-apoptotic proteins is the primary mediator of interactions with anti-apoptotic (pro-survival) family members. For example, it has been proposed that a pro-survival protein, BCL-X_{L}, antagonizes BAK or BAX, both of which promote apoptosis, by binding to their BH3 domain, and a BH3-only protein then relieves this antagonism by similarly binding to BCL-X_{L}; this will prevent the antagonism by competition. There may also be a direct interaction between certain BH3-only proteins and BAX. However, either model strongly suggests that a BH3 mimetic that binds pro-survival proteins will trigger or promote apoptosis.

There are selective interactions between BH3-only proteins and pro-survival proteins. BIM and PUMA bind to all five pro-survival proteins, while BAD and NOXA have complementary binding profiles. Mutated BH3 sequences with altered selectivity patterns have been discovered, as discussed further below.

Potential BH3 mimetics include:
(1) peptides;
(2) modified peptides;
(3) terpyridine-based peptidomimetics;
(4) terephthalamide-based peptidomimetics;
(5) benzoylurea-based peptidomimetics;
(6) obatoclax;
(7) TW37;
(8) (-) gossypol;
(9) gossypol derivatives;
(10) isoxazolidine derivatives;
(11) A-385358;
(12) ABT-737;
(13) ABT-263; and
(14) TM-1206.

General principles applicable to the design of BH3 mimetics include the following: (1) binding occurs between a hydrophobic groove located on Bcl-X_{L} and the BH3 domain of, for example, BAD; (2) the BH3-only protein BAD adopts a helical structure on binding to the hydrophobic groove located on Bcl-X_{L}; and (3) four hydrophobic amino acids located within the BH3 domain at positions i, i +3, i + 7, and i + 11 are essential to the binding of Bad to Bcl-X_{L} and interact in four hydrophobic pockets situated in the Bcl-X_{L} binding groove; the hydrophobic residues in the BH3 domain are highly conserved.

These BH3 mimetics are described in detail below.

Modified peptides include stapled BID BH3 helices, as described in L.D. Walensky et al. "A Stapled BID BH3 Helix Directly Binds and Activates BAX," Mol. Cell 24: 199-210 (2006) and in L. D. Walensky et al., "Activation of Apoptosis in Vivo by a Hydrocarbon-Stapled BH3 Helix," Science 305: 1466-1470 (2004). These stapled helices are produced by substitution of S-pentenylalanine derivatives into the BH3 helix at two positions, as well as the substitution of norleucine for methionine at an adjacent position. A hydrocarbon crosslink was then generated by ruthenium-catalyzed olefin metathesis, leaving a bridged structure with one double bond.

Another alternative for modified peptide BH3 mimetics include the helical peptide-based foldamers described in J.D. Sadowsky et al., "(α/β + α) Peptide Antagonists of BH3 Domain/Bcl-xL Recognition: Toward General Strategies for Foldamer-Based Inhibition of Protein-Protein Interactions," J. Am. Chem. Soc. 129: 139-154 (2007).

Terpyridine-based peptidomimetics are described in J.M. Davis et al., "Synthesis of a 2,3';6',3"-Terpyridine Scaffold as an α-Helix Mimetic," Org. Letters 7: 5404-5408 (2005).

Terephthalamide-based peptidomimetics are described in H. Yin & A.D. Hamilton, "Terephthalamide Derivatives as Mimetics of the Helical Region of Bak Peptide Target Bcl-xL Protein," Bioorq. Med. Chem. Lett. 14: 1375-1379 (2004).

Benzoylurea-based BH3 mimetics are described in United States Patent Application Publication No. 2008/0153802 by Lessene et al..

Obatoclax is 2-(2-((3,5-dimethyl-1*H*-pyrrol-2-yl)methylene)-3-methoxy-2*H*-pyrrol-5-yl)-1*H*-indole and has the structure shown in Formula (II), below.

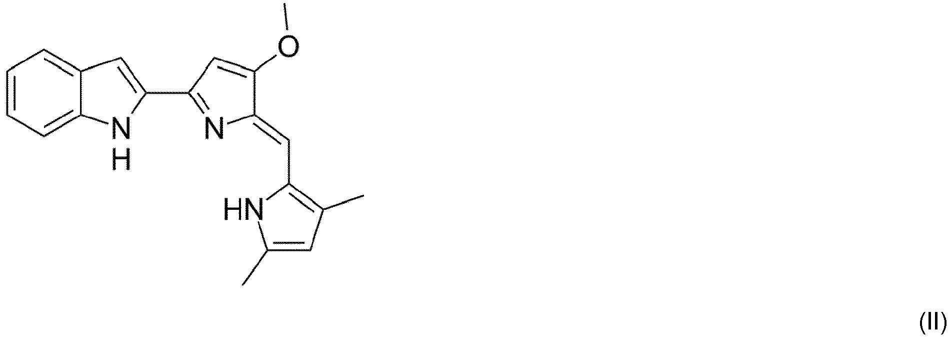

TW37 is *N*-[(2-tert-butyl-benzenesulfonyl)-phenyl]-2,3,4-trihydroxy-5-(2-isopropyl-benzyl)-benzamide and has the structure shown in Formula (III), below, and is described in G.P. Wang et al., "Structure-Based Design of Potent Small Molecule Inhibitors of the Anti-Apoptotic Bcl-2 Proteins," J. Med. Chem. 50: 3163-3166 (2006) and in M.A. Verhaegen et al., "A Novel BH3 Mimetic Reveals a Mitogen-Activated Protein Kinase-Dependent Mechanism of Melanoma Cell Death Controlled by p53 and Reactive Oxygen Species," Cancer Res. 66: 11348-11359 (2006).

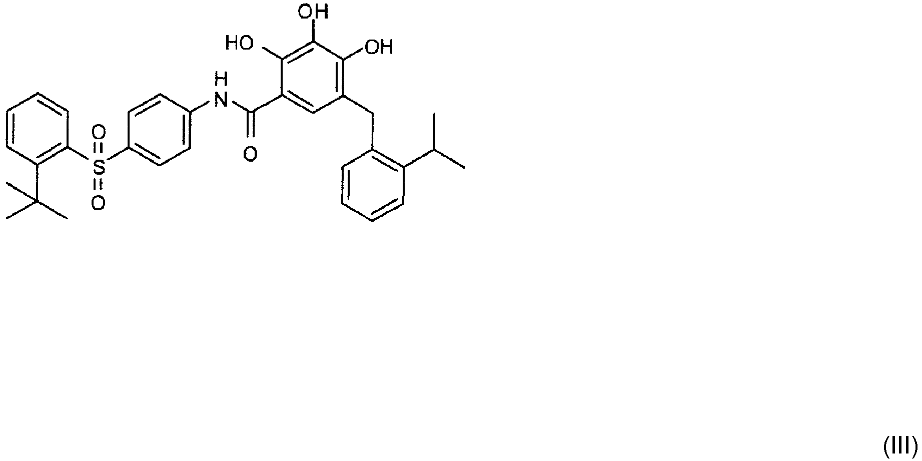

Additional BH3 mimetics include, but are not limited to, analogs and derivatives of TW37, including the following: (1) analogs of TW37 wherein one or more of the hydrogens of the benzene rings are replaced with lower alkyl; and (2) analogs of TW37 wherein one or more of the hydrogens in the hydroxyl groups of the trihydroxyphenyl moiety is replaced with lower alkyl.

ABT-263 is (R)-4-(4-((4'-chloro-4,4-dimethyl-3,4,5,6-tetrahydro-[1,1'-biphenyl]-2-yl)methyl)piperazin-1-yl)-N-((4-((4-morpholino-1-(phenylthio)butan-2-yl)amino)-3-((trifluoromethyl)sulfonyl)phenyl)sulfonyl)benzamide and has the structure shown in Formula (IV), below. The activity of ABT-263 is described in C. Tse et al., "ABT-263: A Potent and Orally Bioavailable Bcl-2 Family Inhibitor," Cancer Res. 68: 3421-3428 (2008) and A.R. Shoemaker et al., "Activity of the Bcl-2 Family Inhibitor ABT-263 in a Panel of Small Cell Lung Cancer Xenograft Models," Clin. Cancer Res. 14: 3268-3277 (2008).

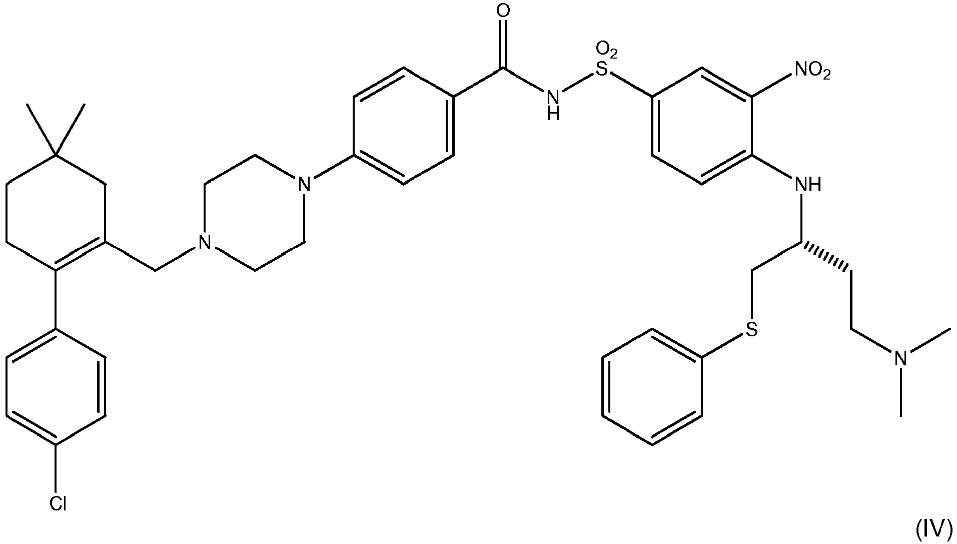

Additional BH3 mimetics include, but are not limited to, analogs or derivatives of ABT-263, including the following: (1) analogs of ABT-263 wherein the chlorine bound to the benzene ring is replaced with fluorine, bromine, or iodine; (2) analogs of ABT-263 wherein one or more of the hydrogens of the benzene rings are replaced with lower alkyl; (3) analogs of ABT-263 wherein one or more of the hydrogens of the piperazinyl moiety are replaced with lower alkyl; and (4) analogs of ABT-263 where the dimethylamino moiety is replaced with another moiety including one or two lower alkyl groups bound to the amino group of the diethylamino moiety.

A compound having partial structural homology with ABT-737 is A-385358. A-385358 is [(R)-4-(3-dimethylamino-1-phenylsulfanylmethyl-propylamino)-N-[4-(4,4-dimethyl-piperidin-1-yl)-benzoyl]-3-nitro-benzenesulfonamide and has the structure shown below as Formula (V). The activity of A-385358 is described in A.R. Shoemaker et al., "A Small Molecule Inhibitor of Bcl-XL Potentiates the Activity of Cytotoxic Drugs in Vitro and in Vivo," Cancer Res. 66: 8731-8739 (2006).

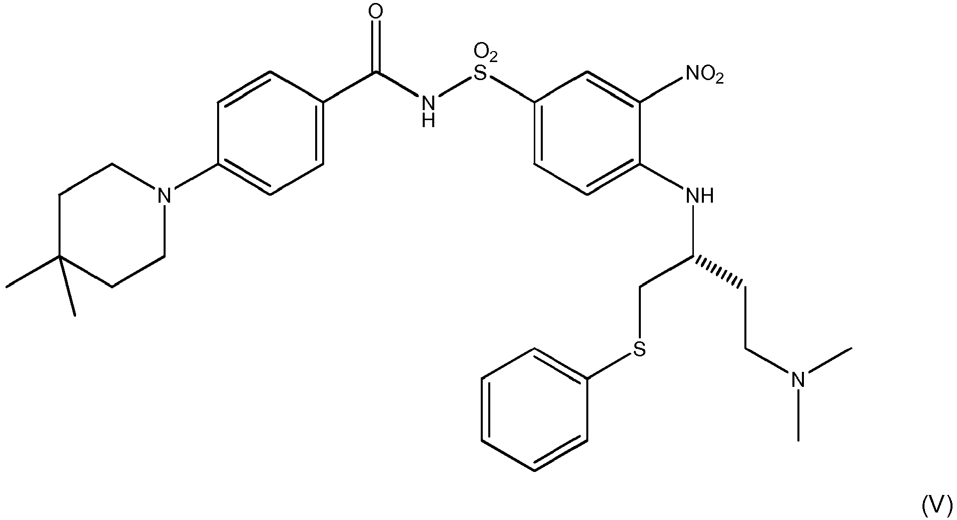

Additional BH3 mimetics described herein include, but are not limited to, analogs or derivatives of A-385358, including the following: (1) analogs of A-385358 wherein one or more of the hydrogens of the benzene rings are replaced with lower alkyl; and (2) analogs of A-385358 where the dimethylamino moiety is replaced with another moiety including one or two lower alkyl groups bound to the amino group of the diethylamino moiety.

The (-) enantiomer of gossypol has been shown to have BH3 mimetic activity; the BH3 mimetic activity of this enantiomer is described in J.P. Qiu et al., "Different Pathways of Cell Killing by Gossypol Enantiomers," Exp. Biol. Med. 227: 398-401 (2002).

The activity of gossypol derivatives and analogs in terms of BH3 mimetic activity is described in G.Z. Tang et al., "Acylpyrogallols as Inhibitors of Antiapoptotic Bcl-2 Proteins," J. Med. Chem. 51: 717-720 (2008).
One particularly significant gossypol derivative is apogossypolone; the BH3 mimetic activity of apogossypolone is described in A.A. Arnold et al., "Preclinical Studies of Apogossypolone: A New Nonpeptidic Pan Small-Molecule Inhibitor of Bcl-2, Bcl-X-L and Mcl-1 Proteins in Follicular Small Cleaved Cell Lymphoma Model," Mol. Cancer 7: 20-30 (2008).

TM-1206 has the structure shown in Formula (VI), below. The BH3 mimetic activity of TM-1206 is described in G.Z. Tang et al. (2007), supra.

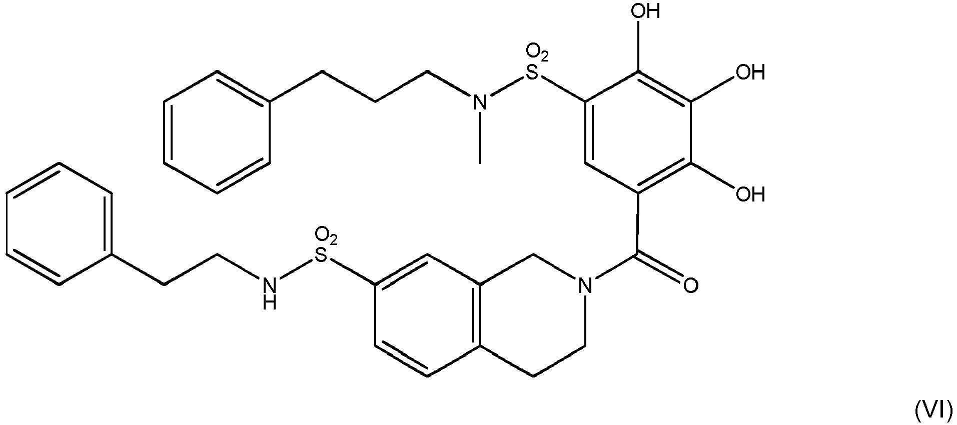

Additional BH3 mimetics described herein include, but are not limited to, analogs or derivatives of TM-1206, including the following: (1) analogs of TM-1206 wherein one or more of the hydrogens of the benzene rings are replaced with lower alkyl; and (2) analogs of TM-1206 wherein one or more of the hydrogens in the hydroxyl groups of the trihydroxyphenyl moiety is replaced with lower alkyl.

Other BH3 mimetics are known in the art.

A series of agents that provide alternative treatment modalities described herein and that act as alkylating agents can treat TKI-resistant malignancies in patients having the germline deletion polymorphism described above. The ability of these alternative agents to treat TKI-resistance malignancies is significant when the magnitude of the problem is considered. As described above, the germline deletion polymorphism described above is considered to account for resistance to TKIs, such as Gleevec, in people of East Asian ancestry (about 15% of cases). This means that out of the approximately 552,000 patients in that category, there are at least about 78,000 lung cancer cases occurring annually in patients carrying the germline deletion polymorphism and thus resistance to TKIs such as Gleevec. In addition, it is estimated that there is at least 1 case of drug-resistant chronic myelocytic leukemia (CML) per 100,000 population, or approximately 2,500 cases annually; this is a very conservative estimate, and the number of cases of drug-resistant CML in populations carrying the germline deletion polymorphism may well be higher.

These agents, as described further below, can also be used in the treatment of other TKI-resistant malignancies in which one or more mutations, either germline mutations or somatic mutations affecting a particular cell or tissue type, prevent phosphorylation of the BIM protein or another protein whose phosphorylation is required for TKI-initated apoptosis.

A class of therapeutic agents described herein that can be employed successfully in patients possessing the germline deletion polymorphism is galactitols, substituted galacitols, dulcitols, and substituted dulcitols, including dianhydrogalactitol, diacetyldianhydrogalactitol, dibromodulcitol, and derivatives and analogs thereof.

These galactitols, substituted galacitols, dulcitols, and substituted dulcitols are either alkylating agents or prodrugs of alkylating agents, as discussed further below.

The structure of dianhydrogalactitol is shown in Formula (VII), below.

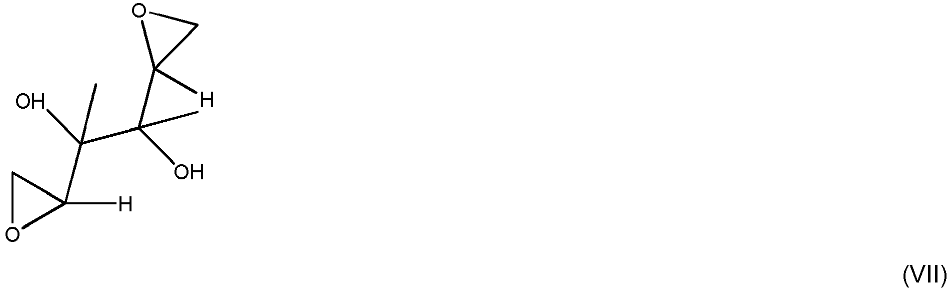

Also described herein are derivatives of dianhydrogalactitol that, for example, have one or both hydrogens of the two hydroxyl groups of dianhydrogalactitol replaced with lower alkyl, have one or more of the hydrogens attached to the two epoxide rings replaced with lower alkyl, or have the methyl groups present in dianhydrogalactitol and that are attached to the same carbons that bear the hydroxyl groups replaced with C₂-C₆ lower alkyl or substituted with, for example, halo groups by replacing a hydrogen of the methyl group with, for example a halo group. As used herein, the term "halo group," without further limitation, refers to one of fluoro, chloro, bromo, or iodo. As used herein, the term "lower alkyl," without further limitation, refers to C₁-C₆ groups and includes methyl. The term "lower alkyl" can be further limited, such as "C₂-C₆ lower alkyl," which excludes methyl. The term "lower alkyl", unless further limited, refers to both straight-chain and branched alkyl groups.

The structure of diacetyldianhydrogalactitol is shown in Formula (VIII), below.

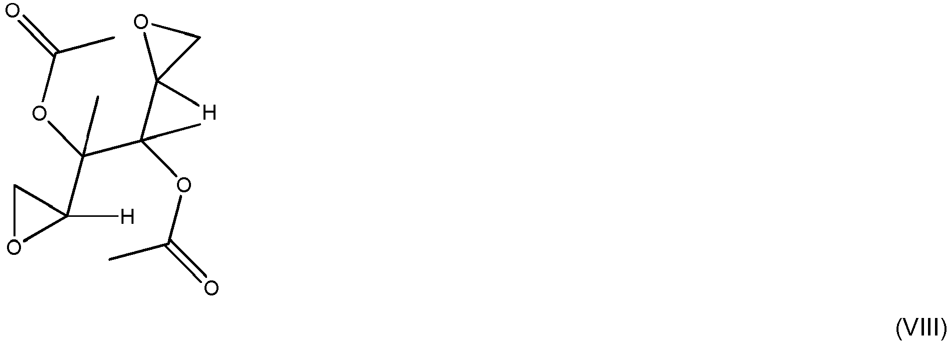

Also described herein are derivatives of diacetyldianhydrogalactitol that, for example, have one or both of the methyl groups that are part of the acetyl moieties replaced with C₂-C₆ lower alkyl, have one or both of the hydrogens attached to the epoxide ring replaced with lower alkyl, or have the methyl groups attached to the same carbons that bear the acetyl groups replaced with lower alkyl or substituted with, for example, halo groups by replacing a hydrogen with, for example, a halo group.

The structure of dibromodulcitol is shown in Formula (IX), below. Dibromodulcitol can be produced by the reaction of dulcitol with hydrobromic acid at elevated temperatures, followed by crystallization of the dibromodulcitol. Some of the properties of dibromodulcitol are described in N.E. Mischler et al., "Dibromoducitol," Cancer Treat. Rev. 6: 191-204 (1979). In particular, dibromodulcitol, as an α, ω-dibrominated hexitol, dibromodulcitol shares many of the biochemical and biological properties of similar drugs such as dibromomannitol and mannitol myleran. Activation of dibromodulcitol to the diepoxide dianhydrogalactitol occurs *in vivo*, and dianhydrogalactitol may represent a major active form of the drug; this means that dibromogalactitol has many of the properties of a prodrug. Absorption of dibromodulcitol by the oral route is rapid and fairly complete. Dibromodulcitol has known activity in melanoma, breast lymphoma (both Hodgkins and non-Hodgkins), colorectal cancer, acute lymphoblastic leukemia and has been shown to lower the incidence of central nervous system leukemia, non-small cell lung cancer, cervical carcinoma, bladder carcinoma, and metastatic hemangiopericytoma.

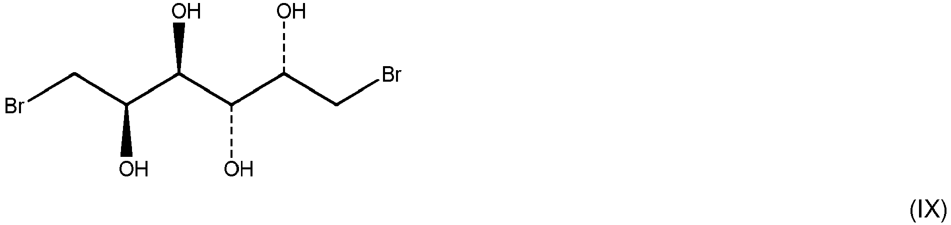

Also described herein are derivatives of dibromodulcitol that, for example, have one or more hydrogens of the hydroxyl groups replaced with lower alkyl, or have one or both of the bromo groups replaced with another halo group such as chloro, fluoro, or iodo.

As described above, and as detailed more generally below, derivatives and analogs of alkylating hexitol derivatives, BH3 mimetics, and other therapeutically active agents employed in methods or compositions described herein can be optionally substituted with one or more groups that do not substantially affect the pharmacological activity of the derivative or analog. These groups are generally known in the art. Definitions for a number of common groups that can be used as optional substituents are described below; however, the omission of any group from these definitions cannot be taken to mean that such a group cannot be used as an optional substituent as long as the chemical and pharmacological requirements for an optional substituent are satisfied.

As used herein, the term "alkyl" refers to an unbranched, branched, or cyclic saturated hydrocarbyl residue, or a combination thereof, of from 1 to 12 carbon atoms that can be optionally substituted; the alkyl residues contain only C and H when unsubstituted. Typically, the unbranched or branched saturated hydrocarbyl residue is from 1 to 6 carbon atoms, which is referred to herein as "lower alkyl." When the alkyl residue is cyclic and includes a ring, it is understood that the hydrocarbyl residue includes at least three carbon atoms, which is the minimum number to form a ring. As used herein, the term "alkenyl" refers to an unbranched, branched or cyclic hydrocarbyl residue having one or more carbon-carbon double bonds. As used herein, the term "alkynyl" refers to an unbranched, branched, or cyclic hydrocarbyl residue having one or more carbon-carbon triple bonds; the residue can also include one or more double bonds. With respect to the use of "alkenyl" or "alkynyl," the presence of multiple double bonds cannot produce an aromatic ring. As used herein, the terms "hydroxyalkyl," "hydroxyalkenyl," and "hydroxyalkynyl," respectively, refer to an alkyl, alkenyl, or alkynyl group including one or more hydroxyl groups as substituents; as detailed below, further substituents can be optionally included. As used herein, the term "aryl" refers to a monocyclic or fused bicyclic moiety having the well-known characteristics of aromaticity; examples include phenyl and naphthyl, which can be optionally substituted. As used herein, the term "hydroxyaryl" refers to an aryl group including one or more hydroxyl groups as substituents; as further detailed below, further substituents can be optionally included. As used herein, the term "heteroaryl" refers to monocyclic or fused bicylic ring systems that have the characteristics of aromaticity and include one or more heteroatoms selected from O, S, and N. The inclusion of a heteroatom permits aromaticity in 5-membered rings as well as in 6-membered rings. Typical heteroaromatic systems include monocyclic C₅-C₆ heteroaromatic groups such as pyridyl, pyrimidyl, pyrazinyl, thienyl, furanyl, pyrrolyl, pyrazolyl, thiazolyl, oxazolyl, triazolyl, triazinyl, tetrazolyl, tetrazinyl, and imidazolyl, as well as the fused bicyclic moieties formed by fusing one of these monocyclic heteroaromatic groups with a phenyl ring or with any of the heteroaromatic monocyclic groups to form a C₈-C₁₀ bicyclic group such as indolyl, benzimidazolyl, indazolyl, benzotriazolyl, isoquinolyl, quinolyl, benzothiazolyl, benzofuranyl, pyrazolylpyridyl, quinazolinyl, quinoxalinyl, cinnolinyl, and other ring systems known in the art. Any monocyclic or fused ring bicyclic system that has the characteristics of aromaticity in terms of delocalized electron distribution throughout the ring system is included in this definition. This definition also includes bicyclic groups where at least the ring that is directly attached to the remainder of the molecule has the characteristics of aromaticity, including the delocalized electron distribution that is characteristic of aromaticity. Typically the ring systems contain 5 to 12 ring member atoms and up to four heteroatoms, wherein the heteroatoms are selected from the group consisting of N, O, and S. Frequently, the monocyclic heteroaryls contain 5 to 6 ring members and up to three heteroatoms selected from the group consisting of N, O, and S; frequently, the bicyclic heteroaryls contain 8 to 10 ring members and up to four heteroatoms selected from the group consisting of N, O, and S. The number and placement of heteroatoms in heteroaryl ring structures is in accordance with the well-known limitations of aromaticity and stability, where stability requires the heteroaromatic group to be stable enough to be exposed to water at physiological temperatures without rapid degradation. As used herein, the term "hydroxheteroaryl" refers to a heteroaryl group including one or more hydroxyl groups as substituents; as further detailed below, further substituents can be optionally included. As used herein, the terms "haloaryl" and "haloheteroaryl" refer to aryl and heteroaryl groups, respedively, substituted with at least one halo group, where "halo" refers to a halogen selected from the group consisting of fluorine, chlorine, bromine, and iodine, typically, the halogen is selected from the group consisting of chlorine, bromine, and iodine; as detailed below, further substituents can be optionally included. As used herein, the terms "haloalkyl," "haloalkenyl," and "haloalkynyl" refer to alkyl, alkenyl, and alkynyl groups, respectively, substituted with at least one halo group, where "halo" refers to a halogen selected from the group consisting of fluorine, chlorine, bromine, and iodine, typically, the halogen is selected from the group consisting of chlorine, bromine, and iodine; as detailed below, further substituents can be optionally included.

As used herein, the term "optionally substituted" indicates that the particular group or groups referred to as optionally substituted may have no non-hydrogen substituents, or the group or groups may have one or more non-hydrogen substituents consistent with the chemistry and pharmacological activity of the resulting molecule. If not otherwise specified, the total number of such substituents that may be present is equal to the total number of hydrogen atoms present on the unsubstituted form of the group being described; fewer than the maximum number of such substituents may be present. Where an optional substituent is attached via a double bond, such as a carbonyl oxygen (C=O), the group takes up two available valences on the carbon atom to which the optional substituent is attached, so the total number of substituents that may be included is reduced according to the number of available valiences. As used herein, the term "substituted," whether used as part of "optionally substituted" or otherwise, when used to modify a specific group, moiety, or radical, means that one or more hydrogen atoms are, each, independently of each other, replaced with the same or different substituent or substituents.

Substituent groups useful for substituting saturated carbon atoms in the specified group, moiety, or radical include, but are not limited to, -Z^{a}, =O, -OZ^{b}, - SZ^{b}, =S⁻, -NZ^{c}Z^{c}, =NZ^{b}, =N-OZ^{b}, trihalomethyl, -CF₃, -CN, -OCN, -SCN, -NO, --NO₂, =N₂, -N₃, -S(O)₂Z^{b}, -S(O)₂NZ^{b}, —S(O₂)O⁻, -S(O₂)OZ^{b}, -OS(O₂)OZ^{b}, - OS(O₂)O⁻ , -OS(O₂)OZ^{b}, -P(O)(O⁻)₂, -P(O)(OZ^{b})(O⁻), -P(O)(OZ^{b})(OZ^{b}), -C(O)Z^{b}, -C(S)Z^{b}, -C(NZ^{b})Z^{b}, -C(O)O-, -C(O)OZ^{b}, -C(S)OZ^{b}, -C(O)NZ^{c}Z^{c}, - C(NZ^{b})NZ^{c}Z^{c}, -OC(O)Z^{b}, -OC(S)Z^{b}, -OC(O)O⁻, ⁻OC(O)OZ^{b}, -OC(S)OZ^{b} - NZ^{b}C(O)Z^{b}, -NZ^{b}C(S)Z^{b}, -NZ^{b}C(O)O⁻, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, -NZ^{b}C(NZ^{b})NZ^{c}Z^{c}, wherein Z^{a} is selected from the group consisting of alkyl, cycloalkyl, heteroalkyl, cycloheteroalkyl, aryl, arylalkyl, heteroaryl and heteroarylalkyl; each Z^{b} is independently hydrogen or Z^{a}; and each Z^{c} is independently Z^{b} or, alternatively, the two Z^{c}'s may be taken together with the nitrogen atom to which they are bonded to form a 4-, 5-, 6-, or 7-membered cycloheteroalkyl ring structure which may optionally include from 1 to 4 of the same or different heteroatoms selected from the group consisting of N, O, and S. As specific examples, -NZ^{c}Z^{c} is meant to include -NH₂, -NH-alkyl, -N-pyrrolidinyl, and -N-morpholinyl, but is not limited to those specific alternatives and includes other alternatives known in the art. Similarly, as another specific example, a substituted alkyl is meant to include - alkylene-O-alkyl, -alkylene-heteroaryl, -alkylene-cycloheteroaryl, -alkylene-C(O)OZ^{b}, -alkylene-C(O)NZ^{b}Z^{b}, and -CH₂-CH₂-C(O)-CH₃, but is not limited to those specific alternatives and includes other alternatives known in the art. The one or more substituent groups, together with the atoms to which they are bonded, may form a cyclic ring, including, but not limited to, cycloalkyl and cycloheteroalkyl.

Similarly, substituent groups described herein useful for substituting unsaturated carbon atoms in the specified group, moiety, or radical include, but are not limited to, -Z^{a}, halo, -O⁻, -OZ^{b}, -SZ^{b}, -S⁻, -NZ^{c}Z^{c}, trihalomethyl, -CF₃, -CN, - OCN, -SCN, -NO, -NO₂, -N₃, -S(O)₂Z^{b}), -S(O₂)0⁻, -S(O₂)OZ^{b}, -OS(O₂)OZ^{b}, -OS(O₂)O-, -P(O)(O⁻)₂, -P(O)(OZ^{b})(O⁻), -P(O)(OZ^{b})(OZ^{b}), -C(O)Z^{b}, -C(S)Z^{b}, - C(NZ^{b})Z^{b}, -C(O)O⁻, -C(O)OZ^{b}, -C(S)OZ^{b}, -C(O)NZ^{c}Z^{c}, -C(NZ^{b})NZ^{c}Z^{c}, -OC(O)Z^{b}, -OC(S)Z^{b}, -OC(O)O⁻, -OC(O)OZ^{b}, -OC(S)OZ^{b}, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, and -NZ^{b}C(NZ^{b})NZ^{c}Z^{c}, wherein Z^{a}, Z^{b}, and Z^{c} are as defined above.

Similarly, substituent groups useful for substituting nitrogen atoms in heteroalkyl and cycloheteroalkyl groups include, but are not limited to, -Z^{a}, halo, -O⁻, -OZ^{b}, -SZ^{b}, -S⁻, -NZ^{c}Z^{c}, trihalomethyl, -CF₃, -CN, -OCN, -SCN, -NO, - NO₂, -S(O)₂Z^{b}, - S(O₂)O-, -S(O₂)OZ^{b}, -OS(O₂)OZ^{b}, -OS(O₂)O⁻, -P(O)(O-)₂, - P(O)(OZ^{b})(O⁻), -P(O)(OZ^{b})(OZ^{b}), -C(O)Z^{b}, -C(S)Z^{b}, -C(NZ^{b})Z^{b}, -C(O)OZ^{b}, - C(S)OZ^{b}, -C(O)NZ^{c}Z^{c}, -C(NZ^{b})NZ^{c}Z^{c}, -OC(O)Z^{b}, -OC(S)Z^{b}, -OC(O)OZ^{b}, - OC(S)OZ^{b}, -NZ^{b}C(O)Z^{b}, -NZ^{b}C(S)Z^{b}, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, and -NZ^{b}C(NZ^{b})NZ^{c}Z^{c}, wherein Z^{a}, Z^{b}, and Z^{c} are as defined above.

The compounds described herein may contain one or more chiral centers and/or double bonds and therefore, may exist as stereoisomers, such as double-bond isomers (i.e., geometric isomers such as E and Z), enantiomers or diastereomers. The disclosure includes each of the isolated stereoisomeric forms (such as the enantiomerically pure isomers, the E and Z isomers, and other alternatives for stereoisomers) as well as mixtures of stereoisomers in varying degrees of chiral purity or percetange of E and Z, including racemic mixtures, mixtures of diastereomers, and mixtures of E and Z isomers. Accordingly, the chemical structures depicted herein encompass all possible enantiomers and stereoisomers of the illustrated compounds including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The disclosure includes each of the isolated stereoisomeric forms as well as mixtures of stereoisomers in varying degrees of chiral purity, including racemic mixtures. It also encompasses the various diastereomers. Other structures may appear to depict a specific isomer, but that is merely for convenience, and is not intended to limit the disclosure to the depicted olefin isomer. When the chemical name does not specify the isomeric form of the compound, it denotes any one of the possible isomeric forms or mixtures of those isomeric forms of the compound.

The compounds may also exist in several tautomeric forms, and the depiction herein of one tautomer is for convenience only, and is also understood to encompass other tautomers of the form shown. Accordingly, the chemical structures depicted herein encompass all possible tautomeric forms of the illustrated compounds. The term "tautomer" as used herein refers to isomers that change into one another with great ease so that they can exist together in equilibrium; the equilibrium may strongly favor one of the tautomers, depending on stability considerations. For example, ketone and enol are two tautomeric forms of one compound.

As used herein, the term "solvate" means a compound formed by solvation (the combination of solvent molecules with molecules or ions of the solute), or an aggregate that consists of a solute ion or molecule, i.e., a compound of the invention, with one or more solvent molecules. When water is the solvent, the corresponding solvate is "hydrate." Examples of hydrate include, but are not limited to, hemihydrate, monohydrate, dihydrate, trihydrate, hexahydrate, and other water-containing species. It should be understood by one of ordinary skill in the art that the pharmaceutically acceptable salt, and/or prodrug of the present compound may also exist in a solvate form. The solvate is typically formed via hydration which is either part of the preparation of the present compound or through natural absorption of moisture by the anhydrous compound of the present invention.

As used herein, the term "ester" means any ester of a present compound in which any of the --COOH functions of the molecule is replaced by a --COOR function, in which the R moiety of the ester is any carbon-containing group which forms a stable ester moiety, including but not limited to alkyl, alkenyl, alkynyl, cycloalkyl, cycloalkylalkyl, aryl, arylalkyl, heterocyclyl, heterocyclylalkyl and substituted derivatives thereof. The hydrolysable esters of the present compounds are the compounds whose carboxyls are present in the form of hydrolysable ester groups. That is, these esters are pharmaceutically acceptable and can be hydrolyzed to the corresponding carboxyl acid in vivo.

In addition to the substituents described above, alkyl, alkenyl and alkynyl groups can alternatively or in addition be substituted by C₁-C₈ acyl, C₂-C₈ heteroacyl, C₆-C₁₀ aryl, C₃-C₈ cycloalkyl, C₃-C₈ heterocyclyl, or C₅-C₁₀ heteroaryl, each of which can be optionally substituted. Also, in addition, when two groups capable of forming a ring having 5 to 8 ring members are present on the same or adjacent atoms, the two groups can optionally be taken together with the atom or atoms in the substituent groups to which they are attached to form such a ring.

"Heteroalkyl," "heteroalkenyl," and "heteroalkynyl" and the like are defined similarly to the corresponding hydrocarbyl (alkyl, alkenyl and alkynyl) groups, but the 'hetero' terms refer to groups that contain 1-3 O, S or N heteroatoms or combinations thereof within the backbone residue; thus at least one carbon atom of a corresponding alkyl, alkenyl, or alkynyl group is replaced by one of the specified heteroatoms to form, respectively, a heteroalkyl, heteroalkenyl, or heteroalkynyl group. For reasons of chemical stability, it is also understood that, unless otherwise specified, such groups do not include more than two contiguous heteroatoms except where an oxo group is present on N or S as in a nitro or sulfonyl group.

While "alkyl" as used herein includes cycloalkyl and cycloalkylalkyl groups, the term "cycloalkyl" may be used herein to describe a carbocyclic non-aromatic group that is connected via a ring carbon atom, and "cycloalkylalkyl" may be used to describe a carbocyclic non-aromatic group that is connected to the molecule through an alkyl linker.

Similarly, "heterocyclyl" may be used to describe a non-aromatic cyclic group that contains at least one heteroatom (typically selected from N, O and S) as a ring member and that is connected to the molecule via a ring atom, which may be C (carbon-linked) or N (nitrogen-linked); and "heterocyclylalkyl" may be used to describe such a group that is connected to another molecule through a linker. The heterocyclyl can be fully saturated or partially saturated, but non-aromatic. The sizes and substituents that are suitable for the cycloalkyl, cycloalkylalkyl, heterocyclyl, and heterocyclylalkyl groups are the same as those described above for alkyl groups. The heterocyclyl groups typically contain 1, 2 or 3 heteroatoms, selected from N, O and S as ring members; and the N or S can be substituted with the groups commonly found on these atoms in heterocyclic systems. As used herein, these terms also include rings that contain a double bond or two, as long as the ring that is attached is not aromatic. The substituted cycloalkyl and heterocyclyl groups also include cycloalkyl or heterocyclic rings fused to an aromatic ring or heteroaromatic ring, provided the point of attachment of the group is to the cycloalkyl or heterocyclyl ring rather than to the aromatic/heteroaromatic ring.

As used herein, "acyl" encompasses groups comprising an alkyl, alkenyl, alkynyl, aryl or arylalkyl radical attached at one of the two available valence positions of a carbonyl carbon atom, and heteroacyl refers to the corresponding groups wherein at least one carbon other than the carbonyl carbon has been replaced by a heteroatom chosen from N, O and S.

Acyl and heteroacyl groups are bonded to any group or molecule to which they are attached through the open valence of the carbonyl carbon atom. Typically, they are C₁-C₈ acyl groups, which include formyl, acetyl, pivaloyl, and benzoyl, and C₂-C₈ heteroacyl groups, which include methoxyacetyl, ethoxycarbonyl, and 4-pyridinoyl.

Similarly, "arylalkyl" and "heteroarylalkyl" refer to aromatic and heteroaromatic ring systems which are bonded to their attachment point through a linking group such as an alkylene, including substituted or unsubstituted, saturated or unsaturated, cyclic or acyclic linkers. Typically the linker is C₁-C₈ alkyl. These linkers may also include a carbonyl group, thus making them able to provide substituents as an acyl or heteroacyl moiety. An aryl or heteroaryl ring in an arylalkyl or heteroarylalkyl group may be substituted with the same substituents described above for aryl groups. Preferably, an arylalkyl group includes a phenyl ring optionally substituted with the groups defined above for aryl groups and a C₁-C₄ alkylene that is unsubstituted or is substituted with one or two C₁-C₄ alkyl groups or heteroalkyl groups, where the alkyl or heteroalkyl groups can optionally cyclize to form a ring such as cyclopropane, dioxolane, or oxacyclopentane. Similarly, a heteroarylalkyl group preferably includes a C₅-C₆ monocyclic heteroaryl group that is optionally substituted with the groups described above as substituents typical on aryl groups and a C₁-C₄ alkylene that is unsubstituted or is substituted with one or two C₁-C₄ alkyl groups or heteroalkyl groups, or it includes an optionally substituted phenyl ring or C₅-C₆ monocyclic heteroaryl and a C₁-C₄ heteroalkylene that is unsubstituted or is substituted with one or two C₁-C₄ alkyl or heteroalkyl groups, where the alkyl or heteroalkyl groups can optionally cyclize to form a ring such as cyclopropane, dioxolane, or oxacyclopentane.

Where an arylalkyl or heteroarylalkyl group is described as optionally substituted, the substituents may be on either the alkyl or heteroalkyl portion or on the aryl or heteroaryl portion of the group. The substituents optionally present on the alkyl or heteroalkyl portion are the same as those described above for alkyl groups generally; the substituents optionally present on the aryl or heteroaryl portion are the same as those described above for aryl groups generally.

"Arylalkyl" groups as used herein are hydrocarbyl groups if they are unsubstituted, and are described by the total number of carbon atoms in the ring and alkylene or similar linker. Thus a benzyl group is a C7-arylalkyl group, and phenylethyl is a C8-arylalkyl.

"Heteroarylalkyl" as described above refers to a moiety comprising an aryl group that is attached through a linking group, and differs from "arylalkyl" in that at least one ring atom of the aryl moiety or one atom in the linking group is a heteroatom selected from N, O and S. The heteroarylalkyl groups are described herein according to the total number of atoms in the ring and linker combined, and they include aryl groups linked through a heteroalkyl linker; heteroaryl groups linked through a hydrocarbyl linker such as an alkylene; and heteroaryl groups linked through a heteroalkyl linker. Thus, for example, C7-heteroarylalkyl would include pyridylmethyl, phenoxy, and N-pyrrolylmethoxy.

"Alkylene" as used herein refers to a divalent hydrocarbyl group; because it is divalent, it can link two other groups together. Typically it refers to -(CH₂)ₙ-where n is 1-8 and preferably n is 1-4, though where specified, an alkylene can also be substituted by other groups, and can be of other lengths, and the open valences need not be at opposite ends of a chain.

In general, any alkyl, alkenyl, alkynyl, acyl, or aryl or arylalkyl group that is contained in a substituent may itself optionally be substituted by additional substituents. The nature of these subtituents is similar to those recited with regard to the primary substituents themselves if the substituents are not otherwise described.

"Amino" as used herein refers to -NH₂, but where an amino is described as "substituted" or "optionally substituted", the term includes NR'R" wherein each R' and R" is independently H, or is an alkyl, alkenyl, alkynyl, acyl, aryl, or arylalkyl group, and each of the alkyl, alkenyl, alkynyl, acyl, aryl, or arylalkyl groups is optionally substituted with the substituents described herein as suitable for the corresponding group; the R' and R" groups and the nitrogen atom to which they are attached can optionally form a 3- to 8-membered ring which may be saturated, unsaturated or aromatic and which contains 1-3 heteroatoms independently selected from N, O and S as ring members, and which is optionally substituted with the substituents described as suitable for alkyl groups or, if NR'R" is an aromatic group, it is optionally substituted with the substituents described as typical for heteroaryl groups.

As used herein, the term "carbocycle," "carbocyclyl," or "carbocyclic" refers to a cyclic ring containing only carbon atoms in the ring, whereas the term "heterocycle" or "heterocyclic" refers to a ring comprising a heteroatom. The carbocyclyl can be fully saturated or partially saturated, but non-aromatic. For example, the carbocyclyl encompasses cycloalkyl. The carbocyclic and heterocyclic structures encompass compounds having monocyclic, bicyclic or multiple ring systems; and such systems may mix aromatic, heterocyclic, and carbocyclic rings. Mixed ring systems are described according to the ring that is attached to the rest of the compound being described.

As used herein, the term "heteroatom" refers to any atom that is not carbon or hydrogen, such as nitrogen, oxygen or sulfur. When it is part of the backbone or skeleton of a chain or ring, a heteroatom must be at least divalent, and will typically be selected from N, O, P, and S.

As used herein, the term "alkanoyl" refers to an alkyl group covalently linked to a carbonyl (C=O) group. The term "lower alkanoyl" refers to an alkanoyl group in which the alkyl portion of the alkanoyl group is C₁-C₆. The alkyl portion of the alkanoyl group can be optionally substituted as described above. The term "alkylcarbonyl" can alternatively be used. Similarly, the terms "alkenylcarbonyl" and "alkynylcarbonyl" refer to an alkenyl or alkynyl group, respectively, linked to a carbonyl group.

As used herein, the term "alkoxy" refers to an alkyl group covalently linked to an oxygen atom; the alkyl group can be considered as replacing the hydrogen atom of a hydroxyl group. The term "lower alkoxy" refers to an alkoxy group in which the alkyl portion of the alkoxy group is C₁-C₆. The alkyl portion of the alkoxy group can be optionally substituted as described above. As used herein, the term "haloalkoxy" refers to an alkoxy group in which the alkyl portion is substituted with one or more halo groups.

As used herein, the term "sulfo" refers to a sulfonic acid (-SO₃H) substituent.

As used herein, the term "sulfamoyl" refers to a substituent with the structure -S(O₂)NH₂, wherein the nitrogen of the NH₂ portion of the group can be optionally substituted as described above.

As used herein, the term "carboxyl" refers to a group of the structure - C(O₂)H.

As used herein, the term "carbamyl" refers to a group of the structure - C(O₂)NH₂, wherein the nitrogen of the NH₂ portion of the group can be optionally substituted as described above.

As used herein, the terms "monoalkylaminoalkyl" and "dialkylaminoalkyl" refer to groups of the structure -Alk₁-NH-Alk₂ and -Alk₁-N(Alk₂)(Alk₃), wherein Alk₁, Alk₂, and Alk₃ refer to alkyl groups as described above.

As used herein, the term "alkylsulfonyl" refers to a group of the structure -S(O)₂-Alk wherein Alk refers to an alkyl group as described above. The terms "alkenylsulfonyl" and "alkynylsulfonyl" refer analogously to sulfonyl groups covalently bound to alkenyl and alkynyl groups, respectively. The term "arylsulfonyl" refers to a group of the structure -S(O)₂-Ar wherein Ar refers to an aryl group as described above. The term "aryloxyalkylsulfonyl" refers to a group of the structure -S(O)₂-Alk-O-Ar, where Alk is an alkyl group as described above and Ar is an aryl group as described above. The term "arylalkylsulfonyl" refers to a group of the structure -S(O)₂-AlkAr, where Alk is an alkyl group as described above and Ar is an aryl group as described above.

As used herein, the term "alkyloxycarbonyl" refers to an ester substituent including an alkyl group wherein the carbonyl carbon is the point of attachment to the molecule. An example is ethoxycarbonyl, which is CH₃CH₂OC(O)-. Similarly, the terms "alkenyloxycarbonyl," "alkynyloxycarbonyl," and "cycloalkylcarbonyl" refer to similar ester substituents including an alkenyl group, alkenyl group, or cycloalkyl group respectively. Similarly, the term "aryloxycarbonyl" refers to an ester substituent including an aryl group wherein the carbonyl carbon is the point of attachment to the molecule. Similarly, the term "aryloxyalkylcarbonyl" refers to an ester substituent including an alkyl group wherein the alkyl group is itself substituted by an aryloxy group.

Other combinations of substituents are known in the art and, are described, for example, in United States Patent No. 8,344,162 to Jung et al.. For example, the term "thiocarbonyl" and combinations of substituents including "thiocarbonyl" include a carbonyl group in which a double-bonded sulfur replaces the normal double-bonded oxygen in the group. The term "alkylidene" and similar terminology refer to an alkyl group, alkenyl group, alkynyl group, or cycloalkyl group, as specified, that has two hydrogen atoms removed from a single carbon atom so that the group is double-bonded to the remainder of the structure.

Dianhydrogalactitol and other substituted hexitols possess a number of advantages for use in treatment of TKI-resistant non-small cell lung carcinoma (NSCLC) and chronic myelogenous leukemia (CML). These agents can suppress the growth of cancer stem cells (CSC) and are resistant to drug inactivation by O⁶-methylguanine-DNA methyltransferase (MGMT). Dianhydrogalactitol is a novel alkylating agent that causes crosslinking of DNA at N⁷.

As detailed below, dianhydrogalactitol and other substituted hexitols can be used to treat malignancies in patients resistant to TKIs.

There is described herein a medicament for use in a method for the treatment of a malignancy in a subject suffering from a malignancy who has a germline deletion polymorphism conferring resistance to TKIs comprising the step of administering a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy.

As detailed above, the malignancy can be chronic myelogenous leukemia (CML) or non-small cell lung carcinoma (NSCLC) or triple-negative breast cancer.

Typically, the confirmation of whether a particular subject suffering from a malignancy has a germline deletion polymorphism conferring resistance to TKIs may be made by performing DNA-paired end tag (DNA-PET) analysis. The connectivity of PET sequences allows detection of deletions. For example, the fragments can be selected to all be of a certain size. After mapping, the PET sequences are thus expected to consistently be a particular distance from each other. A discrepancy from this distance indicates a structural variation between the PET sequences. For example, a deletion in the sequenced genome will have reads that map further away than expected in the reference genome as the reference genome will have a segment of DNA that is not present in the sequenced genome. This can be used to determine the existence of a deletion, such as the germline deletion polymorphism of 2903 bp described above or another deletion that has the effect of conferring resistance to TKIs. Typically, the germline DNA deletion polymorphism causes a splicing variation that leads to expression of an isoform of BIM protein that lacks a BH3 domain and thus inhibits the induction of apoptosis.

There is described herein a medicament for use in a method that combines screening for the germline deletion polymorphism and, if the germline deletion polymorphism is found to exist, treatment of a malignancy resistant to TKIs in a subject.

In general, this method described herein comprises the steps of:
(1) screening for the germline deletion polymorphism in a subject with a malignancy; and
(2) if the germline deletion polymorphism is found to exist in the subject with the malignancy, administering a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy.

As detailed above, the malignancy can be chronic myelogenous leukemia (CML), non-small cell lung carcinoma (NSCLC), or triple-negative breast cancer. It is described herein that other malignancies in which tyrosine kinase activity is associated with abnormal or uncontrolled cellular proliferation can be treated by analogous methods.

These methods described herein can further comprise the step of administering a therapeutically effective quantity of: (1) a BH3 mimetic; or (2) both a BH3 mimetic and a tyrosine kinase inhibitor therapeutic agent in a subject suffering from a malignancy in which the germline deletion polymorphism exists.

Suitable BH3 mimetics are described above. A particularly preferred BH3 mimetic is ABT-737.

Suitable TKI therapeutic agents include imatinib, bosutinib, nilotinib, and dasatinib, all of which target the Bcr-Abl receptor tyrosine kinase. In general, imatinib is preferred. Additional TKI therapeutic agents include erlotinib, afatinib, and dacomitinib, which are EGFR inhibitors (reversible or irreversible) active toward wild-type or mutated EGFR; these agents specifically target the EGFR tyrosine kinase.

It is described herein that the administration of dianhydrogalactitol, a derivative or analog of dianhydrogalactitol, diacetyldianhydrogalactitol, a derivative or analog of dianhydrogalactitol, dibromodulcitol, or a derivative or analog of dibromodulcitol (referred to generally herein as "alkylating hexitol derivatives") to treat a TKI-resistant malignancy can be carried out in accordance with the following principles for achieving improved therapeutic results for the administration of one or more of these hexitol derivatives.

Accordingly, a medicament for use in a method for the treatment of a malignancy is described herein wherein the malignancy is characterized by resistance to at least one tyrosine kinase inhibitor (TKI) due to: (1) at least one mutation in a gene encoding a protein that is a target of at least one TKI; or (2) the presence of at least one additional gene in either a wild-type or mutated state encoding a product that confers resistance to the therapeutic effects of at least one TKI, the method comprising the administration of a therapeutically effective quantity of an alkylating hexitol derivative. Typically, the alkylating hexitol derivative described herein is dianhydrogalactitol, a derivative or analog of dianhydrogalactitol, diacetyldianhydrogalactitol, a derivative or analog of diacetyldianhydrogalactitol, dibromodulcitol, or a derivative or analog of dibromodulcitol.

In one alternative, the additional gene in either a wild-type or mutated state encoding a product that confers resistance to the therapeutic effects of at least one TKI is AHI-1.

As detailed below, these methods described herein can further comprise: (1) the step of administering a therapeutically effective quantity of a BH3 mimetic to the subject; (2) the step of administering a therapeutically effective quantity of a TKI to the subject; (3) the step of administering a therapeutically effective quantity of a JAK2 inhibitor to the subject; (4) the step of administering a therapeutically effective quantity of a STAT5 inhibitor to the subject; (5) the step of administering a therapeutically effective quantity of a Src kinase inhibitor to the subject; or (6) the step of administering a therapeutically effective quantity of a combination of kinase inhibitors to the subject to treat the malignancy; wherein the combination of kinase inhibitors is a combination selected from the group consisting of: (a) a JAK2 inhibitor and a STAT5 inhibitor; (b) a JAK2 inhibitor and a Src inhibitor; (c) a STAT5 inhibitor and a Src inhibitor; and (d) a JAK2 inhibitor, a STAT5 inhibitor, and a Src inhibitor to the subject. In addition, when the method described herein comprises the step of administering a therapeutically effective quantity of a JAK2 inhibitor, a STAT5 inhibitor, a Src inhibitor, or two or more of a JAK2 inhibitor, a STAT5 inhibitor, and a Src inhibitor to the subject, the method can further comprise the step of administering a therapeutically effective quantity of a BH3 mimetic or a TKI to the subject. Suitable BH3 mimetics, TKIs, JAK2 inhibitors, STAT5 inhibitors, and Src inhibitors for use in these methods described herein are described below.

A method for improvement in the therapeutic employment of an alkylating hexitol derivative for treatment of a TKI-resistant malignancy made by alterations to the time that the compound is administered, the use of dose-modifying agents that control the rate of metabolism of the compound, normal tissue protective agents, and other alterations is described herein. General examples described herein include: variations of infusion schedules (e.g., bolus i.v. versus continuous infusion), the use of lymphokines (e.g., G-CSF, GM-CSF, EPO) to increase leukocyte count for improved immune response or for preventing anemia caused by myelosuppressive agents, or the use of rescue agents such as leucovorin for 5-FU or thiosulfate for cisplatin treatment. Specific inventive examples for alkylating hexitol derivatives for treatment of a TKI-resistant malignancy include: continuous i.v. infusion for hours to days; biweekly administration; doses greater than 5 mg/m²/day; progressive escalation of dosing from 1 mg/m²/day based on patient tolerance; doses less than 1 mg/m² for greater than 14 days; use of caffeine to modulate metabolism; use of isoniazid to modulate metabolism; selected and intermittent boost dose administrations; bolus single and multiple doses escalating from 5 mg/m², or oral doses below 30 or above 130 mg/m².

Another aspect of the present invention is, an improvement in the therapeutic employment of an alkylating hexitol derivative for treatment of a TKI-resistant malignancy made by alterations in the route by which the compound is administered is described herein. General examples described herein include: changing route from oral to intravenous administration and vice versa; or the use of specialized routes such as subcutaneous, intramuscular, intraarterial, intraperitoneal, intralesional, intralymphatic, intratumoral, intrathecal, intravesicular, intracranial. Specific inventive examples described herein for alkylating hexitol derivatives for treatment of a TKI-resistant malignancy include: daily; weekly for three weeks, weekly for two weeks, biweekly; biweekly for three weeks with a 1-2 week rest period; intermittent boost dose administration; daily for one week then once per week for multiple weeks; dosing at up to 40 mg/m² for 3 days and then a nadir/recovery period of 18 to 21 days; dosing at a lower level for extended periods (e.g., 21 days); dosing at a higher level; dosing with a nadir/recovery period longer than 21 days; and the use of an alkylating hexitol derivative as a single therapeutic agent.

An improvement in the therapeutic employment of an alkylating hexitol derivative for treatment of a TKI-resistant malignancy made by alterations in the stage of disease at diagnosis/progression that the compound is administered is described herein. General examples described herein include: the use of chemotherapy for non-resectable local disease, prophylactic use to prevent metastatic spread or inhibit disease progression or conversion to more malignant stages. Specific inventive examples described herein for alkylating hexitol derivatives for treatment of a TKI-resistant malignancy include: the use of an alkylating hexitol derivative with angiogenesis inhibitors such as Avastin, a VEGF inhibitor, to prevent or limit metastatic spread, especially in the central nervous system, the use an alkylating hexitol derivative of for newly diagnosed disease, the use of an alkylating hexitol derivative for recurrent disease, and the use of an alkylating hexitol derivative for resistant or refractory disease.

An improvement in the therapeutic employment of an alkylating hexitol derivative for treatment of a TKI-resistant malignancy made by alterations to the type of patient that would best tolerate or benefit from the use of the compound is described herein. General examples described herein include: use of pediatric doses for elderly patients, altered doses for obese patients; exploitation of co-morbid disease conditions such as diabetes, cirrhosis, or other conditions that may uniquely exploit a feature of the compound. Specific inventive examples described herein for alkylating hexitol derivatives for treatment of a TKI-resistant malignancy include: patients with disease conditions with high levels of metabolic enzymes, histone deacetylase, protein kinases, ornithine decarboxylase; patients with disease conditions with low levels of metabolic enzymes, histone deacetylase, protein kinases, ornithine decarboxylase; patients with low or high susceptibility to thrombocytopenia, neutropenia; patients intolerant of GI toxicities; over- or under-expression of jun, GPCR's and signal transduction proteins, VEGF, prostate-specific genes, protein kinases, or telomerase.

An improvement in the therapeutic employment of an alkylating hexitol derivative for treatment of a TKI-resistant malignancy made by more precise identification of a patient's ability to tolerate, metabolize and exploit the use of the compound is described herein. General examples described herein include: biopsy samples of tumors or normal tissues (e.g., glial cells or other cells of the central nervous system) that may also be taken and analyzed to specifically tailor or monitor the use of a particular drug against a gene target; studies of unique tumor gene expression patterns; or analysis of SNPs (single nucleotide polymorphisms), to enhance efficacy or to avoid particular drug-sensitive normal tissue toxicities. Specific inventive examples described herein for alkylating hexitol derivatives for treatment of a TKI-resistant malignancy include: diagnostic tools, techniques, kits and assays to confirm a patient's particular genotype; gene/protein expression chips and analysis; Single Nucleotide Polymorphisms (SNPs) assessment; SNPs for histone deacetylase, ornithine decarboxylase, GPCR's, protein kinases, telomerase, or jun; and identification and the measurement of metabolism enzymes and metabolites.

An improvement in the therapeutic employment of an alkylating hexitol derivative for treatment of a TKI-resistant malignancy made by specialized preparation of a patient prior to or after the use of a chemotherapeutic agent is described herein. General examples described herein include: induction or inhibition of metabolizing enzymes, specific protection of sensitive normal tissues or organ systems. Specific inventive examples described herein for an alkylating hexitol derivative for treatment of a TKI-resistant malignancy include: the use of colchicine or analogs; use of diuretics or uricosurics such as probenecid; use of uricase; non-oral use of nicotinamide; sustained release forms of nicotinamide; use of inhibitors of polyADP ribose polymerase; use of caffeine; leucovorin rescue; infection control; antihypertensives.

An improvement in the therapeutic employment of an alkylating hexitol derivative for treatment of a TKI-resistant malignancy made by use of additional drugs or procedures to prevent or reduce potential side-effects or toxicities is described herein. General examples described herein include: the use of anti-emetics, anti-nausea, hematological support agents to limit or prevent neutropenia, anemia, thrombocytopenia, vitamins, antidepressants, treatments for sexual dysfunction, and other supportive techniques. Specific inventive examples described herein for an alkylating hexitol derivative for treatment of a TKI-resistant malignancy include: the use of colchicine or analogs; use of diuretics or uricosurics such as probenecid; use of uricase; non-oral use of nicotinamide; sustained release forms of nicotinamide; use of inhibitors of poly ADP-ribose polymerase; use of caffeine; leucovorin rescue; use of sustained release allopurinol; non-oral use of allopurinol; bone marrow transplant stimulants, blood, platelet infusions, Neupogen, G-CSF; GM-CSF; pain management; anti-inflammatories; fluids; corticosteroids; insulin control medications; anti-pyretics; anti-nausea treatments; anti-diarrhea treatment; N-acetylcysteine; antihistamines.

An improvement in the therapeutic employment of an alkylating hexitol derivative for treatment of a TKI-resistant malignancy made by the use of monitoring drug levels after dosing in an effort to maximize a patient's drug plasma level, to monitor the generation of toxic metabolites, monitoring of ancillary medicines that could be beneficial or harmful in terms of drug-drug interactions is described herein. General examples described herein include: the monitoring of drug plasma protein binding, and monitoring of other pharmacokinetic or pharmacodynamic variables. Specific inventive examples for an alkylating hexitol derivative for treatment of a TKI-resistant malignancy include: multiple determinations of drug plasma levels; multiple determinations of metabolites in the blood or urine.

An improvement in the therapeutic employment of an alkylating hexitol derivative for treatment of a TKI-resistant malignancy made by exploiting unique drug combinations that may provide a more than additive or synergistic improvement in efficacy or side-effect management is described herein. Specific inventive examples described herein for an alkylating hexitol derivative for treatment of a TKI-resistant malignancy include: in combination with topoisomerase inhibitors; use with fraudulent nucleosides; use with fraudulent nucleotides; use with thymidylate synthetase inhibitors; use with signal transduction inhibitors; use with cisplatin or platinum analogs; use with alkylating agents such as the nitrosoureas (BCNU,Gliadel® wafers, CCNU, nimustine (ACNU), bendamustine (Treanda®)); use with alkylating agents that damage DNA at a different place than does dianhydrogalactitol or another alkylating hexitol derivative (TMZ, BCNU, CCNU, and other alkylating agents all damage DNA at O⁶ of guanine, whereas dianhydrogalactitol cross-links at N⁷); use with a monofunctional alkylating agent; use with a bifunctional alkylating agent; use with anti-tubulin agents; use with antimetabolites; use with berberine; use with apigenin; use with amonafide; use with colchicine or an analog thereof; use with genistein; use with etoposide; use with cytarabine; use with campothecins; use with vinca alkaloids; use with topoisomerase inhibitors; use with 5-fluorouracil; use with curcumin; use with NF-κB inhibitors; use with rosmarinic acid; use with mitoguazone; use with tetrandrine; use with temozolomide (TMZ); use in combination with biological therapies such as antibodies such as Avastin® (a VEGF inhibitor), Rituxan, Herceptin, Erbitux; use in combination with cancer vaccine therapy; use with epigenetic modulators; use with transcription factor inhibitors; use with taxol; use with homoharringtonine; use with pyridoxal; use with spirogermanium; use with caffeine; use with nicotinamide; use with methylglyoxalbisguanylhydrazone; use with Rho kinase inhibitors; use with 1,2,4-benzotriazine oxides; use with an alkylglycerol; use with an inhibitor of a Mer, Ax1, or Tyro-3 receptor kinase; use with an inhibitor of ATR kinase; use with a modulator of Fms kinase, Kit kinase, MAP4K4 kinase, TrkA kinase, or TrkB kinase; use with endoxifen; use with a mTOR inhibitor; use with an inhibitor of Mnk1a kinase, Mkn1b kinase, Mnk2a kinase, or Mnk2b kinase; use with a modulator of pyruvate kinase M2; use with a modulator of phosphoinositide 3-kinases; use with a cysteine protease inhibitor; use with phenformin; use with Sindbis virus-based vectors; use with peptidomimetics that act as mimetics of Smac and inhibit lAPs to promote apoptosis; use with a Raf kinase inhibitor; use with a nuclear transport modulator; use with an acid ceramidase inhibitor and a choline kinase inhibitor; use with tyrosine kinase inhibitors; use with anti-CS1 antibodies; use with inhibitors of protein kinase CK2; use with anti-guanylyl cyclase C (GCC) antibodies; use with histone deacetylase inhibitors; use with cannabinoids; use with glucagon-like peptide-1 (GLP-1) receptor agonists; use with inhibitors of Bcl-2 or Bcl-xL; use with Stat3 pathway inhibitors; use with inhibitors of polo-like kinase 1 (Plk1); use with GBPAR1 activators; use with modulators of serine-threonine protein kinase and poly(ADP-ribose) polymerase (PARP) activity; use with taxanes; use with inhibitors of dihydrofolate reductase; use with inhibitors of aromatase; use with benzimidazole-based anti-neoplastic agents; use with an O6-methylguanine-DNA-methyltransferase (MGMT) inhibitor; use with CCR9 inhibitors; use with acid sphingomyelinase inhibitors; use with peptidomimetic macrocycles; use with cholanic acid amides; use with substituted oxazaphosphorines; use with anti-TWEAK receptor antibodies; use with an ErbB3 binding protein; use with a glutathione S-transferase-activated anti-neoplastic compound; use with substituted phosphorodiamidates; use with inhibitors of MEKK protein kinase; use with COX-2 inhibitors; use with cimetidine and a cysteine derivative; use with anti-IL-6 receptor antibody; use with an antioxidant; use with an isoxazole inhibitor of tubulin polymerization; use with PARP inhibitors; use with Aurora protein kinase inhibitors; use with peptides binding to prostate-specific membrane antigen; use with CD19 binding agents; use with benzodiazepines; use with Toll-like receptor (TLR) agonists; use with bridged bicyclic sulfamides; use with inhibitors of epidermal growth factor receptor kinase; use with a ribonuclease of the T2 family having actin-binding activity; use with myrsinoic acid A or an analog thereof; use with inhibitors of a cyclin-dependent kinase; use with inhibitors of the interaction between p53 and MDM2; use with inhibitors of the receptor tyrosine kinase MET; use with largazole or largazole analogs; use with inhibitors of AKT protein kinase; use with 2'-fluoro-5-methyl-β-L-arabinofuranosyluridine or L-deoxythymidine; use with HSP90 modulators; use with inhibitors of JAK kinases; use with inhibitors of PDK1 protein kinase; use with PDE4 inhibitors; use with inhibitors of proto-oncogene c-Met tyrosine kinase; use with inhibitors of indoleamine 2,3-dioxygenase; use with agents that inhibit expression of ATDC (TRIM29); use with proteomimetic inhibitors of the interaction of nuclear receptor with coactivator peptides; use with antagonists of XIAP family proteins; use with tumor-targeted superantigens; use with inhibitors of Pim kinases; use with inhibitors of CHK1 or CH2 kinases; use with inhibitors of angiopoietin-like 4 protein; use with Smo antagonists; use with nicotinic acetylcholine receptor antagonists; use with farnesyl protein transferase inhibitors; use with adenosine A3 receptor antagonists; use with cancer vaccines; use with a JAK2 inhibitor; or use with a Src inhibitor.

An improvement in the therapeutic employment of an alkylating hexitol derivative for treatment of a TKI-resistant malignancy made by exploiting an alkylating hexitol derivative as a chemosensitizer where no measureable activity is observed when used alone but in combination with other therapeutics a more than additive or synergistic improvement in efficacy is observed is described herein. Specific inventive examples described herein for an alkylating hexitol derivative for treatment of a TKI-resistant malignancy include: as a chemosensitizer in combination with topoisomerase inhibitors; as a chemosensitizer in combination with fraudulent nucleosides; as a chemosensitizer in combination with fraudulent nucleotides; as a chemosensitizer in combination with thymidylate synthetase inhibitors; as a chemosensitizer in combination with signal transduction inhibitors; as a chemosensitizer in combination with cisplatin or platinum analogs; as a chemosensitizer in combination with alkylating agents such as BCNU, Gliadel wafers, CCNU, bendamustine (Treanda), or Temozolomide (Temodar); as a chemosensitizer in combination with anti-tubulin agents; as a chemosensitizer in combination with antimetabolites; as a chemosensitizer in combination with berberine; as a chemosensitizer in combination with apigenin; as a chemosensitizer in combination with amonafide; as a chemosensitizer in combination with colchicine or an analog thereof; as a chemosensitizer in combination with genistein; as a chemosensitizer in combination with etoposide; as a chemosensitizer in combination with cytarabine; as a chemosensitizer in combination with campothecins; as a chemosensitizer in combination with vinca alkaloids; as a chemosensitizer in combination with topoisomerase inhibitors; as a chemosensitizer in combination with 5-fluorouracil; as a chemosensitizer in combination with curcumin; as a chemosensitizer in combination with NF-κB inhibitors; as a chemosensitizer in combination with rosmarinic acid; as a chemosensitizer in combination with mitoguazone; or as a chemosensitizer in combination with tetrandrine.

An improvement in the therapeutic employment of an alkylating hexitol derivative for treatment of a TKI-resistant malignancy made by exploiting an alkylating hexitol derivative as a chemopotentiator where minimal therapeutic activity is observed alone but in combination with other therapeutics unique drug a more than additive or synergistic improvement in efficacy is observed is described herein. Specific inventive examples described herein for an alkylating hexitol derivative for treatment of a TKI-resistant malignancy include: as a chemopotentiator in combination with topoisomerase inhibitors; as a chemopotentiator in combination with fraudulent nucleosides; as a chemopotentiator in combination with thymidylate synthetase inhibitors; as a chemopotentiator in combination with signal transduction inhibitors; as a chemopotentiator in combination with cisplatin or platinum analogs; as a chemopotentiator in combination with alkylating agents such as BCNU, BCNU wafers, Gliadel, or bendamustine (Treanda); as a chemopotentiator in combination with anti-tubulin agents; as a chemopotentiator in combination with antimetabolites; as a chemopotentiator in combination with berberine; as a chemopotentiator in combination with apigenin; as a chemopotentiator in combination with amonafide; as a chemopotentiator in combination with colchicine or an analog thereof; as a chemopotentiator in combination with genistein; as a chemopotentiator in combination with etoposide; as a chemopotentiator in combination with cytarabine; as a chemopotentiator in combination with campothecins; as a chemopotentiator in combination with vinca alkaloids; as a chemopotentiator in combination with topoisomerase inhibitors; as a chemopotentiator in combination with 5-fluorouracil; as a chemopotentiator in combination with curcumin; as a chemopotentiator in combination with NF-κB inhibitors; as a chemopotentiator in combination with rosmarinic acid; as a chemopotentiator in combination with mitoguazone; or as a chemopotentiator in combination with tetrandrine.

An improvement in the therapeutic employment of an alkylating hexitol derivative for treatment of a TKI-resistant malignancy made by drugs, treatments and diagnostics to allow for the maximum benefit to patients treated with a compound is described herein. General examples described herein include: pain management, nutritional support, anti-emetics, anti-nausea therapies, anti-anemia therapy, anti-inflammatories. Specific inventive examples described herein for an alkylating hexitol derivative for treatment of a TKI-resistant malignancy include: use with therapies associated with pain management; use of nutritional support; use of anti-emetics; use of anti-nausea therapies; use of anti-anemia therapy; use of anti-inflammatories: use of antipyretics; use of immune stimulants.

An improvement in the therapeutic employment of an alkylating hexitol derivative for treatment of a TKI-resistant malignancy made by the use of by the use of complementary therapeutics or methods to enhance effectiveness or reduce side effects is described herein. Specific inventive examples described herein for an alkylating hexitol derivative for treatment of a TKI-resistant malignancy include: hypnosis; acupuncture; meditation; herbal medications created either synthetically or through extraction including NF-κB inhibitors (such as parthenolide, curcumin, rosmarinic acid); natural anti-inflammatories (including rhein, parthenolide); immunostimulants (such as those found in Echinacea); antimicrobials (such as berberine); flavonoids, isoflavones, and flavones (such as apigenenin, genistein, genistin, 6"-O-malonylgenistin, 6"-O-acetylgenistin, daidzein, daidzin, 6"-O-malonyldaidzin, 6"-O-acetylgenistin, glycitein, glycitin, 6"-O-malonylglycitin, and 6-O-acetylglycitin); applied kinesiology.

An improvement in the therapeutic employment of an alkylating hexitol derivative for treatment of a TKI-resistant malignancy made by alterations in the pharmaceutical bulk substance is described herein. General examples described herein include: salt formation, homogeneous crystalline structure, pure isomers. Specific inventive examples described herein for an alkylating hexitol derivative for treatment of a TKI-resistant malignancy include: salt formation; homogeneous crystalline structure; pure isomers; increased purity; lower residual solvents and heavy metals.

An improvement in the therapeutic employment of an alkylating hexitol derivative for treatment of a TKI-resistant malignancy made by alterations in the diluents used to solubilize and deliver/present the compound for administration is described herein. General examples described herein include:Cremophor® EL, cyclodextrins for poorly water soluble compounds. Specific inventive examples described herein for an alkylating hexitol derivative for treatment of a TKI-resistant malignancy include: use of emulsions; dimethyl sulfoxide (DMSO); N-methylformamide (NMF); dimethylformamide (DMF); dimethylacetamide (DMA); ethanol; benzyl alcohol; dextrose containing water for injection; Cremophor; cyclodextrins; PEG.

An improvement in the therapeutic employment of an alkylating hexitol derivative for treatment of a TKI-resistant malignancy made by alterations in the solvents used or required to solubilize a compound for administration or for further dilution is described herein. General examples described herein include: ethanol, dimethylacetamide (DMA). Specific inventive examples described herein for an alkylating hexitol derivative for treatment of a TKI-resistant malignancy include: the use of emulsions; dimethylsulfoxide (DMSO); N-methylformamide (NMF); dimethylformamide (DMF); dimethylacetamide (DMA); ethanol; benzyl alcohol; dextrose containing water for injection; Cremophor; PEG; or salt systems.

An improvement in the therapeutic employment of an alkylating hexitol derivative for treatment of a TKI-resistant malignancy made by alterations in the materials/excipients, buffering agents, or preservatives required to stabilize and present a chemical compound for proper administration is described herein. General examples described herein include: mannitol, albumin, EDTA, sodium bisulfite, benzyl alcohol. Specific inventive examples described herein for an alkylating hexitol derivative for treatment of a TKI-resistant malignancy include: the use of mannitol; the use of albumin; the use of EDTA; the use of sodium bisulfite; the use of benzyl alcohol; the use of carbonate buffers; the use of phosphate buffers; the use of polyethylene glycol (PEG); the use of vitamin A; the use of vitamin D; the use of vitamin E; the use of esterase inhibitors; the use of cytochrome P450 inhibitors; the use of multi-drug resistance (MDR) inhibitors; the use of organic resins; the use of detergents; the use of perillyl alcohol or an analog thereof; or the use of activators of channel-forming receptors.

An improvement in the therapeutic employment of an alkylating hexitol derivative for treatment of a TKI-resistant malignancy made by alterations in the potential dosage forms of the compound dependent on the route of administration, duration of effect, plasma levels required, exposure to side-effect normal tissues and metabolizing enzymes is described herein. General examples decribed herein include: tablets, capsules, topical gels, creams, patches, suppositories. Specific inventive examples described herein for use of alkylating hexitol derivatives include: the use of tablets; capsules; topical gels; topical creams; patches; suppositories; lyophilized dosage fills; the use of immediate-release formulations; the use of slow-release formulations; the use of controlled-release formulations; or the use of liquid in capsules.

An improvement in the therapeutic employment of an alkylating hexitol derivative for treatment of a TKI-resistant malignancy made by alterations in the dosage forms, container/closure systems, accuracy of mixing and dosage preparation and presentation is described herein. General examples described herein include: amber vials to protect from light, stoppers with specialized coatings. Specific inventive examples described herein for an alkylating hexitol derivative for treatment of a TKI-resistant malignancy include: the use of amber vials to protect from light; stoppers with specialized coatings to improve shelf-life stability.

An improvement in the therapeutic employment of an alkylating hexitol derivative for treatment of a TKI-resistant malignancy made by the use of delivery systems to improve the potential attributes of a pharmaceutical product such as convenience, duration of effect, reduction of toxicities is described herein. General examples described herein include: nanocrystals, bioerodible polymers, liposomes, slow release injectable gels, microspheres. Specific inventive examples described herein for an alkylating hexitol derivative for treatment of a TKI-resistant malignancy include: the use of oral dosage forms; the use of nanocrystals; the use of nanoparticles; the use of cosolvents; the use of slurries; the use of syrups, the use of bioerodible polymers; the use of liposomes; the use of slow release injectable gels; the use of microspheres; or the use of targeting compositions with epidermal growth factor receptor-binding peptides.

An improvement in the therapeutic employment of an alkylating hexitol derivative for treatment of a TKI-resistant malignancy made by alterations to the parent alkylating hexitol derivative with covalent, ionic, or hydrogen bonded moieties to alter the efficacy, toxicity, pharmacokinetics, metabolism, or route of administration is described herein. General examples described herein include: polymer systems such as polyethylene glycols, polylactides, polyglycolides, amino acids, peptides, or multivalent linkers. Specific inventive examples described herein for an alkylating hexitol derivative for treatment of a TKI-resistant malignancy include: the use of polymer systems such as polyethylene glycols; the use of polylactides; the use of polyglycolides; the use of amino acids; the use of peptides; the use of multivalent linkers; the use of immunoglobulins; the use of cyclodextrin polymers; the use of modified transferrin; the use of hydrophobic or hydrophobic-hydrophilic polymers; the use of conjugates with a phosphonoformic acid partial ester; the use of conjugates with a cell-binding agent incorporating a charged cross-linker; or the use of conjugates with β-glucuronides through a linker.

An improvement in the therapeutic employment of an alkylating hexitol derivative for treatment of a TKI-resistant malignancy made by alterations to the parent structure of a molecule with additional chemical functionalities that may alter efficacy, reduce toxicity, improve pharmacological performance, be compatible with a particular route of administration, or alter the metabolism of the therapeutic agent is described herein. General examples described herein include: alteration of side chains to increase or decrease lipophilicity, additional chemical functionalities to alter reactivity, electron affinity, binding capacity; salt forms. Specific inventive examples described herein for an alkylating hexitol derivative for treatment of a TKI-resistant malignancy include: alteration of side chains to increase or decrease lipophilicity; additional chemical functionalities to alter reactivity, electron affinity, or binding capacity; or salt forms.

An improvement in the therapeutic employment of an alkylating hexitol derivative for treatment of a TKI-resistant malignancy made by alterations to the molecule such that improved pharmaceutical performance is gained with a variant of the active molecule in that after introduction into the body a portion of the molecule is cleaved to reveal the preferred active molecule is described herein. General examples described herein include: enzyme sensitive esters, dimers, Schiff bases. Specific inventive examples described herein for an alkylating hexitol derivative for treatment of a TKI-resistant malignancy include: the use of enzyme sensitive esters; the use of dimers; the use of Schiff bases; the use of pyridoxal complexes; the use of caffeine complexes; the use of nitric oxide-releasing prodrugs; the use of prodrugs with fibroblast activation protein α-cleavable oligopeptides; the use of prodrugs that are products of reaction with an acetylating or carbamylating agent; the use of prodrugs that are hexanoate conjugates; the use of prodrugs that are polymer-agent conjugates; or the use of prodrugs that are subject to redox activation.

An improvement in the therapeutic employment of an alkylating hexitol derivative for treatment of a TKI-resistant malignancy made by the use of additional compounds, biological agents that, when administered in the proper fashion, a unique and beneficial effect can be realized is described herein. General examples described heein include: inhibitors of multi-drug resistance, specific drug resistance inhibitors, specific inhibitors of selective enzymes, signal transduction inhibitors, repair inhibition. Specific inventive examples described herein for an alkylating hexitol derivative for treatment of a TKI-resistant malignancy include: the use of inhibitors of multi-drug resistance; the use of specific drug resistance inhibitors; the use of specific inhibitors of selective enzymes; the use of signal transduction inhibitors; the use of repair inhibition; or the use of topoisomerase inhibitors with non-overlapping side effects.

An improvement in the therapeutic employment of an alkylating hexitol derivative for treatment of a TKI-resistant malignancy made by the use of an alkylating hexitol derivative in combination as sensitizers/potentiators with biological response modifiers is described herein. General examples described herein include: use in combination as sensitizers/potentiators with biological response modifiers, cytokines, lymphokines, therapeutic antibodies, antisense therapies, gene therapies. Specific inventive examples for an alkylating hexitol derivative for treatment of a TKI-resistant malignancy include: use in combination as sensitizers/potentiators with biological response modifiers; use in combination as sensitizers/potentiators with cytokines; use in combination as sensitizers/potentiators with lymphokines; use in combination as sensitizers/potentiators with therapeutic antibodies; use in combination as sensitizers/potentiators with antisense therapies such as Avastin®, Herceptin®, Rituxan®, Herceptin®, and Erbitux®; use in combination as sensitizers/potentiators with gene therapies; use in combination as sensitizers/potentiators with ribozymes; or use in combination as sensitizers/potentiators with RNA interference.

An improvement in the therapeutic employment of an alkylating hexitol derivative for treatment of a TKI-resistant malignancy made by exploiting the selective use of an alkylating hexitol derivative to overcome developing or complete resistance to the efficient use of biotherapeutics is described herein. General examples described herein include: tumors resistant to the effects of biological response modifiers, cytokines, lymphokines, therapeutic antibodies, antisense therapies, gene therapies. Specific inventive examples described herein for an alkylating hexitol derivative for treatment of a TKI-resistant malignancy include: use against tumors resistant to the effects of biological response modifiers; use against tumors resistant to the effects of cytokines; use against tumors resistant to the effects of lymphokines; use against tumors resistant to the effects of therapeutic antibodies; use against tumors resistant to the effects of antisense therapies; use against tumors resistant to the effects of therapies such as Avastin®, Herceptin®, Rituxan®, Herceptin®, and Erbitux®; use against tumors resistant to the effects of gene therapies; use against tumors resistant to the effects of ribozymes; or use against tumors resistant to the effects of RNA interference.

An improvement in the therapeutic employment of an alkylating hexitol derivative for treatment of a TKI-resistant malignancy made by exploiting the use of the alkylating hexitol derivative in combination with ionizing radiation, phototherapies, heat therapies, or radio-frequency generated therapies is described herein. General examples described herein include: hypoxic cell sensitizers, radiation sensitizers/protectors, photosensitizers, radiation repair inhibitors. Specific inventive examples described herein for an alkylating hexitol derivative for treatment of a TKI-resistant malignancy include: use in combination with ionizing radiation; use in combination with hypoxic cell sensitizers; use in combination with radiation sensitizers/protectors; use in combination with photosensitizers; use in combination with radiation repair inhibitors; use in combination with thiol depletion; use in combination with vaso-targeted agents; use in combination with radioactive seeds; use in combination with radionuclides; use in combination with radiolabeled antibodies; or use in combination with brachytherapy. Improvements in the efficacy of such radiation therapy or the ability to exert a synergistic effect by combining radiation therapy with the administration of an alkylating hexitol derivative are significant.

An improvement in the therapeutic employment of an alkylating hexitol derivative for treatment of a TKI-resistant malignancy made by optimizing its utility by determining the various mechanisms of action, biological targets of a compound for greater understanding and precision to better exploit the utility of the molecule is described herein. Specific inventive examples described herein for an alkylating hexitol derivative for treatment of a TKI-resistant malignancy include: use with inhibitors of poly-ADP ribose polymerase; use with agents that affect vasculature; use with agents that promote vasodilation; use with oncogenic targeted agents; use with signal transduction inhibitors; use with agents inducing EGFR inhibition; use with agents inducing Protein Kinase C inhibition; use with agents inducing Phospholipase C down-regulation; use with agents inducing jun down-regulation; use with agents modulating expression of histone genes; use with agents modulating expression of VEGF; use with agents modulating expression of ornithine decarboxylase; use with agents modulating expression of jun D; use with agents modulating expression of v-jun; use with agents modulating expression of GPCRs; use with agents modulating expression of protein kinase A; use with agents modulating expression of telomerase, use with agents modulating expression of prostate specific genes; use with agents modulating expression of protein kinases other than protein kinase A; use with agents modulating expression of histone deacetylase.

An improvement in the therapeutic employment of an alkylating hexitol derivative for treatment of a TKI-resistant malignancy made by more precise identification and exposure of the compound to those select cell populations where the compound's effect can be maximally exploited is described herein. Specific inventive examples described herein for an alkylating hexitol derivative for treatment of a TKI-resistant malignancy include: use against radiation sensitive cells; use against radiation resistant cells; or use against energy depleted cells.

An improvement in the therapeutic employment of an alkylating hexitol derivative for treatment of a TKI-resistant malignancy made by use of agents to enhance activity of the alkylating hexitol derivativeis described herein. General examples described herein include: use with nicotinamide, caffeine, tetandrine, or berberine. Specific inventive examples described herein for an alkylating hexitol derivative for treatment of a TKI-resistant malignancy include: use with nicotinamide; use with caffeine; use with tetandrine; or use with berberine.

It is described herein that these improvements in the therapeutic employment of an alkylating hexitol derivative can also be employed in: (1) the treatment of an *AHI1-*associated malignancy, such as a malignancy characterized by a mutation in or dysregulation of *AHI1*; and (2) the treatment of triple-negative breast cancer.

A method to improve the efficacy and/or reduce the side effects of the administration of an alkylating hexitol derivative for treatment of a TKI-resistant malignancy is described herein comprising the steps of:
(1) identifying at least one factor or parameter associated with the efficacy and/or occurrence of side effects of the administration of an alkylating hexitol derivative for treatment of a TKI-resistant malignancy; and
(2) modifying the factor or parameter to improve the efficacy and/or reduce the side effects of the administration of the alkylating hexitol derivative for treatment of the TKI-resistant malignancy.

Typically, a factor or parameter described herein is selected from the group consisting of:
(1) dose modification;
(2) route of administration;
(3) schedule of administration;
(4) indications for use;
(5) selection of disease stage;
(6) other indications;
(7) patient selection;
(8) patient/disease phenotype;
(9) patient/disease genotype;
(10) pre/post-treatment preparation
(11) toxicity management;
(12) pharmacokinetic/pharmacodynamic monitoring;
(13) drug combinations;
(14) chemosensitization;
(15) chemopotentiation;
(16) post-treatment patient management;
(17) alternative medicine/therapeutic support;
(18) bulk drug product improvements;
(19) diluent systems;
(20) solvent systems;
(21) excipients;
(22) dosage forms;
(23) dosage kits and packaging;
(24) drug delivery systems;
(25) drug conjugate forms;
(26) compound analogs;
(27) prodrugs;
(28) multiple drug systems;
(29) biotherapeutic enhancement;
(30) biotherapeutic resistance modulation;
(31) radiation therapy enhancement;
(32) novel mechanisms of action;
(33) selective target cell population therapeutics.
(34) use with an agent enhancing its activity.

As detailed above, it is described herein that these improvements in the therapeutic employment of an alkylating hexitol derivative can also be employed in: (1) the treatment of an *AHI1*-associated malignancy, such as a malignancy characterized by a mutation in or dysregulation of *AHI1*; and (2) the treatment of triple-negative breast cancer. Further details about the use of alkylating hexitol derivatives in the treatment of an *AHI1*-associated malignancy and in the treatment of triple-negative breast cancer are provided below.

As detailed above, the alkylating hexitol derivative described herein is typically a galactitol, a substituted galactitol, a dulcitol, or a substituted dulcitol, such as, but not limited to, dianhydrogalactitol, diacetyldianhydrogalactitol, dibromodulcitol, and derivatives and analogs thereof. These compounds described herein are either alkylating agents or prodrugs of alkylating agents.

These alkylating hexitol derivatives described herein include, but are not limited to: (1) dianhydrogalactitol; (2) derivatives of dianhydrogalactitol that, for example, have the hydrogen of the hydroxyl groups replaced with a lower alkyl, have the hydrogen attached to the epoxide ring replaced with a lower alkyl, or have the methyl groups attached to the same carbons that bear the hydroxyl groups replaced with a lower alkyl or substituted with, for example, halo groups; (3) diacetyldianhydrogalactitol; (4) derivatives of diacetyldianhydrogalactitol that, for example, have the methyl groups that are part of the acetyl moieties replaced with a lower alkyl, have the hydrogen attached to the epoxide ring replaced with a lower alkyl, or have the methyl groups attached to the same carbons that bear the acetyl groups replaced with a lower alkyl or substituted with, for example, halo groups; (5) dibromodulcitol; and (6) derivatives of dibromodulcitol that, for example, have one or more hydrogens of the hydroxyl groups replaced with a lower alkyl, or have one or both of the bromo groups replaced with another halo group such as chloro or fluoro.

When the improvement described herein is made by dose modification, the dose modification can be, but is not limited to, at least one dose modification selected from the group consisting of:
(a) continuous i.v. infusion for hours to days;
(b) biweekly administration;
(c) doses greater than 5 mg/m²/day;
(d) progressive escalation of dosing from 1 mg/m²/day based on patient tolerance;
(e) use of caffeine to modulate metabolism;
(f) use of isonazid to modulate metabolism;
(g) selected and intermittent boosting of dosage administration;
(h) administration of single and multiple doses escalating from 5mg/m²/day via bolus;
(i) oral dosages of below 30 mg/m²;
(j) oral dosages of above 130 mg/m²;
(k) oral dosages up to 40 mg/m² for 3 days and then a nadir/recovery period of 18-21 days;
(l) dosing at a lower level for an extended period (e.g., 21 days);
(m) dosing at a higher level;
(n) dosing with a nadir/recovery period longer than 21 days;
(o) the use of an alkylating hexitol derivative as a single cytotoxic agent;
(p) immediate release dosing;
(q) slow release dosing; and
(r) controlled release dosing.

The use of immediate release dosing is described in United States Patent No. 8,299,052 by Flanner et al. The use of slow release dosing is described in United States Patent No. 8,303,986 to Vergnault et al. The use of controlled release dosing is described in United States Patent No. 8,304,577 to Dzierba et al. Controlled release described herein dosing can be achieved by the use of biodegradable polymers such as, but not limited to, polylactic acid, polyepsilon caprolactone, polyhydroxy butyric acid, polyorthoesters, polyacetals, polydihydropyrans, polycyanoacrylates, and cross-linked or amphipathic block copolymers of hydrogels.

When the improvement described herein is made by route of administration, the route of administration can be, but is not limited to, at least one route of administration selected from the group consisting of:
(a) topical administration;
(b) oral administration;
(c) slow release oral delivery;
(d) intrathecal administration;
(e) intraarterial administration;
(f) continuous infusion;
(g) intermittent infusion;
(h) intravenous administration, such as intravenous administration for 30 minutes;
(i) administration through a longer infusion;
(j) administration through IV push; and
(k) intraperitoneal administration.

When the improvement described herein is made by schedule of administration, the schedule of administration can be, but is not limited to, at least one schedule of administration selected from the group consisting of:
(a) daily administration;
(b) weekly administration;
(c) weekly administration for three weeks;
(d) biweekly administration;
(e) biweekly administration for three weeks with a 1-2 week rest period;
(f) intermittent boost dose administration; and
(g) daily administration for one week for multiple weeks.

When the improvement described herein is made by selection of disease stage, the selection of disease stage can be, but is not limited to, at least one selection of disease stage selected from the group consisting of:
(a) use in an appropriate disease stage for a TKI-resistant malignancy;
(b) use for newly diagnosed disease;
(c) use for recurrent disease;
(d) use for resistant or refractory disease;
(e) use for treatment of an *AHI1*-associated malignancy;
(f) use for treatment of triple-negative breast cancer; and
(g) use for treatment of metastatic disease.

When the improvement described herein is made by patient selection, the patient selection can be, but is not limited to, a patient selection carried out by a criterion selected from the group consisting of:
(a) selecting patients with a disease condition characterized by a high level of a metabolic enzyme selected from the group consisting of histone deacetylase and ornithine decarboxylase;
(b) selecting patients with a low or high susceptibility to a condition selected from the group consisting of thrombocytopenia and neutropenia;
(c) selecting patients intolerant of GI toxicities;
(d) selecting patients characterized by over- or under-expression of a gene selected from the group consisting of c-Jun, a GPCR, a signal transduction protein, VEGF, a prostate-specific gene, and a protein kinase;
(e) selecting patients based on the *BIM* co-deletion; and
(f) selecting patients based on the existence of a mutation in or dysregulation of *AHI1.*

The cellular proto-oncogene *c-Jun* encodes a protein that, in combination with c-Fos, forms the AP-1 early response transcription factor. This proto-oncogene plays a key role in transcription and interacts with a large number of proteins affecting transcription and gene expression. It is also involved in proliferation and apoptosis of cells that form part of a number of tissues, including cells of the endometrium and glandular epithelial cells. G-protein coupled receptors (GPCRs) are important signal transducing receptors. The superfamily of G protein coupled receptors includes a large number of receptors. These receptors are integral membrane proteins characterized by amino acid sequences that contain seven hydrophobic domains, predicted to represent the transmembrane spanning regions of the proteins. They are found in a wide range of organisms and are involved in the transmission of signals to the interior of cells as a result of their interaction with heterotrimeric G proteins. They respond to a diverse range of agents including lipid analogues, amino acid derivatives, small molecules such as epinephrine and dopamine, and various sensory stimuli. The properties of many known GPCR are summarized in S. Watson & S. Arkinstall, "The G-Protein Linked Receptor Facts Book" (Academic Press, London, 1994). GPCR receptors include, but are not limited to, acetylcholine receptors, β-adrenergic receptors, β₃-adrenergic receptors, serotonin (5-hydroxytryptamine) receptors, dopamine receptors, adenosine receptors, angiotensin Type II receptors, bradykinin receptors, calcitonin receptors, calcitonin gene-related receptors, cannabinoid receptors, cholecystokinin receptors, chemokine receptors, cytokine receptors, gastrin receptors, endothelin receptors, γ-aminobutyric acid (GABA) receptors, galanin receptors, glucagon receptors, glutamate receptors, luteinizing hormone receptors, choriogonadotrophin receptors, follicle-stimulating hormone receptors, thyroid-stimulating hormone receptors, gonadotrophin-releasing hormone receptors, leukotriene receptors, Neuropeptide Y receptors, opioid receptors, parathyroid hormone receptors, platelet activating factor receptors, prostanoid (prostaglandin) receptors, somatostatin receptors, thyrotropin-releasing hormone receptors, vasopressin and oxytocin receptors.

Abelson Helper Integration Site-1 (*AHI1*) is a novel oncogene that has been found to be deregulated in several leukemic cell lines, including CML (chronic myelocytic leukemia). AHI1 enhances the effect of BCR-ABL1 *in vivo* and induces a number of kinases, including JAK2, STAT5, and Src family kinases, eventually mediating response or resistance to thymidine kinase inhibitors (TKIs). A number of studies have shown that *AHI1* overexpression in primitive hematopoietic cells confers a growth advantage *in vitro* to such cells, can induce leukemia *in vivo,* and enhance the effects of BCR-ABL1. Studies with cells from patients with CML have also shown that *AHI1* also contributes to BCR-ABL1-induced malignant transformation by mediating TKI resistance. In addition, mutations within the kinase domain of the ABL1 protein can also contribute to resistance to imatinib and other TKIs; the most frequently found mutation is a point mutation involving a single amino acid substitution, T315I (T.B. Balci et al., "AHI1 Gene Expression Levels and BCR-ABL1 T315I Mutations in Chronic Myeloid Leukemia Patients," Hematology 16: 357-360 (2011)). The *AHI1* locus was initially identified as a common helper provirus integration site in Abelson pre-B-cell lymphomas and was shown to be closely linked to the *c-myb* proto-oncogene. Because no significant alteration of *c-myb* expression was found in Abelson murine leukemia virus-induced pre-B-lymphomas harboring a provirus inserted within the *AHI1* locus, this suggests that this locus includes at least one other gene whose dysregulation is involved in tumor formation. This gene at this locus is the *AHI1* gene. The proviral insertions were found at the 3'-end of the gene in an inverse transcriptional orientation, with most of the proviral insertions being located around or downstream of the last exon of the gene; another insertion was found within intron 22 of the gene. In addition, another previously identified provirus insertion site, Mis-2, was found to map within intron 16 of the *AHI1* gene. The AHI1 cDNA encodes a protein of 1047 amino acid residues. The predicted AHI1 protein is a modular protein that contains one SH3 motif and seven WD-40 repeats. The *AHI1* gene is highly conserved in mammals and encodes two major RNA species of 5 and 4.2 kb and several other, shorter, splicing variants. The *AHI1* gene is expressed in mouse embryos and in several organs of the mouse and rat; notably, expression occurs at high levels in the brain and testes. In tumor cells harboring insertional mutations in *AHI1*, truncated *AHI1*/viral fused transcripts have been identified, including some splicing variants with deletion of the SH3 domain. *AHI1* exhibits several features of a signaling molecule and is believed to play an important role in signal transduction in normal cells. *AHI1* is also likely to be involved in tumor development, possibly in cooperation with other oncogenes (such as v-*abl* and *c-myc*) or with a tumor suppressor gene (*Nf1*), since *AHI1* insertion sites were identified in tumors harboring v-*abl* defective retroviruses or a *c-myc* transgene or in tumors exhibiting deletion of *Nf1.* The full-length human *AHI1* gene consists of 29 exons (i.e., exons 1 to 33, excluding exons 24, 28, 29, and 32); the termination codon is located in exon 33. However, there is a truncated isoform that lacks the SH3 domain and is made up of 24 exons (exons 1 to 24 only) and contains an in-frame termination codon in exon 24. There is a second truncated isoform that is made up of 32 exons (exons 1 to 33, excluding exon 24), but with a termination codon in exon 28. Some of the 3'-end insertional mutations resulting from provirus insertion can alter the normal splicing machinery and can force alternative splicing within viral or intronic sequences close to them, thus deleting exons that would normally code for carboxyl-terminal regions of the AHI1 protein. Genetic evidence suggests that provirus insertional mutation of the *AHI1* gene contributes to tumor formation in a number of different cell lineages. The *AHI1* locus was identified as a common provirus integration site, initially in Abelson (*v*-*abl*-induced) pre-B lymphomas and later in *c-myc* induced T-cell lymphomas in MMTV^{D}/myc Tg mice, suggesting that it may cooperate in some way with these oncogenes to induce tumor formation. This locus has also been found to be rearranged by provirus insertion in other types of tumors, namely in pre B-cell tumors of Eµ/myc Tg mice and in acute myeloid leukemia in *Nf1* heterozygous mice. Similarly, the *Mis-2* locus was identified as a common provirus insertion site in Moloney MuLV-induced rat T-cell lymphomas (thymomas). The fact that these provirus insertional mutations are not random and have been identified in a relatively high proportion of tumors suggests that the mutations have been selected during the oncogenic process and that they are involved in the oncogenic process. The role of the insertional mutations in the oncogenic process is supported by the several examples of common provirus insertional sites that have been found to activate proto-oncogenes in nondefective retrovirus-induced tumors. The analysis of RNA in tumors harboring mutations in the *AHI1* gene suggests that the mechanism by which these insertional mutations may enhance the oncogenic potential of the *AHI1* gene is through the generation of truncated forms of the *AHI1* RNA with the capacity to code for truncated AHI1 proteins. These truncated RNAs are specific to the tumors analyzed and have not been found in nonrearranged tumors. These are likely to be involved in the transformation process. The deletion of sequences coding for the carboxyl-terminus of the protein, including the SH3 domain, may significantly affect the interaction of AHI1 with other proteins, possibly converting this molecule to a dominant-negative mutant. Alternatively, truncated AHI1 proteins may represent aberrant gain-of-function mutants, if the SH3 domain binds intramolecularly, or if the SH3 domain binds to an inhibitor. In nonreceptor tyrosine kinases, such as the proto-oncogene c-src or *c-abl,* deletion or mutation of their SH3 domain generally leads to the oncogenic activation of their tyrosine kinase, suggesting that the SH3 domain binds to an inhibitor of the kinase activity. Thus, the SH3 deletion in AHI1 may uncover some domains and allow novel protein interactions or may prevent the binding of an inhibitor. The fact that *AHI1* cDNA with the potential to encode very similar truncated proteins to the ones detected in tumors have been isolated from normal tissues suggest that truncated AHI1 proteins may not necessarily be oncogenic in each tissue. Rather, the inappropriate, dysregulated, or increased expression of truncated AHI1 proteins in some specific cell types may contribute to the transformation process (X. Jiang et al. "Ahi-1, a Novel Gene Encoding a Modular Protein with WD40-Repeat and SH3 Domains, Is Targeted by the Ahi-1 and Mis-2 Provirus Integrations," J. Virol. 76: 9046-9059 (2002)). The *AHI1* locus is also involved in the development of the acute myeloid leukemia (AML) that arises in *NF-1* heterozygous mice and in Moloney murine leukemia virus (Mo-MuLV)-induced rat T-cell lymphomas. Although the function of the AHI1 protein has not been completely determined, it clearly plays a role in signaling through several pathways. The AHI1 protein is known to have multiple Src homology 3 (SH3) binding sites, an SH3 domain, and multiple tryptophan-aspartic acid 40 (WD40)-repeat domains. There at least three human isoforms of this protein. The shorter human isoform II lacks the SH3 domain, and human isoform III, although also shorter than the full-length isoform I, contains additional coding sequences not present in either isoform I or isoform II. The *AHI1* gene is therefore subject to alternative splicing, even in normal cells. However, mutant forms of *AHI1* can serve as cooperating oncogenes, particularly in the development of human hematopoietic malignancies. Transcription of *AHI1* was found to be most active in the most primitive types of normal hematopoietic cells and was then down-regulated during their early differentiation. Marked deregulation of *AHI1* expression, with greatly increased levels of expression, was seen in a broad spectrum of human leukemic cell lines and in cells obtained directly from Philadelphia chromosome-positive (Ph⁺) but not Ph⁻ leukemias. This strongly suggests that alterations in expression of *AHI1* are important in the development of Ph⁺ leukemias. During normal human hematopoietic cell differentiation, there was an overall 6-fold decrease in the expression of *AHI1* from the most primitive lin⁻CD34⁺CD38⁻ subset to the most mature lin⁺CD34⁻ cells. Splicing perturbations were also seen, including relative upregulation of isoforms I and II. Isoform II lacks the SH3 domain and is a truncated protein that may have distinct properties, possibly including gain-of-function activities; the loss of the SH3 domain may disrupt the normal signaling function of the AHI1 protein (X. Jiang et al., "Deregulated Expression in Ph+ Human Leukemias of AHI-1, a Gene Activated by Insertional Mutagenesis in Mouse Models of Leukemia," Blood 103: 3897-3904 (2004)).

Additionally, the AHI1 protein interacts with the BCR-ABL fusion protein and other proteins involved in hematopoiesis and regulation of hematopoiesis. This interaction is described in L. L. Zhou et al. "AHI-1 Interacts with BCR-ABL and Modulates BCR-ABL Transforming Activity and Imatinib Response to CML Stem/Progenitor Cells," J. Exp. Med. 11: 2657-2671 (2005). Specifically, chronic myelocytic leukemia (CML) is initiated and propagated by a rare population of CML stem cells that have acquired a *BCR-ABL* fusion gene, which encodes a chimeric oncoprotein (BCR-ABL) that displays constitutively elevated tyrosine kinase activity that drives CML pathogenesis. This involves, among other effects, deregulation of cellular proliferation and apoptosis control through effects on multiple signaling pathways, including the Ras, phosphatidylinositol 3-kinase (PI3K), JAK-STAT, and NF-κB pathways. As detailed above, imatinib mesylate, an inhibitor of the BCR-ABL tyrosine kinase, frequently used for treatment of CML, is subject to the development of resistance to the tyrosine kinase inhibitor, leading to relapses or treatment failures. Although other tyrosine kinase inhibitors such as dasatinib and nilotinib may be useful in cases in which resistance has developed or is likely to develop to imatinib, there is a still a need to prevent the development of resistance by a mechanism that does not depend on the specific interactions between a tyrosine kinase inhibitor and the BCR-ABL tyrosine kinase.

Recent studies have demonstrated that CML stem/progenitor cells in chronic phase patients are less responsive to IM and other tyrosine kinase inhibitors and are a critical target population for the development of resistance to imatinib mesylate. Such CML stem/progenitor cells have the property of genetic instability and frequently give rise to imatinib mesylate-resistant mutants *in vitro.*

As indicated above, the *Ahi-1* gene (in mouse) or its conserved human homolog (*AHI-1*) is a novel gene that was identified by provirus insertional mutagenesis in v-abl-induced mouse pre-B cell lymphoma as a candidate cooperate oncogene. The mouse *Ahi-1* gene encodes a unique protein with a SH3 domain, multiple SH3 binding sites, and a WD40-repeat domain, which are all known to be important mediators of protein-protein interactions, suggesting that the normal Ahi-1 protein (in mouse) or the human homolog AHI-1 protein has novel signaling activities and that its deregulation could affect specific cellular signaling pathways. The conserved human homolog AHI-1 has an additional coiled-coil domain in its amino-terminal region. The expression of the *Ahi-1* gene (in mice) or the *AHI-1* gene (in humans) has been demonstrated to be regulated at multiple stages of hematopoiesis in a manner that is highly conserved between mice and humans. In general, these genes, in both mice and humans, are expressed at their highest levels in the most primitive hematopoietic cells and then are rapidly downregulated as the cells begin to differentiate. In general, deregulation of *AHI-1* expression has been seen in a number of human leukemic cell lines, particularly in a CML cell line (K562) and in Philadelphia chromosome-positive (Ph⁺ BCR-ABL⁺) primary leukemic cells, but not Ph⁻ cells, especially in highly enriched leukemic stem cells from patients with CML. In addition, levels of *BCR-ABL* transcripts are highly elevated in the same CML stem cell population, suggesting that it may be important to cooperative activities of AHI-1 and BCR-ABL to generate a permanently expanding clone of deregulated stem cells at the early stage of leukemia development.

Overexpression of the mouse gene *Ahi-1* alone in primitive hematopoietic cells was found to confer a proliferative advantage *in vitro* and to induce a lethal leukemia *in vivo*; these effects were enhanced by *BCR-ABL.* Stable suppression of the homologous human gene *AHI-1* by small interfering RNA (siRNA) in *BCR-ABL-*transduced primitive human cord blood and primitive leukemic cells from CML patients reduces their growth autonomy *in vitro* and therefore would be expected to make such cells less leukemogenic. One alternative for siRNA is the siRNA molecules described in A. Ringrose et al., "Evidence for an Oncogenic Role of AHI-1 in Sezary Syndrome, A Leukemic Variant of Human Cutaneous T-Cell Lymphoma," Leukemia 20: 1593-1601 (2006). The oligonucleotides encoding these siRNAs were 5'-GATCCCCGTGATGATCCCGACACTATTTCAAGAGAATAGTGTCGGGATCATCACTT TTTA-3' (SEQ ID NO: 12) and 5'-AGCTTAAAAAGTGATGATCCCGACACTATTCTCTTGAAATAGTGTCGGGATCATCA CGGG-3' (SEQ ID NO: 13). In addition, overexpression of *Ahi-1* (in mice) induces abnormal differentiation (including lineage switching) Thus, overexpression of *Ahi-1* alone in mice in IL-3-dependent hematopoietic cells leads to strong transforming activity both *in vitro* and *in vivo,* and this is additive with the effects of *BCR-ABL.* The overexpression of *Ahi-1* confers a growth advantage on mouse hematopoietic stem/progenitor cells and enhances the effects of *BCR-ABL.* The overexpression was monitored by Q-RT-PCR analysis of mRNA transcripts. Moreover, elevated *Ahi-1* expression has been obverved in cells transduced with *BCR-ABL* alone without transduction with *Ahi-1* itself, although the level of expression was less than for cells transduced with *Ahi-1.*

Additionally, the effect of either suppression or overexpression of the human homolog *AHI-1* in K562 cells, a cell line derived from a patient with CML and characterized by highly increased expression of *AHI-1* was investigated, with suppression of *AHI-1* expression restoring growth control to some extent and reducing growth-factor-dependent proliferation, the size of colonies that were formed, and the ability to form clones from single cells. By contrast, overexpression of *AHI-1* resulted in sharply increased colony-forming ability compared with control cells; restored expression of *AHI-1* gene or AHI-1 protein in cells subject to siRNA interference reversed growth deficiencies attributable to suppression of *AHI-1.* Analogous results were seen *in vivo* with variations of expression in *AHI-1.*

Similarly, *in vivo,* suppression of *AHI-1* expression in primitive *BCR-ABL-*transduced human CB cells and primary CML stem/progenitor cells reduced their growth autonomy. The suppression was performed by employing lentiviral RNA interference. The results suggest that AHI-1 may play a role in overproduction of myeloid cells in CML.

Additionally, *AHI-1* transcript levels were evaluated in pretreatment lin⁻ CD34⁺ cells from CML chronic phase patients with subsequent clinical responses to imatinib mesylate therapy (both responders and nonresponders) and from patients in blast crisis. Increased levels of *AHI-1* expression were observed in lin⁻CD34⁺ stem/progenitor cells from all patient samples compared with lin⁻CD34⁺ normal BM cells. Cells from imatinib mesylate nonresponders expressed higher levels of *AHI-1* transcripts than cells from responders. The overexpression could be suppressed by lentiviral RNA interference. There was a greater reduction in colony-forming cells induced by lentiviral RNA interference in transduced primary CML cells from nonresponders or from patients in blast crisis than from responders to imatinib mesylate.

The cooperative effects of *Ahi-1* were investigated in a *BCR-ABL-*transduced BaF3 cell line in which the level of expression of p210^{BCR-ABL} can be variably downregulated by exposure to doxycycline. Reduction in BCR-ABL protein expression in the presence of doxycycline resulted in a corresponding decrease in growth-factor independence of BaF3 cells in both liquid suspension cultures and in semisolid cultures *in vitro*; this suggests that the reduction of BCR-ABL protein expression is correlated with a reduction in tumorigenic potential. Similarly, downregulation of *BCR-ABL* expression completely inhibited colony-forming cell generation in semisolid cultures in the absence of IL-3. However, introduction of *Ahi-1* into cells with inhibited *BCR-ABL* expression enabled them to grow continuously in liquid suspension culture, to have fewer Annexin V⁺ apoptotic cells, and to produce more factor-independent colony-forming cells than cells transduced with *BCR-ABL* alone. This is consistent with an increase in tumorigenic potential caused by the expression of *Ahi-1* in these cells. In summary, these results demonstrate the ability of *Ahi-1* to reverse *in vitro* growth deficiencies resulting from downregulation of *BCR-ABL* and provide evidence of the regulatory role of *Ahi-1* in *BCR-ABL*-mediated transformation.

Additionally, it has also been demonstrated that coexpression of *Ahi-1* in *BCR-ABL*-inducible cells sustains tyrosine phosphorylation of BCR-ABL and enhances activation of JAK2 and STAT5. Specifically, tyrosine phosphorylation of p210^{BCR-ABL} was not significantly suppressed in cells that had been cotransduced with both *Ahi-1* and *BCR-ABL* even in the presence of doxycycline. Similarly, BCR-ABL protein expression was also suppressed to a lesser degree in cotransduced cells than in cells transduced with *BCR-ABL* only, and Ahi-1 protein expression was found to be higher in cotransduced cells than in cells transduced with *BCR-ABL* only. There were also increased levels of phosphorylation of JAK2, STAT5, NF-κB p65 (at Ser-563 and Ser-468), and Src (at Tyr-416) in *BCR-ABL-*inducible cells and and *Ahi*-1-cotransduced *BCR-ABL-*inducible cells compared with control BaF3. Furthermore, phosphorylation of most downstream proteins was downregulated when BCR-ABL expression was inhibited by doxycycline, but sustained phosphorylation of JAK2 and STAT5 was consistently observed in cotransduced cells in the presence of IL-3 and doxycycline. In the absence of IL-3, phosphorylation of JAK2 and STAT5 was reduced in cotransduced cells when BCR-ABL expression was suppressed. A similar, albeit less pronounced, finding of sustained phosphorylation of Src was also observed, particularly in Ahi-1⁺ BCR-ABL cells in the presence of IL-3. These results suggest that Ahi-1 may play a regulatory role in the mediation of BCR-ABL-activity associated with enhanced activation of JAK2 and STAT5 through the IL-3 signaling pathway, and also in the activation of Src.

Additionally, a physical interaction was detected between AHI-1 and ABL in CML cells by coimmunoprecipitation. Tyrosine phosphorylated p210^{BCR-ABL} could be detected in K562 cells using an antiphosphotyrosine antibody. This protein complex is also associated with tyrosine-phosphorylated JAK2. An antigenic peptide derived from the sequence of AHI-1 specifically blocked the ability of the AHI-1 antibody to precipitate both tyrosine-phosphorylated BCR-ABL and JAK2; an unrelated peptide had no effect. This interaction complex was also found to be modulated by the tyrosine kinase activity of BCR-ABL, as treatment of the cells with imatinib mesylate resulted in the inability to detect both tyrosine-phosphorylated BCR-ABL and JAK2. These results indicate that AHI-1 and BCR-ABL can interact and form a complex involving tyrosine-phosphorylated JAK2.

The interaction of BCR-ABL and AHI-1 together with JAK2 mediates the imatinib mesylate sensitivity/resistance of BCR-ABL⁺ cells. In experiments in which *BCR-ABL*-transduced BaF3 cells and cells cotransduced with *Ahi-1* were treated with various doses of imatinib mesylate, *BCR-ABL*-transduced cells showed a significant reduction in colony-forming cell output in response to imatinib mesylate treatment in the presence or absence of IL-3. However, BaF3 cells cotransduced with both *BCR-ABL* and *Ahi-1* showed no response to imatinib mesylate and produced as many colony-forming cells in the presence of IL-3 as were produced by the same cells without treatment with imatinib mesylate. Cotransduced cells also displayed greater resistance to imatinib mesylate in terms of colony-forming cell production in the absence of IL-3, although these cells were more sensitive to imatinib mesylate treatment than cells in the presence of IL-3. These results indicate that *Ahi-1* is capable of overcoming IM-induced growth suppression in BCR-ABL⁺ cells when IL-3 signaling is activated in these cells. Similar results were seen in human K562 cells with regard to the overexpression or suppression of *AHI-1.* Overexpression resulted in greater resistance to imatinib mesylate treatment, while suppression by lentiviral RNA interference resulted in increased sensitivity to imatinib mesylate. However, overexpression even in the presence of lentiviral RNA interference restored resistance to imatinib mesylate. Western-blot analysis revealed increased tyrosine-phosphorylated BCR-ABL, JAK2, and STAT5 in cells with *AHI-1* overexpression and reduced levels of these phosphorylated proteins in cells with suppression of *AHI-1* expression; when an AHI-1 construct was reintroduced into cells in which lentiviral RNA interference had been performed, the phosphorylation of BCR-ABL, JAK2, and STAT5 increased. Additionally, expression of AHI-1 not only modulates phosphorylation of BCR-ABL, JAK2, and STAT5 in BCR-ABL⁺ K562 cells, but also regulates protein expression of these genes, as demonstrated by significantly enhanced expression of these proteins when *AHI-1* is overexpressed, reduced expression when *AHI-1* is suppressed, and restored expression in AHI-1-suppressed cells where *AHI-1* expression has been rescued by introduction of an AHI-1 construct.

Consistent with these observations were observations on the sensitivity of lin⁻CD34⁺ CML stem/progenitor cells with and without suppression of *AHI-1* expression of *AHI-1* expression to imatinib mesylate, dasatinib, and nilotinib. Suppression of *AHI-1* expression increased sensitivity to all three tyrosine kinase inhibitors; however, under all conditions, the cells were more sensitive to dasatinib than to the other two tyrosine kinase inhibitors. These results suggest that *AHI-1* plays an important role in modulating sensitivity to imatinib mesylate and other selective BCR-ABL tyrosine kinase inhibitors in BCR-ABL⁺ CML cells.

An important observation is that coexpression of *Ahi-1* in *BCR-ABL-*inducible cells can rescue growth factor-independent cell growth that is inhibited by down-regulation of *BCR-ABL.* Interestingly, this renewed GF independence with introduction of *Ahi-1* appears to be regulated by sustained phosphorylation of BCR-ABL, rather than its continual expression, as these effects were observed in cotransduced cells where *BCR-ABL* expression was inducibly suppressed *in vitro.* These results suggest that that physical interaction between Ahi-1 (in mice) or AHI-1 (in humans) and BCR-ABL may stabilize a protein-protein interaction complex that enables constitutively active BCR-ABL tyrosine kinase activity and further alters specific downstream BCR-ABL signaling pathways that deregulate cellular proliferation and apoptosis control. This is further supported by identification of JAK2 as an associated protein in this interaction complex and observation of enhanced activity of the JAK2-STAT5 pathway in *BCR-ABL*-inducible cells with cotransduction of *Ahi-1* in the presence of GF stimulation with IL-3. It is known that BCR-ABL signaling closely mimics signaling pathways of cytokine receptors and that both IL-3/GM-CSF receptor activation and the BCR-ABL oncoprotein can induce a tyrosine phosphorylation cascade of which numerous proteins, including JAK2 and STAT5, are common substrates. Interestingly, *BCR-ABL*-expressing cells have many similarities to cells induced by IL-3 stimulation or cells with forced IL-3 overexpression. It has been shown that BCR-ABL can interact with the common β chain of IL-3/GM-CSF receptor and constitutively activate JAK2. In particular, increased phosphorylation of STAT5, which was previously thought to be an immediate function of the BCR-ABL oncoprotein, has now been shown in primary CML CD34⁺ progenitor cells to occur largely as a consequence of BCR-ABL-induced activation of IL-3 autostimulation, leading to activation of STAT5. It has been reported that targeted disruption of the *STAT5A* and *STAT5B* genes reduces myeloid progenitor numbers, suggesting a nonredundant role for STAT5 in primitive normal hematopoiesis. It also has been further suggested that autocrine production of GM-CSF may contribute to IM and NL-resistance in BCR-ABL⁺ progenitors through activation of JAK2 and STAT5 pathway, and inhibition of *STAT5* expression by a shRNA approach significantly reduced colony formation of CD34⁺ CML progenitor cells in vitro. In addition, JAK2 is known to interact with the C-terminal region of BCR-ABL, and recent studies further show that mouse hematopoietic cells transformed by the T315I mutant of *BCR-ABL* can be induced to undergo apoptosis by a JAK2 inhibitor, (E)-N-benzyl-2-cyano-3-(3,4-dihydroxyphenyl)acrylamide (AG490). Collectively, these results indicate that activation of the JAK2-STAT5 pathway in CML stem/progenitors is likely to be an important mechanism contributing to responses to BCR-ABL-targeted therapies and identification of Ahi-1/AHI-1 as a novel mediator involved in this pathway suggests AHI-1 alone or in combination with JAK2 and STAT5, as potential additional therapeutic targets. A potential mechanism for a physical interaction between Ahi-1/AHI-1 and BCR-ABL is revealed based on their molecular structures, which are compatible with specific protein-protein interactions. Ahi-1 may interact with BCR-ABL through its SH3 domain or its SH3 binding sites (i.e., the SH3 domain of one protein interacting with the SH3 binding sites of the other) or through the SH2 domain of BCR-ABL if Ahi-1 is tyrosine phosphorylated (Ahi-1 contains two potential tyrosine phosphorylation sites). In addition, Ahi-1 may bind to a SH2-containing protein that is a substrate of BCR-ABL, thus forming a complex, as it is known that BCR-ABL is extensively tyrosine-phosphorylated, providing numerous, potential docking sites for SH2 domain-containing proteins. Moreover, Ahi-1 may interact with multiple domains of BCR-ABL, as demonstrated by other BCR-ABL-interacting proteins. These results, namely that coexpression of *Ahi-1* in *BCR-ABL-* transduced cells can completely rescue imatinib mesylate-induced suppression of cell growth in the presence of IL-3 suggests that Ahi-1 may not be a direct substrate of the BCR-ABL tyrosine kinase, but rather a modular protein that forms a stable protein interaction complex with other tyrosine phosphorylated proteins to mediate IL-3-dependent BCR-ABL and JAK2-STAT5 activities. In addition, this protein interaction complex seems to be disrupted by suppression of tyrosine phosphorylation of BCR-ABL by imatinib mesylate. Additionally, it has been observed that enhanced phosphorylation of Src was observed in *Ahi-1* coexpressed *BCR-ABL*-inducible cells in the presence of IL-3, suggesting that other kinases are also activated with stimulation of IL-3 when BCR-ABL and AHI-1 are coexpressed. This finding also explains the observation that CML progenitor cells with suppression of *AHI-1* experienced greater inhibition of colony-forming cell generation in response to dasatinib, a more potent thymidine kinase inhibitor that also inhibits Src activity.

A medicament for use in a method for the treatment of a malignancy in a subject suffering from a malignancy who has a germline deletion polymorphism conferring resistance to thymidine kinase inhibitors (TKIs) the medicament comprising: (1) a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy; and (2) a therapeutically effective quantity of a JAK2 inhibitor to the subject to treat the malignancy is described herein.

A JAK2 inhibitor described herein can be, but is not limited to, (E)-N-benzyl-2-cyano-3-(3,4-dihydroxyphenyl)acrylamide (AG490), ruxolitinib, tofacitinib, tofacitinib citrate, N-tert-butyl-3-(5-methyl-2-(4-(2-(pyrrolidin-1-yl)ethoxy)phenylamino)pyrimidin-4-ylamino)benzenesulfonamide (TG-101348), (S)-5-chloro-N2-(1-(5-fluoropyrimidin-2-yl)ethyl)-N4-(5-methyl-1H-pyrazol-3-yl)pyrimidine-2,4-diamine (AZD1480), N-(cyanomethyl)-4-(2-(4-morpholinophenylamino)pyrimidin-4-yl)benzamide (CYT387), baricitinib, (S,E)-3-(6-bromopyridin-2-yl)-2-cyano-N-(1-phenylethyl)acrylamide (WP1066), S-ruxolitinib, N-tert-butyl-3-(5-methyl-2-(4-(4-methylpiperazin-1-yl)phenylamino)pyrimidin-4-ylamino)benzenesulfonamide (TG101209), N-[3-(4-methyl-1-piperazinyl)phenyl]-8-[4-(methylsulfonyl)phenyl]-[1,2,4]triazolo[1,5-a]pyridin-2-amine (CEP33779), 8-(3,5-difluoro-4-(morpholinomethyl)phenyl)-2-(1-(piperidin-4-yl)-1H-pyrazol-4-yl)quinoxaline (NVP-BSK805), (S)-5-fluoro-2-(1-(4-fluorophenyl)ethylamino)-6-(5-methyl-1H-pyrazol-3-ylamino)nicotinonitrile (AZ 960), 3-(4-chloro-2-fluorobenzyl)-2-methyl-N-(3-methyl-1H-pyrazol-5-yl)-8-(morpholinomethyl)imidazo[1,2-b]pyridazin-6-amine (LY2784544), 1-cyclopropyl-3-(3-(5-(morpholinomethyl)-1H-benzo[d]imidazol-2-yl)-1H-pyrazol-4-yl)urea (AT9283), pacritinib (SB1518), (S)-N-(4-(2-((4-morpholinophenyl)amino)pyrimidin-4-yl)phenyl)pyrrolidine-2-carboxamide (XL019), and N-tert-butyl-3-(5-methyl-2-(4-(2-(pyrrolidin-1-yl)ethoxy)phenylamino)pyrimidin-4-ylamino)benzenesulfonamide (TG101348). Other JAK2 inhibitors are known in the art, and are described in United States Patent No. 8,367,078 to Sayeski et al., including 2-methyl-1-phenyl-4-pyridin-2-yl-2-(2-pyridin-2-ylethyl)butan-1-one; 3-[5-[(4-oxo-4-phenyl-butan-2-ylidene)amino]pentylimino]-1-phenyl-butan-1-one; 2-(diethylaminomethyl)-4-[4-[3-(diethylaminomethyl)-4-hydroxy-phenyl]hex-3-en-3-yl]phenol; 2-dibutoxyphosphoryloxypentanenitrile; ytterbium(+3) cation trihydroxide; and 4-[(1S)-6,7-diethoxy-1,2,3,4-tetrahydroisoquinolin-1-yl]benzonitrile. Still other JAK2 inhibitors are described in United States Patent No. 8,354,408 to Bourke et al., including 7-iodo-N-(4-morpholinophenyl)thieno[3,2-d]pyrimidin-2-amine; 7-(4-aminophenyl)-N-(4-morpholinophenyl)thieno[3,2-d]pyrimidin-2-amine; N-(4-(2-(4-morpholinophenylamino)thieno[3,2-d]pyrimidin-7-yl)phenyl)acrylamide; 7-(3-aminophenyl)-N-(4-morpholinophenyl)thieno[3,2-d]pyrimidin-2-amine; N-(3-(2-(4-morpholinophenylamino)thieno[3,2-d]pyrimidin-7-yl)phenyl)acrylamide; methyl 2-(4-morpholinophenylamino)thieno[3,2-d]pyrimidine-7-carboxylate; 7-(4-amino-3-methoxyphenyl)-N-(4-morpholinophenyl)thieno[3,2-d]pyrimidin-2-amine; 4-(2-(4-morpholinophenylamino)thieno[3,2-d]pyrimidin-7-yl)benzenesulfonamide; N,N-dimethyl-3-(2-(4-morpholinophenylamino)thieno[3,2-d]pyrimidin-7-yl)benzenesulfonamide; 1-ethyl-3-(2-methoxy-4-(2-(4-morpholinophenylamino)thieno[3,2-d]pyrimidin-7-yl)phenyl)urea; N-(4-(2-(4-morpholinophenylamino)thieno[3,2-d]pyrimidin-7-yl) phenyl)methanesulfonamide; 2-methoxy-4-(2-(4-morpholinophenylamino)thieno[3,2-d]pyrimidin-7-yl)phenol; 2-cyano-N-(3-(2-(4-morpholinophenylamino)thieno[3,2-d]pyrimidin-7-yl)phenyl)acetamide; N-(cyanomethyl)-2-(4-morpholinophenylamino)thieno[3,2-d]pyrimidine-7-carboxamide; N-(3-(2-(4-morpholinophenylamino)thieno[3,2-d]pyrimidin-7-yl)phenyl)methanesulfonamide; 1-ethyl-3-(4-(2-(4-morpholinophenylamino)thieno[3,2-d]pyrimidin-7-yl)-2-(trifluoromethoxy)phenyl)urea; N-(3-nitrophenyl)-7-phenylthieno[3,2-d]pyrimidin-2-amine; 7-iodo-N-(3-nitrophenyl)thieno[3,2-d]pyrimidin-2-amine; N1-(7-(2-ethylphenyl)thieno[3,2-d]pyrimidin-2-yl) benzene-1,3-diamine; tert-butyl-3-(2-(4-morpholinophenylamino)thieno[3,2-d]pyrimidin-7-yl)benzenesulfonamide; N1-(7-iodothieno[3,2-d]pyrimidin-2-yl)benzene-1,3-diamine; 7-(4-amino-3-(trifluoromethoxy)phenyl)-N-(4-morpholinophenyl)thieno[3,2-d]pyrimidin-2-amine; 7-(2-ethylphenyl)-N-(4-morpholinophenyl)thieno[3,2-d]pyrimidin-2-amine; N-(3-(2-(4-morpholinophenylamino)thieno[3,2-d]pyrimidin-7-yl)phenyl)acetamide; N-(cyanomethyl)-N-(3-(2-(4-morpholinophenylamino)thieno[3,2-d]pyrimidin-7-yl)phenyl)methanesulfonamide; N-(cyanomethyl)-N-(4-(2-(4-morpholinophenylamino)thieno[3,2-d]pyrimidin-7-yl)phenyl)methanesulfonamide; N-(3-(5-methyl-2-(4-morpholinophenylamino)-5H-pyrrolo[3,2-d]pyrimidin-7-yl)phenyl)methanesulfonamide; 4-(5-methyl-2-(4-morpholinophenylamino)-5H-pyrrolo[3,2-d]pyrimidin-7-yl)benzenesulfonamide; N-(4-(5-methyl-2-(4-morpholinophenylamino)-5H-pyrrolo[3,2-d]pyrimidin-7-yl)phenyl)methanesulfonamide; 7-iodo-N-(4-morpholinophenyl)-5H-pyrrolo[3,2-d]pyrimidin-2-amine; 7-(2-isopropylphenyl)-N-(4-morpholinophenyl)thieno[3,2-d]pyrimidin-2-amine; 7-bromo-N-(4-morpholinophenyl)thieno[3,2-d]pyrimidin-2-amine; N7-(2-isopropylphenyl)-N2-(4-morpholinophenyl)thieno[3,2-d]pyrimidine-2,7-diamine; N7-(4-isopropylphenyl)-N2-(4-morpholinophenyl)thieno[3,2-d]pyrimidine-2,7-diamine; 7-(5-amino-2-methylphenyl)-N-(4-morpholinophenyl)thieno[3,2-d]pyrimidin-2-amine; N-(cyanomethyl)-4-(2-(4-morpholinophenylamino)thieno[3,2-d]pyrimidin-7-yl)benzamide; 7-iodo-N-(3-morpholinophenyl)thieno[3,2-d]pyrimidin-2-amine; 7-(4-amino-3-nitrophenyl)-N-(4-morpholinophenyl)thieno[3,2-d]pyrimidin-2-amine; 7-(2-methoxypyridin-3-yl)-N-(4-morpholinophenyl)thieno[3,2-d]pyrimidin-2-amine; (3-(7-iodothieno[3,2-d]pyrimidin-2-ylamino)phenyl)methanol; N-tert-butyl-3-(2-(3-morpholinophenylamino)thieno[3,2-d]pyrimidin-7-yl)benzenesulfonamide; N-tert-butyl-3-(2-(3-(hydroxymethyl)phenylamino)thieno[3,2-d]pyrimidin-7-yl)benzenesulfonamide; N-(4-morpholinophenyl)-7-(4-nitrophenylthio)-5H-pyrrolo[3,2-d]pyrimidin-2-amine; N-tert-butyl-3-(2-(3,4,5-trimethoxyphenylamino)thieno[3,2-d]pyrimidin-7-yl)benzenesulfonamide; 7-(4-amino-3-nitrophenyl)-N-(3,4-dimethoxyphenyl)thieno[3,2-d]pyrimidin-2-amine; N-(3,4-dimethoxyphenyl)-7-(2-methoxypyridin-3-yl)thieno[3,2-d]pyrimidin-2-amine; N-tert-butyl-3-(2-(3,4-dimethoxyphenylamino)thieno[3,2-d]pyrimidin-7-yl)benzenesulfonamide; 7-(2-aminopyrimidin-5-yl)-N-(3,4-dimethoxyphenyl)thieno[3,2-d]pyrimidin-2-amine; N-(3,4-dimethoxyphenyl)-7-(2,6-dimethoxypyridin-3-yl)thieno[3,2-d]pyrimidin-2-amine;. N-(3,4-dimethoxyphenyl)-7-(2,4-dimethoxypyrimidin-5-yl)thieno[3,2-d]pyrimidin-2-amine; 7-iodo-N-(4-(morpholinomethyl)phenyl)thieno[3,2-d]pyrimidin-2-amine; N-tert-butyl-3-(2-(4-(morpholinomethyl)phenylamino)thieno[3,2-d]pyrimidin-7-yl)benzenesulfonamide; 2-cyano-N-(4-methyl-3-(2-(4-morpholinophenylamino)thieno[3,2-d]pyrimidin-7-yl)phenyl)acetamide; ethyl 3-(2-(4-morpholinophenylamino)thieno[3,2-d]pyrimidin-7-yl)benzoate; 7-bromo-N-(4-(2-(pyrrolidin-1-yl)ethoxy)phenyl)thieno[3,2-d]pyrimidin-2-amine; N-(3-(2-(4-(2-(pyrrolidin-1-yl)ethoxy)phenylamino)thieno[3,2-d]pyrimidin-7-yl)phenyl)acetamide; N-(cyanomethyl)-3-(2-(4-morpholinophenylamino)thieno[3,2-d]pyrimidin-7-yl)benzamide; N-tert-butyl-3-(2-(4-morpholinophenylamino)thieno[3,2-d]pyrimidin-7-yl)benzamide; N-tert-butyl-3-(2-(4-(1-ethylpiperidin-4-yloxy)phenylamino)thieno[3,2-d]pyrimidin-7-yl)benzenesulfonamide; tert-butyl 4-(2-(4-(morpholinomethyl)phenylamino)thieno[3,2-d]pyrimidin-7-yl)-1H-pyrazole-1-carboxylate; 7-bromo-N-(4-((4-ethylpiperazin-1-yl)methyl)phenyl)thieno[3,2-d]pyrimidin-2-amine; N-tert-butyl-3-(2-(4-((4-ethylpiperazin-1-yl)methyl)phenylamino)thieno[3,2-d]pyrimidin-7-yl)benzenesulfonamide; N-(4-((4-ethylpiperazin-1-yl)methyl)phenyl)-7-(1H-pyrazol-4-yl)thieno[3,2-d]pyrimidin-2-amine; N-(cyanomethyl)-3-(2-(4-(morpholinomethyl)phenylamino)thieno[3,2-d]pyrimidin-7-yl)benzamide; N-tert-butyl-3-(2-(4-(2-(pyrrolidin-1-yl)ethoxy)phenylamino)thieno[3,2-d]pyrimidin-7-yl)benzenesulfonamide; tert-butyl pyrrolidin-1-yl)ethoxy)phenylamino)thieno[3,2-d]pyrimidin-7-yl)benzylcarbamate; 3-(2-(4-(2-(pyrrolidin-1-yl)ethoxy)phenylamino)thieno[3,2-d]pyrimidin-7-yl)benzenesulfonamide; 7-(3-chloro-4-fluorophenyl)-N-(4-(2-(pyrrolidin-1-yl)ethoxy)phenyl)thieno[3,2-d]pyrimidin-2-amine; tert-butyl 4-(2-(4-(1-ethylpiperidin-4-yloxy)phenylamino)thieno[3,2-d]pyrimidin-7-yl)-1H-pyrazole-1-carboxylate; 7-(benzo[d][1,3]dioxol-5-yl)-N-(4-(morpholinomethyl)phenyl)thieno[3,2-d]pyrimidin-2-amine; tert-butyl 5-(2-(4-(morpholinomethyl)phenylamino)thieno[3,2-d]pyrimidin-7-yl)-1H-indole-1-carboxylate; 7-(2-aminopyrimidin-5-yl)-N-(4-(morpholinomethyl)phenyl)thieno[3,2-d]pyrimidin-2-amine; tert-butyl 4-(2-(4-(morpholinomethyl)phenylamino)thieno[3,2-d]pyrimidin-7-yl)-5,6-dihydropyridine-1(2H)-carboxylate; tert-butyl 4-(2-(4-(morpholinomethyl)phenylamino)thieno[3,2-d]pyrimidin-7-yl)benzylcarbamate; N-(3-(2-(4-(morpholinomethyl)phenylamino)thieno[3,2-d]myrimidin-7-yl)phenyl)acetamide; N-(4-(2-(4-(morpholinomethyl)phenylamino)thieno[3,2-d]pyrimidin-7-yl)phenyl)acetamide; N-(3-(2-(4-(morpholinomethyl)phenylamino)thieno[3,2-d]pyrimidin-7-yl)phenyl)methanesulfonamide; 7-(4-(4-methylpiperazin-1-yl)phenyl)-N-(4-(morpholinomethyl)phenyl)thieno[3,2-d]pyrimidin-2-amine; N-(2-methoxy-4-(2-(4-(morpholinomethyl)phenylamino)thieno[3,2-d]pyrimidin-7-yl)phenyl)acetamide; 7-bromo-N-(3,4,5-trimethoxyphenyl)thieno[3,2-d]pyrimidin-2-amine; (3-(2-(3,4,5-trimethoxyphenylamino)thieno[3,2-d]pyrimidin-7-yl)phenyl)methanol; (4-(2-(3,4,5-trimethoxyphenylamino)thieno[3,2-d]pyrimidin-7-yl)phenyl)methanol; (3-(2-(4-morpholinophenylamino)thieno[3,2-d]pyrimidin-7-yl)phenyl)methanol; (4-(2-(4-morpholinophenylamino)thieno[3,2-d]pyrimidin-7-yl)phenyl)methanol; N-(pyrrolidin-1-yl)ethoxy)phenylamino)thieno[3,2-d]pyrimidin-7-yl)benzyl)methanesulfonamide; tert-butyl 3-(2-(4-(morpholinomethyl)phenylamino)thieno[3,2-d]pyrimidin-7-yl)benzylcarbamate; N-(4-(morpholinomethyl)phenyl)-7-(3-(piperazin-1-yl)phenyl)thieno[3,2-d]pyrimidin-2-amine; 7-(6-(2-morpholinoethylamino)pyridin-3-yl)-N-(3,4,5-trimethoxyphenyl)thieno[3,2-d]pyrimidin-2-amine; 7-(2-ethylphenyl)-N-(4-(pyrrolidin-1-yl)ethoxy)phenyl)thieno[3,2-d]pyrimidin-2-amine; 7-(2-isopropylphenyl)-N-(4-(pyrrolidin-1-yl)ethoxy)phenyl(thieno[3,2-d]pyrimidin-2-amine; 7-(4-(aminomethyl)phenyl)-N-(4-(morpholinomethyl)phenyl)thieno[3,2-d]pyrimidin-2-amine; N-(4-(1-ethylpiperidin-4-yloxy)phenyl)-7-(1H-pyrazol-4-yl)thieno[3,2-d]pyrimidin-2-amine; N-(2,4-dimethoxyphenyl)-7-phenylthieno[3,2-d]pyrimidin-2-amine; 7-bromo-N-(3,4-dimethoxyphenyl)thieno[3,2-d]pyrimidin-2-amine; or N-(3,4-dimethoxyphenyl)-7-phenylthieno[3,2-d]pyrimidin-2-amine. Still other JAK2 kinase inhibitors are described in United States Patent No. 8,309,718 to Li et al., including 4-pyrazolyl-N-arylpyrimidin-2-amines and 4-pyrazolyl-N-heteroarylpyrimidin-2-amines. Still other JAK2 kinase inhibitors are described in United States Patent No. 8,242,274 to Menet et al., including [1,2,4]triazolo[1,5-a]pyridines. Still other JAK2 kinase inhibitors are described in United States Patent No. 8,158,616 to Rodgers et al., including azetidines and cyclobutanes; a preferred compound is 1-(ethylsulfonyl)-3-[4-(7H-pyrrolo[2,3-d]pyrimidin-4-yl)-1H-pyrazol-1-yl]azetidin-3-yl}acetonitrile. Still other JAK2 kinase inhibitors are described in United States Patent No. 8,138,199 to Noronha et al., including biaryl *meta*-pyrimidine compounds. Still other JAK2 kinase inhibitors are described in United States Patent No. 8,053,433 to Rodgers et al., including pyrrolo[2,3-b]pyridin-4-yl-amines and pyrrolo[2,3-b]pyrimidin-5-yl-amines. Still other JAK2 kinase inhibitors are described in United States Patent No. 7,897,600 to Burkholder et al., including 3-(4-chloro-2-fluorobenzyl)-2-methyl-N-(5-methyl-1H-pyrazol-3-yl)-8-(morpholinomethyl)imidazo[1,2-b]pyridazin-6-amine. Still other JAK2 kinase inhibitors are described in United States Patent No. 7,598,257 to Rodgers et al., including heteroaryl substituted pyrrolo[2,3-b]pyridines and heteroaryl substituted pyrrolo[2,3-b]pyrimidines. Still other JAK2 kinase inhibitors are described in United States Patent No. 7,355,677 to Rodgers et al., including pyrrolo[2,3-b]pyridine-4-yl amines and pyrrolo[2,3-b]pyrimidin-4-yl amines. Oher JAK2 kinase inhibitors are known in the art.

The method described herein can further comprise the step of administering a therapeutically effective quantity of a BH3 mimetic to the subject. Alternatively, the method described herein can further comprise the step of administering therapeutically effective quantity of a tyrosine kinase inhibitor to the subject.

A medicament for use in a method for the treatment of a malignancy in a subject suffering from a malignancy who has a germline deletion polymorphism conferring resistance to thymidine kinase inhibitors (TKIs) the medicament comprising: (1) a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy; and (2) a therapeutically effective quantity of a STAT5 inhibitor to the subject to treat the malignancy is described herein.

STAT5 inhibitors described herein include, but are not limited to, N'-((4-oxo-4H-chromen-3-yl)methylene)nicotinohydrazide and pimozide. Other STAT 5 inhibitors are disclosed in United States Patent Application Publication No. 2011/0144043 by Frank, including pyrimethamine, guanabenz acetate, alprenolol hydrochloride, nifuroxazide, solanine alpha, fluoxetine hydrochloride, ifosfamide, pyrvinium pamoate, moricizine hydrochloride, 3,3'-oxybis[tetrahydrothiophene, 1,1,1',1'-tetraoxide], 2-(1,8-naphthyridin-2-yl)phenol, and 3-(2-hydroxyphenyl)-3-phenyl-N,N-dipropylpropanamide.

The method described herein can further comprise the step of administering a therapeutically effective quantity of a BH3 mimetic to the subject. Alternatively, the method can further comprise the step of administering therapeutically effective quantity of a tyrosine kinase inhibitor to the subject.

A medicament for use in a method for the treatment of a malignancy in a subject suffering from a malignancy who has a germline deletion polymorphism conferring resistance to thymidine kinase inhibitors (TKIs) the medicament comprising: (1) a therapeutically effective quantity of a therapeutic agent selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy; and (2) a therapeutically effective quantity of a Src inhibitor to the subject to treat the malignancy is described herein.

Src inhibitors described herein include dasatinib, saracatinib, bosutinib, N-benzyl-2-(5-(4-(2-morpholinoethoxy)phenyl)pyridin-2-yl)acetamide (KX2-391), CGP76030, and 4-methyl-3-(1-methyl-6-(pyridin-3-yl)-1H-pyrazolo[3,4-d]pyrimidin-4-ylamino)-N-(3-(trifluoromethyl)phenyl)benzamide (NVP-BHG712). Src kinase inhibitors are described in M. Missbach et al., "Substituted 5,7-diphenyl-pyrrolo[2,3-d]pyrimidines: Potent Inhibitors of the Tyrosine Kinase c-Src," Bioorg. Med. Chem. Lett. 10: 945-949 (2000), Other Src inhibitors are known in the art and are described in United States Patent No. 8,389,525 by Desai et al., including α-[[6-(4-bromophenyl)-3-cyano-4-(trifluoromethyl)-2-pyridinyl]thio]-benzeneacetic acid, 1,4-dihydro-2-[[[4-(methoxycarbonyl)phenyl]methyl]thio]-5-methyl-4-oxo-thieno-[2,3-d]pyrimidine-6-carboxylic acid, 6-hydroxy-7-oxo-7H-benzo[e]perimidine-4-sulfonic acid, 7-ethoxy-11H-indeno[1,2-b]quinoxalin-11-one, 5-bromo-1,3-dihydro-3-hydroxy-3-[2-oxo-2-(5,6,7,8-tetrahydro-2-naphthalenyl)ethyl]-2H-indol-2-one, 1-(4-fluorophenyl)-2-(9H-thioxanthen-9-yl)-1,3-butanedione, 2-[[(2-chloro-6-fluorophenyl)methyl]thio]-3-(3-pyridinyl)-4(3H)-quinazolinone, 2,7-dinitro-oxime-9H-fluoren-9-one, and 3-(9H-fluoren-9-ylmethyl) ester-3,4-thiazolidinedicarboxylic acid. United States Patent No. 8,283,441 to Xie et al., describes peptides comprising a helix or helix-like structure that binds to the Src kinase domain. United States Patent No. 8,236,799 to Hangauer, Jr., describes biaryl compounds that are inhibitors of Src kinase. United States Patent No. 8,088,768 to Hangauer, Jr., et al., describes naphthalene-based Src inhibitor scaffolds, isoquinoline-based Src inhibitor scaffolds, and indole-based Src inhibitor scaffolds. United States Patent No. 8,080,252 to Byzova et al., describes 3-(4,5,6,7-tetrahydroinol-2-ylmethylidene)-2-indolinone derivatives as Src inhibitors, including, 2-oxo-3(4,5,6,7-tetrahydro-1-H-indol-2-ylmethylene)-2,3-dihydro-1-H-indole-5-sulfonic acid dimethylamide and 2-oxo-3(4,5,6,7-tetrahydro-1-H-indol-2-ylmethylene)-2,3-dihydro-1H-indole-5-sulfonic acid amide. United States Patent No. 8,058,283 to Honold et al., describes 7H-pyrido[3,4-d]pyrimidin-8-ones as Src inhibitors. United States Patent No. 7,982,037 to Bebbington et al., describes pyrazole compounds as Src kinase inhibitors. United States Patent No. 7,951,820 to Bebbington et al., describes triazole compounds as Src kinase inhibitors. United States Patent No. 7,919,625 to Boschelli et al., describes 4-anilino-3-quinolinecarbonitriles as Src inhibitors, including 4-[(2,4-dichloro-5-methoxyphenyl)amino]-6-methoxy-7-[3-(4-methyl-1-piperazinyl)propoxy]-3-quinolinecarbonitrile. United States Patent No. 7,842,712 to Aronov et al., describes indazolinones as Src inhibitors. United States Patent No. 7,842,701 to Fukumoto et al., describes pyrazoloquinolone derivatives as Src inhibitors, including 3-amino-2-(2-chloro-5-hydroxyphenyl)-7-(3-morpholin-4-ylpropoxy)-2,5-dihydro-4H-pyrazolo[4,3-c]quinolin-4-one, 3-amino-2-(2-chloro-5-hydroxyphenyl)-7-(2-morpholin-4-ylethoxy)-2,5-dihydro-4H-pyrazolo [4,3-c]quinolin-4-one, 3-amino-2-(5-hydroxy-2-m ethylphenyl)-7-(3-morpholin-4-2,5-dihydro-4H-pyrazolo[4,3-c]quinolin-4-one, 3-amino-2-(5-hydroxy-2-ylpropoxy) methylphenyl)-7-(2-morpholin-4-ylethoxy)-2,5-dihydro-4H-pyrazolo[4,3-c]quinolin-4-one. United States Patent No. 7,786,113 to Honold et al., describes heterocyclic carbamate derivatives as Src inhibitors, including (2-phenyl-1H-pyrrolo[2,3-b]pyridin-5-yl)-carbamic acid isopropyl ester; {2-[3-(2-methoxy-ethoxy)-phenyl]-1H-pyrrolo[2,3-b]pyridin-5-yl}-carbamic acid isopropyl ester; (2-phenyl-1H-pyrrolo[2,3-b]pyridin-5-yl)-carbamic acid 2,2-dimethyl-propyl ester; {2-[4-(2-methoxy-ethoxy)-phenyl]-1H-pyrrolo[2,3-b]pyridin-5-yl}-carbamic acid ethyl ester; {2-[4-(2-methoxy-ethoxy)-phenyl]-1H-pyrrolo[2,3-b]pyridin-5-yl}-carbamic acid allyl ester; {2-[3-(2-methoxy-ethoxy)-phenyl]-1H-pyrrolo[2,3-b]pyridin-5-yl}-carbamic acid ethyl ester; {2-[3-(2-methoxy-ethoxy)-phenyl]-1H-pyrrolo[2,3-b]pyridin-5-yl}-carbamic acid allyl ester; (2-phenyl-1H-pyrrolo[2,3-b]pyridin-5-yl)-carbamic acid benzyl ester; {2-[3-(2-methoxy-ethoxy)-phenyl]-1H-pyrrolo[2,3-b]pyridin-5-yl}-carbamic acid isobutyl ester; {2-[3-(2-methoxy-ethoxy)-phenyl]-1H-pyrrolo[2,3-b]pyridin-5-yl}-carbamic acid 2-chloro-benzyl ester; (2-phenyl-1H-pyrrolo[2,3-b]pyridin-5-yl)-carbamic acid ethyl ester; (2-phenyl-1H-pyrrolo[2,3-b]pyridin-5-yl)-carbamic acid 2-chloro-benzyl ester; (2-phenyl-1H-pyrrolo[2,3-b]pyridin-5-yl)-carbamic acid allyl ester; (2-phenyl-1H-pyrrolo[2,3-b]pyridin-5-yl)-carbamic acid isobutyl ester; {2-[3-(2-methoxy-ethoxy)-phenyl]-1H-pyrrolo[2,3-b]pyridin-5-yl}-carbamic acid 2,2-dimethyl-propyl ester; {2-[3-(2-methoxy-ethoxy)-phenyl]-1H-pyrrolo[2,3-b]pyridin-5-yl}-carbamic acid benzyl ester; {2-[4-(2-methoxy-ethoxy)-phenyl]-1H-pyrrolo[2,3-b]pyridin-5-yl}-carbamic acid isopropyl ester; {2-[4-(2-methoxy-ethoxy)-phenyl]-1H-pyrrolo[2,3-b]pyridin-5-yl}-carbamic acid benzyl ester; {2-[4-(2-methoxy-ethoxy)-phenyl]-1H-pyrrolo[2,3-b]pyridin-5-yl}-carbamic acid isobutyl ester; [2-(3-acetylamino-phenyl)-1H-pyrrolo[2,3-b]pyridin-5-yl]-carbamic acid isopropyl ester; [2-(4-acetylamino-phenyl)-1H-pyrrolo[2,3-b]pyridin-5-yl]-carbamic acid allyl ester; [2-(3-acetylamino-phenyl)-1H-pyrrolo[2,3-b]pyridin-5-yl]-carbamic acid 2-chloro-benzyl ester; [2-(3-acetylamino-phenyl)-1H-pyrrolo[2,3-b]pyridin-5-yl]-carbamic acid benzyl ester; [2-(3-acetylamino-phenyl)-1H-pyrrolo[2,3-b]pyridin-5-yl]-carbamic acid 2,2-dimethyl-propyl ester; [2-(4-acetylamino-phenyl)-1H-pyrrolo[2,3-b]pyridin-5-yl]-carbamic acid 2,2-dimethyl-propyl ester; [2-(3-acetylamino-phenyl)-1H-pyrrolo[2,3-b]pyridin-5-yl]-carbamic acid ethyl ester; [2-(3-acetylamino-phenyl)-1H-pyrrolo[2,3-b]pyridin-5-yl]-carbamic acid allyl ester; [2-(3-acetylamino-phenyl)-1H-pyrrolo[2,3-b]pyridin-5-yl]-carbamic acid isobutyl ester; (2-phenyl-3H-imidazo[4,5-b]pyridin-6-yl)-carbamic acid 1-methyl-allyl ester; (2-phenyl-1H-imidazo[4,5-b]pyridin-6-yl)-carbamic acid isopropyl ester; (2-phenyl-3H-imidazo[4,5-b]pyridin-6-yl)-carbamic acid cyclohexyl ester; (2-phenyl-3H-imidazo[4,5-b]pyridin-6-yl)-carbamic acid isobutyl ester; (2-phenyl-3H-imidazo[4,5-b]pyridin-6-yl)-carbamic acid allyl ester; (2-phenyl-3H-imidazo[4,5-b]pyridin-6-yl)-carbamic acid tert-butyl ester; (2-phenyl-3H-imidazo[4,5-b]pyridin-6-yl)-carbamic acid cyclopentyl ester; (2-phenyl-3H-imidazo[4,5-b]pyridin-6-yl)-carbamic acid ethyl ester; (2-phenyl-3H-imidazo[4,5-b]pyridin-6-yl)-carbamic acid sec-butyl ester; (2-phenyl-3H-imidazo[4,5-b]pyridin-6-yl)-carbamic acid 1-ethyl-propyl ester; (2-phenyl-3H-imidazo[4,5-b]pyridin-6-yl)-carbamic acid 2,2,2-trifluoro-1-methyl-ethyl ester; (2-phenyl-3H-imidazo[4,5-b]pyridin-6-yl)-carbamic acid 2,2-dimethyl-propyl ester; (2-phenyl-3H-imidazo[4,5-b]pyridin-6-yl)-carbamic acid 1-phenyl-ethyl ester; (2-phenyl-1H-imidazo[4,5-b]pyridin-6-yl)-carbamic acid propyl ester; (2-phenyl-3H-imidazo[4,5-b]pyridin-6-yl)-carbamic acid 1-methyl-prop-2-ynyl ester; (2-phenyl-3H-imidazo[4,5-b]pyridin-6-yl)-carbamic acid 1-methyl-but-2-ynyl ester; (2-phenyl-3H-imidazo[4,5-b]pyridin-6-yl)-carbamic acid cyclobutyl ester; (2-phenyl-3H-imidazo[4,5-b]pyridin-6-yl)-carbamic acid 1,3-dimethyl-butyl ester; (2-phenyl-3H-imidazo[4,5-b]pyridin-6-yl)-carbamic acid 1,2-dimethyl-propyl ester; {2-[3-(3-methoxy-propionylamino)-phenyl]-3H-imidazo[4,5-b]pyridin-6-yl}-carbamic acid isopropyl ester; (2-phenyl-3H-imidazo[4,5-b]pyridin-6-yl)-carbamic acid (E)-1-methyl-but-2-enyl ester; (2-phenyl-3H-imidazo[4,5-b]pyridin-6-yl)-carbamic acid 1,2-dimethyl-allyl ester; {2-[4-(2-diethylamino-ethoxy)-phenyl]-3H-imidazo[4,5-b]pyridin-6-yl}-carbamic acid isopropyl ester; 2-[3-(2-methoxy-ethoxy)-phenyl]-3H-imidazo[4,5-b]pyridin-6-yl}-carbamic acid isopropyl ester; {2-[4-(2-methoxy-ethoxy)-phenyl]-3H-imidazo[4,5-b]pyridin-6-yl}-carbamic acid isopropyl ester; [2-(3-nitro-phenyl)-3H-imidazo[4,5-b]pyridin-6-yl]-carbamic acid isopropyl ester; [2-(4-morpholin-4-yl-phenyl)-3H-imidazo[4,5-b]pyridin-6-yl]-amic acid isopropyl ester; {2-[4-(4-methyl-piperazin-1-yl)-phenyl]-3H-imidazo[4,5-b]pyridin-6-yl}-carbamic acid isopropyl ester; {2-[3-(2-hydroxy-ethyl)-phenyl]-3H-imidazo[4,5-b]pyridin-6-yl}-carbamic acid isopropyl ester; [2-(4-nitro-phenyl)-3H-imidazo[4,5-b]pyridin-6-yl]-carbamic acid isopropyl ester; [2-(4-sulfamoyl-phenyl)-3H-imidazo[4,5-b]pyridin-6-yl]-carbamic acid isopropyl ester; [2-(4-methylsulfanyl-phenyl)-3H-imidazo[4,5-b]pyridin-6-yl]-carbamic acid isopropyl ester; [2-(3-amino-phenyl)-3H-imidazo[4,5-b]pyridin-6-yl]-carbamic acid isopropyl ester; [2-(4-amino-phenyl)-3H-imidazo[4,5-b]pyridin-6-yl]-carbamic acid isopropyl ester; [2-(3-acetylamino-phenyl)-3H-imidazo[4,5-b]pyridin-6-yl]-carbamic acid isopropyl ester; [2-(3-methanesulfonylamino-phenyl)-3H-imidazo[4,5-b]pyridin-6-yl]-carbamic acid isopropyl ester; [2-(3-methylsulfinyl-phenyl)-3H-imidazo[4,5-b]pyridin-6-yl]-carbamic acid isopropyl ester; [2-(3-methylsulfonyl-phenyl)-3H-imidazo[4,5-b]pyridin-6-yl]-carbamic acid isopropyl ester; [2-(4-methanesulfonyl-phenyl)-3H-imidazo[4,5-b]pyridin-6-yl]-carbamic acid isopropyl ester; [2-(4-methanesulfinyl-phenyl)-3H-imidazo[4,5-b]pyridin-6-yl]-carbamic acid isopropyl ester; [2-(3,4-difluoro-phenyl)-3H-imidazo[4,5-b]pyridin-6-yl]-carbamic acid isopropyl ester; (2-{4-[bis-(2-methoxy-ethyl)-amino]-3-fluoro-phenyl}-3H-imidazo[4,5-b]pyridin-6-yl)-carbamic acid isopropyl ester; 3-(6-isopropoxycarbonylamino-3H-imidazo[4,5-b]pyridin-2-yl)-benzoic acid; {2-[3-(2-methoxy-I-methoxymethyl-ethylcarbamoyl)-phenyl]-3H-imidazo[4,5-b]pyridin-6-yl}-carbamic acid isopropyl ester; {2-[3-(3-methoxy-propylcarbamoyl)-phenyl]-3H-imidazo[4,5-b]pyridin-6-yl}-carbamic acid isopropyl ester; (2-thiophen-2-yl-3H-imidazo[4,5-b]pyridin-6-yl)-carbamic acid isopropyl ester; (2-thiophen-3-yl-3H-imidazo[4,5-b]pyridin-6-yl)-carbamic acid isopropyl ester; [2-(2-methyl-pyridin-4-yl)-3H-imidazo[4,5-b]pyridin-6-yl]-carbamic acid isopropyl ester; [2-(6-methyl-pyridin-3-yl)-3H-imidazo[4,5-b]pyridin-6-yl]-carbamic acid isopropyl ester; [2-(1H-benzoimidazol-5-yl)-3H-imidazo[4,5-b]pyridin-6-yl]-carbamic acid isopropyl ester; [2-(2-chloro-pyridin-4-yl)-3H-imidazo[4,5-b]pyridin-6-yl]-carbamic acid isopropyl ester; and {2-[2-(3-Methoxy-propylamino)-pyridin-4-yl]-3H-imidazo[4,5-b]pyridin-6-yl}-carbamic acid isopropyl ester. United States Patent No. 7,776,878 to Boyd et al., describes heterocyclic benzylamino derivatives as Src inhibitors, including (1-phenyl-ethyl)-(2-phenyl-3H-imidazo[4,5-b]pyridin-6-yl)-amine; benzyl-{2-[3-(2-methoxy-ethoxy)-phenyl]-1H-pyrrolo[2,3-b]pyridin-5-yl}-amine; {2-[3-(2-methoxy-ethoxy)-phenyl]-1H-pyrrolo[2,3-b]pyridin-5-yl}-(2-methylbenzyl)-amine; benzyl-(2-phenyl-1H-pyrrolo[2,3-b]pyridin-5-yl)-amine; (2-methyl-benzyl)-(2-phenyl-1H-pyrrolo[2,3-b]pyridin-5-yl)-amine; N-[3-(5-benzylamino-1H-pyrrolo[2,3-b]pyridin-2-yl)-phenyl]-acetamide; N-{3-[5-(2-methyl-benzylamino)-1H-pyrrolo[2,3-b]pyridin-2-yl]-phenyl}-acetamide; N-[4-(5-benzylamino-1H-pyrrolo[2,3-b]pyridin-2-yl)-phenyl]-acetamide; and N-{4-[5-(2-methyl-benzylamino)-1H-pyrrolo[2,3-b]pyridin-2-yl]-phenyl}-acetamide. United States Patent No. 7,691,853 to Bebbington et al., describes pyrazole compounds as Src kinase inhibitors. United States Patent No. 7,655,601 to Engh et al., discloses amide derivatives of 3-phenyl dihydropyrimido[4,5-d]pyrimidinones as Src kinase inhibitors. United States Patent No. 7,625,913 to Bebbington et al., describes pyrazole compounds as Src kinase inhibitors, including (5-methyl-2H-pyrazol-3-yl)-(6-phenyl-2-phenylamino-pyrimidin-4-yl)-amine; (5-cyclopropyl-2H-pyrazol-3-yl)-(6-phenyl-2-phenylamino-pyrimidin-4-yl)-amine; (5-cyclopropyl-2H-pyrazol-3-yl)-[2-(3-methylphenylamino)-6-phenyl-pyrimidin-4-yl]-amine; [2-(4-cyanomethylphenylamino)-6-phenyl-pyrimidin-4-yl]-(5-cyclopropyl-2H-pyrazol-3-yl)-amine; (5-cyclopropyl-2H-pyrazol-3-yl)-[6-phenyl-2-(pyridin-3-ylmethylamino)-pyrimidin-4-yl]-amine; [2-(3-chlorophenyl)amino-6-(3-nitrophenyl)-pyrimidin-4-yl]-(5-methyl-2H-pyrazol-3-yl)-amine; [2-(3-chlorophenyl)amino-6-(3,4,5-trimethoxyphenyl)-pyrimidin-4-yl]-(5-methyl-2H-pyrazol-3-yl)-amine; (5-methyl-2H-pyrazol-3-yl)-[2-(4-sulfamoylphenylamino)-6-(3,4,5-trimethoxyphenyl)-pyrimidin-4-yl]-amine; [2-(benzimidazol-2-ylamino-)-6-ethyl-pyrimidin-4-yl]-(5-methyl-2H-pyrazol-3-yl)-amine; [2-(4-chlorophenyl)amino-6-ethyl-pyrimidin-4-yl]-(5-methyl-2H-pyrazol-3-yl)-amine; (5-tert-butyl-2H-pyrazol-3-yl)-[2-(3-chlorophenyl)amino-6-(3-nitrophenyl)-pyrimidin-4-yl]-amine; [2-(3-chlorophenyl)amino-6-(3-nitrophenyl)-pyrimidin-4-yl]-(5-phenyl-2H-pyrazol-3-yl)-amine; [5-(furan-2-yl)-2H-pyrazol-3-yl]-(6-phenyl-2-phenylamino-pyrimidin-4-yl)-amine; [2-(4-chlorophenyl)amino-5,6-dimethyl-pyrimidin-4-yl]-(5-methyl-2H-pyrazol-3-yl)-amine; (5,6-dimethyl-2-phenylamino-pyrimidin-4-yl)-(5-methyl-2H-pyrazol-3-yl)-amine; [2-(4-chlorophenyl)amino-6-methoxymethyl-pyrimidin-4-yl]-(5-methyl-2H-pyrazol-3-yl)-amine; [2-(benzimidazol-2-ylamino)-6-methoxymethyl-pyrimidin-4-yl]-(5-methyl-2H-pyrazol-3-yl)-amine; and (6-methoxymethyl-2-phenylamino-pyrimidin-4-yl)-(5-methyl-2H-pyrazol-3-yl)-amine. United States Patent No. 7,622,472 to Lee discloses Src inhibitors, including N-(2-chloro-6-methylphenyl)-2-[[6-[4-(2-hydroxyethyl)-1-piperazinyl]-2-methyl-4-pyrimidinyl]amino]-5-thiazolecarboxamide. United States Patent No. 7,618,964 to Honold et al., describes benzamide derivatives as Src kinase inhibitors, including 2-chloro-N-(2-phenyl-1H-pyrrolo[2,3-b]pyridin-5-yl)-benzamide; 2-chloro-N-{2-[3-(2-methoxy-ethoxy)-phenyl]-1H-pyrrolo[2,3-b]pyridin-5-yl}-benzamide; 2-methoxy-N-(2-phenyl-1H-pyrrolo[2,3-b]pyridin-5-yl)-benzamide; 2,4-dichloro-N-(2-phenyl-1H-pyrrolo[2,3-b]pyridin-5-yl)-benzamide; 2-chloro-6-methyl-N-(2-phenyl-1H-pyrrolo[2,3-b]pyridin-5-yl)-benzamide; N-[2-(3-acetylamino-phenyl)-1H-pyrrolo[2,3-b]pyridin-5-yl]-4-methoxy-benzamide; 2-methyl-N-(2-phenyl-1 H-pyrrolo[2,3-b]pyridin-5-yl)-benzamide; 2-chloro-5-methoxy-N-{2-[3-(2-methoxy-ethoxy)-phenyl]-1H-pyrrolo[2,3-b]pyridin-5-yl}-benzamide; 2,4-dichloro-N-{2-[3-(2-methoxy-ethoxy)-phenyl]-1H-pyrrolo[2,3-b]pyridin-5-yl}-benzamide; 4-methoxy-N-{2-[3-(2-methoxy-ethoxy)-phenyl]-1H-pyrrolo[2,3-b]pyridin-5-yl}-benzamide; 3,5-dimethoxy-N-{2-[3-(2-methoxy-ethoxy)-phenyl]-1H-pyrrolo[2,3-b]pyridin-5-yl-benzamide; 3,5-dimethoxy-N-(2-phenyl-1H-pyrrolo[2,3-b]pyridin-5-yl)-benzamide; N-{2-[3-(2-methoxy-ethoxy)-phenyl]-1H-pyrrolo[2,3-b]pyridin-5-yl}-2-methyl-benzamide; 2-methoxy-N-{2-[4-(2-methoxy-ethoxy)-phenyl]-1H-pyrrolo[2,3-b]pyridin-5-yl}-benzamide; N-[2-(3-acetylamino-phenyl)-1H-pyrrolo[2,3-b]pyridin-5-yl]-2-chloro-benzamide; N-[2-(3-acetylamino-phenyl)-1H-pyrrolo[2,3-b]pyridin-5-yl]-2,4-dichloro-benzamide; N-[2-(3-acetylamino-phenyl)-1H-pyrrolo[2,3-b]pyridin-5-yl]-2-methoxybenzamide; N-[2-(3-acetylamino-phenyl)-1H-pyrrolo[2,3-b]pyridin-5-yl]-2-chloro-6-methyl-benzamide; N-[2-(3-acetylamino-phenyl)-1H-pyrrolo[2,3-b]pyridin-5-yl]-2-chloro-5-methoxy-benzamide; 2-chloro-N-(2-phenyl-3H-imidazo[4,5-b]pyridin-6-yl)-benzamide; 2-chloro-6-methyl-N-(2-phenyl-3H-imidazo[4,5-b]pyridin-6-yl)-benzamide; 2-bromo-N-(2-phenyl-3H-imidazo[4,5-b]pyridin-6-yl)-benzamide; 2-methyl-5-nitro-N-(2-phenyl-3H-imidazo[4,5-b]pyridin-6-yl)-benzamide; 2-chloro-5-nitro-N-(2-phenyl-3H-imidazo[4,5-b]pyridin-6-yl)-benzamide; N-(2-phenyl-3H-imidazo[4,5-b]pyridin-6-yl)-benzamide; 2-chloro-N-{2-[3-(3-methoxy-propionylamino)-phenyl]-3H-imidazo[4,5-b]pyridin-6-yl}-benzamide; 5-amino-2-methyl-N-(2-phenyl-3H-imidazo[4,5-b]pyridin-6-yl)-benzamide; 5-amino-2-chloro-N-(2-phenyl-3H-imidazol[4,5-b]pyridin-6-yl)-benzamide; 2-chloro-N-{2-[4-(2-diethylamino-ethoxy)-phenyl]-3H-imidazo[4,5-b]pyridin-6-yl}-benzamide; 2-chloro-N-{2-[4-(2-methoxy-ethoxy)-phenyl]-3H-imidazo[4,5-b]pyridin-6-yl}-benzamide; 2-chloro-N-{2-[3-(2-methoxy-ethoxy)-phenyl]-3H-imidazo[4,5-b]pyridin-6-yl}-benzamide; 2-chloro-N-[2-(3-nitro-phenyl)-3H-imidazo[4,5-b]pyridin-6-yl]-benzamide; 2-chloro-N-[2-(4-morpholin-4-yl-phenyl)-3H-imidazo[4,5-b]pyridin-6-yl]-benzamide; 2-chloro-N-{2-[4-(4-methyl-piperazin-1-yl)-phenyl]-3H-imidazo[4,5-b]pyridin-6-yl}-benzamide; 2-chloro-N-{2-[3-(2-hydroxy-ethyl)-phenyl]-3H-imidazo[4,5-b]pyridin-6-yl}-benzamide; 3-[6-(2-chloro-benzoylamino)-3H-imidazo[4,5-b]pyridin-2-yl]-benzoic acid; 3-(6-(2-chlorobenzoylamino)-3H-imidazo[4,5-b]pyridin-2-yl)-N-(3-methoxy-propyl)-benzamide; 3-(6-(2-chlorobenzoylamino)-3H-imidazo[4,5-b]pyridin-2-yl)-N-isopropyl-benzamide; 2-chloro-N-(2-{3-[2-methoxy-1-methoxymethyl-ethylcarbamoyl]-phenyl}-3H-imidazo[4,5-b]pyridin-6-yl)-benzamide; 2-chloro-N-[2-(3-methylsulfanyl-phenyl)-3H-imidazo[4,5-b]pyridin-6-yl]-benzamide; 2-chloro-N-[2-(4-sulfamoyl-phenyl)-3H-imidazo[4,5-b]pyridin-6-yl]-benzamide; 2-chloro-N-[2-(4-nitrophenyl)-3H-imidazo[4,5-b]pyridin-6-yl]-benzamide; 2-chloro-N-[2-(4-methylsulfanyl-phenyl)-3H-imidazo[4,5-b]pyridin-6-yl]-benzamide; 2-chloro-N-[2-(3-methanesulfinyl-phenyl)-3H-imidazo[4,5-b]pyridin- -6-yl]-benzamide; 2-chloro-N-[2-(4-methanesulfonylphenyl)-3H-imidazo[4,5-b]pyridin-6-yl]-benzamide; N-[2-(3-amino-phenyl)-3H-imidazo[4,5-b]pyridin-6-yl]-2-chloro-benzamide; N-[2-(3-acetylamino-phenyl)-3H-imidazo[4,5-b]pyridin-6-yl]-2-chloro-benzamide; N-(2-{4-[bis-(2-methoxy-ethyl)-amino]-3-fluoro-phenyl}-3H-imidazo[4,5-b]pyridin-6-yl)-2-chloro-benzamide; 2-chloro-N-(2-thiophen-2-yl-3H-imidazo[4,5-b]pyridin-6-yl)-benzamide; 2-chloro-N-(2-thiophen-3-yl-3H-imidazo[4,5-b]pyridin-6-yl)-benzamide; 2-chloro-N-[2-(2-methyl-pyridin-4-yl)-3H-imidazo[4,5-b]pyridin-6-yl]-benzamide; 2-chloro-N-[2-(6-methyl-pyridin-3-yl)-3H-imidazo[4,5-b]pyridin-6-yl]-benzamide; N-[2-(1H-benzoimidazol-5-yl)-3H-imidazo[4,5-b]pyridin-6-yl]-2-chloro-benzamide; 2-chloro-N-[2-(6-morpholin-4-yl-pyridin-3-yl)-3H-imidazo[4,5-b]pyridin-6-yl]-benzamide; and 2-chloro-N-{2-[2-(3-methoxy-propylamino)-pyridin-4-yl]-3H-imidazo[4,5-b]pyridin-6-yl}-benzamide. United States Patent No. 7,583,767 to Xiao et al., describes substituted pyrazole compounds as Src inhibitors. United States Patent No. 7,550,589 to Honold et al., describes 6-(2-alkyl-phenyl)-pyrido[2,3-d]pyrimidines as Src inhibitors, including 2-(4-morpholin-4-yl-phenylamino)-6-(2-trifluoromethyl-phenyl)-pyrido[2,3-d]pyrimidine-7-carboxylic acid (pyrrolidin-2-ylmethyl)-amide; 2-(3-acetylamino-phenylamino)-6-(2-trifluoromethyl-phenyl)-pyrido[2,3-d]p- yrimidine-7-carboxylic acid (pyrrolidin-2-ylmethyl)-amide; 2-(3-methanesulfonylamino-phenylamino)-6-(2-trifluoromethyl-phenyl)-pyrido[2,3-d]pyrimidine-7-carboxylic acid (pyrrolidin-2-ylmethyl)-amide; 2-(4,4-dioxo-3,4-dihydro-2H-4lambda*6*-benzo[1,4]oxathiin-6-ylamino)-6-(2-trifluoromethyl-phenyl)-pyrido[2,3-d]pyrimidine-7-carboxylic acid (pyrrolidin-2-ylmethyl)-amide; 2-(4-morpholin-4-yl-phenylamino)-6-(2-trifluoromethyl-phenyl)-pyrido[2,3-d]pyrimidine-7-carboxylic acid (2-methanesulfonylamino-ethyl)-amide; 2-(3-acetylamino-phenylamino)-6-(2-trifluoromethyl-phenyl)-pyrido[2,3-d]pyrimidine-7-carboxylic acid (2-methanesulfonylamino-ethyl)-amide; 2-(3-methanesulfonylamino-phenylamino)-6-(2-trifluoromethyl-phenyl)-pyrido[2,3-d]pyrimidine-7-carboxylic acid (2-methanesulfonylamino-ethyl)-amide; and 2-(4,4-dioxo-3,4-dihydro-2H-4lambda*6*-benzo[1,4]oxathiin-6-ylamino)-6-(2-trifluoromethyl-phenyl)-pyrido[2,3-d]pyrimidine-7-carboxylic acid (2-methanesulfonylamino-ethyl)-amide. United States Patent No. 7,531,536 to Bebbington et al., describes pyrazole compounds as Src kinase inhibitors. United States Patent No. 7,494,993 to Engh et al., discloses amide derivatives of 7-amino-3-phenyl-dihydropyrimido[4,5-d]pyrimidinones. United States Patent No. 7,479,561 to Boschelli et al., describes 4-(2,4-dichloro-5-methoxyphenyl)amino-6-methoxy-7-{[5-substituted-amino)methyl]-3-furyl}-3-quinolinecarbonitriles as Src inhibitors, including 4-[(2,4-dichloro-5-methoxyphenyl)amino]-6-methoxy-7-{5-[(4-methylpiperazin-1-yl)methyl]-3-furyl}-3-quinolinecarbonitrile; 4-[(2,4-dichloro-5-methoxyphenyl)amino]-7-{5-[(dimethylamino)methyl]-3-furyl{-3-quinolinecarbonitrile; 4-[(2,4-dichloro-5-methoxyphenyl)amino]-6-methoxy-7-[5-(morpholin-4-ylmethyl)]-3-furyl]-3-quinolinecarbonitrile; 4-[(2,4-dichloro-5-methoxyphenyl)amino]-6-methoxy-7-{5-[(4-phenylpiperazin-1-yl)methyl]-3-furyl}-3-quinolinecarbonitrile; 4-[(2,4-dichloro-5-methoxyphenyl)amino]-6-methoxy-7-(5-{[4-(2,4-dimethoxyphenyl)piperazin-1-yl]methyl}-3-furyl)-3-quinolinecarbonitrile; 4-[(2,4-dichloro-5-methoxyphenyl)amino]-6-methoxy-7-[5-(pyrrolidin-1-ylmethyl)-3-furyl]quinoline-3-carbonitrile; 4-[(2,4-dichloro-5-methoxyphenyl)amino]-6-methoxy-7-[5-(piperidin-1-ylmethyl)-3-furyl]quinoline-3-carbonitrile; 4-[(2,4-dichloro-5-methoxyphenyl)amino]-7-{5-[(diethylamino)methyl]-3-furyl)-6-methoxyquinoline-3-carbonitrile; 4-[(2,4-dichloro-5-methoxyphenyl)amino]-7-{5-[(4-ethyipiperazin-1-yl)methyl]-3-furyl)-6-methoxyquinoline-3-carbonitrile; 4-[(2,4-dichloro-5-methoxyphenyl)amino]-6-methoxy-7-(5-{[4-(1-methylpiperidin-4-yl)piperazin-1-yl]methyl}-3-furyl)quinoline-3-carbonitrile; 4-[(2,4-dichloro-5-methoxyphenyl)amino]-6-methoxy-7-{5-[(4-pyrrolidin-1-ylpiperidin-1-yl)methyl]-3-furyl}quinoline-3-carbonitrile; 7-{5-[(4-butylpiperazin-1-yl)methyl]-3-furyl}-4-[(2,4-dichloro-5-methoxyphenyl)amino]-6-methoxyquinoline-3-carbonitrile; 4-[(2,4-dichloro-5-methoxyphenyl)amino]-6-methoxy-7-(5-{[4-(2-morpholin-4-ylethyl)piperazin-1-yl]methyl}-3-furyl)quinoline-3-carbonitrile; 7-{5-[(4-benzylpiperazin-1-yl)methyl]-3-furyl}-4-[(2,4-dichloro-5-methoxyphenyl)amino]-6-methoxyquinoline-3-carbonitrile; 4-[(2,4-dichloro-5-methoxyphenyl)amino]-6-methoxy-7-(5-{[4-(2-phenylethyl)piperazin-1-yl]methyl}-3-furyl)quinoiine-3-carbonitrile; 4-[(2,4-dichloro-5-methoxyphenyl)amino]-7-{5-[(dipropylamino)methyl]-3-furyl}-6-methoxyquinoline-3-carbonitrile; 4-[(2,4-dichloro-5-methoxyphenyl)amino]-7-{5-[(1,1-dioxidothiomorpholin-4-yl)methyl]-3-furyl}-6-methoxyquinoline-3-carbonitrile; 4-[(2,4-dichloro-5-methoxyphenyl)amino]-6-methoxy-7-{5-[(1-oxidothiomorpholin-4-yl)methyl]-3-furyl}quinoline-3-carbonitrite; 7-{5-[(4-cyclohexylpiperazin-1-yl)methyl]-3-furyl}-4-[(2,4-dichloro-5-methoxyphenyl)amino]-6-methoxyquinoline-3-carbonitrile; 4-[(2,4-dichloro-5-methoxyphenyl)amino]-6-methoxy-7-(5-{[4-(4-methoxyphenyl)piperazin-1-yl]methyl}-3-furyl)quinoline-3-carbonitrile; 4-[(2,4-dichloro-5-methoxyphenyl)amino]-6-methoxy-7-{5-[(4-pyridin-4-ylpiperazin-1-yl)methyl]-3-furyl}quinoline-3-carbonitrile; 4-[(2,4-dichloro-5-methoxyphenyl)amino]-6-methoxy-7-(5-{[4-(4-methylphenyl)piperazin-1-yl]methyl}-3-furyl)quinoline-3-carbonitrile; 7-(5-{[4-(4-chlorophenyl)piperazin-1-yl]methyl}-3-furyl)-4-[(2,4-dichloro-5-methoxyphenyl)amino]-6-methoxyquinoline-3-carbonitrile; 4-[(2,4-dichloro-5-methoxyphenyl)amino]-7-(5-{[4-(4-hydroxyphenyl)piperazin-1-yl]methyl}-3-furyl)-6-methoxyquinoline-3-carbonitrile; 4-[(2,4-dichloro-5-methoxyphenyl)amino]-6-methoxy-7-[5-({4-[4-(trifluoromethyl)phenyl]piperazin-1-yl}methyl)-3-furyl]quinoline-3-carbonitrile; 4-[(2,4-dichloro-5-methoxyphenyl)amino]-6-methoxy-7-[5-(thiomorpholin-4-ylmethyl)-3-furyl]quinoline-3-carbonitrile; 4-[(2,4-dichloro-5-methoxyphenyl)amino]-7-(5-{[[2-(dimethylamino)ethyl](methyl)amino]methyl}-3-furyl)-6-methoxyquinoline-3-carbonitrile; 4-[(2,4-dichloro-5-methoxyphenyl)amino]-7-}5-[(4-isopropylpiperazin-1-yl)methyl]-3-furyl}-6-methoxyquinoline-3-carbonitrile; 4-[(2,4-dichloro-5-methoxyphenyl)amino]-6-methoxy-7-{5-[(4-methyl-1,4-diazepan-1-yl)methyl]-3-furyl)quinoline-3-carbonitrile; 4-[(2,4-dichloro-5-methoxyphenyl)amino]-6-methoxy-7-(5-{[4-(2-methoxyethyl)piperazin-1-yl]methyl}-3-furyl)quinoline-3-carbonitrile; 4-[(2,4-dichloro-5-methoxyphenyl)amino]-7-(5-{[4-{2-hydroxyethyl)piperazin-1-yl]methyl)-3-furyl)-6-methoxyquinoline-3-carbonitrile; 4-[(2,4-dichloro-5-methoxyphenyl)amino]-7-(5-{[4-(2,6-dimethyiphenyl)piperazin-1-yl]methyl}-3-furyl)-6-methoxyquinoline-3-carbonitrile; 4-[(2,4-dichloro-5-methoxyphenyl)amino]-7-[5-({4-[3-(diethylamino)propyl]piperazin-1-yl}methyl)-3-furyl]-6-methoxyquinoline-3-carbonitrile; 4-[(2,4-dichloro-5-methoxyphenyl)amino]-6-methoxy-7-(5-{[4-(pyridin-4-ylmethyl)piperazin-1-yl]methyl}-3-furyl)quinoline-3-carbonitrile; and 4-[(2,4-dichloro-5-methoxyphenyl)amino]-7-(5-{[4-(2,6-dimethyiphenyl)piperazin-1-yl]methyl}-3-furyl)-6-methoxyquinoline-3-carbonitrile. United States Patent No. 7,473,691 to Davies et al., describes pyrazole compounds as Src inhibitors. United States Patent No. 7,417,148 to Boschelli et al., discloses 4-anilino-3-quinolinecarbonitriles as Src inhibitors, including 4-[(2,4-dichloro-5-methoxyphenyl)amino]-6-methoxy-7-[3-(4-methyl-1-piperazinyl)propoxy]-3-quinolinecarbonitrile; 4-[(2,4-dichloro-5-methoxyphenyl)amino]-7-[3-(4-ethyl-1-piperazinyl)propoxy]-6-methoxy-3-quinolinecarbonitrile; 4-[(2,4-dichloro-5-methoxyphenyl)amino]-6-methoxy-7-[2-(4-methyl-1-piperazinyl)ethoxy]-3-quinolinecarbonitrile; 4-[(2,4-dichloro-5-methoxyphenyl)amino]-7-[2-(4-ethyl-1-piperazinyl)ethoxy]-6-methoxy-3-quinolinecarbonitrile; 4-[(2,4-dichloro-5-methoxyphenyl)amino]-6-methoxy-7-[(1-methylpiperidin-4-yl)methoxy]-3-quinolinecarbonitrile; 4-[(2,4-dichloro-5-methoxyphenyl)amino]-6-methoxy-7-[2-(1-methylpiperidin-4-yl)ethoxy]-3-quinolinecarbonitrile; 4-[(2,4-dichloro-5-methoxyphenyl)amino]-6-methoxy-7-[3-(1-methylpiperidin-4-yl)propoxy]quinoline-3-carbonitrile; 4-[(2,4-dichloro-5-methoxyphenyl)amino]-7-[(1-ethylpiperidin-4-yl)methoxy]-6-methoxyquinoline-3-carbonitrile; 4-[(2,4-dichloro-5-methoxyphenyl)amino]-6-ethoxy-7-[3-(4-methylpiperazin-1-yl)propoxy]quinoline-3-carbonitrile; 4-[(2,4-dichloro-5-methoxyphenyl)amino]-6-ethoxy-7-[(1-methylpiperidin-4-yl)methoxy]quinoline-3-carbonitrile; 4-[(2,4-dichloro-5-methoxyphenyl)amino]-6-ethoxy-7-[3-(4-ethylpiperazin-1-yl)propoxy]quinoline-3-carbonitrile; 4-[(2,4-dichloro-5-methoxyphenyl)amino]-6-ethoxy-7-[3-(1-methylpiperidin-4-yl)propoxy]quinoline-3-carbonitrile; 4-[(2,4-dichloro-5-methoxyphenyl)amino]-6-ethoxy-7-[2-(4-methyl-1-piperazinyl)ethoxy]quinoline-3-carbonitrile; 4-[(2,4-dichloro-5-methoxyphenyl)amino]-6-ethoxy-7-[2-(1-methylpiperidin-4-yl)ethoxy]quinoline-3-carbonitrile; 4-[(2,4-dichloro-5-methoxyphenyl)amino]-6-methoxy-7-[3-(4-propyl-1-piperazinyl)propoxy]-3-quinolinecarbonitrile; 4-[(2,4-dichlorophenyl)amino]-6-methoxy-7-[(1methylpiperidin-4-yl)methoxy]-3-quinolinecarbonitrile; 6-methoxy-7-[(1-methylpiperidin-4-yl)methoxy]-4-[(3,4,5-trimethoxyphenyl)amino]quinoline-3-carbonitrile; 4-[(2-chloro-5-methoxyphenyl)amino]-6-methoxy-7-[(1-methylpiperidin-4-yl)methoxy]quinoline-3-carbonitrile; 6-methoxy-4-[(5-methoxy-2-methylphenyl)amino]-7-[(1-methylpiperidin-4-yl)methoxy]quinoline-3-carbonitrile; 4-[(2,4-dimethylphenyl)amino]-6-methoxy-7-[(1-methylpiperidin-4-yl)methoxy]quinoline-3-carbonitrile; 6-methoxy-4-[(5-methoxy-2,4-dimethylphenyl)amino]-7-[(1-methylpiperidin-4-yl)methoxy]quinoline-3-carbonitrile; and 4-[(2,4-dichloro-5-ethoxyphenyl)amino]-6-methoxy-7-[(1-methylpiperidin-4-yl)methoxy]quinoline-3-carbonitrile. United States Patent No. 7,390,815 to Davies et al., describes pyrazole compounds as Src inhibitors. United States Patent No. 7,285,556 to Benish et al., describes thienopyridine compounds as Src inhibitors, including 2-thiazolecarboxaldehyde(7-methylthieno[3,2-d]pyrimidin-4-yl)hydrazone; 1 H-imidazole-4-carboxaldehyde(7-methylthieno[3,2-d]pyrimidin-4-yl)hydrazone; 2-chlorobenzaldehyde(7-methylthieno[3,2-d]pyrimidin-4-yl)hydrazone; 3-bromobenzaldehyde(7-methylthieno[3,2-d]pyrimidin-4-yl)hydrazone; 4-fluorobenzaldehyde(6,7-dimethylthieno[3,2-d]pyrimidin-4-yl)hydrazone; 1-(3-pyridinyl)ethanone(7-methylthieno[3,2-d]pyrimidin-4-yl)hydrazone; 3-methyl-2-thiophenecarboxaldehyde(7-methylthieno[3,2-d]pyrimidin-4-yl)hydrazone; 4-propoxybenzaldehyde(7-methylthieno[3,2-d]pyrimidin-4-yl)hydrazone; 3-propoxybenzaldehyde(7-methylthieno[3,2-d]pyrimidin-4-yl)hydrazone; 1H-indole-5-carboxaldehyde(7-methylthieno[3,2-d]pyrimidin-4-yl)hydrazone; ethanal(7-methylthieno[3,2-d]pyrimidin-4-yl)hydrazone; 4-(methylthio)benzaldehyde(7-methylthieno[3,2-d]pyrimidin-4-yl)hydrazone; propanal(7-methylthieno[3,2-d]pyrimidin-4-yl)hydrazone; butanal(7-methyithieno[3,2-d]pyrimidin-4-yl)hydrazone; pentanal(7-methylthieno[3,2-d]pyrimidin-4-yl)hydrazone; tetrahydro-3-furancarboxaldehyde(7-methylthieno[3,2-d]pyrimidin-4-yl)hydrazone; 3-cyclohexene-1-carboxaldehyde(7-methylthieno[3,2-d]pyrimidin-4-yl)hydrazone; E-2-Butenal(7-methylthieno[3,2-d]pyrimidin-4-yl)hydrazone; benzeneacetaldehyde(7-methylthieno[3,2-d]pyrimidin-4-yl)hydrazone; 3-pyridinecarboxaldehyde(6-bromothieno[3,2-d]pyrimidin-4-yl)hydrazone; 3-pyridinecarboxaldehyde(6-(3-thienyl)thieno[3,2-d]pyrimidin-4-yl)hydrazone; 2-thiophenecarboxaldehyde(pyrido[3',2':4,5]thieno[3,2-d]pyrimidin-4-yl)hydrazone; 4-carboxybenzaldehyde(pyrido[3',2':4,5]thieno[3,2-d]pyrimidin-4-yl)hydrazone; 3-hydroxy-4-methoxybenzaldehyde(pyrido[3',2':4,5]thieno[3,2-d]pyrimidin-4-yl)hydrazone; 3-carboxybenzaldehyde(7-methylthieno[3,2-d]pyrimidin-4-yl)hydrazone; 4-pyridinecarboxaldehyde(6-phenylthieno[3,2-d]pyrimidin-4-yl)hydrazone; 3-pyridinecarboxaldehyde(7-ethylthieno[3,2-d]pyrimidin-4-yl)hydrazone; 3-pyridinecarboxaldehyde(7-propylthieno[3,2-d]pyrimidin-4-yl)hydrazone; 3-pyridinecarboxaldehyde(6-(4-fluorophenyl)thieno[3,2-d]pyrimidin-4-yl)hydrazone; 4-carboxybenzaldehyde(6-(4-fluorophenyl)thieno[3,2-d]pyrimidin-4-yl)hydrazone; 3-pyridinecarboxaldehyde(6-(4-methanesulphonylphenyl)thieno-[3,2-d]pyrimidin-4-yl)hydrazone; 4-carboxybenzaldehyde(6-(4-methanesulphonylphenyl)thieno-[3,2-d]pyrimidin-4-yl)hydrazone; 3-pyridinecarboxaldehyde(6-(3-chlorophenyl)thieno[3,2-d]pyrimidin-4-yl)hydrazone; 4-carboxybenzaldehyde(6-(3-chlorophenyl)thieno[3,2-d]pyrimidin-4-yl)hydrazone; 3-pyridinecarboxaldehyde(6-(4-chlorophenyl)thieno[3,2-d]pyrimidin-4-yl)hydrazone; 4-carboxybenzaldehyde(6-(4-chlorophenyl)thieno[3,2-d]pyrimidin-4--yl)hydrazone; 3-pyridinecarboxaldehyde(6-(2-chlorophenyl)thieno[3,2-d]pyrimidin-4-yl)hydrazone; 4-carboxybenzaldehyde(6-(2-chlorophenyl)thieno[3,2-d]pyrimidin-4-yl)hydrazone; 2-thiophenecarboxaldehyde(6-(2-chlorophenyl)thieno[3,2-d]pyrimidin-4-yl)hydrazone; 3-hydroxy-4-methoxybenzaldehyde(6-(2-chlorophenyl)thieno[3,2-d]pyrimidin-4-yl)hydrazone; 3-pyridinecarboxaldehyde(7-methyl-6-phenylthieno[3,2-d]pyrimidin-4-yl)hydrazone; 4-carboxybenzaldehyde(7-methyl-6-phenylthieno[3,2-d]pyrimidin-4-yl)hydrazone; 2-thiophenecarboxaldehyde(7-methyl-6-phenylthieno[3,2-d]pyrimidin-4-yl)hydrazone; 3-hydroxy-4-methoxybenzaldehyde(7-methyl-6-phenylthieno[3,2-d]pyrimidin-4-yl)hydrazone; 3-pyridinecarboxaldehyde(6-iodo-7-methylthieno[3,2-d]pyrimidin-4-yl)hydrazone; 4-carboxybenzaldehyde(6-iodo-7-methylthieno[3,2-d]pyrimidin-4-yl)hydrazone; 2-thiophenecarboxaldehyde(6-iodo-7-methylthieno[3,2-d]pyrimidin-4-yl)hydrazone; 3-hydroxy-4-methoxybenzaldehyde(6-iodo-7-methylthieno[3,2-d]pyrimidin-4-yl)hydrazone; 3-pyridinecarboxaldehyde(7-methyl-6-(3-thienyl)thieno[3,2-d]pyrimidin-4-yl)hydrazone; 4-carboxybenzaldehyde(7-methyl-6-(3-thienyl)thieno[3,2-d]pyrimidin-4-yl)hydrazone; 2-thiophenecarboxaldehyde(7-methyl-6-(3-thienyl)thieno[3,2-d]pyrimidin-4-yl)hydrazone; 3-hydroxy-4-methoxybenzaldehyde(7-methyl-6-(3-thienyl)thieno[3,2-d]pyrimidin-4-yl)hydrazone; 3-pyridinecarboxaldehyde(6-(2-chlorophenyl)7-methylthieno[3,2-d]pyrimidin-4-yl)hydrazone; 4-carboxybenzaldehyde(6-(2-chlorophenyl)7-methylthieno[3,2-d]pyrimidin-4-yl)hydrazone; 2-thiophenecarboxaldehyde(6-(2-chlorophenyl)7-methylthieno[3,2-d]pyrimidin-4-yl)hydrazone; 3-pyridinecarboxaldehyde(6-iodothieno[3,2-d]pyrimidin-4-yl)hydrazone; 4-carboxybenzaldehyde(6-iodothieno[3,2-d]pyrimidin-4-yl)hydrazone; 2-thiophenecarboxaldehyde(6-iodothieno[3,2-d]pyrimidin-4-yl)hydrazone; 3-hydroxy-4-methoxybenzaldehyde(6-iodothieno[3,2-d]pyrimidin-4-yl)hydrazone; 3-pyridinecarboxaldehyde(6-(4-methoxyphenyl)thieno[3,2-d]pyrimidin-4-yl)hydrazone; 4-carboxybenzaldehyde(6-(4-methoxyphenyl)thieno[3,2-d]pyrimidin-4-yl)hydrazone; 2-thiophenecarboxaldehyde(6-(4-methoxyphenyl)thieno[3,2-d]pyrimidin-4-yl)hydrazone; 3-hydroxy-4-methoxybenzaldehyde(6-(4-methoxyphenyl)thieno[3,2-d]pyrimidin-4-yl)hydrazone; 3-pyridinecarboxaldehyde(6-(4-methoxyphenyl)-7-methylthieno[3,2-d]pyrimidin-4-yl)hydrazone; 4-carboxybenzaldehyde(6-(4-methoxyphenyl)-7-methylthieno[3,2-d]pyrimidin-4-yl)hydrazone; 2-thiophenecarboxaldehyde(6-(4-methoxyphenyl)-7-methylthieno[3,2-d]pyrimidin-4-yl)hydrazone; 3-hydroxy-4-methoxybenzaldehyde(6-(4-methoxyphenyl)-7-methylthieno[3,2-d]pyrimidin-4-yl)hydrazone; 3-pyridinecarboxaldehyde(6-(3-methoxyphenyl)-7-methylthieno[3,2-d]pyrimidin-4-yl)hydrazone; 4-carboxybenzaldehyde(6-(3-methoxyphenyl)-7-methylthieno[3,2-d]pyrimidin-4-yl)hydrazone; 2-thiophenecarboxaldehyde(6-(3-methoxyphenyl)-7-methylthieno[3,2-d]pyrimidin-4-yl)hydrazone; 3-hydroxy-4-methoxybenzaldehyde(6-(3-methoxyphenyl)-7-methylthieno[3,2-d]pyrimidin-4-yl)hydrazone; 3-pyridinecarboxaldehyde(6-(2-methoxyphenyl)-7-methylthieno[3,2-d]pyrimidin-4-yl)hydrazone; 4-carboxybenzaldehyde(6-(2-methoxyphenyl)-7-methylthieno[3,2-d]pyrimidin-4-yl)hydrazone; 2-thiophenecarboxaldehyde(6-(2-methoxyphenyl)-7-methylthieno[3,2-d]pyrimidin-4-yl)hydrazone; 3-hydroxy-4-methoxybenzaldehyde(6-(2-methoxyphenyl)-7-methylthieno[3,2-d]pyrimidin-4-yl)hydrazone; 3-pyridinecarboxaldehyde(6-(4-hydroxymethylphenyl)-7-methylthieno[3,2-d]pyrimidin-4-yl)hydrazone; 4-carboxybenzaldehyde(6-(4-hydroxymethylphenyl)-7-methylthieno[3,2-d]pyrimidin-4-yl)hydrazone; 2-thiophenecarboxaldehyde(6-(4-hydroxymethylphenyl)-7-methylthieno[3,2-d]pyrimidin-4-yl)hydrazone; 3-hydroxy-4-methoxybenzaldehyde(6-(4-hydroxymethylphenyl)-7-methylthieno[3,2-d]pyrimidin-4-yl)hydrazone; 3-pyridinecarboxaldehyde(6-(3-hydroxymethylphenyl)-7-methylthieno[3,2-d]pyrimidin-4-yl)hydrazone; 4-carboxybenzaldehyde(6-(3-hydroxymethylphenyl)-7-methylthieno[3,2-d]pyrimidin-4-yl)hydrazone; 2-thiophenecarboxaldehyde(6-(3-hydroxymethylphenyl)-7-methylthieno[3,2-d]pyrimidin-4-yl)hydrazone; 3-hydroxy-4-methoxybenzaldehyde(6-(3-hydroxymethylphenyl)-7-methylthieno[3,2-d]pyrimidin-4-yl)hydrazone; 3-pyridinecarboxaldehyde(7-methyl-6-(trans-2-phenylvinyl)-thieno[3,2-d]pyrimidin-4-yl)hydrazone; 4-carboxybenzaldehyde(7-methyl-6-(trans-2-phenylvinyl)-thieno[3,2-d]pyrimidin-4-yl)hydrazone; 3-hydroxy-4-methoxybenzaldehyde(7-methyl-6-(trans-2-phenylvinyl)-thieno[3,2-d]pyrimidin-4-yl)hydrazone; 3-pyridinecarboxaldehyde(6-(4-carboxyphenyl)-7-methylthieno[3,2-d]pyrimidin-4-yl)hydrazone; 2-thiophenecarboxaldehyde(6-(4-carboxyphenyl)-7-methylthieno[3,2-d]pyrimidin-4-yl)hydrazone; 3-hydroxy-4-methoxybenzaldehyde(6-(4-carboxyphenyl)-7-methylthieno[3,2-d]pyrimidin-4-yl)hydrazone; 3-pyridinecarboxaldehyde(6-(1-hydroxy-1-phenyl)methyl-7-methylthieno[3,2-d]pyrimidin-4-yl)hydrazone; 4-carboxybenzaldehyde(6-(1-hydroxy-1-phenyl)methyl-7-methylthieno[3,2-d]pyrimidin-4-yl)hydrazone; 2-thiophenecarboxaldehyde(6-(1-hydroxy-1-phenyl)methyl-7-methylthieno[3,2-d]pyrimidin-4-yl)hydrazone; 3-hydroxy-4-methoxybenzaldehyde(6-(1-hydroxy-1-phenyl)methyl-7-methylthieno[3,2-d]pyrimidin-4-yl)hydrazone; 3-oyridinecarboxaldehyde(6-(1-hydroxy-1-[3-thienyl])methyl-7-methylthieno[3,2-d]pyrimidin-4-yl)hydrazone; 4-carboxybenzaldehyde(6-(1-hydroxy-1-[3-thienyl])methyl-7-methylthieno[3,2-d]pyrimidin-4-yl)hydrazone; 2-thiophenecarboxaldehyde(6-(1-hydroxy-1-[3-thienyl])methyl-7-methylthieno[3,2-d]pyrimidin-4-yl)hydrazone; 3-hydroxy-4-methoxybenzaldehyde(6-(1-hydroxy-1-[3-thienyl])methyl-7-methylthieno[3,2-d]pyrimidin-4-yl)hydrazone; 3-pyridinecarboxaldehyde (6-carboxy-7-methylthieno[3,2-d]pyrimidin-4-yl)hydrazone; 4-carboxybenzaldehyde (6-carboxy-7-methylthieno[3,2-d]pyrimidin-4-yl)hydrazone; 2-thiophenecarboxaldehyde (6-carboxy-7-methylthieno[3,2-d]pyrimidin-4-yl)hydrazone; 3-hydroxy-4-methoxybenzaldehyde(6-carboxy-7-methylthieno[3,2-d]pyrimidin-4-yl)hydrazone; 3-pyridinecarboxaldehyde(6-(1-cyclohexyl-1-hydroxy)methyl-7-methylthieno[3,2-d]pyrimidin-4-yl)hydrazone; 4-carboxybenzaldehyde(6-(1-cyclohexyl-1-hydroxy)methyl-7-methylthieno[3,2-d]pyrimidin-4-yl)hydrazone; 2-thiophenecarboxaldehyde(6-(1-cyclohexyl-1-hydroxy)methyl-7-methylthieno[3,2-d]pyrimidin-4-yl)hydrazone; 3-hydroxy-4-methoxybenzaldehyde(6-(1-cyclohexyl-1-hydroxy)methyl-7-methylthieno[3,2-d]pyrimidin-4-yl)hydrazone; 3-pyridinecarboxaldehyde(6-(1-benzyl-1-phenyl)methyl-7-methylthieno[3,2-d]pyrimidin-4-yl)hydrazone; 2-thiophenecarboxaldehyde(6-(1-benzyl-1-phenyl)methyl-7-methylthieno[3,2-d]pyrimidin-4-yl)hydrazone; 3-hydroxy-4-methoxybenzaldehyde(6-(1-benzyl-1-phenyl)methyl-7-methylthieno[3,2-d]pyrimidin-4-yl)hydrazone; 3-pyridinecarboxaldehyde(6-hydroxymethyl-7-methylthieno[3,2-d]pyrimidin-4-yl)hydrazone; 4-carboxybenzaldehyde(6-hydroxymethyl-7-methylthieno[3,2-d]pyrimidin-4-yl)hydrazone; 3-hydroxy-4-methoxybenzaldehyde(6-hydroxymethyl-7-methylthieno[3,2-d]pyrimidin-4-yl)hydrazone; 3-pyridinecarboxaldehyde(6-(1-hydroxy-1-[3,4,5-trimethoxyphenyl])methyl-7-methylthieno[3,2-d]pyrimidin-4-yl)hydrazone; 4-carboxybenzaldehyde(6-(1-hydroxy-1-[3,4,5-trimethoxyphenyl])methyl-7-methylthieno[3,2-d]pyrimidin-4-yl)hydrazone; 2-thiophenecarboxaldehyde(6-(1-hydroxy-1-[3,4,5-trimethoxyphenyl]) methyl-7-methylthieno[3,2-d]pyrimidin-4-yl)hydrazone; 3-hydroxy-4-methoxybenzaldehyde(6-(1-hydroxy-1-[3,4,5-trimethoxyphenyl])methyl-7-methylthieno[3,2-d]pyrimidin-4-yl)hydrazone; 3-pyridinecarboxaldehyde(6-(1-hydroxy-1-[3-pyridyl])methyl-7-methylthieno[3,2-d]pyrimidin-4-yl)hydrazone; 4-carboxybenzaldehyde(6-(1-hydroxy-1-[3-pyridyl])methyl-7-methylthieno[3,2-d]pyrimidin-4-yl)hydrazone; 2-thiophenecarboxaldehyde(6-(1-hydroxy-1-[3-pyridyl])methyl-7-methylthieno[3,2-d]pyrimidin-4-yl)hydrazone; 3-hydroxy-4-methoxybenzaldehyde(6-(1-hydroxy-1-[3-pyridyl])methyl-7-methylthieno[3,2-d]pyrimidin-4-yl)hydrazone; 3-pyridinecarboxaldehyde(6-(1-hydroxy-1-[2-thienyl])methyl-7-methylthieno[3,2-d]pyrimidin-4-yl)hydrazone; 4-carboxybenzaldehyde(6-(1-hydroxy-1-[2-thienyl])methyl-7-methylthieno[3,2-d]pyrimidin-4-yl)hydrazone; 2-thiophenecarboxaldehyde(6-(1-hydroxy-1-[2-thienyl])methyl-7-methylthieno[3,2-d]pyrimidin-4-yl)hydrazone; and 3-hydroxy-4-methoxybenzaldehyde(6-(1-hydroxy-1-[2-thienyl])methyl-7-methylthieno[3,2-d]pyrimidin-4-yl)hydrazone. United States Patent No. 7,276,519 to Boschelli et al., describes thieno[3,2-b]pyridine-6-carbonitriles and thieno[2,3-b]pyridine-5-carbonitriles as Src inhibitors, including 3-bromo-7-[(2,4-dichloro-5-methoxyphenyl)amino]thieno[3,2-b]pyridine-6-carbonitrile; 7-[(2,4-dichloro-5-methoxyphenyl)amino]-3-(4-formylphenyl)thieno[3,2-b]pyridine-6-carbonitrile; 7-[(2,4-dichloro-5-methoxyphenyl)amino]-3-{4-[(dimethylamino)methyl]phenyl}thieno[3,2-b]pyridine-6-carbonitrile; 7-[(2,4-dichloro-5-methoxyphenyl)amino]-3-{4-[(4-methylpiperazin-1-yl)methyl]phenyl}thieno[3,2-b]pyridine-6-carbonitrile; and 7-[(2,4-dichloro-5-methoxyphenyl)amino]-3-[4-(morpholin-4-ylmethyl)phenyl]thieno[3,2-b]pyridine-6-carbonitrile. United States Patent No. 7,262,200 to Aronov et al., describes indazolinone compounds as Src inhibitors. United States Patent No. 7,226,920 to Arnost et al., describes aminotriazole compounds as Src inhibitors. United States Patent No. 7,189,732 to Honold et al., describes pyrido[2,3-d]pyrimidine dichloro-phenyl derivatives as Src inhibitors, including 6-(2,6-dichloro-phenyl)-2-(4-morpholin-4-yl-phenylamino)-pyrido[2,3-d]pyrimidine-7-carboxylic acid (2-methanesulfonylamino-ethyl)-amide; 6-(2,6-dichloro-phenyl)-2-[4-(2-hydroxy-ethoxy)-phenylamino]-pyrido[2,3-d]pyrimidine-7-carboxylic acid (2-methanesulfonylamino-ethyl)-amide; 6-(2,6-dichloro-phenyl)-2-(3-methanesulfonylamino-phenylamino)-pyrido[2,3-d]pyrimidine-7-carboxylic acid (2-methanesulfonylamino-ethyl)-amide; 6-(2,6-dichloro-phenyl)-2-[3-(2-hydroxy-ethylsulfanyl)-phenylamino]-pyrido[2,3-d]pyrimidine-7-carboxylic acid (2-methanesulfonylamino-ethyl)-amide; 6-(2,6-dichloro-phenyl)-2-[4-(4-methyl-piperazin-1-yl)-phenylamino]-pyrido[2,3-d]pyrimidine-7-carboxylic acid (2-methanesulfonylaminoethyl)-amide; and 6-(2,6-dichloro-phenyl)-2-(3-methylsulfanyl-phenylamino)-pyrido[2,3-d]pyrimidine-7-carboxylic acid (2-methanesulfonylamino-ethyl)-amide. United States Patent No. 7,169,781 to Honold et al., describes imidazole derivatives as Src inhibitors, including 2-(2,6-dichlorophenyl)-4-(3-bromophenyl)-5-(2-[4-(2-diethylamino-ethoxy)phenylamino]pyrimidin-4-yl)-N--H-imidazole; 2-(2,6-dichlorophenyl)-4-(3-chlorophenyl)-5-(2-[4-(2-diethylamino-ethoxy)phenylamino]pyrimidin-4-yl)-N--H-imidazole; 2-(2,6-dichlorophenyl)-4-(3-chlorophenyl)-5-(2-[4-(2-hydroxyethoxy)phenyl-amino]pyrimidin-4-yl)-N--H-imidazole; 2-(2,6-dichlorophenyl)-4-(3-chlorophenyl)-5-(2-[4-dimethylaminophenyl-amino]pyrimidin-4-yl)-N--H-imidazole; 2-(2,6-dichlorophenyl)-4-(3-chlorophenyl)-5-(2-[4-(N-(2-hydroxyethyl)-sulfamoyl)phenylamino]-pyrimidin-4-yl)-N--H-imidazole; 2-(2,6-dichloro-4-hydroxymethylphenyl)-4-(3-chlorophenyl)-5-(2-[4-(2-diethylaminoethoxy)-phenylamino]pyrimidin-4-yl)-N--H-imidazole; 2-(2,6-dichloro-4-hydroxymethylphenyl)-4-(3-chlorophenyl)-5-(2-[4-(2-hydroxyethoxy)-phenylamino]pyrimidin-4-yl)-N--H-imidazole; 2-(2,6-dichloro-4-[2-hydroxyethoxy]phenyl)-4-(3-chlorophenyl)-5-(2-[4-(2-diethylaminoethoxy)-phenylamino]pyrimidin-4-yl)-N--H-imidazole; 2-(2,6-dichloro-4-[2-hydroxyethoxy]phenyl)-4-(3-chlorophenyl)-5-(2-[4-methylsufinyl-phenylamino]pyrimidin-4-yl)-N--H-imidazole; 2-(2,6-dichloro-4-[2-hydroxyethoxy]phenyl)-4-(3-chlorophenyl)-5-(2-[4-(N-(2-hydroxyethyl)-sulfamoyl)phenylamino]pyrimidin-4-yl)-N--H-imidazole; 2-(2,6-dichlorophenyl)-4-(4-chlorophenyl)-5-(2-[4-(2-diethylaminoethoxy)phenylamino]pyrimidin-4-yl)-N--H-imidazole; 2-(2,6-dichlorophenyl)-4-(4-chlorophenyl)-5-(2-[4-hydroxyphenyl-amino]pyrimidin-4-yl)-N--H-imidazole; 2-(2,6-dichlorophenyl)-4-(4-chlorophenyl)-5-(2-[4-methoxyphenyl-amino]pyrimidin-4-yl)-N--H-imidazole; 2-(2,6-dichlorophenyl)-4-(4-chlorophenyl)-5-(2-[4-ethoxyphenyl-amino]pyrimidin-4-yl)-N--H-imidazole; 2-(2,6-dichlorophenyl)-4-(3-ethynylphenyl)-5-(2-[4-(2-diethylamino-ethoxy)phenylamino]pyrimidin-4-yl)-N--H-imidazole; 2-(2,6-dichlorophenyl)-4-(3-ethynylphenyl)-5-(2-[4-(2-hydroxyethoxy)-phenylamino]pyrimidin-4-yl)-N--H-imidazole; and 2-(2,6-dichloro-4-[2-hydroxyethoxy]phenyl)-4-(3-ethynylphenyl)-5-(2-[4-(2-diethylaminoethoxy)-phenylamino]pyrimidin-4-yl)-N--H-imidazole. United States Patent No. 7,163,941 to Honold et al., describes pyrido[2,3-d]pyrimidin-7-carboxylic acids as Src inhibitors, including 6-(2-bromo-phenyl)-2-(4-morpholin-4-yl-phenylamino)-pyrido[2,3-d]pyrimidine-7-carboxylic acid [2-(3H-imidazol-4-yl)-ethyl]-amide; 6-(2-bromo-phenyl)-2-(4-morpholin-4-yl-phenylamino)-pyrido[2,3-d]pyrimidine-7-carboxylic acid (S)-piperidin-3-ylamide; 6-(2-bromo-phenyl)-2-(3-methylsulfanyl-phenylamino)-pyrido[2,3-d]pyrimidine-7-carboxylic acid (S)-piperidin-3-ylamide; 6-(2-Bromo-phenyl)-2-(4-morpholin-4-yl-phenylamino)-pyrido[2,3-d]pyrimidine-7-carboxylic acid (2-sulfamoyl-ethyl)-amide, 6-(2-bromo-phenyl)-2-(3-methylsulfanyl-phenylamino)-pyrido[2,3-d]pyrimidine-7-carboxylic acid (2-sulfamoyl-ethyl)-amide; 6-(2-bromo-phenyl)-2-(4-morpholin-4-yl-phenylamino)-pyrido[2,3-d]pyrimidine-7-carboxylic acid (2-hydroxyethyl)-amide; 6-(2-bromo-phenyl)-2-(4-fluoro-phenylamino)-pyrido[2,3-d]pyrimidine-7-carboxylic acid (2-dimethylamino-ethyl)-amide; 6-(2-bromo-phenyl)-2-(4-fluoro-phenylamino)-pyrido[2,3-d]pyrimidine-7-carboxylic acid (2-methoxy-ethyl)-amide; 6-(2-bromo-phenyl)-2-(4-fluoro-phenylamino)-pyrido[2,3-d]pyrimidine-7-carboxylic acid (3-dimethylamino-propyl)-amide; 6-(2-bromo-phenyl)-2-(4-fluoro-phenylamino)-pyrido[2,3-d]pyrimidine-7-carboxylic acid (3-dimethylamino-2,2-dimethyl-propyl)-amide; 6-(2-bromo-phenyl)-2-(4-fluoro-phenylamino)-pyrido[2,3-d]pyrimidine-7-carboxylic acid (2-acetylamino-ethyl)-amide; 6-(2-bromo-phenyl)-2-(4-fluoro-phenylamino)-pyrido[2,3-d]pyrimidine-7-carboxylic acid (2-methylamino-ethyl)-amide; 6-(2-bromo-phenyl)-2-(4-fluoro-phenylamino)-pyrido[2,3-d]pyrimidine-7-carboxylic acid carbamoylmethyl-amide; 6-(2-bromo-phenyl)-2-(4-fluoro-phenylamino)-pyrido[2,3-d]pyrimidine-7-carboxylic acid (2-dimethylamino-1-methyl-ethyl)-amide; 6-(2-bromo-phenyl)-2-(4-fluoro-phenylamino)-pyrido[2,3-d]pyrimidine-7-carboxylic acid methylcarbamoylmethyl-amide; 6-(2-bromo-phenyl)-2-(4-fluoro-phenylamino)-pyrido[2,3-d]pyrimidine-7-carboxylic acid (2-dimethylamino-propyl)-amide; 6-(2-bromophenyl)-2-(4-fluoro-phenylamino)-pyrido[2,3-d]pyrimidine-7-carboxylic acid dimethylcarbamoylmethyl-amide; 6-(2-bromo-phenyl)-2-(4-fluoro-phenylamino)-pyrido[2,3-d]pyrimidine-7-carboxylic acid (3-methylamino-propyl)-amide; 6-(2-bromophenyl)-2-(4-fluoro-phenylamino)-pyrido[2,3-d]pyrimidine-7-carboxylic acid (2-sulfamoyl-ethyl)-amide; 6-(2-bromo-phenyl)-2-(4-fluoro-phenylamino)-pyrido[2,3-d]pyrimidine-7-carboxylic acid (2-hydroxy-ethyl)-amide; 6-(2-bromo-phenyl)-2-(4-fluoro-phenylamino)-pyrido[2,3-d]pyrimidine-7-carboxylic acid (3-hydroxy-propyl)-amide; (S)-6-(2-bromophenyl)-2-(4-fluoro-phenylamino)-pyrido[2,3-d]pyrimidine-7-carboxylic acid (2,3-dihydroxy-propyl)-amide; (R)-6-(2-bromo-phenyl)-2-(4-fluoro-phenylamino)-pyrido[2,3-d]pyrimidine-7-carboxylic acid (2,3-dihydroxy-propyl)-amide; 6-(2-bromo-phenyl)-2-(4-fluoro-phenylamino)-pyrido[2,3-d]pyrimidine-7-carboxylic acid (2-methanesulfinyl-ethyl)-amide; 6-(2-bromo-phenyl)-2-(4-fluoro-phenylamino)-pyrido[2,3-d]pyrimidine-7-carboxylic acid [2-(2-hydroxy-ethanesulfinyl)-ethyl]-amide; 6-(2-bromo-phenyl)-2-(4-fluoro-phenylamino)-pyrido[2,3-d]pyrimidine-7-carboxylic acid (2-morpholin-4-yl-ethyl)-amide; 6-(2-bromo-phenyl)-2-(4-fluoro-phenylamino)-pyrido[2,3-d]pyrimidine-7-carboxylic acid [2-(2-oxo-imidazolidin-1-yl)-ethyl]-amide; (R)-6-(2-bromo-phenyl)-2-(4-fluoro-phenylamino)-pyrido[2,3-d]pyrimidine-7-carboxylic acid (pyrrolidin-2-ylmethyl)-amide; 6-(2-bromo-phenyl)-2-(4-fluoro-phenylamino)-pyrido[2,3-d]pyrimidine-7-carboxylic acid [3-(2-oxo-pyrrolidin-1-yl)-propyl]-amide; 6-(2-bromo-phenyl)-2-(4-fluoro-phenylamino)-pyrido[2,3-d]pyrimidine-7-carboxylic acid (3-morpholin-4-yl-propyl)-amide; 6-(2-bromo-phenyl)-2-(4-fluoro-phenylamino)-pyrido[2,3-d]pyrimidine-7-carboxylic acid [2-(1-methyl-pyrrolidin-2-yl)-ethyl]-amide; 6-(2-bromo-phenyl)-2-(4-fluoro-phenylamino)-pyrido[2,3-d]pyrimidine-7-carboxylic acid [2-(pyridin-2-ylamino)-ethyl]-amide; 6-(2-bromo-phenyl)-2-(4-fluoro-phenylamino)-pyrido[2,3-d]pyrimidine-7-carboxylic acid [2-(3H-imidazol-4-yl)-ethyl]-amide; 6-(2-bromo-phenyl)-2-(4-fluoro-phenylamino)-pyrido[2,3-d]pyrimidine-7-carboxylic acid (1,5-dimethyl-1H-pyrazol-3-ylmethyl)-amide; 6-(2-bromo-phenyl)-2-(4-fluoro-phenylamino)-pyrido[2,3-d]pyrimidine-7-carboxylic acid ((S)-pyrrolidin-2-ylmethyl)-amide; 6-(2-bromo-phenyl)-2-(3-methylsulfanyl-phenylamino)-pyrido[2,3-d]pyrimidine-7-carboxylic acid (2-dimethylamino-ethyl)-amide; 6-(2-bromophenyl)-2-(4-morpholin-4-yl-phenylamino)-pyrido[2,3-d]pyrimidine-7-carboxylic acid (2-dimethylamino-ethyl)-amide; 6-(2-bromo-phenyl)-2-(4-morpholin-4-yl-phenylamino)-pyrido[2,3-d]pyrimidine-7-carboxylic acid (2-methylamino-ethyl)-amide; 6-(2-bromophenyl)-2-[4-(2-diethylamino-ethoxy)-phenylamino]-pyrido[2,3-d]pyrimidine-7-carboxylic acid (2-dimethylamino-ethyl)-amide; 6-(2-bromo-phenyl)-2-(4-morpholin-4-yl-phenylamino)-pyrido[2,3-d]pyrimidine-7-carboxylic acid piperidin-4-ylamide; 6-(2-bromophenyl)-2-(3-methylsulfanyl-phenylamino)-pyrido[2,3-d]pyrimidine-7-carboxylic acid (2-methylamino-ethyl)-amide; (R)-6-(2-bromo-phenyl)-2-(4-fluoro-phenylamino)-pyrido[2,3-d]pyrimidine-7-carboxylic acid piperidin-3-ylamide; (S)-6-(2-bromo-phenyl)-2-(4-fluoro-phenylamino)-pyrido[2,3-d]pyrimidine-7-carboxylic acid piperidin-3-ylamide; 6-(2-bromophenyl)-2-(4-fluoro-phenylamino)-pyrido[2,3-d]pyrimidine-7-carboxylic acid piperidin-4-ylamide; 1-[6-(2-bromo-phenyl)-2-(4-fluoro-phenylamino)-pyrido[2,3-d]pyrimidine-7-carbonyl]semicarbazide; 6-(2-bromo-phenyl)-2-(4-fluoro-phenylamino)-pyrido[2,3-d]pyrimidine-7-carboxylic acid N'-(2-dimethylamino-acetyl)-hydrazide; 6-(2-bromophenyl)-2-(4-fluoro-phenylamino)-pyrido[2,3-d]pyrimidine-7-carboxylic acid (1-methyl-piperidin-4-yl)-amide; (S)-6-(2-bromo-phenyl)-2-(4-fluoro-phenylamino)-pyrido[2,3-d]pyrimidine-7-carboxylic acid pyrrolidin-3-ylamide; (R)-6-(2-bromo-phenyl)-2-(4-fluoro-phenylamino)-pyrido[2,3-d]pyrimidine-7-carboxylic acid pyrrolidin-3-ylamide; 6-(2-bromo-phenyl)-2-(4-fluoro-phenylamino)-pyrido[2,3-d]pyrimidine-7-car- boxylic acid (1-aza-bicyclo[2.2.2]oct-3-yl)-amide; 6-(2-bromo-phenyl)-2-(4-fluoro-phenylamino)-pyrido[2,3-d]pyrimidine-7-carboxylic acid (1H-pyrazol-3-yl)-amide; 6-(2-bromo-phenyl)-2-(4-fluoro-phenylamino)-pyrido[2,3-d]pyrimidine-7-carboxylic acid (2-methyl-2H-pyrazol-3-yl)-amide; 6-(2-bromo-phenyl)-2-(4-fluoro-phenylamino)-pyrido[2,3-d]pyrimidine-7-carboxylic acid (4-carbamoyl-1H-pyrazol-3-yl)-amide; 6-(2-bromophenyl)-2-(4-morpholin-4-yl-phenylamino)-pyrido[2,3-d]pyrimidine-7-carboxylic acid amide; 6-(2-bromo-phenyl)-2-[4-(2-diethylamino-ethoxy)-phenylamino]-pyrido[2,3-d]pyrimidine-7-carboxylic acid amide; 6-(2-bromo-phenyl)-2-(3-methylsulfanyl-phenylamino)-pyrido[2,3-d]pyrimidine-7-carboxylic acid amide; 6-(2-bromo-phenyl)-2-(4-sulfamoyl-phenylamino)-pyrido[2,3-d]pyrimidine-7-carboxylic acid amide; 6-(2-bromophenyl)-2-(3-methylsulfamoylmethyl-phenylamino)-pyrido[2,3-d]pyrimidine-7-carboxylic acid amide; 6-(2-bromo-phenyl)-2-[3-(2-hydroxy-ethanesulfonyl)-phenylamino]-pyrido[2,-3-d]pyrimidine-7-carboxylic acid amide; 6-(2-bromo-phenyl)-2-(3-methanesulfonyl-phenylamino)-pyrido[2,3-d]pyrimidine-7-carboxylic acid amide; 6-(2-bromo-phenyl)-2-(3-methanesulfonyl-phenylamino)-pyrido[2,3-d]pyrimidine-7-carbonitrile; 6-(2-bromophenyl)-2-[4-(2-diethylamino-ethoxy)-phenylamino]-pyrido[2,3-d]pyrimidine-7-carbonitrile; 6-(2-bromo-phenyl)-2-[4-(2-hydroxy-ethoxy)-phenylamino]-pyrido[2,3-d]pyrimidine-7-carbonitrile; 6-(2-bromo-phenyl)-2-[4-(2-ethylamino-ethoxy)-phenylamino]-pyrido[2,3-d]pyrimidine-7-carbonitrile; 6-(2-bromo-phenyl)-2-(3-methanesulfinyl-phenylamino)-pyrido[2,3-d]pyrimidine-7-carbonitrile; and 6-(2-bromophenyl)-2-[3-(2-hydroxy-ethanesulfonyl)-phenylamino]-pyrido[2,3-d]pyrimidine-7-carbonitrile; 6-(2-bromo-phenyl)-2-(4-morpholin-4-yl-phenylamino)-pyrido[2,3-d]pyrimidine-7-carboxylic acid (2-methanesulfonylamino-ethyl)-amide; 6-(2-bromophenyl)-2-(3-methanesulfonylamino-phenylamino)-pyrido[2,3-d]pyrimidine-7-carboxylic acid (2-methanesulfonylamino-ethyl)-amide; 6-(2-bromo-phenyl)-2-[4-(2-hydroxy-ethoxy)-phenylamino]-pyrido[2,3-d]pyrimidine-7-carboxylic acid (2-methanesulfonylamino-ethyl)-amide; 6-(2-bromo-phenyl)-2-[4-(4-methyl-piperazin-1-yl)-phenylamino]-pyrido[2,3-d]pyrimidine-7-carboxylic acid (2-methanesulfonylamino-ethyl)-amide; 6-(2-bromo-phenyl)-2-(3-methoxy-phenylamino)-pyrido[2,3-d]pyrimidine-7-carboxylic acid (2-methanesulfonylamino-ethyl)-amide; 2-(3-acetylamino-phenylamino)-6-(2-bromo-phenyl)-pyrido[2,3-d]pyrimidine-7-carboxylic acid (2-methanesulfonylaminoethyl)-amide; 6-(2-bromo-phenyl)-2-(4,4-dioxo-3,4-dihydro-2H-4lambda*6*-benzo[1,4]oxathiin-6-ylamino)-pyrido[2,3-d]pyrimidine-7-carboxylic acid (2-methanesulfonylamino-ethyl)-amide; 6-(2-bromo-phenyl)-2-[3-(2-hydroxy-ethylsulfanyl)-phenylamino]-pyrido[2,3-d]pyrimidine-7-carboxylic acid (2-methanesulfonylamino-ethyl)-amide; 6-(2-bromo-phenyl)-2-(3-hydroxymethyl-2,3-dihydro-benzo[1,4]dioxin-6-ylamino)-pyrido[2,3-d]pyrimidine-7-carboxylic acid (2-methanesulfonyl-amino-ethyl)-amide; 6-(2-bromo-phenyl)-2-(4-fluoro-phenylamino)-pyrido[2,3-d]pyrimidine-7-carboxylic acid (piperidin-2-ylmethyl)-amide; 6-(2-bromo-phenyl)-2-(4-methanesulfinyl-phenylamino)-pyrido[2,3-d]pyrimidine-7-carboxylic acid (2-hydroxy-ethyl)-amide; 6-(2-bromo-phenyl)-2-(3-methanesulfinyl-phenylamino)-pyrido[2,3-d]pyrimidine-7-carboxylic acid (2-hydroxy-ethyl)-amide; 6-(2-bromo-phenyl)-2-[3-(2-hydroxy-ethylsulfamoyl)-phenylamino]-pyrido[2,3-d]pyrimidine-7-carboxylic acid (2-hydroxy-ethyl)-amide; 6-(2-bromo-phenyl)-2-[4-(2-hydroxy-ethylsulfamoyl)-phenylamino]-pyrido[2,3-d]pyrimidine-7-carboxylic acid (2-hydroxy-ethyl)-amide; 6-(2-bromo-phenyl)-2-(4-methanesulfinyl-phenylamino)-pyrido[2,3-d]pyrimidine-7-carboxylic acid (2-sulfamoyl-ethyl)-amide; 6-(2-bromo-phenyl)-2-(3-methanesulfinyl-phenylamino)-pyrido[2,3-d]pyrimidine-7-carboxylic acid (2-sulfamoyl-ethyl)-amide; 6-(2-bromo-phenyl)-2-[4-(2-hydroxy-ethoxy)-phenylamino]-pyrido[2,3-d]pyrimidine-7-carboxylic acid (2-sulfamoyl-ethyl)-amide; 6-(2-bromo-phenyl)-2-(3-methanesulfonyl-phenylamino)-pyrido[2,3-d]pyrimidine-7-carboxylic acid (2-sulfamoyl-ethyl)-amide; 6-(2-bromo-phenyl)-2-(3-hydroxymethyl-phenylamino)-pyrido[2,3-d]pyrimidine-7-carboxylic acid (2-hydroxy-ethyl)-amide; 6-(2-bromo-phenyl)-2-(3-hydroxymethyl-phenylamino)-pyrido[2,3-d]pyrimidine-7-carboxylic acid (2-sulfamoyl-ethyl)-amide; 6-(2-bromo-phenyl)-2-(4,4-dioxo-3,4-dihydro-2H-4lambda*6*-benzo[1,4]oxathiin-6-ylamino)-pyrido[2,3-d]pyrimidine-7-carboxylic acid (2-sulfamoyl-ethyl)-amide; 6-(2-bromo-phenyl)-2-(4,4-dioxo-3,4-dihydro-2H-4lambda*6*-benzo[1,4]oxathiin-6-ylamino)-pyrido[2,3-d]pyrimidine-7-carboxylic acid (pyrrolidin-2-ylmethyl)-amide HCI salt; 6-(2-bromo-phenyl)-2-(3-methanesulfonylamino-phenylamino)-pyrido[2,3-d]pyrimidine-7-carboxylic acid (pyrrolidin-2-ylmethyl)-amide HCI salt; and 2-(3-acetylamino-phenylamino)-6-(2-bromo-phenyl)-pyrido[2,3-d]pyrimidine-7-carboxylic acid (pyrrolidin-2-ylmethyl)-amide HCI salt. United States Patent No. 7,129,351 to Luk et al., describes pyrimido compounds as Src inhibitors, including (+)-3-(2-bromo-phenyl)-7-[4-(2-diethylamino-ethoxy)-phenylamino]-1,4-dimethyl-3,4-dihydro-1H-pyrimido[4,5-d]pyrimidin-2-one and (-)-3-(2-bromo-phenyl)-7-[4-(2-diethylamino-ethoxy)-phenylamino]-1,4-dimethyl-3,4-dihydro-1H-pyrimido[4,5-d]pyrimidin-2-one. United States Patent No. 7,115,739 to Bebbington et al., describes triazole compounds as Src inhibitors. United States Patent No. 7,098,330 to Bebbington et al., describes pyrazolylamine-substituted quinolone compounds as Src inhibitors. United States Patent No. 7,091,345 to Cai et al., describes amino-substituted dihydropyrimido[4,5-d]pyrimidinone derivatives as Src inhibitors. United States Patent No. 7,087,603 to Bebbington et al., describes pyrazole compounds as Src inhibitors. United States Patent No. 7,008,948 to Bebbington et al., describes fused pyrimidyl pyrazole compounds as Src inhibitors. United States Patent No. 6,989,385 to Bebbington et al., describes pyrazole compounds as Src inhibitors, including {2-[(2-hydroxyethyl)phenylamino]-quinazolin-4-yl}-(5-methyl-2H-pyrazol-3-- yl)-amine; [2-(methylphenylamino)-quinazolin-4-yl]-(5-methyl-2H-pyrazol-3-yl)-amine; (5-methyl-2H-pyrazol-3-yl)-{2-[N-methyl-N-(pyridin-3-ylmethyl)amino]-quinazolin-4-yl}-amine; (5-methyl-2H-pyrazol-3-yl)-(2-phenylamino-quinazolin-4-yl)-amine; (2-Benzylamino-quinazolin-4-yl)-(5-methyl-2H-pyrazol-3-yl)amine; (2-cyclohexylamino-quinazolin-4-yl)-(5-methyl-2H-pyrazol-3-yl)-amine; [2-(2,3-dihydrobenzo[1,4]dioxin-6-ylamino)-quinazolin-4-yl]-(5-methyl-2H-pyrazol-3-yl)-amine; (2-cyclohexylmethylamino-quinazolin-4-yl)-(5-methyl-2H-pyrazol-3-yl)-amine; [2-(1H-indazol-6-ylamino)-quinazolin-4-yl]-(5-methyl-2H-pyrazol-3-yl)-amine; (5-methyl-2H-pyrazol-3-yl)-[2-(pyridin-3-ylmethylamino)-quinazolin-4-yl]-amine; [2-(3-chlorophenylamino)-quinazolin-4-yl]-(5-methyl-2H-pyrazol-3-yl)-amine; [2-(4-chlorophenylamino)-quinazolin-4-yl]-(5-methyl-2H-pyrazol-3-yl)-amine; [2-(4-fluorobenzylamino)-quinazolin-4-yl]-(5-methyl-2H-pyrazol-3-yl)-amine; {2-[2-(2-hydroxyethyl)phenylamino]-quinazolin-4-yl}-(5-methyl-2H-pyrazol-3-yl)-amine; [2-(4-cyanomethylphenylamino)-quinazolin-4-yl]-(5-methyl-2H-pyrazol-3-yl)-amine; [2-(3-hydroxymethylphenylamino)-quinazolin-4-yl]-(5-methyl-2H-pyrazol-3-yl)-amine; [2-(3-hydroxyphenylamino)-quinazolin-4-yl]-(5-methyl-2H-pyrazol-3-yl)-amine; [2-(3-hydroxyphenylamino)-quinazolin-4-yl]-(5-methyl-2H-pyrazol-3-yl)-amine; (5-cyclopropyl-2H-pyrazol-3-yl)-(2-phenylamino-quinazolin-4-yl)-amine; (5-cyclopropyl-2H-pyrazol-3-yl)-[2-(3-methylphenylamino)-quinazolin-4-yl]-amine; (5-cyclopropyl-2H-pyrazol-3-yl)-[2-(6-methoxypyridin-3-ylamino)-quinazolin-4-yl]-amine; (5-cyclopropyl-2H-pyrazol-3-yl)-[2-(indan-5-ylamino)-quinazolin-4-yl]-amine; (5-cyclopropyl-2H-pyrazol-3-yl)-[2-(1H-indol-6-ylamino)-quinazolin-4-yl]-amine; [2-(4-acetamido-3-methylphenylamino)-quinazolin-4-yl]-(5-cyclopropyl-2H-pyrazol-3-yl)-amine; [2-(4-chloro-3-methylphenylamino)-quinazolin-4-yl]-(5-cyclopropyl-2H-pyrazol-3-yl)-amine; (5-cyclopropyl-2H-pyrazol-3-yl)-[2-(4-ethylphenylamino)-quinazolin-4-yl]-amine; (5-cyclopropyl-2H-pyrazol-3-yl)-[2-(4-propylphenylamino)-quinazolin-4-yl]-amine; (5-cyclopropyl-2H-pyrazol-3-yl)-{2-[4-(2-hydroxyethyl)phenylamino]-quinazolin-4-yl}-amine; (5-cyclopropyl-2H-pyrazol-3-yl)-(2-phenylamino-quinazolin-4-yl)-amine; [2-(2-cyclohexylethylamino)-quinazolin-4-yl]-(5-cyclopropyl-2H-pyrazol-3-yl)-amine; [2-(4-carboxymethoxyphenylamino)-quinazolin-4-yl]-(5-cyclopropyl-2H-pyrazol-3-yl)-amine; [2-(4-cyanomethylphenylamino)-quinazolin-4-yl]-(5-cyclopropyl-2H-pyrazol-3-yl)-amine; [2-(benzothiazol-6-ylamino)-quinazolin-4-yl]-(5-cyclopropyl-2H-pyrazol-3-yl)-amine; (5-cyclopropyl-2H-pyrazol-3-yl)-[2-(3,4-dimethylphenylamino)-quinazolin-4-yl]-amine; (5-cyclopropyl-2H-pyrazol-3-yl)-[2-(2-phenoxyethylamino)-quinazolin-4-yl]-amine; (5-cyclopropyl-2H-pyrazol-3-yl)-[2-(thiophen-2-methylamino)-quinazolin-4-yl]-amine; [2-(4-carboxymethylphenylamino)-quinazolin-4-yl]-(5-cyclopropyl-2H-pyrazol-3-yl)-amine; (5-cyclopropyl-2H-pyrazol-3-yl)-[2-(1H-indazol-5-ylamino)-quinazolin-4-yl]-amine; (5-cyclopropyl-2H-pyrazol-3-yl)-[2-(pyridin-3-ylmethylamino)-quinazolin-4-yl]-amine; (5-cyclopropyl-2H-pyrazol-3-yl)-[2-(3-methoxycarbonylphenylamino)-quinazolin-4-yl]-amine; [2-(3-carboxyphenylamino)-quinazolin-4-yl]-(5-cyclopropyl-2H-pyrazol-3-yl)-amine; (5-cyclopropyl-2H-pyrazol-3-yl)-[2-(3-ethylphenylamino)-quinazolin-4-yl]-amine; (5-cyclopropyl-2H-pyrazol-3-yl)-[2-(2,3-dimethylphenylamino)-quinazolin-4-yl]-amine; (5-cyclopropyl-2H-pyrazol-3-yl)-[2-(3,4-dimethoxyphenylamino)-quinazolin-4-yl]-amine; (5-cyclopropyl-2H-pyrazol-3-yl)-[2-(3-methoxyphenylamino)-quinazolin-4-yl]-amine; (5-methyl-2H-pyrazol-3-yl)-(2-phenylamino-5,6,7,8-tetrahydroquinazolinin-4-yl)-amine; [2-(biphenyl-3-ylamino)-quinazolin-4-yl]-(5-cyclopropyl-2H-pyrazol-3-yl)-amine; (5-cyclopropyl-2H-pyrazol-3-yl)-[2-(3-phenylprop-1-ylamino)-quinazolin-4-yl]-amine; [2-(4-acetamido-3-methylphenylamino)-quinazolin-4-yl]-(5-methyl-2H-pyrazo-1-3-yl)-amine; (5-cyclopropyl-2H-pyrazol-3-yl)-[2-(indan-2-ylamino)-quinazolin-4-yl]-amine; [2-(3-methylphenylamino)-quinazolin-4-yl]-(5-methyl-2H-pyrazol-3-yl)-amine; [2-(2-chloro-5-methylphenylamino)-quinazolin-4-yl]-(5-methyl-2H-pyrazol-3-yl)-amine; (5-cyclopropyl-2H-pyrazol-3-yl)-{2-[4-(morpholin-1-yl)phenylamino]-quinazolin-4-yl}-amine; [2-(benzothiazol-6-ylamino)-quinazolin-4-yl]-(5-methyl-2H-pyrazol-3-yl)-amine; [2-(3,4-dimethylphenylamino)-quinazolin-4-yl]-(5-methyl-2H-pyrazol-3-yl)-amine; [2-(3-ethylphenylamino)-quinazolin-4-yl]-(5-methyl-2H-pyrazol-3-yl)-amine; [2-(3-methoxyphenylamino)-quinazolin-4-yl]-(5-methyl-2H-pyrazol-3-yl)-amine; [2-(4-acetamido-3-cyanophenylamino)-quinazolin-4-yl]-(5-methyl-2H-pyrazol-3-yl)-amine ; [2-(2-methoxybiphenyl-5-ylamino)-quinazolin-4-yl]-(5-methyl-2H-pyrazol-3-yl)-amine; [2-(4-acetamidophenylamino)-quinazolin-4-yl]-(5-methyl-2H-pyrazol-3-yl)-amine; [2-(4-tert-butoxycarbonylamino-phenylamino)-quinazolin-4-yl]-(5-methyl-2H-pyrazol-3-yl)-amine; [2-(4-cyanophenylamino)-quinazolin-4-yl]-(5-methyl-2H-pyrazol-3-yl)-amine; (5-methyl-2H-pyrazol-3-yl)-[2-(6-oxo-6,10-dihydro-4aH-benzo[c]chromen-2-ylamino)-quinazolin-4-yl]-amine; [2-(biphenyl-3-ylamino)-quinazolin-4-yl]-(5-methyl-2H-pyrazol-3-yl)-amine; [2-(4-methoxycarbonylmethyl-3-methylphenylamino)-quinazolin-4-yl]-(5-methyl-2H-pyrazol-3-yl)-amine; [2-(4-carboxymethyl-3-methylphenylamino)-quinazolin-4-yl]-(5-methyl-2H-pyrazol-3-yl)-amine; [2-(4-aminophenylamino)-quinazolin-4-yl]-(5-methyl-2H-pyrazol-3-yl)-amine; [2-(4-bromophenylamino)-quinazolin-4-yl]-(5-methyl-2H-pyrazol-3-yl)-amine; [2-(4-isobutyrylamino-phenylamino)-quinazolin-4-yl]-(5-methyl-2H-pyrazol-3-yl)-amine; (5-ethyl-2H-pyrazol-3-yl)-[2-(5-ethyl-2H-pyrazol-3-ylamino)-quinazolin-4-yl]-amine; (1H-indazol-3-yl)-(2-phenylamino-quinazolin-4-yl)-amine; (1H-indazol-3-yl)-[2-(3-trifluoromethylphenylamino)-quinazolin-4-yl]-amine; (1H-indazol-3-yl)-[2-(4-trifluoromethylphenylamino)-quinazolin-4-yl]-amine; [2-(adamantan-2-ylamino)-quinazolin-4-yl]-(1H-indazol-3-yl)-amine; (1H-indazol-3-yl)-(2-methyl-phenyl-amino-quinazolin-4-yl)-amine; [2-(2-chloro-phenyl)-amino-quinazolin-4-yl]-(1H-indazol-3-yl)-amine; (1H-indazol-3-yl)-[2-(2-trifluoromethylphenylamino)-quinazolin-4-yl]-amine; [2-(4-cyanomethylphenylamino)-quinazolin-4-yl]-(1H-indazol-3-yl)-amine; [2-(4-chlorophenylamino)-5,6,7,8-tetrahydroquinazolinin-4-yl]-(5-methyl-2H-pyrazol-3-yl)-amine; (5-methyl-2H-pyrazol-3-yl)-(2-phenylamino-6,7,8,9-tetrahydro-5H-cycloheptapyrimidin-4-yl)-amine; [2-(benzimidazol-2-ylamino)-7-benzyl-5,6,7,8-tetrahydro-pyrido[3,4-d]pyrimidin-4-yl]-(5-methyl-2H-pyrazol-3-yl)-amine; (7-benzyl-2-phenylamino-5,6,7,8-tetrahydro-pyrido[3,4-d]pyrimidin-4-yl)-(5-methyl-2H-pyrazol-3-yl)-amine; [6-benzyl-2-(4-chlorophenylamino)-5,6,7,8-tetrahydro-pyrido[4,3-d]pyrimidin-4-yl]-(5-methyl-2H-pyrazol-3-yl)-amine; [2-(benzimidazol-2-ylamino)-6-benzyl-5,6,7,8-tetrahydro-pyrido[4,3-d]pyrimidin-4-yl]-(5-methyl-2H-pyrazol-3-yl)-amine; (6-benzyl-2-phenylamino-5,6,7,8-tetrahydro-pyrido[4,3-d]pyrimidin-4-yl)-(5-methyl-2H-pyrazol-3-yl)-amine; (5-methyl-2H-pyrazol-3-yl)-(2-phenylamino-5,6,7,8-tetrahydro-pyrido[3,4-d]pyrimidin-4-yl)-amine; [2-(4-cyanomethylphenylamino)-quinazolin-4-yl]-(1H-pyrazolo[3,4-b]pyridin-3-yl)-amine; [2-(4-cyanobenzylamino)-quinazolin-4-yl]-(1H-pyrazolo[3,4-b]pyridin-3-yl)-amine; [2-(4-cyanomethylphenylamino)-quinazolin-4-yl]-(4-fluoro-1H-indazol-3-yl)-amine; [2-(4-cyanophenylamino)-quinazolin-4-yl]-(1H-indazol-3-yl)-amine; and [2-(4-cyanobenzylamino)-quinazolin-4-yl]-(1H-indazol-3-yl)-amine. United States Patent No. 6,987,116 to Boschelli et al., describes thieno[3,2-b]pyridine-6-carbonitriles and thieno[2,3-b]pyridine-5-carbonitriles as Src inhibitors. United States Patent No. 6,696,452 to Davies et al., describes pyrazole compounds as Src inhibitors. United States Patent No. 6,664,247 to Bebbington et al., describes pyrazole compounde as Src inhibitors, including (5-cyclopropyl-2H-pyrazol-3-yl)-[2-(naphtalen-2-ylsulfanyl)-6-phenylpyrimidin-4-yl]-amine; (5-cyclopropyl-2H-pyrazol-3-yl)-[2-(3-methoxycarbonyl-phenylylsulfanyl)-6-phenylpyrimidin-4-yl]-amine; (5-cyclopropyl-2H-pyrazol-3-yl)-[2-(naphthalen-2-ylsulfanyl)-pyrimidin-4-yl]-amine; (5-cyclopropyl-2H-pyrazol-3-yl)-[5,6-dimethyl-2-(naphthalen-2-ylsulfanyl)-pyrimidin-4-yl]-amine; (5-cyclopropyl-2H-pyrazol-3-yl)-[5-methyl-2-(naphthalen-2-ylsulfanyl)-pyrimidin-4-yl]-amine; (5-cyclopropyl-2H-pyrazol-3-yl)-[6-methyl-2-(naphthalen-2-ylsulfanyl)-pyrimidin-4-yl]-amine; (5-cyclopropyl-2H-pyrazol-3-yl)-[6-(morpholin-4-yl)-2-(naphthalen-2-ylsulfanyl)-pyrimidin-4-yl]-amine; (5-cyclopropyl-2H-pyrazol-3-yl)-[6-(1-methylpiperazin-4-yl)-2-(naphthalen-2yl-sulfanyl)-pyrimidin-4-yl]-amine; [6-(2,6-dimethylphenyl)-2-(naphthalen-2-yl-sulfanyl)-pyrimidin-4-yl]-(5-methyl-2H-pyrazol-3-yl)-amine; [6-(2-methylphenyl)-2-(naphthalen-2-ylsulfanyl)-pyrimidin-4-yl]-(5-methyl-2H-pyrazol-3-yl)-amine; [2-(4-acetamido-phenylsulfanyl)-6-phenyl-pyrimidin-4-yl]-(5-methyl-2H-pyrazol-3-yl)-amine; (5-methyl-2H-pyrazol-3-yl)-[2-(naphthalen-2-ylsulfanyl)-6-phenyl-pyrimidin-4-yl]-amine; [2-(4-isobutyrylylamino-phenylsulfanyl)-6-phenylpyrimidin-4-yl]-(5-methyl-2H-pyrazol-3-yl)-amine; [6-(4-methylpiperazin-1-yl)-2-methylsulfanyl-pyrimidin-4-yl]-(5-methyl-2H-pyrazol-3-yl)-amine; (5-methyl-2H-pyrazol-3-yl)-[6-phenyl-2-(4-propionylamino-phenylsulfanyl)-pyrimidin-4-yl]-amine; [2-(4-cyclopropanecarbonylamino-phenylsulfanyl)-6-phenylpyrimidin-4-yl]-(5-methyl-2H-pyrazol-3-yl)-amine; (5-methyl-2H-pyrazol-3-yl)-{6-phenyl-2-[4-(propane-1-sulfonylamino)-phenylsulfanyl]-pyrimidin-4-yl}-amine; [2-(4-ethanesulfonylamino-phenylsulfanyl)-6-phenyl-pyrimidin-4-yl]-(5-methyl-2H-pyrazol-3-yl)-amine; [2-(4-acetamidophenyl-sulfanyl)-6-(2-methylphenyl)-pyrimidin-4-yl]-(5-methyl-2H-pyrazol-3-yl)-amine; [2-(4-isobutanecarbonylamino-phenyl-sulfanyl)-6-phenyl-pyrimidin-4-yl]-(5-methyl-2H-pyrazol-3-yl)-amine; [2-(4-acetamido-phenyl-sulfanyl)-5-methyl-6-phenyl-pyrimidin-4-yl]-(5-methyl-2H-pyrazol-3-yl)-amine; [2-(4-acetamido-phenyl-sulfanyl)-6-(4-methoxyphenyl)-pyrimidin-4-yl]-(5-methyl-2H-pyrazol-3-yl)-amine; [6-(3-acetamidophenyl)-2-(4-acetamido-phenyl-sulfanyl)-pyrimidin-4-yl]-(5-methyl-2H-pyrazol-3-yl)-amine; [2-(4-isopropanesulfonylamino-phenyl-sulfanyl)-6-phenyl-pyrimidin-4-yl]-(5-methyl-2H-pyrazol-3-yl)-amine; {2-[4-(2-dimethylamino-acetylamino)-phenylsulfanyl]-6-phenyl-pyrimidin-4-yl}-(5-methyl-2H-pyrazol-3-yl)-amine; [2-(3-chloro-benzylsulfanyl)-6-morpholin-4-yl-pyrimidin-4-yl]-(5-methyl-2H- pyrazol-3-yl)-amine; [2-(3-chloro-benzylsulfanyl)-6-(2-methoxy-ethylamino)-pyrimidin-4-yl]-(5-methyl-2H-pyrazol-3-yl)-amine; [2-benzylsulfanyl-6-(4-methylpiperazin-1-yl)-pyrimidin-4-yl]-(5-methyl-2H-pyrazol-3-yl)-amine; [2-benzylsulfanyl-6-morpholin-4-yl-pyrimidin-4-yl]-(5-methyl-2H-pyrazol-3-yl)-amine; [2-(3-chloro-benzylsulfanyl)-6-(4-methylpiperazin-1-yl)-pyrimidin-4-yl]-(5-methyl-2H-pyrazol-3-yl)-amine; [2-(4-methoxy-benzylsulfanyl)-6-(4-methylpiperazin-1-yl)-pyrimidin-4-yl]-(5-methyl-2H-pyrazol-3-yl)-amine; [2-(4-acetamidophenyl-sulfanyl)-6-tert-butyl-pyrimidin-4-yl]-(5-methyl-2H-pyrazol-3-yl)-amine; (5-cyclopropyl-2H-pyrazol-3-yl)-[6-phenyl-2-(4-propionylamino-phenyl-sulfanyl)-pyrimidin-4-yl]-amine; [2-(3-chloro-benzylsulfanyl)-6-(piperidin-1-yl)-pyrimidin-4-yl]-(5-methyl-2H-pyrazol-3-yl)-amine; (5-methyl-2H-pyrazol-3-yl)-{2-[4-(morpholinesulfonyl)-benzylsulfanyl]-6-morpholin-4-yl-pyrimidin-4-yl}-amine; {6-(2-methoxy-ethylamino)-2-[4-(morpholinesulfonyl)-benzylsulfanyl]-pyrimidin-4-yl}-(5-methyl-2H-pyrazol-3-yl)-amine; {6-(4-methylpiperazin-1-yl)-2-[4-(morpholinesulfonyl)-benzylsulfanyl]-pyrimidin-4-yl}-(5-methyl-2H-pyrazol-3-yl)-amine; [6-methoxymethyl-2-(4-propionylamino-phenyl-sulfanyl)-pyrimidin-4-yl]-(5-methyl-2H-pyrazol-3-yl)-amine; [2-(4-methoxycarbonyl-phenyl-sulfanyl)-6-methoxymethyl-pyrimidin-4-yl]-(5-methyl-2H-pyrazol-3-yl)-amine; [2-(3,5-dimethoxy-benzylsulfanyl)-6-morpholin-4-yl-pyrimidin-4-yl]-(5-methyl-2H-pyrazol-3-yl)-amine; [2-(3,5-dimethoxy-benzylsulfanyl)-6-pyrrolidin-4-yl-pyrimidin-4-yl]-(5-methyl-2H-pyrazol-3-yl)-amine; (5-methyl-2H-pyrazol-3-yl)-[6-morpholin-4-yl-2-(naphthalene-2-yl-methylsulfanyl)-pyrimidin-4-yl]-amine; {2-(4-acetamido-phenyl-sulfanyl)-6-[4-(3-dimethylamino-propoxy)phenyl]-pyrimidin-4-yl}-(5-methyl-2H-pyrazol-3-yl)-amine; [2-(4-acetamidophenylsulfanyl)-6-(morpholin-4-yl)-pyrimidin-4-yl]-(5-methyl-2H-pyrazol-3-yl)-amine; [6-hydroxymethyl-2-(4-propionylamino-phenyl-sulfanyl)-pyrimidin-4-yl]-(5-methyl-2H-pyrazol-3-yl)-amine; [2-(4-acetamido-phenyl-sulfanyl)-pyrimidin-4-yl]-(5-methyl-2H-pyrazol-3-yl)-amine; [6-(1-butoxycarbonyl)-2-(4-propionylamino-phenyl-sulfanyl)pyrimidin-4-yl]-(5-methyl-2H-pyrazol-3-yl)-amine; and [6-methoxycarbonyl-2-(4-propionylamino-phenyl-sulfanyl)-pyrimidin-4-yl]-(5-methyl-2H-pyrazol-3-yl)-amine. United States Patent No. 6,660,731 to Bebbington et al., describes pyrazole compounds as Src inhibitors. United States Patent No. 6,653,301 to Bebbington et al., describes pyrazole compounds as Src inhibitors, including (5-cyclopropyl-2H-pyrazol-3-yl)-[2-(naphtalen-2-ylsulfanyl)-6-phenylpyrimidin-4-yl]-amine; (5-cyclopropyl-2H-pyrazol-3-yl)-[2-(3-methoxycarbonyl-phenylylsulfanyl)-6-phenylpyrimidin-4-yl]-amine; (5-cyclopropyl-2H-pyrazol-3-yl)-[2-(naphthalen-2-ylsulfanyl)-pyrimidin-4-yl]-amine; (5-cyclopropyl-2H-pyrazol-3-yl)-[5,6-dimethyl-2-(naphthalen-2-ylsulfanyl)-pyrimidin-4-yl]-amine; (5-cyclopropyl-2H-pyrazol-3-yl)-[5-methyl-2-(naphthalen-2-ylsulfanyl)-pyrimidin-4-yl]-amine; (5-cyclopropyl-2H-pyrazol-3-yl)-[6-methyl-2-(naphthalen-2-ylsulfanyl)-pyrimidin-4-yl]-amine; (5-cyclopropyl-2H-pyrazol-3-yl)-[6-(morpholin-4-yl)-2-(naphthalen-2-ylsulfanyl)-pyrimidin-4-yl]-amine; (5-cyclopropyl-2H-pyrazol-3-yl)-[6-(1-methylpiperazin-4-yl)-2-(naphthalen-2-ylsulfanyl)-pyrimidin-4-yl]-amine; [6-(2,6-dimethylphenyl)-2-(naphthalen-2-ylsulfanyl)-pyrimidin-4-yl]-(5-methyl-2H-pyrazol-3-yl)-amine; [6-(2-methylphenyl)-2-(naphthalen-2-ylsulfanyl)-pyrimidin-4-yl]-(5-methyl-2H-pyrazol-3-yl)-amine; [2-(4-acetamido-phenylsulfanyl)-6-phenyl-pyrimidin-4-yl]-(5-methyl-2H-pyrazol-3-yl)-amine; (5-methyl-2H-pyrazol-3-yl)-[2-(naphthalen-2-ylsulfanyl)-6-phenyl-pyrimidin-4-yl]-amine; [2-(4-isobutyrylamino-phenylsulfanyl)-6-phenylpyrimidin-4-yl]-(5-methyl-2 H-pyrazol-3-yl)-amine; [6-(4-methylpiperazin-1-yl)-2-methylsulfanyl-pyrimidin-4-yl]-(5-methyl-2H-pyrazol-3-yl)-amine; (5-methyl-2H-pyrazol-3-yl)-[6-phenyl-2-(4-propionylamino-phenylsulfanyl)-pyrimidin-4-yl]-amine; [2-(4-cyclopropanecarbonylamino-phenylsulfanyl)-6-phenylpyrimidin-4-yl]-(5-methyl-2H-pyrazol-3-yl)-amine; (5-methyl-2H-pyrazol-3-yl)-{6-phenyl-2-[4-(propane-1-sulfonylamino)-phenylsulfanyl]-pyrimidin-4-yl}-amine; [2-(4-ethanesulfonylamino-phenylsulfanyl)-6-phenyl-pyrimidin-4-yl]-(5-methyl-2H-pyrazol-3-yl)-amine; [2-(4-acetamidophenyl-sulfanyl)-6-(2-methylphenyl)-pyrimidin-4-yl]-(5-methy l-2H-pyrazol-3-yl)-amine; [2-(4-isobutanecarbonylamino-phenyl-sulfanyl)-6-phenyl-pyrimidin-4-yl]-(5-methyl-2H-pyrazol-3-yl)-amine; [2-(4-acetamido-phenyl-sulfanyl)-5-methyl-6-phenyl-pyrimidin-4-yl]-(5-methyl-2H-pyrazol-3-yl)-amine; [2-(4-acetamido-phenyl-sulfanyl)-6-(4-methoxyphenyl)-pyrimidin-4-yl]-(5-methyl-2H-pyrazol-3-yl)-amine; [6-(3-acetamidophenyl)-2-(4-acetamido-phenyl-sulfanyl)-pyrimidin-4-yl]-(5-methyl-2H-pyrazol-3-yl)-amine; [2-(4-isopropanesulfonylamino-phenyl-sulfanyl)-6-phenyl-pyrimidin-4-yl]-(5-methyl-2H-pyrazol-3-yl)-amine; {2-[4-(2-dimethylamino-acetylamino)-phenylsulfanyl]-6-phenyl-pyrimidin-4-yl}-(5-methyl-2H-pyrazol-3-yl)-amine; [2-(3-chloro-benzylsulfanyl)-6-morpholin-4-yl-pyrimidin-4-yl]-(5-methyl-2H-pyrazol-3-yl)-amine; [2-(3-chloro-benzylsulfanyl)-6-(2-methoxy-ethylamino)-pyrimidin-4-yl]-(5-methyl-2H-pyrazol-3-yl)-amine; [2-benzylsulfanyl-6-(4-methylpiperazin-1-yl)-pyrimidin-4-yl]-(5-methyl-2H-pyrazol-3-yl)-amine; [2-benzylsulfanyl-6-morpholin-4-yl-pyrimidin-4-yl]-(5-methyl-2H-pyrazol-3-yl)-amine; [2-(3-chloro-benzylsulfanyl)-6-(4-methylpiperazin-1-yl)-pyrimidin-4-yl]-(5-methyl-2H-pyrazol-3-yl)-amine; [2-(4-methoxy-benzylsulfanyl)-6-(4-methylpiperazin-1-yl)-pyrimidin-4-yl]-(5-methyl-2H-pyrazol-3-yl)-amine; [2-(4-acetamidophenyl-sulfanyl)-6-tert-butyl-pyrimidin-4-yl]-(5-methyl-2H-pyrazol-3-yl)-amine; (5-cyclopropyl-2H-pyrazol-3-yl)-[6-phenyl-2-(4-propionylamino-phenyl-sulfanyl)-pyrimidin-4-yl]-amine; [2-(3-chloro-benzylsulfanyl)-6-(piperidin-1-yl)-pyrimidin-4-yl]-(5-methyl-2H-pyrazol-3-yl)-amine; (5-methyl-2H-pyrazol-3-yl)-{2-[4-(morpholinesulfonyl)-benzylsulfanyl]-6-morpholin-4-yl-pyrimidin-4-yl}-amine; {6-(2-methoxy-ethylamino)-2-[4-(morpholinesulfonyl)-benzylsulfanyl]-pyrimidin-4-yl}-(5-methyl-2H-pyrazol-3-yl)-amine; {6-(4-methylpiperazin-1-yl)-2-[4-(morpholinesulfonyl)-benzylsulfanyl]-pynmidin-4-yl}-(5-methyl-2H-pyrazol-3-yl)-amine; [6-methoxymethyl-2-(4-propionylamino-phenyl-sulfanyl)-pyrimidin-4-yl]-(5-methyl-2H-pyrazol-3-yl)-amine; [2-(4-methoxycarbonyl-phenyl-sulfanyl)-6-methoxymethyl-pyrimidin-4-yl]-(5-methyl-2H-pyrazol-3-yl)-amine; [2-(3,5-dimethoxy-benzylsulfanyl)-6-morpholin-4-yl-pyrimidin-4-yl]-(5-methyl-2H-pyrazol-3-yl)-amine; [2-(3,5-dimethoxy-benzylsulfanyl)-6-pyrrolidin-4-yl-pyrimidin-4-yl]-(5-methyl-2H-pyrazol-3-yl)-amine; (5-methyl-2H-pyrazol-3-yl)-[6-morpholin-4-yl-2-(naphthalene-2-yl-methylsulfanyl)-pyrimidin-4-yl]-amine; {2-(4-acetamido-phenyl-sulfanyl)-6-[4-(3-dimethylamino-propoxy)phenyl]-pyrimidin-4-yl}-(5-methyl-2H-pyrazol-3-yl)-amine; [2-(4-acetamidophenylsulfanyl)-6-(morpholin-4-yl)-pyrimidin-4-yl]-(5-methyl-2H-pyrazol-3-yl)-amine; [6-hydroxymethyl-2-(4-propionylamino-phenyl-sulfanyl)-pyrimidin-4-yl]-(5-methyl-2H-pyrazol-3-yl)-amine; [2-(4-acetamido-phenyl-sulfanyl)-pyrimidin-4-yl]-(5-methyl-2H-pyrazol-3-yl)-amine; [6-(1-butoxycarbonyl)-2-(4-propionylamino-phenyl-sulfanyl)pyrimidin-4-yl]-(5-methyl-2H-pyrazol-3-yl)-amine; and [6-methoxycarbonyl-2-(4-propionylamino-phenyl-sulfanyl)-pyrimidin-4-yl]-(5-methyl-2H-pyrazol-3-yl)-amine. United States Patent No. 6,653,300 to Bebbington et al., describes pyrazole compounds as Src inhibitors, including (5-methyl-2H-pyrazol-3-yl)-[2-(naphthalen-2-yloxy)-quinazolin-4-yl]-amine; (5-methyl-2H-pyrazol-3-yl)-(2-phenoxy-quinazolin-4-yl)-amine; (5-cyclopropyl-2H-pyrazol-3-yl)-[2-(5,6,7,8-tetrahydronaphthalen-2-yloxy)-quinazolin-4-yl]-amine; (5-cyclopropyl-2H-pyrazol-3-yl)-[2-(3-methylphenoxy)-quinazolin-4-yl]-amine; [2-(3-methoxyphenoxy)-quinazolin-4-yl]-(5-methyl-2H-pyrazol-3-yl)-amine; [2-(3,4-dimethoxyphenoxy)-quinazolin-4-yl]-(5-methyl-2H-pyrazol-3-yl)-amine; [2-(benzo[1,3]dioxol-5-yloxy)-quinazolin-4-yl]-(5-methyl-2H-pyrazol-3-yl)-amine; [2-(3-methoxycarbonylphenoxy)-quinazolin-4-yl]-(5-methyl-2H-pyrazol-3-yl)-amine; (5-cyclopropyl-2H-pyrazol-3-yl)-(2-phenoxymethyl-quinazolin-4-yl)-amine; (2-benzyloxymethyl-quinazolin-4-yl)-(5-cyclopropyl-2H-pyrazol-3-yl)-amine; (2-benzyl-quinazolin-4-yl)-(5-cyclopropyl-2H-pyrazol-3-yl)-amine; (5-cyclopropyl-2H-pyrazol-3-yl)-(2-methyl-quinazolin-4-yl)-amine; [2-(4-chlorophenoxymethyl)-6,7,8,9-tetrahydro-5H-cycloheptapyrimidin-4-yl]-(5-methyl-2H-pyrazol-3-yl)-amine; [2-(4-chlorophenoxymethyl)-5,6,7,8-tetrahydro-quinazolin-4-yl]-(5-methyl-2H-pyrazol-3-yl)-amine; [2-(2-chlorophenoxymethyl)-6-methyl-pyrimidin-4-yl]-(5-phenyl-2H-pyrazol-3-yl)-amine; [2-(2-chlorophenoxymethyl)-6-methyl-pyrimidin-4-yl]-[5-(furan-2-yl)-2H-pyrazol-3-yl]-amine; (6-methyl-2-phenoxymethyl-pyrimidin-4-yl)-(5-phenyl-2H-pyrazol-3-yl)-amine; [5-(furan-2-yl)-2H-pyrazol-3-yl]-(6-methyl-2-phenoxymethyl-pyrimidin-4-yl)-amine; [5-(furan-2-yl)-2H-pyrazol-3-yl]-(6-methyl-2-phenylsulfanylmethyl-pyrimidin-4-yl)-amine; [6-methyl-2-(4-methyl-phenylsulfanylmethyl)-pyrimidin-4-yl]-(5-phenyl-2H-pyrazol-3-yl)-amine; [5-(furan-2-yl)-2H-pyrazol-3-yl]-[6-methyl-2-(4-methyl-phenylsulfanylmethyl)-pyrimidin-4-yl]-amine; [2-(4-fluoro-phenoxymethyl)-6-methyl-pyrimidin-4-yl]-(5-phenyl-2H-pyrazol-3-yl)-amine; [2-(4-fluoro-phenoxymethyl)-6-methyl-pyrimidin-4-yl]-[5-(furan-2-yl)-2H-pyrazol-3-yl]-amine; (6-ethyl-2-phenylsulfanylmethyl-pyrimidin-4-yl)-(5-methyl-2H-pyrazol-3-yl)-amine; (6-ethyl-2-phenoxymethyl-pyrimidin-4-yl)-(5-methyl-2H-pyrazol-3-yl)-amine; [6-ethyl-2-(4-fluorophenoxymethyl)-pyrimidin-4-yl]-(5-methyl-2H-pyrazol-3-yl)-amine; [6-ethyl-2-(1-methyl-1-phenyl-ethyl)-pyrimidin-4-yl]-(5-methyl-2H-pyrazol-3-yl)-amine; [2-(4-chlororophenoxymethyl)-6-methyl-pyrimidin-4-yl]-(5-phenyl-2H-pyrazol-3-yl)-amine; [2-(4-chlororophenoxymethyl)-6-methyl-pyrimidin-4-yl]-(5-methyl-2H-pyrazol-3-yl)-amine; [2-(4-chlororophenoxymethyl)-6-methoxymethyl-pyrimidin-4-yl]-(5-methyl-2H-pyrazol-3-yl)-amine; [2-(4-chlororophenoxymethyl)-6-methy-pyrimidin-4-yl]-[5-(furan-2-yl)-2H-pyrazol-3-yl]-amine; (5-methyl-2H-pyrazol-3-yl)-(2-phenylsulfanylmethyl-5,6,7,8-tetrahydro-quinazolin-4-yl)-amine; [2-(4-methylphenylsulfanylmethyl)-6,7,8,9-tetrahydro-5H-cycloheptapyrimidin-4-yl]-(5-methyl-2H-pyrazol-3-yl)-amine; [2-(1-methyl-1-phenyl-ethyl)-6,7,8,9-tetrahydro-5H-cycloheptapyrimidin-4-yl]-(5-methyl-2H-pyrazol-3-yl)-amine; [2-(2,6-dichlorobenzyl)-5,6,7,8-tetrahydro-quinazolin-4-yl]-(5-methyl-2H-pyrazol-3-yl)-amine; [7-benzyl-2-(2,6-dichlorobenzyl)-5,6,7,8-tetrahydropyrido[3,4-d]pyrimidin-4-yl]-(5-methyl-2H-pyrazol-3-yl)-amine; [6-benzyl-2-(4-chlorophenoxymethyl)-5,6,7,8-tetrahydro-pyrido[4,3-d]pyrimidin-4-yl]-(5-methyl-2H-pyrazol-3-yl)-amine; [2-(4-chlorophenoxymethyl)-5,6,7,8-tetrahydro-pyrido[4,3-d]pyrimidin-4-yl]-(5-methyl-2H-pyrazol-3-yl)-amine; [2-(2,6-dichlorobenzyl)-6-methyl-pyrimidin-4-yl]-(5-methyl-2H-pyrazol-3-yl)-amine; [2-(2,6-dichlorobenzyl)-5,6-dimethyl-pyrimidin-4-yl]-(5-methyl-2H-pyrazol-3-yl)-amine; (1H-indazol-3-yl)-[2-(2-phenyl-cyclopropyl)-quinazolin-4-yl]-amine; (7-fluoro-1H-indazol-3-yl)-[2-(2-phenyl-cyclopropyl)-quinazolin-4-yl]-amine; (5-fluoro-1H-indazol-3-yl)-[2-(2-phenylcyclopropyl)-quinazolin-4-yl]-amine; (5-methyl-1H-pyrazol-3-yl)-[2-(2-phenylcyclopropyl)-quinazolin-4-yl]-amine; [6-ethyl-2-(1-methyl-1-phenyl-ethyl)-pyrimidin-4-yl]-(5-methyl-1H-pyrazol-3-yl)-amine; [6-methyl-2-(1-methyl-1-phenyl-ethyl)-pyrimidin-4-yl]-(5-methyl-1H-pyrazol-3-yl)-amine; [6-methyl-2-(1-phenyl-cyclopropyl)-pyrimidin-4-yl]-(5-methyl-1H-pyrazol-3-yl)-amine; and [6-ethyl-2-(1-methyl-1-phenyl-propyl)-pyrimidin-4-yl]-(5-methyl-1 H-pyrazol-3-yl)-amine. United States Patent No. 6,638,926 to Davies et al., describes pyrazole compounds as Src inhibitors. United States Patent No. 6,613,736 to Knegtel et al., describes pyrazole compounds as Src inhibitors. United States Patent No. 6,610,677 to Davies et al., describes pyrazole compounds as Src inhibitors. United States Patent No. 6,503,914 to Benish et al., describes thienopyrimidine compounds as Src inhibitors, including benzaldehyde(7-methylthieno[3,2-d]pyrimidin-4-yl)hydrazone; 4-methoxybenzaldehyde(7-methylthieno[3,2-d]pyrimidin-4-yl)hydrazone; 4-pyridinecarboxaldehyde(7-methylthieno[3,2-d]pyrimidin-4-yl)hydrazone; 3,4-dimethoxybenzaldehyde(7-methylthieno[3,2-d]pyrimidin-4-yl)hydrazone; 3,5-dimethoxybenzaldehyde(7-methylthieno[3,2-d]pyrimidin-4-yl)hydrazone; 3-chlorobenzaldehyde(7-methylthieno[3,2-d]pyrimidin-4-yl)hydrazone; 3,4-dihydroxybenzaldehyde(7-methylthieno[3,2-d]pyrimidin-4-yl)hydrazone; 3-pyridinecarboxaldehyde(7-methylthieno[3,2-d]pyrimidin-4-yl)hydrazone; 2-thiophenecarboxaldehyde(7-methylthieno[3,2-d]pyrimidin-4-yl)hydrazone; 1H-pyrrole-2-carboxaldehyde(7-methylthieno[3,2-d]pyrimidin-4-yl)hydrazone; 2-furancarboxaldehyde(7-methylthieno[3,2-d]pyrimidin-4-yl)hydrazone; 3-hydroxybenzaldehyde(7-methylthieno[3,2-d]pyrimidin-4-yl)hydrazone; 3-thiophenecarboxaldehyde(7-methylthieno[3,2-d]pyrimidin-4-yl)hydrazone; 1H-imidazole-2-carboxaldehyde(7-methylthieno[3,2-d]pyrimidin-4-yl)hydrazone; 4-ethoxybenzaldehyde(7-methylthieno[3,2-d]pyrimidin-4-yl)hydrazone; 4-hydroxy-3-nitrobenzaldehyde(7-methylthieno[3,2-d]pyrimidin-4-yl)hydrazone; 3-ethoxy-4-hydroxybenzaldehyde(7-methylthieno[3,2-d]pyrimidin-4-yl)hydrazone; 3-hydroxy-4-methoxybenzaldehyde(7-methylthieno[3,2-d]pyrimidin-4-yl)hydrazone; 3-fluorobenzaldehyde(7-methylthieno[3,2-d]pyrimidin-4-yl)hydrazone; 4-hydroxy-3-methoxybenzaldehyde(7-methylthieno[3,2-d]pyrimidin-4-yl)hydrazone; 3-chloro-4-hydroxybenzaldehyde(7-methylthieno[3,2-d]pyrimidin-4-yl)hydrazone; 4-fluorobenzaldehyde(6-phenylthieno[3,2-d]pyrimidin-4-yl)-hydrazone; 3-pyridinecarboxaldehyde(thieno[3,2-d]pyrimidin-4-yl)hydrazone; 5-methyl-1H-imidazole-4-carboxaldehyde(7-methylthieno[3,2-d]pyrimidin-4-yl)hydrazone; 5-methyl-2-thiophenecarboxaldehyde(7-methylthieno[3,2-d]pyrimidin-4-yl)hydrazone; 4-cyanobenzaldehyde(7-methylthieno[3,2-d]pyrimidin-4-yl)hydrazone; 3-cyanobenzaldehyde(7-methylthieno[3,2-d]pyrimidin-4-yl)hydrazone; 3-methoxybenzaldehyde(7-methylthieno[3,2-d]pyrimidin-4-yl)hydrazone; 3-ethoxybenzaldehyde(7-methylthieno[3,2-d]pyrimidin-4-yl)hydrazone; cyclopropanecarboxaldehyde(7-methylthieno[3,2-d]pyrimidin-4-yl)hydrazone; 3-pyridinecarboxaldehyde(7-bromothieno[3,2-d]pyrimidin-4-yl)hydrazone; and 3-pyridinecarboxaldehyde(6-phenylthieno[3,2-d]pyrimidin-4-yl)hydrazone. United States Patent No. 6,100,254 to Budde et al., describes compounds based on a 1,4-benzodiazepin-2-one nucleus as Src inhibitors.

The method described herein can further comprise the step of administering a therapeutically effective quantity of a BH3 mimetic to the subject. Alternatively, the method described herein can further comprise the step of administering therapeutically effective quantity of a tyrosine kinase inhibitor to the subject.

A medicament for use in a method for the treatment of a malignancy in a subject suffering from a malignancy who has a germline deletion polymorphism conferringresistance to thymidine kinase inhibitors (TKIs) the medicament comprising: selected from the group consisting of dianhydrogalactitol, a derivative or analog of dianhydrogalactitol, diacetyldianhydrogalactitol, a derivative or analog of diacetyldianhydrogalactitol, dibromodulcitol, and a derivative or analog of dibromodulcitol to the subject to treat the malignancy; and (2) a therapeutically effective quantity of a combination of kinase inhibitors to the subject to treat the malignancy; wherein the combination of kinase inhibitors is a combination selected from the group consisting of: (1) a JAK2 inhibitor and a STAT5 inhibitor; (2) a JAK2 inhibitor and a Src inhibitor; (3) a STAT5 inhibitor and a Src inhibitor; and (4) a JAK2 inhibitor, a STAT5 inhibitor, and a Src inhibitor is described herein.

The method described herein can further comprise the step of administering a therapeutically effective quantity of a BH3 mimetic to the subject. Alternatively, the method can further comprise the step of administering therapeutically effective quantity of a tyrosine kinase inhibitor to the subject.

The description of a particular kinase inhibitor as a JAK2 inhibitor, a STAT5 inhibitor, or a Src inhibitor does not preclude the kinase inhibitor described herein being referenced or described having inhibitory activity toward another kinase, such as one or more of JAK2, STAT5, Src, or the kinase activity associated with the BCR-ABL fusion protein.

When the improvement described herein is made by analysis of patient or disease phenotype, the analysis of patient or disease phenotype can be, but is not limited to, a method of analysis of patient or disease phenotype carried out by a method selected from the group consisting of:
(a) use of a diagnostic tool, a diagnostic technique, a diagnostic kit, or a diagnostic assay to confirm a patient's particular phenotype;
(b) use of a method for measurement of a marker selected from the group consisting of histone deacetylase, ornithine decarboxylase, VEGF, a protein that is a gene product of a prostate specific gene, a protein that is a gene product of jun, a protein kinase, desmoglein-3, and a caspase-derived neo-epitope;
(c) surrogate compound dosing; and
(d) low dose pre-testing for enzymatic status.

The measurement of the protein desmoglein-3 as a marker of metastasis of a tumor to lymph nodes and the selection of appropriate therapy based on the amount of desmoglein-3 in a sample from a subject is described in United States Patent Application Publication No. 2012/0087892 by Gutkind et al..

The measurement of caspase-derived neo-epitopes as an indicator of apoptosis, including apoptosis induced by anti-neoplastic agents, is described in United States Patent Application Publication No. 2012/0028266 by Wells et al..

When the improvement described herein is made by analysis of patient or disease genotype, the analysis of patient or disease genotype can be, but is not limited to, a method of analysis of patient or disease genotype carried out by a method selected from the group consisting of:
(a) use of a diagnostic tool, a diagnostic technique, a diagnostic kit, or a diagnostic assay to confirm a patient's particular genotype;
(b) use of a gene chip;
(c) use of gene expression analysis;
(d) use of single nucleotide polymorphism (SNP) analysis; and
(e) measurement of the level of a metabolite or a metabolic enzyme.

The use of gene chips is described in A.J. Lee & S. Ramaswamy, "DNA Microarrays in Biological Discovery and Patient Care" in Essentials of Genomic and Personalized Medicine (G.S. Ginsburg & H.F. Willard, eds., Academic Press, Amsterdam, 2010), ch. 7, pp. 73-88.

When the method is the use of single nucleotide polymorphism (SNP) analysis, the SNP analysis can be carried out on a gene selected from the group consisting of histone deacetylase, ornithine decarboxylase, VEGF, a prostate specific gene, c-Jun, and a protein kinase. The use of SNP analysis is described in S. Levy and Y.-H. Rogers, "DNA Sequencing for the Detection of Human Genome Variation" in Essentials of Genomic and Personalized Medicine (G.S. Ginsburg & H.F. Willard, eds., Academic Press, Amsterdam, 2010), ch. 3, pp. 27-37.

Still other genomic techniques such as copy number variation analysis and analysis of DNA methylation can be employed. Copy number variation analysis is described in C. Lee et al., "Copy Number Variation and Human Health" in Essentials of Genomic and Personalized Medicine (G.S. Ginsburg & H.F. Willard, eds., Academic Press, Amsterdam, 2010), ch. 5, pp. 46-59. DNA methylation analysis is described in S. Cottrell et al., "DNA Methylation Analysis: Providing New Insight into Human Disease" in Essentials of Genomic and Personalized Medicine (G.S. Ginsburg & H.F. Willard, eds., Academic Press, Amsterdam, 2010), ch. 6, pp. 60-72.

When the improvement described herein is made by pre/post-treatment preparation, the pre/post-treatment preparation can be, but is not limited to, a method of pre/post treatment preparation selected from the group consisting of:
(a) the use of colchicine or an analog thereof;
(b) the use of a uricosuric;
(c) the use of uricase;
(d) the non-oral use of nicotinamide;
(e) the use of a sustained-release form of nicotinamide;
(f) the use of an inhibitor of poly-ADP ribose polymerase;
(g) the use of caffeine;
(h) the use of leucovorin rescue;
(i) infection control; and
(j) the use of an anti-hypertensive agent.

Uricosurics described herein include, but are not limited to, probenecid, benzbromarone, and sulfinpyrazone. A particularly preferred uricosuric described herein is probenecid. Uricosurics, including probenecid, may also have diuretic activity.

Poly-ADP ribose polymerase inhibitors are described in G.J. Southan & C. Szabó, "Poly(ADP-Ribose) Inhibitors," Curr. Med. Chem. 10: 321-240 (2003), and include nicotinamide, 3-aminobenzamide, substituted 3,4-dihydroisoquinolin-1(2H)-ones and isoquinolin-1(2H)-ones, benzimidazoles, indoles, phthalazin-1(2H)-ones, quinazolinones, isoindolinones, phenanthridinones, and other compounds.

Leucovorin rescue comprises administration of folinic acid (leucovorin) to patients in which methotrexate has been administered. Leucovorin is a reduced form of folic acid that bypasses dihydrofolate reductase and restores hematopoietic function. Leucovorin can be administered either intravenously or orally.

In one alternative, wherein the pre/post treatment is the use of a uricosuric, the uricosuric is probenecid or an analog thereof.

When the improvement described herein is made by toxicity management, the toxicity management can be, but is not limited to, a method of toxicity management selected from the group consisting of:
(a) the use of colchicine or an analog thereof;
(b) the use of a uricosuric;
(c) the use of uricase;
(d) the non-oral use of nicotinamide;
(e) the use of a sustained-release form of nicotinamide;
(f) the use of an inhibitor of poly-ADP ribose polymerase;
(g) the use of caffeine;
(h) the use of leucovorin rescue;
(i) the use of sustained-release allopurinol;
(j) the non-oral use of allopurinol;
(k) the use of bone marrow transplants;
(l) the use of a blood cell stimulant;
(m) the use of blood or platelet infusions;
(n) the administration of an agent selected from the group consisting of filgrastim (Neupogen®), G-CSF, and GM-CSF;
(o) the application of a pain management technique;
(p) the administration of an anti-inflammatory agent;
(q) the administration of fluids;
(r) the administration of a corticosteroid;
(s) the administration of an insulin control medication;
(t) the administration of an antipyretic;
(u) the administration of an anti-nausea treatment;
(v) the administration of an anti-diarrheal treatment;
(w) the administration of N-acetylcysteine;
(x) the administration of an antihistamine; and
(y) the administration of agents for reduction of gastric toxicity.

Filgrastim is a granulocytic colony-stimulating factor (G-CSF) analog produced by recombinant DNA technology that is used to stimulate the proliferation and differentiation of granulocytes and is used to treat neutropenia; G-CSF can be used in a similar manner. GM-CSF is granulocyte macrophage colony-stimulating factor and stimulates stem cells to produce granulocytes (eosinophils, neutrophils, and basophils) and monocytes; its administration is useful to prevent or treat infection.

Anti-inflammatory agents are well known in the art and include corticosteroids and non-steroidal anti-inflammatory agents (NSAIDs). Corticosteroids with anti-inflammatory activity include, but are not limited to, hydrocortisone, cortisone, beclomethasone dipropionate, betamethasone, dexamethasone, prednisone, methylprednisolone, triamcinolone, fluocinolone acetonide, and fludrocortisone. Non-steroidal anti-inflammatory agents include, but are not limited to, acetylsalicylic acid (aspirin), sodium salicylate, choline magnesium trisalicylate, salsalate, diflunisal, sulfasalazine, olsalazine, acetaminophen, indomethacin, sulindac, tolmetin, diclofenac, ketorolac, ibuprofen, naproxen, flurbiprofen, ketoprofen, fenoprofin, oxaprozin, mefenamic acid, meclofenamic acid, piroxicam, meloxicam, nabumetone, rofecoxib, celecoxib, etodolac, nimesulide, aceclofenac, alclofenac, alminoprofen, amfenac, ampiroxicam, apazone, araprofen, azapropazone, bendazac, benoxaprofen, benzydamine, bermoprofen, benzpiperylon, bromfenac, bucloxic acid, bumadizone, butibufen, carprofen, cimicoxib, cinmetacin, cinnoxicam, clidanac, clofezone, clonixin, clopirac, darbufelone, deracoxib, droxicam, eltenac, enfenamic acid, epirizole, esflurbiprofen, ethenzamide, etofenamate, etoricoxib, felbinac, fenbufen, fenclofenac, fenclozic acid, fenclozine, fendosal, fentiazac, feprazone, filenadol, flobufen, florifenine, flosulide, flubichin methanesulfonate, flufenamic acid, flufenisal, flunixin, flunoxaprofen, fluprofen, fluproquazone, furofenac, ibufenac, imrecoxib, indoprofen, isofezolac, isoxepac, isoxicam, licofelone, lobuprofen, lomoxicam, lonazolac, loxaprofen, lumaricoxib, mabuprofen, miroprofen, mofebutazone, mofezolac, morazone, nepafanac, niflumic acid, nitrofenac, nitroflurbiprofen, nitronaproxen, orpanoxin, oxaceprol, oxindanac, oxpinac, oxyphenbutazone, pamicogrel, parcetasal, parecoxib, parsalmide, pelubiprofen, pemedolac, phenylbutazone, pirazolac, pirprofen, pranoprofen, salicin, salicylamide, salicylsalicylic acid, satigrel, sudoxicam, suprofen, talmetacin, talniflumate, tazofelone, tebufelone, tenidap, tenoxicam, tepoxalin, tiaprofenic acid, tiaramide, tilmacoxib, tinoridine, tiopinac, tioxaprofen, tolfenamic acid, triflusal, tropesin, ursolic acid, valdecoxib, ximoprofen, zaltoprofen, zidometacin, and zomepirac, and the salts, solvates, analogues, congeners, bioisosteres, hydrolysis products, metabolites, precursors, and prodrugs thereof.

The clinical use of corticosteroids is described in B.P. Schimmer & K.L. Parker, "Adrenocorticotropic Hormone; Adrenocortical Steroids and Their Synthetic Analogs; Inhibitors of the Synthesis and Actions of Adrenocortical Hormones" in Goodman & Gilman's The Pharmacological Basis of Therapeutics (L.L. Brunton, ed., 11th ed., McGraw-Hill, New York, 2006), ch. 59, pp. 1587-1612.

Anti-nausea treatments described herein include, but are not limited to, ondansetron, metoclopramide, promethazine, cyclizine, hyoscine, dronabinol, dimenhydrinate, diphenhydramine, hydroxyzine, medizine, dolasetron, granisetron, palonosetron, ramosetron, domperidone, haloperidol, chlorpromazine, fluphenazine, perphenazine, prochlorperazine, betamethasone, dexamethasone, lorazepam, and thiethylperazine.

Anti-diarrheal treatments described herein include, but are not limited to, diphenoxylate, difenoxin, loperamide, codeine, racecadotril, octreoside, and berberine.

N-acetylcysteine is an antioxidant and mucolytic that also provides biologically accessible sulfur.

Agents for reduction of gastric toxicity described herein include, but are not limited to, ferruginol (C. Areche et al., "Gastroprotective Activity of Ferruginol in Mice and Rats: Effects on Gastric Secretion, Endogenous Prostaglandins and Non-Protein Sulfhydryls," J. Pharm. Pharmacol. 60: 245-251 (2008)).

When the improvement described herein is made by pharmacokinetic/pharmacodynamic monitoring, the pharmacokinetic/pharmacodynamic monitoring can be, but is not limited to a method selected from the group consisting of:
(a) multiple determinations of blood plasma levels; and
(b) multiple determinations of at least one metabolite in blood or urine.

Typically, determination of blood plasma levels or determination of at least one metabolite in blood or urine is carried out by immunoassays. Methods for performing immunoassays are well known in the art, and include radioimmunoassay, ELISA (enzyme-linked immunosorbent assay), competitive immunoassay, immunoassay employing lateral flow test strips, and other assay methods.

When the improvement described herein is made by drug combination, the drug combination can be, but is not limited to, a drug combination selected from the group consisting of:
(a) use with fraudulent nucleosides;
(b) use with fraudulent nucleotides;
(c) use with thymidylate synthetase inhibitors;
(d) use with signal transduction inhibitors;
(e) use with cisplatin or platinum analogs;
(f) use with alkylating agents;
(g) use with anti-tubulin agents;
(h) use with antimetabolites;
(i) use with berberine;
(j) use with apigenin;
(k) use with colchicine or an analog thereof;
(l) use with genistein;
(m) use with etoposide;
(n) use with cytarabine;
(o) use with camptothecins;
(p) use with vinca alkaloids;
(q) use with topoisomerase inhibitors;
(r) use with 5-fluorouracil;
(s) use with curcumin;
(t) use with NF-κB inhibitors;
(u) use with rosmarinic acid;
(v) use with mitoguazone;
(w) use with meisoindigo;
(x) use with imatinib;
(y) use with dasatinib;
(z) use with nilotinib;
(aa) use with epigenetic modulators;
(ab) use with transcription factor inhibitors;
(ac) use with taxol;
(ad) use with homoharringtonine;
(ae) use with pyridoxal;
(af) use with spirogermanium;
(ag) use with caffeine;
(ah) use with nicotinamide;
(ai) use with methylglyoxalbisguanylhydrazone;
(aj) use with Rho kinase inhibitors;
(ak) use with 1,2,4-benzotriazine oxides;
(al) use with an alkylglycerol;
(am) use with an inhibitor of a Mer, Ax1, or Tyro-3 receptor kinase;
(an) use with an inhibitor of ATR kinase;
(ao) use with a modulator of Fms kinase, Kit kinase, MAP4K4 kinase, TrkA kinase, or TrkB kinase;
(ap) use with endoxifen;
(aq) use with a mTOR inhibitor;
(ar) use with an inhibitor of Mnk1a kinase, Mkn1b kinase, Mnk2a kinase, or Mnk2b kinase;
(as) use with a modulator of pyruvate kinase M2;
(at) use with a modulator of phosphoinositide 3-kinases;
(au) use with a cysteine protease inhibitor;
(av) use with phenformin;
(aw) use with Sindbis virus-based vectors;
(ax) use with peptidomimetics that act as mimetics of Smac and inhibit lAPs to promote apoptosis;
(ay) use with a Raf kinase inhibitor;
(az) use with a nuclear transport modulator;
(ba) use with an acid ceramidase inhibitor and a choline kinase inhibitor;
(bb) use with tyrosine kinase inhibitors;
(bc) use with anti-CS1 antibodies;
(bd) use with inhibitors of protein kinase CK2;
(be) use with anti-guanylyl cyclase C (GCC) antibodies;
(bf) use with histone deacetylase inhibitors;
(bg) use with cannabinoids;
(bh) use with glucagon-like peptide-1 (GLP-1) receptor agonists;
(bi) use with inhibitors of Bcl-2 or Bcl-xL;
(bj) use with Stat3 pathway inhibitors;
(bk) use with inhibitors of polo-like kinase 1 (Plk1);
(bl) use with GBPAR1 activators;
(bm) use with modulators of serine-threonine protein kinase and poly(ADP-ribose) polymerase (PARP) activity;
(bn) use with taxanes;
(bo) use with inhibitors of dihydrofolate reductase;
(bp) use with inhibitors of aromatase;
(bq) use with benzimidazole-based anti-neoplastic agents;
(br) use with an O6-methylguanine-DNA-methyltransferase (MGMT) inhibitor;
(bs) use with CCR9 inhibitors;
(bt) use with acid sphingomyelinase inhibitors;
(bu) use with peptidomimetic macrocycles;
(bv) use with cholanic acid amides;
(bw) use with substituted oxazaphosphorines;
(bx) use with anti-TWEAK receptor antibodies;
(by) use with an ErbB3 binding protein;
(bz) use with a glutathione S-transferase-activated anti-neoplastic compound;
(ca) use with substituted phosphorodiamidates;
(cb) use with inhibitors of MEKK protein kinase;
(cd) use with COX-2 inhibitors;
(ce) use with cimetidine and a cysteine derivative;
(cf) use with anti-IL-6 receptor antibody;
(cg) use with an antioxidant;
(ch) use with an isoxazole inhibitor of tubulin polymerization;
(ci) use with PARP inhibitors;
(cj) use with Aurora protein kinase inhibitors;
(ck) use with peptides binding to prostate-specific membrane antigen;
(cl) use with CD19 binding agents;
(cm) use with benzodiazepines;
(cn) use with Toll-like receptor (TLR) agonists;
(co) use with bridged bicyclic sulfamides;
(cp) use with inhibitors of epidermal growth factor receptor kinase;
(cq) use with a ribonuclease of the T2 family having actin-binding activity;
(cr) use with myrsinoic acid A or an analog thereof;
(cs) use with inhibitors of a cyclin-dependent kinase;
(ct) use with inhibitors of the interaction between p53 and MDM2;
(cu) use with inhibitors of the receptor tyrosine kinase MET;
(cv) use with largazole or largazole analogs;
(cw) use with inhibitors of AKT protein kinase;
(cx) use with 2'-fluoro-5-methyl-β-L-arabinofuranosyluridine or L-deoxythymidine;
(cy) use with HSP90 modulators;
(cz) use with inhibitors of JAK kinases;
(da) use with inhibitors of PDK1 protein kinase;
(db) use with PDE4 inhibitors;
(de) use with inhibitors of proto-oncogene c-Met tyrosine kinase;
(df) use with inhibitors of indoleamine 2,3-dioxygenase;
(dg) use with agents that inhibit expression of ATDC (TRIM29);
(dh) use with proteomimetic inhibitors of the interaction of nuclear receptor with coactivator peptides;
(di) use with antagonists of XIAP family proteins;
(dj) use with tumor-targeted superantigens;
(dk) use with inhibitors of Pim kinases;
(dl) use with inhibitors of CHK1 or CHK2 kinases;
(dm) use with inhibitors of angiopoietin-like 4 protein;
(dn) use with Smo antagonists;
(do) use with nicotinic acetylcholine receptor antagonists;
(dp) use with farnesyl protein transferase inhibitors;
(dq) use with adenosine A3 receptor antagonists;
(dr) use with a cancer vaccine;
(ds) use with a JAK2 inhibitor; and
(dt) use with a Src inhibitor.

These drug combinations are in addition to the use of an alkylating hexitol derivative together with a BH3 mimetic, described above.

Additionally, as described further below, an alkylating hexitol derivative described herein can be used together with an agent modulating the expression of the *AHI1* gene or modulating the activity of AHI1 protein.

Topoisomerase inhibitors described herein include, but are not limited to, irinotecan, topotecan, camptothecin, lamellarin D, amsacrine, etoposide, etoposide phosphate, teniposide, doxorubicin, and 4-[2-(3,5-dioxo-1-piperazinyl)-1-methylpropyl]piperazine-2,6-dione (ICRF-193).

Fraudulent nucleosides described herein include, but are not limited to, cytosine arabinoside, gemcitabine, and fludarabine; other fraudulent nucleosides are known in the art.

Fraudulent nucleotides described herein include, but are not limited to, tenofovir disoproxil fumarate and adefovir dipivoxil; other fraudulent nucleotides are known in the art.

Thymidylate synthetase inhibitors described herein include, but are not limited to, raltitrexed, pemetrexed, nolatrexed, ZD9331, GS7094L, fluorouracil, and BGC 945.

Signal transduction inhibitors are described in A.V. Lee et al., "New Mechanisms of Signal Transduction Inhibitor Action: Receptor Tyrosine Kinase DownRegulation and Blockade of Signal Transactivation," Clin. Cancer Res. 9: 516s (2003).

Alkylating agents described herein include, but are not limited to, Shionogi 254-S, aldo-phosphamide analogues, altretamine, anaxirone, Boehringer Mannheim BBR-2207, bendamustine, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)₂, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, melphalan, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromustine, Tanabe Seiyaku TA-077, tauromustine, temozolomide, teroxirone, tetraplatin and trimelamol, as described in United States Patent No. 7,446,122 by Chao et al..

Anti-tubulin agents described herein include, but are not limited to, vinca alkaloids, taxanes, podophyllotoxin, halichondrin B, and homohalichondrin B.

Antimetabolites described herein include, but are not limited to: methotrexate, pemetrexed, 5-fluorouracil, capecitabine, cytarabine, gemcitabine, 6-mercaptopurine, and pentostatin, alanosine, AG2037 (Pfizer), 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrill-Dow DDFC, deazaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

Berberine has antibiotic activity and prevents and suppresses the expression of pro-inflammatory cytokines and E-selectin, as well as increasing adiponectin expression.

Apigenin is a flavone that can reverse the adverse effects of cyclosporine and has chemoprotective activity, either alone or derivatized with a sugar.

Colchicine is a tricyclic alkaloid that exerts its activity by binding to the protein tubulin. Analogs of colchicine described herein include, but are not limited to, cholchiceinamide, *N*-desacetylthiocolchicine, demecolcine, *N*-acetyliodocolchinol, trimethylcolchicinie acid (TMCA) methyl ether, *N*-acetylcolchinol, TMCA ethyl ether, isocolchicine, isocolchiceinamide, iso-TMCA methyl ether, colchiceine, TMCA, *N*-benzoyl TMCA, colchicosamide, colchicoside, colchinol and colchinoic acid (M.H. Zweig & C.F. Chignell, "Interaction of Some Colchicine Analogs, Vinblastine and Podophyllotoxin with Rat Brain Microtubule Protein," Biochem. Pharmacol. 22: 2141-2150 (1973) and B. Yang et al., "Syntheses and Biological Evaluation of Ring C-Modified Colchicine Analogs," Bioorg. Med. Chem. Lett. 20: 3831-3833 (2010)).

Genistein is an isoflavone with the systemic name 5,7-dihydroxy-3-(4-hydroxyphenyl)chromen-4-one. Genistein has a number of biological activities, including activation of PPARs, inhibition of several tyrosine kinases, inhibition of topoisomerase, antioxidative activity, activation of Nrf2 antioxidative response, activation of estrogen receptor beta, and inhibition of the mammalian hexose transporter GLUT2.

Etoposide is an anticancer agent that acts primarily as a topoisomerase II inhibitor. Etoposide forms a ternary complex with DNA and the topoisomerase II enzyme, prevents re-ligation of the DNA strands and thus induces DNA strand breakage and promotes apoptosis of the cancer cells.

Cytarabine is a nucleoside analog replacing the ribose with arabinose. It can be incorporated into DNA and also inhibits both DNA and RNA polymerases and nucleotide reductase. It is particularly useful in the treatment of acute myeloid leukemia and acute lymphocytic leukemia,

Camptothecins include camptothecin, homocamptothecin, topotecan, irinotecan, DB 67, BNP 1350, exatecan, lurtotecan, ST 1481, and CKD 602. These compounds act as topoisomerase I inhibitors and block DNA synthesis in cancer cells.

Vinca alkaloids described herein include vinblastine, vincristine, vindesine, and vinorelbine.

Topoisomerase inhibitors described herein include topoisomerase I inhibitors and topoisomerase II inhibitors. Topoisomerase I inhibitors described herein include the camptothecins and lamellarin D. Topoisomerase II inhibitors described herein include, in addition to amonafide and derivatives and analogs thereof, etoposide, teniposide, doxorubicin, daunorubicin, mitoxantrone, amsacrine, ellipticines, and aurintricarboxylic acid. A number of plant-derived naturally-occurring phenolic compounds, such as genistein, quercetin, and resveratrol, exhibit inhibitory activity toward both topoisomerase I and topoisomerase II.

5-fluorouracil is a base analog that acts as a thymidylate synthase inhibitor and thereby inhibits DNA synthesis. When deprived of a sufficient supply of thymidine, rapidly dividing cancer cells die by a process known as thymineless death.

Curcumin is believed to have anti-neoplastic, anti-inflammatory, antioxidant, anti-ischemic, anti-arthritic, and anti-amyloid properties and also has hepatoprotective activity.

NF-κB inhibitors described herein include, but are not limited to bortezomib.

Rosmarinic acid is a naturally-occurring phenolic antioxidant that also has anti-inflammatory activity.

Mitoguazone is an inhibitor of polyamine biosynthesis through competitive inhibition of S-adenosylmethionine decarboxylase.

Meisoindigo is active via several, possibly novel mechanisms of action. It has cell cycle specific effects, including arrest in G(0)/G1 for AML cell lines and G2/M arrest for HT-29 colorectal cell lines. It also stimulates apoptosis through a number of mechanisms, including the upregulation of p21 and p27 and the downregulation of Bcl-2 in primary AML cells, as well as upregulation of Bak and Bax in AML cells (DKO insensitive to chemotherapy), and a novel caspase-dependent pathway in K562 cells. Meisoindigo also has effects on mitochondria, but with no change in Bcl-2, Bax, and Bid protein expression. Meisoindigo also stimulates the cleavage of pro-caspase 3, 8, 9 and PARP in HL-60 myeloid cells. Meisoindigo also is directed to multiple cellular targets, which are possibly synergistic and complementary. For example, it promotes differentiation of human myeloblastic leukemic cells, accompanied by downregulation of c-myb gene expression. It also promotes inhibition of DNA and RNA synthesis in W256 cells, microtubule assembly, glycogen synthase kinase-3β (GSK-3β) (at 5-50 nM), CDK1/cyclin B, and CDK5/p25 (tau microtubule protein phosphorylation). Additionally, meisoindigo decreases β-catenin and c-myc (HL-60 cells, but not in K562), affects the Wnt pathway through inhibiting GSK-3β and downregulating β-catenin and c-myc protein expression. Meisoindigo also promotes upregulation of CD11 b, promoting myeloid differentiation, and upregulation of AHI-1 in Jurkat cells (inducing phosphorylation of c-Myb). Furthermore, meisoindigo exhibits antiangiogenic effects, including decreased VEGF protection, VCAM-1, tubule formulation in HUVEC, and ECV304 apoptosis.

Imatinib is an inhibitor of the receptor tyrosine kinase enzyme ABL and is used to treat chronic myelogenous leukemia, gastrointestinal stromal tumors, and other hyperproliferative disorders.

Dasatinib is an inhibitor of BCR/ABL and Src family tyrosine kinases and is used to treat chronic myelogenous leukemia and acute lymphoblastic leukemia.

Nilotinib is another tyrosine kinase inhibitor approved for the treatment of chronic myelogenous leukemia; it inhibits the kinases BCR/ABL, KIT, LCK, EPHA3, and a number of other kinases. The use of nilotinib is described in United States Patent Application Publication No. 2011/0028422 by Aloyz et al..

Epigenetic modulators include polyamine-based epigenetic modulators, such as the polyamine-based epigenetic modulators described in S.K. Sharma et al., "Polyamine-Based Small Molecule Epigenetic Modulators," Med. Chem. Commun. 3: 14-21 (2012), and L.G. Wang & J.W. Chiao, "Prostate Cancer Chemopreventive Activity of Phenethyl Isothiocyanate Through Epigenetic Regulation (Review), Int. J. Oncol. 37: 533-539 (2010).

Transcription factor inhibitors include 1-(4-hexaphenyl)-2-propane-1-one, 3-fluoro-4-[[2-hydroxy-2-(5,5,8,8-tetramethyl-5,6,7,8,-tetrahydro-2-naphthalenyl)acetyl]amino]-benzoic acid (BMS 961), 4-[5-[8-(1-Methylethyl)-4-phenyl-2-quinolinyl]-1*H*-pyrrolo-2-benzoic acid (ER-50891), 7-Ethenyl-2-(3-fluoro-4-hydroxyphenyl)-5-benzoxazolol (ERB 041), and other compounds. Trascription factor inhibitors are described in T. Berg, "Inhibition of Transcription Factors with Small Organic Molecules," Curr. Opin. Chem. Biol. 12: 464-471 (2008).

Tetrandrine has the chemical structure 6,6',7,12-tetramethoxy-2,2'-dimethyl-1 β-berbaman and is a calcium channel blocker that has anti-inflammatory, immunologic, and antiallergenic effects, as well as an anti-arrhythmic effect similar to that of quinidine. It has been isolated from *Stephania tetranda* and other Asian herbs.

VEGF inhibitors include bevacizumab (Avastin™), which is a monoclonal antibody against VEGF, itraconazole, and suramin, as well as batimastat and marimastat, which are matrix metalloproteinase inhibitors, and cannabinoids and derivatives thereof.

Cancer vaccines are being developed. Typically, cancer vaccines are based on an immune response to a protein or proteins occurring in cancer cells that does not occur in normal cells. Cancer vaccines include Pnovenge™ for metastatic hormone-refractory prostate cancer, Oncophage™ for kidney cancer, CimaVax-EGF™ for lung cancer, MOBILAN™, Neuvenge™ for Her2/neu expressing cancers such as breast cancer, colon cancer, bladder cancer, and ovarian cancer,Stimuvax™ for breast cancer, and others. Cancer vaccines are described in S. Pejawar-Gaddy & O. Finn, "Cancer Vaccines: Accomplishments and Challenges," Crit. Rev. Oncol. Hematol. 67: 93-102 (2008).

The use of methylglyoxalbisguanylhydrazone in cancer therapy has been described in D.D. Von Hoff, "MGBG: Teaching an Old Drug New Tricks," Ann. Oncol. 5: 487-493 (1994).

The use of Rho kinase inhibitors, such as (R)-(+)-N-(4-pyridyl)-4-(1-aminoethyl)benzamide, ethacrynic acid, 4-[2(2,3,4,5,6-pentafluorophenyl)acryloyl]cinnamic acid, (+)-trans-4-(1-aminoethyl)-1-(4-pyridylcarbamoyl)cyclohexane, (+)-10 trans-N-(1H-pyrrolo[2,3-b]pyridin-4-yl)-4-(1-aminoethyl)cyclohexanecarboxamide, and (R)-(+)-N-(1H-pyrrolo[2,3-b]pyridin-4-yl)-4-(1-aminoethyl)benzamide, as described in United States Patent No. 6,930,115 to Fujii et al..

The use of 1,2,4-benzotriazine oxides, such as 3-hydroxy-1,2,4-benzotriazine 1,4-dioxide, 3-amino-7-trifluoromethyl-1,2,4-benzotriazine 1-oxide, 3-amino-7-carbamyl-1,2,4-benzotriazine 1-oxide, 7-acetyl-3-amino-1,2,4-benzotriazine 1-oxide oxime, 3-amino-6(7)decyl-1,2,4-benzotriazine 1,4-dioxide, 1,2,4-benzotriazine dioxide, 7-chloro-3-hydroxy-1,2,4-benzotriazine 1,4-dioxide, 7-nitro-3-amino-1,2,4-benzotriazine 1,4-dioxide, 3-(3-N,N-diethylaminopropylamino)-1,2,4-benzotriazine 1,4-dioxide, 7-nitro-3-(2-N,N-diethylaminoethylamino)-1,2,4-benzotriazine 1,4-dioxide, 7-allyloxy-1,2,4-benzotriazine 1,4-dioxide, 7-(3-N-ethylacetamido-2-acetoxypropoxy) 1,2,4-benzotriazine 1,4-dioxide, 7-nitro-1,2,4-benzotriazine 1,4-dioxide. 3-propyl-1,2,4-benzotriazine 1,4-dioxide, and 3-(1-hydroxyethyl)-1,2,4-benzotriazine 1,4-dioxide, as described in United States Patent No. 6,277,835 by Brown.

The use of alkylglycerols is described in United States Patent No. 6,121,245 to Firshein.

The use of inhibitors of Mer, Ax1, or Tyro-3 receptor tyrosine kinase is described in United States Patent Application Publication No. 2012/0230991 by Graham et al.. These inhibitors can be antibodies, including monoclonal antibodies, or fusion proteins.

The use of inhibitors of ATR kinase is described in United States Patent Application Publication No. 2012/0177748 by Charrier et al.. These inhibitors of ATR kinase are substituted pyridine compounds such as 2-amino-N-phenyl-5-(3-pyridyl)pyridine-3-carboxamide, 5-(4-(methylsulfonyl)phenyl-3-(5-phenyl-1,3,4-oxadiazol-2-yl)pyridine-2-amine, and 5-(1-ethylsulfonyl-3,6-dihydro-2H-pyridin-4-yl)-3-(5-phenyl-1,3,4-oxadiazol-2-yl)pyridine-2-amine.

The use of compounds that modulate the activity of one or more of Fms kinase, Kit kinase, MAP4K4 kinase, TrkA kinase, or TrkB kinase is described in United States Patent Application Publication No. 2012/0165329 by Ibrahim et al.. These compounds include (6-methoxy-pyridin-3-ylmethyl)[5-(7H-pyrrolo [2,3-d] pyrimidin-5-ylmethyl)-pyrimidin-2-yl]-amine, (5-fluoro-2-methoxy-pyridin-3-ylmethyl)-[5-(7H-pyrrolo[2,3-d]pyrimidin-5-ylmethyl)-pyrimidin-2-y]-amine, and (5-fluoro-6-methoxy-pyridin-3-ylmethyl)-[5-(7H-pyrrolo[2,3-d]pyrimidin-5-ylmethyl)-pyrimidin-2-yl]-amine. Compounds that inhibit Trk kinases, particularly TrkA, are described in United States Patent Application Publication No. 2011/0301133 by Wu et al..

The use of endoxifen is described in United States Patent Application Publication No. 2012/0164075 by Ahmad et al..

The use of a mTOR inhibitor is described in United States Patent Application Publication No. 2012/0129881 by Burke et al.. Suitable mTOR inhibitors include, but are not limited to, 40-O-(2-hydroxyethyl)rapamycin. These mTOR inhibitors can be used together with Raf kinase inhibitors, as described in United States Patent Application Publication No. 2011/0301184 by Lane. Raf kinase inhibitors are also described in United States Patent Application Publication No. 2010/0286178 by Ibrahim et al.; these compounds include, but are not limited to, propane-1-sulfonic acid {2,4-difluoro-3-[5-(2-methoxy-pyrimidin-5-yl)-1H-pyrrolo[2,3-b]pyridine-3-carbonyl]-phenyl}-amide, propane-1-sulfonic acid [3-(5-cyano-1H-pyrrolo[2,3-b]pyridine-3-carbonyl)-2,4-difluoro-phenyl]-amide, propane-1-sulfonic acid [3-(5-cyano-1H-pyrrolo[2,3-b]pyridine-3-carbonyl)-2-fluoro-phenyl]-amide, N-[3-(5-cyano-1H-pyrrolo[2,3-b]pyridine-3-carbonyl)-2,4-difluoro-phenyl]-2,5-difluoro-benzenesulfonamide, N-[3-(5-cyano-1H-pyrrolo[2,3-b]pyridine-3-carbonyl)-2,4-difluoro-phenyl]-3-fluoro-benzenesulfonamide, pyrrolidine-1-sulfonic acid [3-(5-cyano-1H-pyrrolo[2,3-b]pyridine-3-carbonyl)-2,4-difluoro-phenyl]-amide, and N,N-dimethylamino-sulfonic acid [3-(5-cyano-1H-pyrrolo[2,3-b]pyridine-3-carbonyl)-2,4-difluoro-phenyl]-amide. These mTOR inhibitors can also be used together with compounds that elevate pAkt levels in malignant cells, as described in United States Patent Application Publication No. 2009/0274698 by Bhagwat et al.. A number of compounds that elevate pAkt levels are described, including chemotherapeutic agents, analogs of rapamycin, and other agents. The use of mTOR inhibitors is also described in United States Patent No. 8,268,819 to Jin et al.; these mTOR inhibitors are hexahydrooxazinopterine compounds.

The use of an inhibitor of Mnk1a kinase, Mnk1b kinase, Mnk2a kinase, or Mnk2b kinase is described in United States Patent Application Publication No. 2012/0128686 by Austen et al.. These compounds include thienopyrimidines. Additional thienopyrimidine inhibitors of one or more of these kinases are described in United States Patent Application Publication No. 2011/0212103 by Heckel et al. and in United States Patent Application Publication No. 2011/0212102 by Lehmann-Lintz et al..

The use of a modulator of pyruvate kinase M2 is described in United States Patent Application Publication 2012/0122885 by Salituro et al.. Suitable modulators of pyruvate kinase M2 include, but are not limited to, 1-(3-chloro-5-(trifluoromethyl)pyridin-2-yl)-N-(3,5-dimethylphenyl)-1H-imidazole-5-sulfonamide; 1-(3-chloro-5-(trifluoromethyl)pyridin-2-yl)-N-(5-methoxyphenyl)-1H-imidazole-5-sulfonamide; and N-(4-methoxyphenyl)-1-(5-(trifluoromethyl)pyridine-2-yl)-H-imidazole-5-sulfonamide.

The use of a modulator of a phosphoinositide 3-kinase is described in United States Patent Application Publication No. 2012/0122838 by Ren et al.. Inhibitors of phosphoinositide 3-kinase are also described in United States Patent Application Publication No. 2010/0209420 by Lamb et al., and in United States Patent Application Publication No. 2009/0209340 by Buhret al.; these inhibitors include pyridopyrimidones. Inhibitors of phosphoinositide 3-kinase are also described in United States Patent No. 8,242,104 to Blaquiere et al.; these inhibitors include benzoxazepines. Inhibitors of phosphoinositide 3-kinase are also described in United States Patent No. 8,193,182 to Ren et al.; these inhibitors include isoquinolin-1(2H)-ones. Inhibitors of phosphoinositide 3-kinase are also described in United States Patent No. 7,928,428 to Do et al.; these inhibitors include benzopyrans and benzoxepines.

The use of a cysteine protease inhibitor is described in United States Patent Application Publication No. 2012/0114765 by Cao et al.. Suitable cysteine protease inhibitors include, but are not limited to, 1-[5-(2,4-dichlorophenylsulfanyl)-4-nitro-2-thienyl]ethanone, 1-[5-(2,4-difluorophenylsulfanyl)-4-nitro-2-thienyl]ethanone, and 1-{4-nitro-5-[2-(trifluoromethyl)phenylsulfanyl]-2-thienyl}ethanone.

The use of phenformin is described in United States Patent Application Publication No. 2012/0114676 by Thompson et al..

The use of Sindbis-based virus vectors is described in United States Patent Application Publication No. 2011/0318430 by Meruelo et al.. These vectors are capable of binding to solid tumors that express higher levels of high affinity laminin receptors.

The use of peptidomimetics that act as mimetics of Smac and inhibit lAPs to promote apoptosis is described in United States Patent Application Publication No. 2011/0305777 by Condon et al..

The use of nuclear transport modulators, especially inhibitors of Crm1, is described in United States Patent Application Publication No. 2011/0275607 by Shacham et al.. These inhibitors of Crm1 include, but are not limited to, (Z)-3-[3-(3-chlorophenyl)[1,2,4]-triazol-1-yl]-acrylic acid ethyl ester, (E)-3-[3-(3-chlorophenyl)[1,2,4]-triazol-1-yl]-acrylic acid ethyl ester, (Z)-3-[3-(3-chlorophenyl)-[1,2,4]-triazol-1-yl]-acrylic acid isopropyl ester, (E)-3-[3-(3-chlorophenyl)-[1,2,4]-triazol-1-yl]-acrylic acid isopropyl ester, (Z)-3-[3-(3-chlorophenyl)-[1,2,4]-triazol-1-yl]-acrylic acid *t*-butyl ester, (Z)-3-[3-(3-chlorophenyl)-[1,2,4]-triazol-1-yl]-acrylic acid *t*-butyl ester, (E)-3-[3-(3-chlorophenyl)-[1,2,4]-triazol-1-yl]-N-phenyl-acrylamide, (E)-N-(2-chlorophenyl)-3-[3-(3-chlorophenyl)-[1,2,4]-triazol-1-yl]-acrylamide, (4-{(E)-3-[3-(3-chlorophenyl)[1,2,4]-triazol-1-yl]-acryloylamino}-phenyl-)-carbamic acid *t*-butyl ester, (E)-3-[3-(3-chlorophenyl)-[1,2,4]-triazol-1-yl]-N-(4-methoxyphenyl)-acrylamide, (E)-3-[3-(3-chlorophenyl)-[1,2,4]-triazol-1-yl]-N-methyl-N-phenyl-acrylamide, and (E)-N-(4-aminophenyl)-3-[3-(3-chlorophenyl)-[1,2,4]-triazol-1-yl]-acrylamide.

The use of tyrosine kinase inhibitors is described in United States Patent Application Publication No. 2011/0206661 by Zhang et al., which is directed to trimethoxyphenyl inhibitors of tyrosine kinase, and in United States Patent Application Publication No. 2011/0195066, which is directed to quinoline inhibitors of tyrosine kinase. The use of tyrosine kinase inhibitors is also described in United States Patent Application Publication No. 2011/053968 by Zhang et al., which is directed to aminopyridine inhibitors of tyrosine kinase. The use of tyrosine kinase inhibitors is also described in United States Patent Application Publication No. 2010/0291025, which is directed to indazole inhibitors of tyrosine kinase. The use of tyrosine kinase inhibitors is also described in United States Patent Application Publication No. 2010/0190749 by Ren et al.; these tyrosine kinase inhibitors are benzoxazole compounds; compounds of this class can also inhibit mTOR and lipid kinases such as phosphoinositide 3-kinases. The use of tyrosine kinase inhibitors is also described in United States Patent No. 8,242,270 by Lajeunesse et al.; these tyrosine kinase inhibitors are 2-aminothiazole-5-aromatic carboxamides.

The use of an acid ceramidase inhibitor and a choline kinase inhibitor is described in United States Patent Application Publication No. 2011/0256241 by Ramirez de Molina et al..

The use of anti-CS1 antibodies is described in United States Patent Application Publication No. 2011/0165154 by Afar.

The use of protein kinase CK2 inhibitors is described in United States Patent Application Publication No. 2011/0152240 by Haddach et al.. These protein kinase CK2 inhibitors include pyrazolopyrimidines. Additional protein kinase CK2 inhibitors, including tricyclic compounds, are described in United States Patent Application Publication No. 2011/0071136 by Haddach et al.; these protein kinase CK2 inhibitors may also inhibit Pim kinases or other kinases. Additional protein kinase CK2 inhibitors, including heterocycle-substituted lactams, are also described in United States Patent Application Publication No. 2011/0071115 by Haddach et al.; these protein kinase CK2 inhibitors may also inhibit Pim kinases or other kinases.

The use of anti-guanylyl cyclase C (GCC) antibodies is described in United States Patent Application Publication No. 2011/0110936 by Nam et al..

The use of histone deacetylase inhibitors is described in United States Patent Application Publication No. 2011/0105474 by Thaler et al.. These histone deacetylase inhibitors described herein include, but are not limited to, (E)-N-hydroxy-3-{4-[(E)-3-(4-methyl-piperazin-1-yl)-3-oxo-propenyl]-phenyl}-acrylamide; (E)-N-hydroxy-3-{3-[(E)-3-(4-methyl-piperazin-1-yl)-3-oxo-propenyl]-phenyl}-acrylamide; (E)-N-hydroxy-3-{3-[(E)-3-oxo-3-(4-phenyl-piperazin-1-yl)-propenyl]-phenyl}-acrylamide; (E)-3-[3-((E)-3-[1,4']bipiperidinyl-1'-yl-3-oxo-propenyl)-phenyl]-N-hydroxy-acrylamide; (E)-N-hydroxy-3-{3-[(E)-3-oxo-3-(cis-3,4,5-trimethyl-piperazin-1-yl)-propenyl]-phenyl}-acrylamide; (E)-3-{3-[(E)-3-((1S,4S)-5-methyl-2,5-diaza-bicyclo[2.2.1]hept-2-yl)-3-oxo-propenyl]-phenyl}-N-hydroxy-acrylamide; (E)-N-hydroxy-3-{4-[(E)-3-oxo-3-(4-phenyl-piperazin-1-yl)-propenyl]-phenyl}-acrylamide; (E)-3-[4-((E)-3-[1,4']bipiperidinyl-1'-yl-3-oxo-propenyl)-phenyl]-N-hydroxy-acrylamide; (E)-N-hydroxy-3-{4-[(E)-3-oxo-3-(cis-3,4,5-trimethyl-piperazin-1-yl)-propenyl]-phenyl}-acrylamide; (E)-N-hydroxy-3-{4-[(E)-3-oxo-3-((1S,4S)-5-methyl-2,5-diaza-bicyclo[2.2.1]hept-2-yl)-propenyl]-phenyl}-acrylamide; (E)-N-hydroxy-3-{5-[(E)-3-oxo-3-(4-phenyl-piperazin-1-yl)-propenyl]-pyridin-2-yl}-acrylamide; (E)-N-hydroxy-3-{5-[(E)-3-(4-methyl-piperazin-1-yl)-3-oxo-propenyl]-pyridin-2-yl}-acrylamide; (E)-N-hydroxy-3-{6-[(E)-3-oxo-3-(4-phenyl-piperazin-1-yl)-propenyl]-pyridin-2-yl}-acrylamide; (E)-N-hydroxy-3-{6-[(E)-3-(4-methyl-piperazin-1-yl)-3-oxo-propenyl]-pyridin-2-yl}-acrylamide; (E)-3-(6-{(E)-3-[4-(3-chloro-phenyl)-piperazin-1-yl]-3-oxo-propenyl}-pyridin-2-yl)-N-hydroxy-acrylamide; (E)-3-{6-[(E)-3-(4-benzoyl-piperazin-1-yl)-3-oxo-propenyl]-pyridin-2-yl}-N-hydroxy-acrylamide hydrochloride; (E)-3-(6-{(E)-3-[4-(2-chloro-phenyl)-piperazin-1-yl]-3-oxo-propenyl}-pyridin-2-yl)-N-hydroxy-acrylamide hydrochloride; (E)-N-hydroxy-3-{6-[(E)-3-oxo-3-(4-phenyl-piperidin-1-yl)-propenyl]-pyridin-2-yl}-acrylamide hydrochloride; (E)-N-hydroxy-3-{6-[(E)-3-oxo-3-(4-pyrimidin-2-yl-piperazin-1-yl)-propenyl]-pyridin-2-yl}-acrylamide hydrochloride; (E)-3-(6-{(E)-3-[4-(4-chloro-phenyl)-piperazin-1-yl]-3-oxo-propenyl}-pyridin-2-yl)-N-hydroxy-acrylamide hydrochloride; and (E)-3-{6-[(E)-3-(4-benzyl-piperazin-1-yl)-3-oxo-propenyl]-pyridin-2-yl}-N-hydroxy-acrylamide hydrochloride. Additional histone deacetylase inhibitors, including spirocyclic derivatives, are described in United States Patent Application Publication No. 2011/039840 by Varasi et al.. Prodrugs of histone deacetylase inhibitors are described in United States Patent No. 8,227,636 to Miller et al.. Histone deacetylase inhibitors are described in United States Patent No. 8,222,451 to Kozikowski et al.. Histone deacetylase inhibitors, including disubstituted aniline compounds, are also described in United States Patent No. 8,119,685 to Heidebrecht et al.. Histone deacetylase inhibitors, including aryl-fused spirocyclic compounds, are also described in United States Patent No. 8,119,852 to Hamblett et al..

The use of cannabinoids is disclosed in United States Patent Application Publication No. 2011/0086113 by Velasco Diez et al.. Suitable cannabinoids described herein include, but are not limited to, tetrahydrocannabinol and cannabidiol.

The use of glucagon-like peptide-1 (GLP-1) receptor agonists is described in United States Patent Application Publication No. 2011/0046071 by Karasik et al.. A suitable GLP-1 receptor agonist described herein is exendin-4.

The use of inhibitors of anti-apoptotic proteins BcI-2 or BcI-xL is described in United States Patent Application Publication No. 2011/0021440 by Martin et al..

The use of Stat3 pathway inhibitors is described in United States Patent Application Publication No. 2010/0310503 by Li et al.. These Stat3 pathway inhibitors described herein include, but are not limited to, 2-(1-hydroxyethyl)-naphtho[2,3-b]furan-4,9-dione, 2-acetyl-7-chloro-naphtho[2,3-b]furan-4,9-dione, 2-acetyl-7-fluoro-naphtho[2,3-b]furan-4,9-dione, 2-acetylnaphtho[2,3-b]furan-4,9-dione, and 2-ethyl-naphtho[2,3-b]furan-4,9-dione.

The use of inhibitors of polo-like kinase 1 (Plk1) is described in United States Patent Application Publication No. 2010/0278833 by Stengel et al.. These inhibitors described herein include, but are not limited to, thiophene-imidazopyridines, including, but not limited to, 5-(6-chloro-1H-imidazo[4,5-c]pyridin-1-yl)-3-{[2-(trifluoromethyl)benzyl]oxy}thiophene-2-carboxamide, 5-(1H-imidazo[4,5-c]pyridin-1 -yl)-3-{[2-(trifluoromethyl)benzyl]oxy}thiophene-2-carboxamide, 5-(3H-imidazo[4,5-c]pyridin-3-yl)-3-{[2-(trifluoromethyl)benzyl]oxy}thiophene-2-carboxamide, 1-(5-carbamoyl-4-{[2-(trifluoromethyl)benzyl]oxy}-2-thienyl)-N-(2-methoxyethyl)-1H-imidazo[4,5-c]pyridine-6-carboxamide, 1-(5-carbamoyl-4-{[2-(trifluoromethyl)benzyl]oxy}-2-thienyl)-N-(2-morpholin-4-ylethyl)-1H-imidazo[4,5-c]pyridine-6-carboxamide, 5-{6-[diethylamino)methyl]-1 H-imidazo[4,5-c]pyridin-1-yl}-3-{[2-(trifluoromethyl)benzyl]oxy}thiophene-2-carboxamide, 5-{6-[(cyclopropylamino)methyl]-1H-imidazo[4,5-c]pyridin-1-yl}-3-{[2-(trifluoromethyl)benzyl]oxy}thiophene-2-carboxamide, 5-{6-[(4-methylpiperazin-1-yl)methyl]-1H-imidazo[4,5-c]pyridin-1 -yl}-3-{[2-(trifluoromethyl)benzyl]oxy}thiophene-2-carboxamide, and 5-[6-(hydroxymethyl)-1H-imidazo[4,5-c]pyridin-1-yl]-3-{[2-(trifluoromethyl)benzyl]oxy}thiophene-2-carboxamide.

The use of GBPAR1 activators is described in United States Patent Application Publication No. 2010/0261758 by Arista et al.. These GBPAR1 activators described herein include, but are not limited to, heterocyclic amides. These compounds include, but are not limited to, N-(3,5-dichlorophenyl)-3-methyl-N-naphthalen-2-ylmethyl-isonicotinamide, (3,5-dichlorophenyl)-N-(2-methoxybenzyl)-3-methyl-isonicotinamide, 3-methyl-N-phenyl-N-pyridin-3-ylmethyl-isonicotinamide, N-naphthalen-2-ylmethyl-1-oxy-N-phenyl-isonicotinamide, N-(3,5-dichlorophenyl)-3-methyl-N-(2-trifluoromethoxybenzyl)-isonicotinamide, 4-methyl-oxazole-5-carboxylic acid benzyl-phenylamide, N-benzyl-N-phenylisonicotinamide, N-benzyl-N-p-tolylisonicotinamide, N-benzyl-2-fluoro-N-phenylisonicotinamide, N-benzyl-3,5-dichloro-N-phenylisonicotinamide, N-benzyl-2-chloro-N-phenyl-isonicotinamide, N-benzyl-2-chloro-6-methyl-N-phenyl-isonicotinamide, N-benzyl-3-methyl-N-phenylisonicotinamide, N-benzyl-3-chloro-N-phenyl-isonicotinamide, N-benzyl-2,5-dichloro-N-phenyl-isonicotinamide, N-benzyl-2-methyl-N-phenyl-isonicotinamide, N-benzyl-2-cyano-N-phenyl-isonicotinamide, N-benzyl-N-phenethyl-isonicotinamide, N-benzyl-N-(2-fluoromethoxy-phenyl)-isonicotinamide, and N-benzyl-N-(4-chlorophenyl)-isonicotinamide. Additional GBPAR1 activators are described in United States Patent Application Publication No. 2010/0048579 by Arista, including pyridazine, pyridine, and pyrane derivatives.

The use of modulators of serine-threonine protein kinase and poly(ADP-ribose) polymerase (PARP) activity is described in United States Patent Application Publication No. 2009/0105233 by Chua et al. and in United States Patent Application Publication No. 2010/0173013 by Drygin et al.. The serine-threonine protein kinase can be, but is not limited to, CK2, CK2α2, Pim-1, CDK1/cyclinB, c-RAF, Mer, MELK, DYRK2, Flt3, Flt3 (D835Y), Flt4, HIPK3, HIPK2, and ZIPK.

The use of taxanes is described in United States Patent Application Publication No. 2010/0166872 by Singh et al.. The taxane described herein can be, but is not limited to, paclitaxel or docitaxel.

The use of inhibitors of dihydrofolate reductase is described in United States Patent Application Publication No. 2010/0150896 by Gant et al.. These inhibitors of dihydrofolate reductase described herein include, but are not limited to, diaminoquinazolines.

The use of inhibitors of aromatase is described in United States Patent Application Publication No. 2010/0111901 by Gant et al.. These inhibitors of aromatase described herein include, but are not limited to, triazoles.

The use of benzimidazole-based anti-neoplastic agents is described in United States Patent Application Publication No. 2010/0098691 by Goh et al.. The benzimidazole-based anti-neoplastic agent described herein can be, but is not limited to, (E)-3-[1-(3-dimethylamino-2,2-dimethyl-propyl)-2-isopropyl-1H-benzimidazol-5-yl]-N-hydroxy-acrylamide, (E)-3-[2-butyl-1-(3-dimethylamino-2,2-dimethyl-propyl)-1 H-benzimidazol-5-yl]-N-hydroxy-acrylamide, (E)-3-[1-(3-dimethylamino-2,2-dimethyl-propyl)-2-(2-methylsulfanyl-ethyl)-1H-benzimidazol-5-yl]-N-hydroxy-acrylamide, (E)-3-[1-(3-dimethylamino-2,2-dimethyl-propyl)-2-ethoxymethyl-1H-benzimidazol-5-yl]-N-hydroxy-acrylamide, (E)-3-[1-(3-dimethylamino-2,2-dimethylpropyl)-2-isobutyl-1H-benzimidazol-5-yl]-N-hydroxy-acrylamide, (E)-3-[1-(2-diethylamino-ethyl)-2-isobutyl-1H-benzimidazol-5-yl]-N-hydroxy-acrylamide, (E)-3-[2-butyl-1-(2-diethylamino-ethyl)-1H-benzimidazol-5-yl]-N-hydroxy-acrylamide, (E)-3-[2-but-3-ynyl-1-(3-dimethylamino-2,2-dimethyl-propyl)-1H-benzimidazol-5-yl]-N-hydroxy-acrylamide, (E)-3-[2-but-3-enyl-1-(3-dimethylamino-2,2-dimethyl-propyl)-1H-benzimidazol-5-yl]-N-hydroxy-acrylamide, (E)-3-[2-but-3-enyl-1-(2-diethylamino-ethyl)-1H-benzimidazol-5-yl]-N-hydroxy-acrylamide, (E)-3-[2-but-3-ynyl-1-(2-diethylaminoethyl)-1H-benzimidazol-5-yl]-N-hydroxy-acrylamide, (E)-3-[1-(3-dimethylamino-2,2-dimethyl-propyl)-2-(3,3,3-trifluoro-propyl)-1H-benzimidazol-5-yl]-N-hydroxy-acrylamide (E)-3-[1-(2-diethylamino-ethyl)-2-(3,3,3-trifluoro-propyl)-1H-benzimidazol-5-yl]-N-hydroxy-acrylamide, (E)-3-[1-(2-diethylamino-ethyl)-2-ethoxymethyl-1H-benzimidazol-5-yl]-N-hydroxy-acrylamide, (E)-3-[1-(3-dimethylamino-2,2-dimethyl-propyl)-2-methyl-1 H-benzimidazol-5-yl]-N-hydroxy-acrylamide, (E)-3-[1-(2-diethylamino-ethyl)-2-(2,2-dimethyl-propyl)-1H-benzimidazol-5-yl]-N-hydroxy-acrylamide, (E)-N-hydroxy-3-[1-(3-isopropylamino-propyl)-2-(3,3,3-trifluoro-propyl)-1-H-benzimidazol-5-yl]-acrylamide, (E)-3-[2-(2,2-dimethyl-propyl)-1-(2-isopropylamino-ethyl)-1H-benzimidazol-5-yl]-N-hydroxy-acrylamide, (E)-3-[1-(2-diisopropylamino-ethyl)-2-(2,2-dimethyl-propyl)-1 H-benzimidazol-5-yl]-N-hydroxy-acrylamide, (E)-3-[1-(2-diisopropylamino-ethyl)-2-isobutyl-1H-benzimidazol-5-yl]-N-hydroxy-acrylamide, (E)-3-[1-(3-dimethylamino-2,2-dimethylpropyl)-2-hex-3-enyl-1H-benzimidazol-5-yl]-N-hydroxy-acrylamide, (E)-3-[1-(3-dimethylamino-2,2-dimethyl-propyl)-2-(2,4,4-trimethyl-pentyl)-1H-benzimidazol-5-yl]-N-hydroxy-acrylamide, (E)-3-[2-cyclohexyl-1-(3-dimethylamino-2,2-dimethyl-propyl)-1H-benzimidazol-5-yl]-N-hydroxy-acrylamide, (E)-3-[2-bicyclo[2.2.1]hept-5-en-2-yl-1-(3-dimethylamino-2,2-dimethyl-propyl)-1H-benzimidazol-5-yl]-N-hydroxy-acrylamide, (E)-3-[1-(2-diethylamino-ethyl)-2-hex-3-enyl-1H-benzimidazol-5-yl]-N-hydroxy-acrylamide, (E)-3-[1-(2-diisopropylamino-ethyl)-2-hex-3-enyl-1 H-benzimidazol-5-yl]-N-hydroxy-acrylamide, (E)-3-[2-hex-3-enyl-1-(2-isopropylamino-ethyl)-1H-benzimidazol-5-yl]-N-hydroxy-acrylamide, (E)-3-[2-hex-3-enyl-1-(3-isopropylamino-propyl)-1H-benzimidazol-5-yl]-N-hydroxy-acrylamide, (E)-3-[1-(2-ethylamino-ethyl)-2-hex-3-enyl-1H-benzimidazol-5-yl]-N-hydroxy-acrylamide, (E)-3-[1-(2-diethylamino-ethyl)-2-hexyl-1H-benzimidazol-5-yl]-N-hydroxy-acrylamide, (E)-N-hydroxy-3-[1-(3-isopropylamino-propyl)-2-(2,4,4-trimethyl-pentyl)-1H-benzimidazol-5-yl]-acrylamide, (E)-3-[2-(2,2-dimethylpropyl)-1-(3-isopropylamino-propyl)-1H-benzimidazol-5-yl]-N-hydroxy-acrylamide, (E)-3-[1-(2-diisopropylamino-ethyl)-2-(3,3,3-trifluoro-propyl)-1H-benzimidazol-5-yl]-N-hydroxy-acrylamide, and (E)-N-hydroxy-3-[2-isobutyl-1-(2-isopropylamino-ethyl)-1H-benzimidazol-5-yl]-acrylamide.

The use of O6-methylguanine-DNA-methyltransferase (MGMT) inhibitors is described in United States Patent Application 2010/0093647 by Liu et al.. Suitable MGMT inhibitors described herein include, but are not limited to, O⁶-benzylguanine, O⁶-2-fluoropyridinylmethylguanine, O⁶-3-iodobenzyl guanine, O⁶-4-bromophenylguanine, O⁶-5-iodophenylguanine O⁶-benzyl-8-oxoguanine, O⁶-(*p*-chlorobenzyl)guanine, O⁶-(*p-*methylbenzyl)guanine, O⁶-(*p*-bromobenzyl)guanine, O⁶-(*p*-isopropylbenzyl)guanine, O⁶-(3,5-dimethylbenzyl)guanine, O⁶-(*p*-n-butylbenzyl)guanine, O⁶-(*p-*hydroxymethybenzyl)guanine, O⁶-benzylhypoxanthine, N²-acetyl-O⁶-benzylguanine, N²-acetyl-O⁶-benzyl-8-oxo-guanine, 2-amino-6-(p-methyl-benzyl-thio)purine, 2-amino-6-(benzyloxy)-9-[(ethoxycarbonyl)methyl]purine, 2-amino-6-(benzyloxy)-9-(pivaloyloxymethyl)purine, 2-amino-6-(benzyl-thio)purine, O⁶-benzyl-7,8-dihydro-8-oxoguanine, 2,4,5-triamino-6-benzyloxyprimidine, O⁶-benzyl-9-[(3-oxo-5α-androstan-17β-yloxycarbonylmethyl]guanine, O⁶-benzyl-9-[(3-oxo-4-androsten-17β-yloxycarbonyl)methyl(guanine, 8-amino-O⁶-benzylguanine (8-amino-BG), 2,4-diamino-6-benzyloxy-5-nitrosopyrimidine, 2,4-diamino-6-benzyloxy-5-nitropyrimidine, and 2-amino-4-benzyloxy-5-nitropyrimidine.

The use of CCR9 inhibitors is described in United States Patent Application Publication No. 2010/0075963 by Lehr et al.. These CCR9 inhibitors described herein include, but are not limited to, benzylsulfonylindoles.

The use of acid sphingomyelinase inhibitors is described in United States Patent Application Publication No. 2010/0022482 by Baumann et al.. Typically, these compounds are biphenyl derivatives.

The use of peptidomimetic macrocycles is described in United States Patent Application Publication No. 2009/0275519 by Nash et al..

The use of cholanic acid amides is described in United States Patent Application Publication No. 2009/0258847 by Schreiner et al.. These cholanic acid amides described herein include, but are not limited to, substituted 4-(3-hydroxy-10,13-hydroxymethyl-hexadecahydro-cyclopenta(a)-phenanthren-17-yl)pentanoic acid amides.

The use of substituted oxazaphosphorines is described in United States Patent Application Publication No. 2009/0202540. The oxazaphosphorine described herein can be, but is not limited to, ifosphamide and cyclophosphamide.

The use of anti-TWEAK receptor antibodies is described in United States Patent Application Publication No. 2009/0074762 by Culp. The TWEAK receptor is a member of the tumor necrosis receptor superfamily and is expressed on the surface of cancer cells in a number of solid tumors.

The use of ErbB3 binding protein is described in United States Patent Application Publication No. 2008/0269133 by Zhang et al..

The use of a glutathione S-transferase-activated (GST-activated) anti-neoplastic compound is described in United States Patent Application Publication No. 2008/0166428 by Brown et al.. A preferred GST-activated anti-neoplastic compound is canfosfamide.

The use of substituted phosphorodiamidates is described in United States Patent Application Publication No. 2008/0125398 by Ma et al., which describes 2-{[2-(substituted amino)ethyl]sulfonyl}ethyl N,N,N',N'-tetrakis(2-chloroethyl)-phosphorodiamidates, and in United States Patent Application Publication No. 2008/0125397 by Lui et al., which describes 2-({2-oxo-2-[(pyridin-3-ylmethyl)amino]ethyl}sulfonyl)ethyl N,N,N',N'-tetrakis(2-chloroethyl)phosphorodiamidate. The use of substituted phosphorodiamidates is also described in United States Patent Application Publication No. 2008/0039429 by Allen et al., which describes sulfonylethyl and thioethyl phosphorodiamidates.

The use of inhibitors of MEKK protein kinase is described in United States Patent Application Publication No. 2006/0100226 by Sikorski et al. These inhibitors described herein include, but are not limited to, 2-thiopyrimidinones, such as 2-[3-(3,4-dichloro-benzylamino)-benzylsulfanyl]-4-(3-methoxy-phenyl)-6-oxo-1,6-dihydro-pyrimidine-5-carbonitrile, 2-[3-(3,4-dichloro-benzylamino)-benzylsulfanyl]-4-(3,4-dimethoxy-phenyl)-6-oxo-1,6-dihydro-pyrimidine-5-carbonitrile, and 2-[3-(3,4-dichloro-benzylamino)-benzylsulfanyl-4-(4-methoxy-3-thiophen-2-yl-phenyl)-6-oxo-1,6-dihydro-pyrimidine-5-carbonitrile.

The use of COX-2 inhibitors is described in United States Patent Application Publication No. 2004/0072889 by Masferrer et al. Suitable COX-2 inhibitors described herein include, but are not limited to, celecoxib, parecoxib, deracoxib, rofecoxib, etoricoxib, valdecoxib, and meloxicam.

The use of cimetidine and N-acetylcysteine is described in United States Patent Application Publication No. 2003/0158118 by Weidner. In one disclosure, derivatives of cimetidine or N-acetylcysteine can also be used.

The use of an anti-IL-6 receptor antibody is described in United States Patent Application Publication No. 2002/0131967 by Nakamura et al.. The antibody can be a humanized antibody.

The use of an antioxidant is described in United States Patent Application Publication No. 2001/0049349 by Chinery et al. Suitable antioxidants described herein include, but are not limited to, pyrrolidinedithiocarbamate, probucol (4,4'-(isopropylidenedithio)bis(2,6-di-*t*-butylphenol), vitamin C, vitamin E, and 6-hydroxy-2,5,7,8-tetramethylchroman-2-carboxylic acid.

The use of an isoxazole inhibitor of tubulin polymerization is described in United States Patent No. 8,269,017 by Sun et al. Suitable isoxazole inhibitors of tubulin polymerization described herein include, but are not limited to, 2-amino-N-(2-methoxy-5-[5-(3,4,5-trimethoxyphenyl)-isoxazol-4-yl)-phenyl)acetamide hydrochloride; 2-amino-3-hydroxy-N-(2-methoxy-5-[5-(3,4,5-trimethoxyphenyl)isoxazol-4-yl)-phenyl)propanamide hydrochloride; 2-amino-N-(2-methoxy-5-[5-(3,4,5-trimethoxyphenyl)isoxazol-4-yl)-phenyl)propanamide; 2-amino-N-(2-methoxy-5-[5-(3,4,5-trimethoxyphenyl)-isoxazol-4-yl)-phenyl)-4-(methylthio)butanamide hydrochloride; 2-amino-N-(2-methoxy-5-[5-(3,4,5-trimethoxyphenyl)-isoxazol-4-yl)-phenyl)butanamide; 2-amino-N-(2-methoxy-5-[5-(3,4,5-trimethoxyphenyl)-isoxazol-4-yl)-phenyl)-3-phenylpropanamide hydrochloride; 2-amino-N-(2-methoxy-5-[5-(3,4,5-trimethoxyphenyl)-isoxazol-4-yl)-phenyl)-4-methylpentanamide hydrochloride; 2-amino-N-(2-methoxy-5-[5-(3,4,5-trimethoxy-phenyl)-isoxazol-4-yl)-phenyl)-3-(4-methoxyphenyl)propanamide hydrochloride; 1-{2-methoxy-5-[5-(3,4,5-trimethoxyphenyl)-isoxazol-4-yl]-phenylcarbamoyl}-2-methyl-propyl-ammonium chloride; 1-{2-methoxy-5-[5-(3,4,5-trimethoxyphenyl)-isoxazol-4-yl]-phenylcarbamoyl}-2-methyl-butylammonium chloride; 2-hydroxy-1-{2-methoxy-5-[5-(3,4,5-trimethoxyphenyl)-isoxazol-4-yl]-phenylcarbamoyl}-propyl-ammonium chloride; 2-(4-hydroxy-phenyl)-1-{2-methoxy-5-[5-(3,4,5-trimethoxyphenyl)-isoxazol-4-yl]-phenylcarbamoyl}-ethyl-ammonium chloride; C-{2-methoxy-5-[5-(3,4,5-trimethoxyphenyl)-isoxazol-4-yl]-phenylcarbamoyl}-C-phenylmethyl-ammonium chloride; 2-(1H-indol-2-yl)-1-{2-methoxy-5-[5-(3,4,5-trimethoxyphenyl)-isoxazol-4-yl]-phenylcarbamoyl}-ethyl-ammonium chloride; 2-benzofuran-2-yl-1-{2-methoxy-5-[5-(3,4,5-trimethoxyphenyl)-isoxazol-4-yl]-phenylcarbamoyl}-ethyl-ammonium chloride; 2-carboxyl-1-{2-methoxy-5-[5-(3,4,5-trimethoxyphenyl)-isoxazol-4-yl]-phenylcarbamoyl}-ethyl-ammonium chloride; 3-carboxyl-1-{2-methoxy-5-[5-(3,4,5-trimethoxyphenyl)-isoxazol-4-yl]-phenylcarbamoyl}-propyl-ammonium chloride; 3-carbamoyl-1-{2-methoxy-5-[5-(3,4,5-trimethoxyphenyl)-isoxazol-4-yl]-phenylcarbamoyl}-propyl-ammonium chloride; 2-carbamoyl-1-{2-methoxy-5-[5-(3,4,5-trimethoxyphenyl)-isoxazol-4-yl]-phenylcarbamoyl}-ethyl-ammonium chloride; and 2-(3H-imidazol-4-yl)-1-{2-methoxy-5-[5-(3,4,5-trimethoxyphenyl)-isoxazol-4-yl]-phenylcarbamoyl}-ethyl-ammonium chloride.

The use of pyridazinone PARP inhibitors is described in United States Patent No. 8,268,827 by Branca et al..
Pyridazinone PARP inhibitors include, but are not limited to, 6-{4-fluoro-3-[(3-oxo-4-phenylpiperazin-1-yl)carbonyl]benzyl}-4,5-dimethyl-3-oxo-2,3-dihydropyridazin-1-ium trifluoroacetate; 6-{3-[(4-cyclohexyl-3-oxopiperazin-1-yl)carbonyl]-4-fluorobenzyl}-4,5-dimethyl-3-oxo-2,3-dihydropyridazin-1-ium trifluoroacetate; 6-{3-[(4-cyclopentyl-3-oxopiperazin-1-yl)carbonyl]-4-fluorobenzyl}-4,5-dimethylpyridazin-3(2H)-one; 6-{4-fluoro-3-[(3-oxo-4-phenylpiperazin-1-yl)carbonyl]benzyl}-4,5-dimethylpyridazin-3(2H)-one hydrochloride; 4-ethyl-6-{4-fluoro-3-[(3-oxo-4-phenylpiperazin-1-yl)carbonyl]benzyl}pyridazin-3(2H)-one trifluoroacetate; 6-{3-[(4-cyclohexyl-3-oxopiperazin-1-yl)carbonyl]-4-fluorobenzyl}-4-ethylpyridazin-3(2H)-one trifluoroacetate; 3-{4-fluoro-3-[(4-methyl-3-oxopiperazin-1-yl)carbonyl]benzyl}-4,5-dimethyl-6-oxo-1,6-dihydropyridazin-1-ium trifluoroacetate; 3-(4-fluoro-3-{[4-(4-fluorobenzyl)-3-oxopiperazin-1-yl]carbonyl}benzyl)-4,5-dimethyl-6-oxo-1,6-dihydropyridazin-1-ium trifluoroacetate; 6-(3-{[4-(2-chlorophenyl)-3-oxopiperazin-1-yl]carbonyl}-4-fluorobenzyl)-4,5-dimethyl-3-oxo-2,3-dihydropyridazin-1-ium trifluoroacetate; 6-(3-{[4-(3-chloro-4-fluorophenyl)-3-oxopiperazin-1-yl]carbonyl}-4-fluorobenzyl)-4,5-dimethyl-3-oxo-2,3-dihydropyridazin-1-ium trifluoroacetate; and 6-(3-{[4-(3,4-difluorophenyl)-3-oxopiperazin-1-yl]carbonyl}-4-fluorobenzyl)-4,5-dimethyl-3-oxo-2,3-dihydropyridazin-1-ium trifluoroacetate. Other PARP inhibitors are described in United States Patent No. 8,143,447 by Moore et al.; these compounds include nitrobenzamide derivatives.

The use of Aurora protein kinase inhibitors is described in United States Patent No. 8,268,811 to Mortimore et al.. The Aurora protein kinase inhibitors include, but are not limited to, thiazoles and pyrazoles. The use of Aurora protein kinase inhibitors is also described in United States Patent No. 8,129,399 to Binch et al.; these Aurora protein kinase inhibitors include, but are not limited to, aminopyridines.

The use of peptides binding to prostate-specific membrane antigen (PSMA) is described in United States Patent No. 8,258,256 to Denmeade et al..

The use of CD19 binding agents is described in United States Patent No. 8,242,252 to McDonagh et al.. These CD19 binding agents described herein include, but are not limited to, anti-CD19 antibodies.

The use of benzodiazepines is described in United States Patent No. 8,242,109 to Glick.

The use of Toll-like receptor (TLR) agonists is described in United States Patent No. 8,242,106 to Howbert et al.. Suitable TLR agonists described herein include, but are not limited to, (1E, 4E)-2-amino-N,N-dipropyl-8-(4-(pyrrolidine-1-carbonyl)phenyl)-3H-benzo[b]azepine-4-carboxamide.

The use of bridged bicyclic sulfamides is described in United States Patent No. 8,242,103 to Lewis et al..

The use of inhibitors of epidermal growth factor receptor (EGFR) kinase is described in United States Patent No. 8,242,080 to Kuriyan et al.. Typically, these inhibitors of EGFR kinase target the asymmetric activating dimer interface.

The use of ribonucleases of the T2 family having actin-binding activity is described in United States Patent No. 8,236,543 to Roiz et al.. Typically, the ribonuclease binds actin in either its active or inactive ribonucleolytic form.

The use of myrsinoic acid A or an analog thereof is described in United States Patent No. 8,232,318 to Lee et al..

The use of an inhibitor of a cyclin-dependent kinase is described in United States Patent No. 8,227,605 to Shipps et al.; these inhibitors include, but are not limited to, 2-aminothiazole-4-carboxylic amides. Use of an inhibitor of a cyclin-dependent kinase is also described in United States Patent No. 7,700,773 to Mallams et al.; these inhibitors include, but are not limited to, 4-cyano, 4-amino, and 4-aminomethyl derivatives of pyrazolo[1,5-a]pyridine, pyrazolo[1,5-c]pyrimidine, and 2H-indazole compounds and 5-cyano, 5-amino, and 5-aminomethyl derivatives of imidazo[1,2-a]pyridine and imidazo[1,5-a]pyrazine compounds.

The use of an inhibitor of the interaction between p53 and MDM2 is described in United States Patent No. 8,222,288 to Wang et al..

The use of inhibitors of the receptor tyrosine kinase MET is described in United States Patent No. 8,222,269 to Dinsmore et al.. These inhibitors of the receptor tyrosine kinase MET include, but are not limited to, 5H-benzo[4,5]cyclohepta[1,2-b]pyridine derivatives. Inhibitors of the receptor tyrosine kinase MET are also described in United States Patent No. 8,207,186 to Jewell et al.. These compounds described herein include, but are not limited to, benzocycloheptapyridines, including 5H-benzo[4,5]cyclohepta[1,2-b]pyridine derivatives.

The use of largazole or largazole analogs is described in United States Patent No. 8,217,076 to Williams et al..

The use of inhibitors of the protein kinase AKT is described in United States Patent No. 8,207,169 to Furuyama et al.; these inhibitors include, but are not limited to, triazolopyridopyridines, including substituted [1,2,4]triazolo[4',3':1,6]pyrido[2,3-b]pyrazines.

The use of 2'-fluoro-5-methyl-β-L-arabinofuranosyluridine or L-deoxythymidine is described in United States Patent No. 8,207,143 to Cheng.

The use of compounds that modulate HSP90 activity is described in United States Patent No. 8,188,075 to Ying et al.. These compounds described herein include, but are not limited to, substituted triazoles, including 3-(2-hydroxyphenyl)-4-(naphthalen-1-yl)-5-mercaptotriazole; 3-(2,4-dihydroxyphenyl)-4-[4-(2-methoxyethoxy)-naphthalen-1-yl]-5-mercaptotriazole; 3-(2,4-dihydroxyphenyl)-4-(2-methyl-4-bromophenyl)-5-mercaptotriazole; 3-(3,4-dihydroxyphenyl)-4-(6-methoxy-naphthalen-1-yl)-5-mercaptotriazole; 3-(3,4-dihydroxyphenyl)-4-(6-ethoxy-naphthalen-1-yl)-5-mercaptotriazole; 3-(3,4-dihydroxyphenyl)-4-(6-propoxy-naphthalen-1-yl)-5-mercaptotriazole; 3-(2,4-dihydroxy-5-ethyl-phenyl)-4-(5-methoxy-naphthalen-1-yl)-5-mercaptotriazole; 3-(3,4-dihydroxyphenyl)-4-(6-isopropoxy-naphthalen-1-yl)-5-mercaptotriazole; 3-(2,4-dihydroxyphenyl)-4-(2,6-diethylphenyl)-5-mercaptotriazole; 3-(2,4-dihydroxyphenyl)-4-(2-methyl-6-ethylphenyl)-5-mercaptotriazole; 3-(2,4-dihydroxyphenyl)-4-(2,6-diisopropylphenyl)-5-mercaptotriazole; 3-(2,4-dihydroxyphenyl)-4-(1-ethyl-indol-4-yl)-5-mercaptotriazole; and 3-(2,4-dihydroxyphenyl)-4-(2,3-dihydro-benzo[1,4]dioxin-5-yl)-5-mercaptotriazole.

The use of inhibitors of a JAK kinase or PDK kinase is described in United States Patent No. 8,183,245 to Guerin et al. JAK kinases include JAK1, JAK2, JAK3, and TYK2. Suitable inhibitors of these classes of kinases include, but are not limited to, 5-(1-methyl-1H-pyrazol-4-yl)-3-(6-piperazin-1-ylpyrazin-2-yl)-1H-pyrrolo[2,3-b]pyridine; 5-(1-methyl-1H-pyrazol-4-yl)-3-[6-(piperidin-4-yloxy)pyrazin-2-yl]-1H-pyrrolo[2,3-b]pyridine; 3-[6-(cyclohexyloxy)pyrazin-2-yl]-5-(1-methyl-1H-pyrazol-4-yl)-1 H-pyrrolo[2,3-b]pyridine; N-methyl-6-[5-(1-methyl-1H-pyrazol-4-yl)-1H-pyrrolo[2,3-b]pyridin-3-yl]-N-piperidin-4-ylpyrazin-2-amine; 3-[6-(piperidin-4-yloxy)pyrazin-2-yl]-5-(1H-pyrazol-4-yl)-1H-pyrrolo[2,3-b]pyridine; 3-{6-[(3R)-piperidin-3-yloxy]pyrazin-2-yl}-5-(1H-pyrazol-4-yl)-1H-pyrrolo[2,3-b]pyridine; and 3-{6-[(3S)-piperidin-3-yloxy]pyrazin-2-yl}-5-(1H-pyrazol-4-yl)-1H-pyrrolo[2,3-b]pyridine.

The use of inhibitors of phosphodiesterase type IV (PDE4) is described in United States Patent No. 8,158,672 to Muller et al. The inhibitors of PDE4 include fluoroalkoxy-substituted 1,3-dihydroisoindolyl compounds.

The use of inhibitors of c-Met proto-oncogene receptor tyrosine kinase is described in United States Patent No. 8,143,251 to Zhuo et al. These inhibitors include, but are not limited to, triazolotriazines, including [1,2,4]triazolo[4,3-b][1,2,4]triazines. Inhibitors of c-Met proto-oncogene receptor tyrosine kinase are also described in United States Patent No. 8,106,197 to Cui et al.; these inhibitors include aminoheteroaryl compounds.

The use of inhibitors of indoleamine 2,3-dioxygenase is described in United States Patent No. 8,088,803 to Combs et al.; these inhibitors include, but are not limited to, 1,2,5-oxadiazole derivatives.

The use of agents that inhibit ATDC (TRIM29) expression is described in United States Patent No. 8,088,749 to Simeone et al. These agents include oligonucleotides that function via RNA interference.

The use of proteomimetic inhibitors of the interaction of nuclear receptor with coactivator peptides is described in United States Patent No. 8,084,471 to Hamilton et al. These inhibitors include, but are not limited to, 2,3',3"-trisubstituted terphenyls.

The use of antagonists of XIAP family proteins is described in United States Patent No. 7,910,621 to Chen et al. These antagonists include, but are not limited to, embelin.

The use of tumor-targeted superantigens is described in United States Patent No. 7,763,253 to Hedlund et al..

The use of inhibitors of Pim kinases is described in United States Patent No. 7,750,007 to Bearss et al.. These inhibitors described herein include, but are not limited to, imidazo[1,2-b]pyridazine and pyrazolo[1,5-a]pyrimidine compounds.

The use of inhibitors of CHK1 or CHK2 kinases is described in United States Patent No. 7,732,436 to Tepe. These inhibitors described herein include, but are not limited to, indoloazepines and acid amine salts thereof.

The use of inhibitors of angiopoietin-like 4 protein is described in United States Patent No. 7,740,846 to Gerber et al.. These inhibitors described herein include, but are not limited to, antibodies, including monoclonal antibodies.

The use of inhibitors of Smo is described in United States Patent No. 7,691,997 to Balkovec et al.. Smo, or Smoothened, is a mediator of signaling by hedgehog proteins. Suitable inhibitors described herein include, but are not limited to, 5-(1,1-difluoroethyl)-3-(4-{4-methyl-5-[2-(trifluoromethyl)phenyl]-4H-1,2,4-triazol-3-yl}bicyclo [2.2.2]oct-1-yl)-1,2,4-oxadiazole; 5-(3,3-difluorocyclobutyl)-3-(4-{4-methyl-5-[2-(trifluoromethyl)phenyl]-4H-1,2,4-triazol-3-yl}bicyclo[2.2.2]oct-1-yl)-1,2,4-oxadiazole; 5-(1-fluoro-1-methylethyl)-3-(4-{4-methyl-5-[2-(trifluoromethyl)phenyl]-4H-1,2,4-triazol-3-yl}bicyclo[2.2.2]oct-1-yl)-1,2,4-oxadiazole; 2-(1,1-difluoroethyl)-5-(4-{4-methyl-5-[2-(trifluoromethyl)phenyl]-4H-1,2,4-triazol-3-yl}bicyclo[2.2.2]oct-1-yl)-1,3,4-oxadiazole; 2-(3,3-difluorocyclobutyl)-5-(4-{4-methyl-5-[2-(trifluoromethyl)phenyl]-4H-1,2,4-triazol-3-yl}bicyclo[2.2.2]oct-1-yl)-1,3,4-oxadiazole; and 2-(1-fluoro-1-methylethyl)-5-(4-{4-methyl-5-[2-(trifluoromethyl)phenyl]-4H-1,2,4-triazol-3-yl}bicyclo[2.2.2]oct-1-yl)-1,3,4-oxadiazole.

The use of nicotinic acetylcholine receptor antagonists is disclosed in United States Patent No. 7,652,038 to Cooke et al.. Nicotinic acetylcholine receptor antagonists described herein include, but are not limited to, mecamylamine, hexamethonium, dihydro-P-erythroidine, d-tubocurarine, pempidine, chlorisondamine, erysodine, trimethaphan camsylate, pentolinium, bungarotoxin, succinylcholine, tetraethylammonium, trimethaphan, chlorisondamine, and trimethidinium.

The use of farnesyl protein transferase inhibitors is described in United States Patent No. 7,557,107 to Zhu et al.. These farnesyl protein transferase inhibitors include tricyclic compounds.

The use of adenosine A3 receptor antagonists is described in United States Patent No. 6,326,390 to Leung et al.. These adenosine A3 receptor antagonists include tricyclic non-xanthine antagonists and triazoloquinazolines.

United States Patent Application Publication No. 2010/0069458 by Atadja et al., discloses the use of the following additional therapeutic agents, which in one disclosure can be used together with an alkylating hexitol derivative as described above:
(1) ACE inhibitors, including, but not limited to, benazepril, enazepril, captopril, enalapril, fosinopril, lisinopril, moexipril, quinapril, ramipril, perindopril and trandolapril;
(2) adenosine kinase inhibitors, including, but not limited to, 5-iodotubericidin;
(3) adrenal cortex antagonists, including, but not limited to, mitotane;
(4) AKT pathway inhibitors (protein kinase B inhibitors) including, but not limited to, deguelin and 1,5-dihydro-5-methyl-1-β-D-ribofuranosyl-1,4,5,6,8-pentaazaacenaphthylen-3-amine;
(5) angiogenesis inhibitors, including, but not limited to, fumagillin, Shikonin, Tranilast, ursolic acid; suramin; thalidomide, lenalidomide; phthalazines, including, but not limited to, 1-(4-chloroanilino)-4-(4-pyridylmethyl)phthalazine, 1-(4-methylanilino)-4-(4-pyridylmethyl)phthalazine, 1-(3-chloroanilino)-4-(4-pyridylmethyl)phthalazine, 1-anilino-4-(4-pyridylmethyl)phthalazine, 1-benzylamino-4-(4-pyridylmethyl)phthalazine, 1-(4-methoxyanilino)-4-(4-pyridylmethyl)phthalazine, 1-(3-benzyloxyanilino)-4-(4-pyridylmethyl)phthalazine, 1-(3-methoxyanilino)-4-(4-pyridylmethyl)phthalazine, 1-(2-methoxyanilino}-4-(4-pyridylmethyl)phthalazine, 1-(4-trifluoromethylanilino)-4-(4-pyridylmethyl)phthalazine, 1-(4-fluoroanilino)-4-(4-pyridylmethyl)phthalazine, 1-(3-hydroxyanilino)-4-(4-pyridylmethyl)phthalazine, 1-(4-hydroxyanilino)-4-(4-pyridylmethyl)phthalazine, 1-(3-aminoanilino)-4-(4-pyridylmethyl)phthalazine, 1-(3,4-dichloroanilino)-4-(4-pyridylmethyl)phthalazine, 1-(4-bromoanilino)-4-(4-pyridylmethyl)phthalazine, 1-(3-chloro-4-methoxyanilino)-4-(4-pyridylmethyl)phthalazine, 1-(4-cyanoanilino)-4-(4-pyridylmethyl)phthalazine, 1-(3-chloro-4-fluoroanilino)-4-(4-pyridylmethyl)phthalazine, 1-(3-methylanilino)-4-(4-pyridylmethyl)phthalazine, and other phthalazines disclosed in PCT Patent Application Publication No. WO 98/035958 by Bold et al., isoquinolines disclosed in PCT Patent Application Publication No. WO 00/09495 by Altmann et al., including 1-(3,5-dimethylanilino)-4-(pyridin-4-ylmethyl)-isoquinoline; phthalazines disclosed in PCT Patent Application Publication No. WO 00/59509 by Bold et al., including *E*-1-(3-methylanilino)-4-[(2-(pyridin-3-yl)vinyl]phthalazine, *Z*-1-(3-methylanilino)-4-[(2-(pyridin-3-yl)vinyl]phthalazine, 1-(3-methylanilino)-4-[(2-(pyridin-3-yl)ethyl]phthalazine, 1-(3-methylanilino)-4-[{2-(pyridin-4-yl)vinyl]phthalazine, 1-(4-chloro-3-trifluoromethylanilino)-4-[(2-(pyridin-3-yl)ethyl]phthalazine, 1-(4-chloroanilino)-4-[(2-(pyridin-3-yl)ethyl]phthalazine, 1-(3-chlorobenzylamino)-4-[(2-(pyridin-3-yl)ethyl]phthalazine, 1-(4-chloro-3-trifluoromethylanilino)-4-[3-(pyridin-3-yl)propyl]phthalazine, 1-(4-chloroanilino)-4-[3-(pyridin-3-yl)propyl]phthalazine, 1-(3-chloro-5-trifluoromethylanilino)-4-[3-(pyridin-3-yl)propyl]phthalazine, and 1-(4-*tert*-butylanilino)-4-[3-(pyridin-3-yl)propyl]phthalazine; and monoclonal antibodies;
(6) angiostatic steroids, including, but not limited to, anecortave, triamcinolone, hydrocortisone, 11α-epihydrocotisol, cortexolone, 17α-hydroxyprogesterone, corticosterone, desoxycorticosterone, testosterone, estrone, and dexamethasone;
(7) anti-androgens, including, but not limited to, nilutamide and bicalutamide;
(8) anti-estrogens, including, but not limited to, toremifene, letrozole, testolactone, anastrozole, bicalutamide, flutamide, exemestane, tamoxifen, fulvestrant, and raloxifene;
(9) anti-hypercalcemia agents, including, but not limited to, gallium (III) nitrate hydrate and pamidronate disodium;
(10) apoptosis inducers, including, but not limited to, 2-[[3-(2,3-dichlorophenoxy)propyl]amino]-ethanol, gambogic acid, embellin, and arsenic trioxide;
(11) ATI receptor antagonists, including, but not limited to, valsartan;
(12) aurora kinase inhibitors, including, but not limited to, binucleine 2;
(13) aromatase inhibitors, including, but not limited to: (a) steroids, including, but not limited to, atamestane, exemestane, and formestane; and (b) non-steroids, including, but not limited to, aminoglutethimide, roglethimide, pyridoglutethimide, trilostane, testolactone, ketokonazole, vorozole, fadrozole, anastrozole, and letrozole;
(14) bisphosphonates, including, but not limited to, etidronic acid, clodronic acid, tiludronic acid, alendronic acid, ibandronic acid, risedronic acid, and zoledronic acid;
(15) Bruton's tyrosine kinase inhibitors, including, but not limited to, terreic acid;
(16) calcineurin inhibitors, including, but not limited to, cypermethrin, deltamethrin, fenvalerate, and tyrphostin 8;
(17) CaM kinase II inhibitors, including, but not limited to, the 5-isoquinolinesulfonic acid 4-[(2S)-2-[(5-isoquinolinylsulfonyl)methylamino]-3-oxo-3-(4-phenyl-1-piperazinyl)propyl]phenyl ester, and N-[2-[[[3-(4-chlorophenyl)-2-propenyl]methyl]amino]methyl]phenyl]-N-(2-hydroxyethyl)-4-methoxy-benzenesulfonamide;
(18) CD45 tyrosine phosphatase inhibitors, including, but not limited to, [[2-(4-bromophenoxy)-5-nitrophenyl]hydroxymethyl]-phosphonic acid;
(19) CDC25 phosphatase inhibitors, including, but not limited to, 2,3-bis[(2-hydroyethyl)thio]-1,4-naphthalenedione;
(20) CHK kinase inhibitors, including, but not limited to, debromohymenialdisine;
(21) compounds targeting/decreasing a protein or lipid kinase activity; or a protein or lipid phosphatase activity; or further anti-angiogenic compounds, including, but not limited to, protein tyrosine kinase and/or serine and/or threonine kinase inhibitors or lipid kinase inhibitors, including, but not limited to:
   (a) compounds targeting, decreasing or inhibiting the activity of the vascular endothelial growth factor receptors (VEGFR) or of vascular endothelial growth factor (VEGF), including, but not limited to, 7H-pyrrolo[2,3-d]pyrimidine derivatives, including: [6-[4-(4-ethyl-piperazine-1-ylmethyl)-phenyl]-7H-pyrrolo[2,3-d]pyrimidinpyrimidin-4-yl]-(R)-1-phenyl-ethyl)-amine (known as AEE788), BAY 43-9006; and isoquinoline compounds disclosed in PCT Patent Application Publication No. WO 00/09495, such as (4-tert-butyl-phenyl)-94-pyridin-4-ylmethyl-isoquinolin-1-yl)-amine;
   (b) compounds targeting, decreasing or inhibiting the activity of the platelet-derived growth factor-receptor (PDGFR), including, but not limited to: N-phenyl-2-pyrimidine-amine derivatives, e.g., imatinib, SU101, SU6668 and GFB-111;
   (c) compounds targeting, decreasing or inhibiting the activity of the fibroblast growth factor-receptor (FGFR);
   (d) compounds targeting, decreasing or inhibiting the activity of the insulin-like growth factor receptor 1 (IGF-1R), including, but not limited to: the compounds disclosed in WO 02/092599 and derivatives thereof of 4-amino-5-phenyl-7-cyclobutyl-pyrrolo[2,3-d]pyrimidine derivatives;
   (e) compounds targeting, decreasing or inhibiting the activity of the Trk receptor tyrosine kinase family;
   (f) compounds targeting, decreasing or inhibiting the activity of the Axl receptor tyrosine kinase family;
   (g) compounds targeting, decreasing or inhibiting the activity of the c-Met receptor;
   (h) compounds targeting, decreasing or inhibiting the activity of the Ret receptor tyrosine kinase;
   (i) compounds targeting, decreasing or inhibiting the activity of the Kit/SCFR receptor tyrosine kinase;
   (j) compounds targeting, decreasing or inhibiting the activity of the C-kit receptor tyrosine kinases, including, but not limited to, imatinib;
   (k) compounds targeting, decreasing or inhibiting the activity of members of the c-Abl family and their gene-fusion products, e.g., BCR-Abl kinase, such as N-phenyl-2-pyrimidine-amine derivatives, including, but not limited to: imatinib, 6-(2,6-dichlorophenyl)-2-[(4-fluoro-3-methylphenyl)amino]-8-methyl-pyrido[2,3-d]pyrimidin-7(8H)-one (PD180970), methyl-4-[N-(2',5'-dihydroxybenzyl)amino]benzoate (Tyrphostin AG957), 4-[[(2,5-dihydroxyphenyl)methyl]amino]benzoic acid tricyclo[3.3.1.13,7]dec-1-yl ester (adaphostin or NSC 680410), 6-(2,6-dichlorophenyl)-8-methyl-2-(3-methylsulfanylanilino)pyrido[2,3-d]pyrimidin-7-one (PD173955), and desatinib;
   (l) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in United States Patent No. 5,093,330, such as, but not limited to, midostaurin; examples of further compounds include, e.g., UCN-01; safingol, sorafenib, Bryostatin 1; Perifosine; Ilmofosine; 3-[3-[2,5-Dihydro-4-(1-methyl-1*H*-indol-3-yl)-2,5-dioxo-1*H*-pyrrol-3-yl]-1*H*-indol-1-yl]propyl carbamimidothioic acid ester (RO 318220), 3-[(8*S*)-8-[(dimethylamino)methyl]-6,7,8,9-tetrahydropyrido[1,2-*a*]indol-10-yl]-4-(1-methyl-1*H*-indol-3-yl)-1*H*-pyrrole-2,5-dione (RO 320432), 12-(2-cyanoethyl)-6,7,12,13-tetrahydro-13-methyl-5-oxo-5H-indolo[2,3-a]pyrrolo[3,4-c]carbazole (GO 6976); Isis 3521; (S)-13-[(dimethylamino)methyl]-10,11,14,15-tetrahydro-4,9:16, 21-dimetheno-1H, 13H-dibenzo[e,k]pyrrolo[3,4-h][1,4,13]oxadiazacy clohexadecene-1,3(2H)-dione (LY333531), LY379196; isoquinoline compounds, such as those disclosed in PCT Patent Application Publication No. WO 00/09495; farnesyltransferase inhibitors, including, but not limited to, tipifarnib and lonafarnib; 2-(2-chloro-4-iodo-phenylamino)-N-cyclopropylmethoxy-3,4-difluoro-benzamide (PD184352); and QAN697, a PI3K inhibitor;
   (m) compounds targeting, decreasing or inhibiting the activity of protein-tyrosine kinase, such as, but not limited to, imatinib mesylate, a tyrphostin, pyrymidylaminobenzamide and derivatives thereof; a tyrphostin is preferably a low molecular weight (Mᵣ < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810, Tyrphostin AG 99, Tyrphostin AG 213, Tyrphostin AG 1748, Tyrphostin AG 490, Tyrphostin B44, Tyrphostin B44 (+) enantiomer, Tyrphostin AG 555, AG 494, Tyrphostin AG 556; Tyrphostin AG957, and adaphostin (4-{[(2,5-dihydroxyphenyl)methyl]amino}-benzoic acid adamantyl ester or NSC 680410);
   (n) compounds targeting, decreasing or inhibiting the activity of the epidermal growth factor family of receptor tyrosine kinases (EGFR, ErbB2, ErbB3, ErbB4 as homodimers or heterodimers), such as, but not limited to, those compounds, proteins or monoclonal antibodies generically and specifically disclosed in PCT Patent Application Publication No. WO 97/02266 by Traxler et al. such as (R)-6-(4-hydroxyphenyl)-4-[(1-phenylethyl)-amino]-7H-pyrrolo-[2,3-d]pyrimidine, or in European Patent Application Publication No. EP 0564409 by Zimmermann, PCT Patent Application Publication No. WO 99/03854 by Zimmermann et al., European Patent Application Publication No. EP 0520722 by Barker et al., European Patent Application Publication No. EP 0566226 by Barker et al., European Patent Application Publication EP 0787722 by Wissner et al., European Patent Application Publication EP 0837063 by Arnold et al., U.S. Pat. No. 5,747,498 by Schnur et al., PCT Patent Application Publication WO 98/10767 by McMahon et al., PCT Patent Application Publication WO 97/30034 by Barker, PCT Patent Application Publication WO 97/49688 by Schnur, PCT Patent Application Publication WO 97/38983 by Bridges et al., PCT Patent Application Publication WO 96/30347 by Schnur et al., including, but not limited to, N-(3-ethylnylphenyl)-6,7-bis(2-methoxyethoxy)-4-quinazolinamine (CP 358774 or erlotinib), PCT Patent Application Publication WO 96/33980 by Gibson et al., including, but not limited to, *N*-(3-chloro-4-fluoro-phenyl)-7-methoxy-6-(3-morpholin-4-ylpropoxy)quinazolin-4-amine (gefitinib); and PCT Patent Application Publication WO 95/03283 by Barker et al., including, but not limited to, compound 6-amino-4-(3-methylphenyl-amino)-quinazoline (ZM105180); monoclonal antibodies, including, but not limited to trastuzumab and cetuximab; and other small molecule inhibitors, including, but not limited to: canertinib, pelitinib, lapatinib, and 7H-pyrrolo-[2,3-d]pyrimidine derivatives which are disclosed in PCT Patent Application Publication WO 03/013541 by Bold et al.;
(22) compounds which target, decrease or inhibit the activity of a protein or lipid phosphatase, including, but not limited to, inhibitors of phosphatase 1, phosphatase 2A, PTEN or CDC25, such as, but not limited to okadaic acid or a derivative thereof;
(23) compounds which induce cell differentiation processes, including, but not limited to, retinoic acid, α-tocopherol, γ-tocopherol, δ-tocopherol, α-tocotrienol, γ-tocotrienol, and δ-tocotrienol;
(24) cRAF kinase inhibitors, including, but not limited to, 3-(3,5-dibromo-4-hydroxybenzylidene)-5-iodo-1,3-dihydroindol-2-one and 3-(dimethylamino)-N-[3-[(4-hydroxybenzoyl)amino]-4-methylphenyl]-benzamide;
(25) cyclin dependent kinase inhibitors, including, but not limited to, N9-isopropyl-olomoucine; olomoucine; purvalanol B, roascovitine, kenpaullone, and purvalanol A;
(26) cysteine protease inhibitors, including, but not limited to, N-[(1S)-3-fluoro-2-oxo-1-(2-phenyl]ethyl)propyl]amino]-2-oxo-1-(phenylmethyl)ethyl]-4-morpholinecarboxamide;
(27) DNA intercalators, including, but not limited to, plicamycin and dactinomycin;
(28) DNA strand breakers, including, but not limited to, bleomycin;
(29) E3 ligase inhibitors, including, but not limited to, N-((3,3,3-trifluoro-2-trifluoromethyl)propionyl)sulfanilamide;
(30) EDG binders, including, but not limited to, FTY720;
(31) endocrine hormones, including, but not limited to, leuprolide and megestrol acetate;
(32) farnesyltransferase inhibitors, including, but not limited to, α-hydroxyfarnesylphosphonic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-1-methylethyl butanoic acid ester (2S), and manumycin A;
(33) Flk-1 kinase inhibitors, including, but not limited to, 2-cyano-3-[4-hydroxy-3,5-bis(1-methylethyl)phenyl]-N-(3-phenylpropyl)-(2-E)-2-propenamide;
(34) Flt-3 inhibitors, including, but not limited to, N-benzoyl-staurosporine, midostaurin, and N-(2-diethylaminoethyl)-5-[(Z)-(5-fluoro-2-oxo-1H-indol-3-ylidene)methyl]-2,4-dimethyl-1H-pyrrole-3-carboxamide (sunitinib);
(35) gonadorelin agonists, including, but not limited to, abarelix, goserelin, and goserelin acetate;
(36) heparanase inhibitors, including, but not limited to, phosphomannopentaose sulfate (PI-88);
(37) histone deacetylase (HDAC) inhibitors, including, but not limited to, compounds disclosed in PCT Patent Application Publication No. WO 02/22577 by Bair et al., including, but not limited to, N-hydroxy-3-[4-[[(2-hydroxyethyl)[2-(1H-indol-3-yl)ethyl]-amino]methyl]phenyl]-2E-2-propenamide, suberoylanilide hydroxamic acid, 4-(2-amino-phenylcarbamoyl)-benzyl]-carbamic acid pyridine-3-ylmethyl ester and derivatives thereof, butyric acid, pyroxamide, trichostatin A, oxamflatin, apicidin, depsipeptide, depudecin, trapoxin, HC toxin, and sodium phenylbutyrate;
(38) HSP90 inhibitors, including, but not limited to: 17-allylamino,17-demethoxygeldanamycin (17AAG); a geldanamycin derivative; other geldanamycin-related compounds; radicicol; and 5-(2,4-dihydroxy-5-isopropyl-phenyl)-4-(4-morpholin-4-ylmethyl-phenyl)-isoxazole-3-carboxylic acid ethylamide;
(39) IκBα inhibitors (IKKs), including, but not limited to, 3-[(4-methylphenyl)sulfonyl]-(2E)-2-propenenitrile;
(40) insulin receptor tyrosine kinase inhibitors, including, but not limited to, hydroxy-2-naphthalenylmethylphosphonic acid;
(41) c-Jun N-terminal kinase inhibitors, including, but not limited to, pyrazoleanthrone and epigallocatechin gallate;
(42) microtubule binding agents, including, but not limited to: vinblastine sulfate; vincristine sulfate; vindesine; vinorelbine; docetaxel; paclitaxel; discodermolides; colchicines; and epothilones and derivatives thereof, such as epothilone B or a derivative thereof;
(43) mitogen-activated protein (MAP) kinase inhibitors, including, but not limited to, N-[2-[[[3-(4-chlorophenyl)-2-propenyl]methyl]amino]methyl]phenyl]-N-(2-hydroxyethyl)-4-methoxy-benzenesulfonamide;
(44) MDM2 inhibitors, including, but not limited to, *trans*-4-iodo,4'-boranyl-chalcone;
(45) MEK inhibitors, including, but not limited to, bis[amino[2-aminophenyl)thio]methylene]-butanedinitrile;
(46) methionine aminopeptidase inhibitors, including, but not limited to, bengamide and derivatives thereof;
(47) MMP inhibitors, including, but not limited to: actinonin; epigallocatechin gallate; collagen peptidomimetic and non-peptidomimetic inhibitors; tetracycline derivatives such as hydroxamate, batimastat, marimastat, primomastat, TAA211, N-hydroxy-2(R)-[[(4-methoxyphenyl)sulfonyl](3-picolyl)amino]-3-methylbutanamide hydrochloride (MMI270B), and AAJ996;
(48) NGFR tyrosine kinase inhibitors, including, but not limited to, Tyrphostin AG 879;
(49) p38 MAP kinase inhibitors, including, but not limited to, 3-(dimethylamino)-N-[3-[(4-hydroxybenzoyl)amino]-4-methylphenyl]-benzamide;
(50) p56 tyrosine kinase inhibitors, including, but not limited to, 9,10-dihydro-3-hydroxy-1-methoxy-9,10-dioxo-2-anthracenecarboxaldehyde and Tyrphostin 46;
(51) PDGFR tyrosine kinase inhibitors, including, but not limited to, Tyrphostin AG 1296; Tyrphostin 9, 2-amino-4-(1H-indol-5-yl)-1,3-butadiene-1,1,3-tricarbonitrile, and imatinib;
(52) phosphatidylinositol 3-kinase inhibitors, including, but not limited to, wortmannin and quercetin dihydrate;
(53) phosphatase inhibitors, including, but not limited to, cantharidic acid, cantharidin, and (*E*)- N-[4-(2-carboxyethenyl)benzoyl]glycyl-L-α-glutamyl-L-leucinamide;
(54) platinum agents, including, but not limited to, carboplatin, cisplatin, oxaliplatin, satraplatin, and ZD0473;
(55) protein phosphatase inhibitors, including, but not limited to:
   (a) PP1 and PP2A inhibitors, including, but not limited to, cantharidic acid and cantharidin;
   (b) tyrosine phosphatase inhibitors, including, but not limited to, L-P-bromotetramisole oxalate, benzylphosphonic acid, and (5R)-4-hydroxy-5-(hydroxymethyl)-3-(1-oxohexadecyl)-2(5H)-furanone;
(56) PKC inhibitors, including, but not limited to, -[1-[3-(dimethylamino)propyl]-1H-indol-3-yl]-4-(1H-indol-3-yl)-1H-pyrrolo-2,5-dione, sphingosine, staurosporine, Tyrphostin 51, and hypericin;
(57) PKC delta kinase inhibitors, including, but not limited to, rottlerin;
(58) polyamine synthesis inhibitors, including, but not limited to, (RS)-2,5-diamino-2-(difluoromethyl)pentanoic acid (DMFO);
(59) proteasome inhibitors, including, but not limited to, aclacinomycin A, gliotoxin, and bortezomib;
(60) PTP1B inhibitors, including, but not limited to, (E)-N-[4-(2-carboxyethenyl)benzoyl]glycyl-L-α-glutamyl-L-leucinamide;
(61) protein tyrosine kinase inhibitors, including, but not limited to: Tyrphostin AG 126; Tyrphostin AG 1288; Tyrphostin AG 1295; geldanamycin; and genistein;
(62) SRC family tyrosine kinase inhibitors, including, but not limited to, 1-(1,1-dimethylethyl)-3-(1-naphthalenyl)-1H-pyrazolo[3,4-d]pyrimidin-4-amine, and 3-(4-chlorophenyl)-1-(1,1-dimethylethyl)-1H-pyrazolo[3,4-d]pyrimidin-4-amine;
(63) Syk tyrosine kinase inhibitors including, but not limited to, piceatannol;
(64) Janus (JAK-2 and/or JAK-3) tyrosine kinase inhibitors, including, but not limited to, Tyrphostin AG 490, and 2-naphthyl vinyl ketone;
(65) inhibitors of Ras oncogenic isoforms, including, but not limited to, (2S)-2-[[(2S)-2-[(2S,3S)-2-[(2R)-2-amino-3-mercaptopropyl]amino]-3-methylpentyl]oxy]-1-oxo-3-phenylpropyl]amino]-4-(methylsulfonyl)-butanoic acid 1-methylethyl ester (L-744832), DK8G557, and tipifarnib;
(66) retinoids, including, but not limited to, isotretinoin and tretinoin;
(67) ribonucleotide reductase inhibitors, including, but not limited to, hydroxyurea and 2-hydroxy-1H-isoindole-1,3-dione;
(68) RNA polymerase II elongation inhibitors, including, but not limited to, 5,6-dichloro-1-beta-D-ribofuranosylbenzimidazole;
(69) S-adenosylmethionine decarboxylase inhibitors, including, but not limited to, 5-amidino-1-tetralone-2'-amidinohydrazone and other compounds disclosed in United States Patent No. 5,461,076 to Stanek et al.;
(70) serine/threonine kinase inhibitors, including, but not limited to, sorafenib and 2-aminopurine;
(71) compounds which target, decrease, or inhibit the activity or function of serine/threonine mTOR kinase, including, but not limited to, everolimus, temsirolimus, zotarolimus, rapamycin, derivatives and analogs of rapamycin, deforolimus, AP23841, sirolimus, and everolimus;
(72) somatostatin receptor antagonists, including, but not limited to, octreotide and pasireotide (SOM230);
(73) sterol biosynthesis inhibitors, including, but not limited to, terbinadine;
(74) telomerase inhibitors, including, but not limited to, telomestatin; and
(75) topoisomerase inhibitors, including, but not limited to:
   (a) topoisomerase I inhibitors, including, but not limited to, topotecan, gimatecan, irinotecan, camptothecin and its analogues, 9-nitrocamptothecin and the macromolecular camptothecin conjugate PNU-16614, macromolecular camptothecin conjugates described in PCT Patent Application Publication No. WO 99/17804 by Angelucci et al., 10-hydroxycamptothecin acetate salt, etoposide idarubicin hydrochloride, teniposide, doxorubicin; epirubicin hydrochloride, mitoxantrone hydrochloride, and daunorubicin hydrochloride; and
   (b) topoisomerase II inhibitors, including, but not limited to, anthracyclines, such as doxorubicin, including liposomal formulations thereof, daunorubicin, including liposomal formulations thereof, epirubicin, idarubicin, nemorubicin, mitoxantrone, losoxantrone, etoposide, and eniposide;
(76) VEGFR tyrosine kinase inhibitors, including, but not limited to, 3-(4-dimethylaminobenzylidenyl)-2-indolinone; and
(77) RANKL inhibitors, including, but not limited to, denosumab.

When the improvement described herein is made by chemosensitization, the chemosensitization can comprise, but is not limited to, the use of an alkylating hexitol derivative as a chemosensitizer in combination with an agent selected from the group consisting of:
(a) topoisomerase inhibitors;
(b) fraudulent nucleosides;
(c) fraudulent nucleotides;
(d) thymidylate synthetase inhibitors;
(e) signal transduction inhibitors;
(f) cisplatin or platinum analogs;
(g) alkylating agents;
(h) anti-tubulin agents;
(i) antimetabolites;
(j) berberine;
(k) apigenin;
(l) colchicine or an analog of colchicine;
(m) genistein;
(n) etoposide;
(o) cytarabine;
(p) camptothecin;
(q) vinca alkaloids;
(r) 5-fluorouracil;
(s) curcumin;
(t) NF-κB inhibitors;
(u) rosmarinic acid; and
(v) mitoguazone.

When the improvement described herein is made by chemopotentiation, the chemopotentiation can comprise, but is not limited to, the use of an alkylating hexitol derivative as a chemopotentiator in combination with an agent selected from the group consisting of:
(a) fraudulent nucleosides;
(b) fraudulent nucleotides;
(c) thymidylate synthetase inhibitors;
(d) signal transduction inhibitors;
(e) cisplatin or platinum analogs;
(f) alkylating agents;
(g) anti-tubulin agents;
(h) antimetabolites;
(i) berberine;
(j) apigenin;
(k) colchicine or analogs of colchicine;
(l) genistein;
(m) etoposide;
(n) cytarabine;
(o) camptothecins;
(p) vinca alkaloids;
(q) topoisomerase inhibitors;
(r) 5-fluorouracil;
(s) curcumin;
(t) NF-κB inhibitors;
(u) rosmarinic acid;
(v) mitoguazone; and
(w) a biotherapeutic.

In one disclosure, when the chemopotentiation described herein involves chemopotentiation of an alkylating agent by the activity of an alkylating hexitol derivative, the alkylating agent can be selected from the group consisting of BCNU, BCNU wafers (Gliadel), CCNU, bendamustine (Treanda), lomustine, ACNU, and temozolimide (Temodar).

When the agent subject to chemopotentiation described herein is a biotherapeutic, the biotherapeutic can be, but is not limited to, a biotherapeutic selected from the group consisting of Avastin, Herceptin, Rituxan, and Erbitux.

When the improvement described herein is made by post-treatment management, the post-treatment management can be, but is not limited to, a method selected from the group consisting of:
(a) a therapy associated with pain management;
(b) nutritional support;
(c) administration of an anti-emetic;
(d) an anti-nausea therapy;
(e) administration of an anti-inflammatory agent;
(f) administration of an anti-pyretic agent; and
(g) administration of an immune stimulant.

When the improvement described herein is made by alternative medicine/post-treatment support, the alternative medicine/post-treatment support can be, but is not limited to, a method selected from the group consisting of:
(a) hypnosis;
(b) acupuncture;
(c) meditation;
(d) a herbal medication created either synthetically or through extraction; and
(e) applied kinesiology.

In one disclosure, when the method is a herbal medication created either synthetically or through extraction, the herbal medication created either synthetically or through extraction can be selected from the group consisting of:
(a) a NF-κB inhibitor;
(b) a natural anti-inflammatory;
(c) an immunostimulant;
(d) an antimicrobial; and
(e) a flavonoid, isoflavone, or flavone.

When the herbal medication described herein created either synthetically or through extraction is a NF-κB inhibitor, the NF-κB inhibitor can be selected from the group consisting of parthenolide, curcumin, and rosmarinic acid. When the herbal medication described herein created either synthetically or through extraction is a natural anti-inflammatory, the natural anti-inflammatory can be selected from the group consisting of rhein and parthenolide. When the herbal medication described herein created either synthetically or through extraction is an immunostimulant, the immunostimulant can be a product found in or isolated from Echinacea. When the herbal medication described herein created either synthetically or through extraction is an anti-microbial, the anti-microbial can be berberine. When the herbal medication described herein created either synthetically or through extraction is a flavonoid or flavone, the flavonoid, isoflavone, or flavone can be selected from the group consisting of apigenin, genistein, apigenenin, genistein, genistin, 6"-O-malonylgenistin, 6"-O-acetylgenistin, daidzein, daidzin, 6"-O-malonyldaidzin, 6"-O-acetylgenistin, glycitein, glycitin, 6"-O-malonylglycitin, and 6-O-acetylglycitin.

When the improvement described herein is made by a bulk drug product improvement, the bulk drug product improvement can be, but is not limited to, a bulk drug product improvement selected from the group consisting of:
(a) salt formation;
(b) preparation as a homogeneous crystal structure;
(c) preparation as a pure isomer;
(d) increased purity;
(e) preparation with lower residual solvent content; and
(f) preparation with lower residual heavy metal content.

When the improvement described herein is made by use of a diluent, the diluent can be, but is not limited to, a diluent selected from the group consisting of:
(a) an emulsion;
(b) dimethylsulfoxide (DMSO);
(c) N-methylformamide (NMF)
(d) dimethylformamide (DMF)
(e) dimethylacetamide (DMA);
(f) ethanol;
(g) benzyl alcohol;
(h) dextrose-containing water for injection;
(i) Cremophor;
(j) cyclodextrin; and
(k) PEG.

When the improvement described herein is made by use of a solvent system, the solvent system can be, but is not limited to, a solvent system selected from the group consisting of:
(a) an emulsion;
(b) DMSO;
(c) NMF;
(d) DMF;
(e) DMA;
(f) ethanol;
(g) benzyl alcohol;
(h) dextrose-containing water for injection;
(i) Cremophor;
(j) PEG; and
(k) salt systems.

When the improvement described herein is made by use of an excipient, the excipient can be, but is not limited to, an excipient selected from the group consisting of:
(a) mannitol;
(b) albumin;
(c) EDTA;
(d) sodium bisulfite;
(e) benzyl alcohol;
(f) carbonate buffers;
(g) phosphate buffers;
(h) PEG;
(i) vitamin A;
(j) vitamin D;
(k) vitamin E;
(l) esterase inhibitors;
(m) cytochrome P450 inhibitors;
(n) multi-drug resistance (MDR) inhibitors;
(o) organic resins;
(p) detergents;
(q) perillyl alcohol or an analog thereof; and
(r) activators of channel-forming receptors.

Suitable esterase inhibitors described herein include, but are not limited to, ebelactone A and ebelactone B.

Suitable cytochrome P450 inhibitors described herein include, but are not limited to, 1-aminobenzotriazole, N-hydroxy-N'-(4-butyl-2-methylphenyl)formamidine, ketoconazole, methoxsalen, metyrapone, roquefortine C, proadifen, 2,3',4,5'-tetramethylstilbene, and troleandomycin.

Suitable MDR inhibitors described herein include, but are not limited to, 5'-methoxyhydnocarpin, INF 240, INF 271, INF 277, INF 392, INF 55, reserpine, and GG918. MDR inhibitors are described in M. Zloh & S. Gibbons, "Molecular Similarity of MDR9 Inhibitors," Int. J. Mol. Sci. 5: 37-47 (2004).

Suitable organic resins described herein include, but are not limited to, a partially neutralized polyacrylic acid, as described in United States Patent No. 8,158,616 to Rodgers et al..

Suitable detergents described herein include, but are not limited to, nonionic detergents such as a polysorbate or a poloxamer, and are described in PCT Patent Application Publication No. WO/1997/039768 by Bjørn et al..

The use of perillyl alcohol or an analog thereof to improve transport of anti-neoplastic agents is described in United States Patent Application 2012/0219541 by Chen et al..

The use of activators of channel-forming receptors is described in United States Patent Application Publication No. 2010/0311678 by Bean et al.. Such activators of channel-forming receptors described herein include, but are not limited to, capsaicin, lidocaine, eugenol, arvanil (N-arachidonoylvanillamine), anandamide, 2-aminoethoxydiphenyl borate, resiniferatoxin, phorbol 12-phenylacetate 13-acetate 20-homovanillate (PPAHV), olvanil, N-oleoyldopamine, N-arachidonyldopamine, 6'-iodoresiniferatoxin (6'-IRTX), C₁₈ N-acylethanolamines, lipoxygenase derivatives such as 12-hydroperoxyeicosatetraenoic acid, inhibitor cysteine knot (ICK) peptides (vanillotoxins), piperine, N-[2-(3,4-dimethylbenzyl)-3-(pivaloyloxy)propyl]-2-[4-(2-aminoethoxy)-3-methoxyphenyl]acetamide, N-[2-(3,4-dimethylbenzyl)-3-(pivaloyloxy)propyl]-N'-(4-hydroxy-3-methoxybenzyl)thiourea, SU200 N-(4-*t*-butylbenzyl)-N'-(4-hydroxy-3-methoxybenzyl)thiourea), transacin, cinnamaldehyde, allyl-isothiocyanate, diallyl disulfide, icilin, cinnamon oil, wintergreen oil, clove oil, acrolein, mustard oil, ATP, 2-methylthio-ATP, 2' and 3'-O-(4-benzoylbenzoyl)-ATP, ATP-5'-O-(3-thiotriphosphate), menthol, eucalyptol, linalool, geraniol, and hydroxycitronellal.

When the improvement described herein is made by use of a dosage form, the dosage form can be, but is not limited to, a dosage form selected from the group consisting of:
(a) tablets;
(b) capsules;
(c) topical gels;
(d) topical creams;
(e) patches;
(f) suppositories;
(g) lyophilized dosage fills;
(h) immediate-release formulations;
(i) slow-release formulations;
(j) controlled-release formulations; and
(k) liquid in capsules.

Formulation of pharmaceutical compositions in tablets, capsules, and topical gels, topical creams or suppositories is well known in the art and is described, for example, in United States Patent Application Publication No. 2004/0023290 by Griffin et al..

Formulation of pharmaceutical compositions as patches such as transdermal patches is well known in the art and is described, for example, in United States Patent No. 7,728,042 to Eros et al..

Lyophilized dosage fills are also well known in the art. One general method described herein for the preparation of such lyophilized dosage fills, applicable to dibromodulcitol and derivatives thereof, comprises the following steps:
(1) Dissolve the drug in water for injection precooled to below 10° C. Dilute to final volume with cold water for injection to yield a 40 mg/mL solution.
(2) Filter the bulk solution through an 0.2-µm filter into a receiving container under aseptic conditions. The formulation and filtration should be completed in 1 hour.
(3) Fill nominal 1.0 mL filtered solution into sterilized glass vials in a controlled target range under aseptic conditions.
(4) After the filling, all vials are placed with rubber stoppers inserted in the "lyophilization position" and loaded in the prechilled lyophilizer. For the lyophilizer, shelf temperature is set at +5° C and held for 1 hour; shelf temperature is then adjusted to -5° C and held for one hour, and the condenser, set to -60° C, turned on.
(5) The vials are then frozen to 30° C or below and held for no less than 3 hours, typically 4 hours.
(6) Vacuum is then turned on, the shelf temperature is adjusted to -5° C, and primary drying is performed for 8 hours; the shelf temperature is again adjusted to - 5° C and drying is carried out for at least 5 hours.
(7) Secondary drying is started after the condenser (set at -60° C) and vacuum are turned on. In secondary drying, the shelf temperature is controlled at +5° C for 1 to 3 hours, typically 1.5 hours, then at 25°C for 1 to 3 hours, typically 1.5 hours, and finally at 35-40° C for at least 5 hours, typically for 9 hours, or until the product is completely dried.
(8) Break the vacuum with filtered inert gas (e.g., nitrogen). Stopper the vials in the lyophilizer.
(9) Vials are removed from the lyophilizer chamber and sealed with aluminum flip-off seals. All vials are visually inspected and labeled with approved labels.

Immediate-release formulations are described in United States Patent No. 8,148,393 to van Dalen et al.. Immediate-release formulations can include, for example, conventional film-coated tablets.

Slow-release formulations are described in United States Patent No. 8,178,125 to Wen et al.. Slow-release formulations can include, for example, microemulsions or liquid crystals.

Controlled-release formulations are described in United States Patent No. 8,231,898 to Oshlack et al.. Controlled-release formulations can include, for example, a matrix that includes a controlled-release material. Such a controlled-release material described herein can include hydrophilic and/or hydrophobic materials, such as gums, cellulose ethers, acrylic resins, protein derived materials, waxes, shellac, and oils such as hydrogenated castor oil or hydrogenated vegetable oil. However, any pharmaceutically acceptable hydrophobic or hydrophilic controlled-release material which is capable of imparting controlled-release of the mustard-based alkylating agent may be used in accordance with the present invention. Preferred controlled-release polymers include alkylcelluloses such as ethylcellulose, acrylic and methacrylic acid polymers and copolymers, and cellulose ethers, especially hydroxyalkylcelluloses (e.g., hydroxypropylmethylcellulose) and carboxyalkylcelluloses. Preferred acrylic and methacrylic acid polymers and copolymers include methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer, poly(methyl methacrylate), poly(methacrylic acid) (anhydride), polymethacrylate, polyacrylamide, poly(methacrylic acid anhydride), and glycidyl methacrylate copolymers.

When the improvement described herein is made by use of dosage kits and packaging, the dosage kits and packaging can be, but are not limited to, dosage kits and packaging selected from the group consisting of the use of amber vials to protect from light and the use of stoppers with specialized coatings to improve shelf-life stability.

When the improvement described herein is made by use of a drug delivery system, the drug delivery system can be, but is not limited to, a drug delivery system selected from the group consisting of:
(a) oral dosage forms;
(b) nanocrystals;
(c) nanoparticles;
(d) cosolvents;
(e) slurries;
(f) syrups;
(g) bioerodible polymers;
(h) liposomes;
(i) slow-release injectable gels;
(j) microspheres; and
(k) targeting compositions with epidermal growth factor receptor-binding peptides.

Nanocrystals are described in United States Patent No. 7,101,576 to Hovey et al..

Nanoparticles for drug delivery are described in United States Patent No. 8,258,132 to Bosch et al.. Typically, such nanoparticles have an average particle size of the active ingredient of less than about 1000 nm, more preferably, less than about 400 nm, and most preferably, less than about 250 nm. The nanoparticles can be coated with a surface stabilizer, such as, but not limited to, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., the commercially available Tweens® such as e.g., Tween 20® and Tween 80® (ICI Speciality Chemicals)); polyethylene glycols (e.g., Carbowaxes 3550® and 934® (Union Carbide)), polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethyl-cellulose, hydroxypropylmethyl-cellulose phthalate, noncrystalline cellulose, magnesium aluminium silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (e.g., Pluronics F68® and F108®, which are block copolymers of ethylene oxide and propylene oxide); poloxamines (e.g., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation), dialkylesters of sodium sulfosuccinic acid (e.g., Aerosol OT®, which is a dioctyl ester of sodium sulfosuccinic acid (American Cyanamid)), dioctyl sodium sulfosuccinate (DOSS), docusate sodium (Ashland Chem. Co., Columbus, Ohio); Duponol P®, which is a sodium lauryl sulfate (DuPont); Triton X-200®, which is an alkyl aryl polyether sulfonate (Rohm and Haas); Crodestas F-110®, which is a mixture of sucrose stearate and sucrose distearate (Croda Inc.); p-isononylphenoxy-poly-(glycidol), also known as Olin-IOG® or Surfactant 10-G® (Olin Chemicals, Stamford, Conn.); Crodestas SL-40® (Croda, Inc.); and SA9OHCO, which is C₁₈H₃₇CH₂(CON(CH₃)--0CH₂(CHOH)₄(CH₂OH)₂ (Eastman Kodak Co.); decanoyl-N-methylglucamide; *n*-decyl β-D-glucopyranoside; *n*-decyl β-D-maltopyranoside; *n*-dodecyl β-D-glucopyranoside; *n*-dodecyl β-D-maltoside; heptanoyl-N-methyl-glucamide; *n*-heptyl-β-D-glucopyranoside; *n*-heptyl β-D-thioglucoside; *n*-hexyl β-D-glucopyranoside; nonanoyl-N-methylglucamide; *n*-nonanoyl β-D-glucopyranoside; octanoyl-N-methylglucamide; *n*-octyl β-D-glucopyranoside; and octyl β-D-thioglucopyranoside. Nanoparticles for drug delivery are also described in United States Patent Application Publication No. 2010/209479 by Carroll et al.. These nanoparticles include carbon nanoparticles such as carbon nanotubes.

Pharmaceutically acceptable cosolvents are described in United States Patent No. 8,207,195 to Navratil et al., and include, but are not limited to, water, methanol, ethanol, 1-propanol, isopropanol, 1-butanol, isobutanol, *t*-butanol, acetone, methyl ethyl ketone, acetonitrile, ethyl acetate, benzene, toluene, xylene(s), ethylene glycol, dichloromethane, 1,2-dichloroethane, N-methylformamide, N,N-dimethylformamide, N-methylacetamide, pyridine, dioxane, and diethyl ether.

Slurries for use in pharmaceutical formulations are described in United States Patent Application Publication No. 2006/0229277 by Laxminarayan.

Syrups for use in pharmaceutical formulations are described in United States Patent No. 8,252,930 to Stoit et al.. Such syrups can include the active ingredient and a syrup-forming component such as sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents.

Bioerodible polymers are described in United States Patent No. 7,318,931 to Okumu et al.. A bioerodible polymer decomposes when placed inside an organism, as measured by a decline in the molecular weight of the polymer over time. Polymer molecular weights can be determined by a variety of methods including size exclusion chromatography (SEC), and are generally expressed as weight averages or number averages. A polymer is bioerodible if, when in phosphate buffered saline (PBS) of pH 7.4 and a temperature of 37° C, its weight-average molecular weight is reduced by at least 25% over a period of 6 months as measured by SEC. Useful bioerodible polymers include polyesters, such as poly(caprolactone), poly(glycolic acid), poly(lactic acid), and poly(hydroxybutryate); polyanhydrides, such as poly(adipic anhydride) and poly(maleic anhydride); polydioxanone; polyamines; polyamides; polyurethanes; polyesteramides; polyorthoesters; polyacetals; polyketals; polycarbonates; polyorthocarbonates; polyphosphazenes; poly(malic acid); poly(amino acids); polyvinylpyrrolidone; poly(methyl vinyl ether); poly(alkylene oxalate); poly(alkylene succinate); polyhydroxycellulose; chitin; chitosan; and copolymers and mixtures thereof.

Liposomes are well known as drug delivery vehicles. Liposome preparation is described in European Patent Application Publication No. EP 1332755 by Weng et al.. Liposomes can incorporate short oligopeptide sequences capable of targeting the EGFR receptor, as described in United States Patent Application Publication 2012/0213844 by Huang et al.. Alternatively, liposomes can include nuclear localization signal/fusogenic peptide conjugates and form targeted liposome complexes, as described in United States Patent Application Publication 2012/0183596 to Boulikas.

Slow release injectable gels are known in the art and are described, for example, in B. Jeong et al., "Drug Release from Biodegradable Injectable Thermosensitive Hydrogel of PEG-PLGA-PEG Triblock Copolymers," J. Controlled Release 63: 155-163 (2000).

The use of microspheres for drug delivery is known in the art and is described, for example, in H. Okada & H. Taguchi, "Biodegradable Microspheres in Drug Delivery," Crit. Rev. Ther. Drug Carrier Sys. 12: 1-99 (1995).

The use of targeting compositions with epidermal growth factor receptor-binding peptides is described in United States Patent Application Publication No. 2010/0151003 by Trikha et al..

When the improvement described herein is made by use of a drug conjugate form, the drug conjugate form can be, but is not limited to, a drug conjugate form selected from the group consisting of:
(a) a polymer system;
(b) polylactides;
(c) polyglycolides;
(d) amino acids;
(e) peptides;
(f) multivalent linkers;
(g) immunoglobulins;
(h) cyclodextrin polymers;
(i) modified transferrin;
(j) hydrophobic or hydrophobic-hydrophilic polymers;
(k) conjugates with a phosphonoformic acid partial ester;
(l) conjugates with a cell-binding agent incorporating a charged cross-linker; and
(m) conjugates with β-glucuronides through a linker.

Polylactide conjugates are well known in the art and are described, for example, in R. Tong & C. Cheng, "Controlled Synthesis of Camptothecin-Polylactide Conjugates and Nanoconjugates," Bioconjugate Chem. 21: 111-121 (2010).

Polyglycolide conjugates are also well known in the art and are described, for example, in PCT Patent Application Publication No. WO 2003/070823 by Elmaleh et al..

Multivalent linkers are known in the art and are described, for example, in United States Patent Application Publication No. 2007/0207952 by Silva et al.. For example, multivalent linkers can contain a thiophilic group for reaction with a reactive cysteine, and multiple nucleophilic groups (such as NH or OH) or electrophilic groups (such as activated esters) that permit attachment of a plurality of biologically active moieties to the linker.

Conjugates with immunoglobulins are disclosed in United States Patent No. 4,925,662 to Oguchi et al.. The conjugates are prepared by use of a cross-linking agent such as carbodiimide, glutaraldehyde, or diethyl malonimidate.

Cyclodextrin polymers, their conjugates with therapeutically active agents, and their administration together with particles are described in United States Patent Application Publication Serial No. 2012/0213854 by Fetzer.

Conjugates with modified transferrin are described in United States Patent Application Publication Serial No. 2011/0288023 by Kamei et al..

Conjugates with hydrophobic or hydrophobic-hydrophilic polymers are described in United States Patent Application Publication No. 2011/0268658 by Crawford et al.. These polymers can include mono-, di-, or tripeptides. These polymers can also include polylactic acid (PLA), polyglycolic acid (PGA), poly (lactic-co-glycolic) acid (PLGA), polycaprolactone (PCL), polydioxanone (PDO), polyanhydrides, polyorthoesters, or chitosan.

Conjugates with a phosphonoformic acid partial ester are described in United States Patent Application Publication No. 2010/227831 by Saha et al..

Conjugates with a cell-binding agent incorporating a charged cross-linker are described in United States Patent No. 8,236,319 to Chari et al..

Conjugates with β-glucuronides through a linker are described in United States Patent No. 8,039,273 to Jeffrey.

Suitable reagents for cross-linking many combinations of functional groups are known in the art. For example, electrophilic groups can react with many functional groups, including those present in proteins or polypeptides. Various combinations of reactive amino acids and electrophiles are known in the art and can be used. For example, N-terminal cysteines, containing thiol groups, can be reacted with halogens or maleimides. Thiol groups are known to have reactivity with a large number of coupling agents, such as alkyl halides, haloacetyl derivatives, maleimides, aziridines, acryloyl derivatives, arylating agents such as aryl halides, and others. These are described in G. T. Hermanson, "Bioconjugate Techniques" (Academic Press, San Diego, 1996), pp. 146-150. The reactivity of the cysteine residues can be optimized by appropriate selection of the neighboring amino acid residues. For example, a histidine residue adjacent to the cysteine residue will increase the reactivity of the cysteine residue. Other combinations of reactive amino acids and electrophilic reagents are known in the art. For example, maleimides can react with amino groups, such as the ε-amino group of the side chain of lysine, particularly at higher pH ranges. Aryl halides can also react with such amino groups. Haloacetyl derivatives can react with the imidazolyl side chain nitrogens of histidine, the thioether group of the side chain of methionine, and the .epsilon.-amino group of the side chain of lysine. Many other electrophilic reagents are known that will react with the ε-amino group of the side chain of lysine, including, but not limited to, isothiocyanates, isocyanates, acyl azides, N-hydroxysuccinimide esters, sulfonyl chlorides, epoxides, oxiranes, carbonates, imidoesters, carbodiimides, and anhydrides. These are described in G.T. Hermanson, "Bioconjugate Techniques" (Academic Press, San Diego, 1996), pp. 137-146. Additionally, electrophilic reagents are known that will react with carboxylate side chains such as those of aspartate and glutamate, such as diazoalkanes and diazoacetyl compounds, carbonydilmidazole, and carbodiimides. These are described in G. T. Hermanson, "Bioconjugate Techniques" (Academic Press, San Diego, 1996), pp. 152-154. Furthermore, electrophilic reagents are known that will react with hydroxyl groups such as those in the side chains of serine and threonine, including reactive haloalkane derivatives. These are described in G. T. Hermanson, "Bioconjugate Techniques" (Academic Press, San Diego, 1996), pp. 154-158. In another alternative disclosure, the relative positions of electrophile and nucleophile (i.e., a molecule reactive with an electrophile) are reversed so that the protein has an amino acid residue with an electrophilic group that is reactive with a nucleophile and the targeting molecule includes therein a nucleophilic group. This includes the reaction of aldehydes (the electrophile) with hydroxylamine (the nucleophile), described above, but is more general than that reaction; other groups can be used as electrophile and nucleophile. Suitable groups are well known in organic chemistry and need not be described further in detail.

Additional combinations of reactive groups for cross-linking are known in the art. For example, amino groups can be reacted with isothiocyanates, isocyanates, acyl azides, N-hydroxysuccinimide (NHS) esters, sulfonyl chlorides, aldehydes, glyoxals, epoxides, oxiranes, carbonates, alkylating agents, imidoesters, carbodiimides, and anhydrides. Thiol groups can be reacted with haloacetyl or alkyl halide derivatives, maleimides, aziridines, acryloyl derivatives, acylating agents, or other thiol groups by way of oxidation and the formation of mixed disulfides. Carboxy groups can be reacted with diazoalkanes, diazoacetyl compounds, carbonyldiimidazole, carbodiimides. Hydroxyl groups can be reacted with epoxides, oxiranes, carbonyldiimidazole, N,N'-disuccinimidyl carbonate, N-hydroxysuccinimidyl chloroformate, periodate (for oxidation), alkyl halogens, or isocyanates. Aldehyde and ketone groups can react with hydrazines, reagents forming Schiff bases, and other groups in reductive amination reactions or Mannich condensation reactions. Still other reactions suitable for cross-linking reactions are known in the art. Such cross-linking reagents and reactions are described in G.T. Hermanson, "Bioconjugate Techniques" (Academic Press, San Diego, 1996).

When the improvement described herein is made by use of a compound analog, the compound analog can be, but is not limited to, a compound analog selected from the group consisting of:
(a) alteration of side chains to increase or decrease lipophilicity;
(b) addition of an additional chemical functionality to alter a property selected from the group consisting of reactivity, electron affinity, and binding capacity; and
(c) alteration of salt form.

When the improvement described herein is made by use of a prodrug system, the prodrug system can be, but is not limited to, a prodrug system selected from the group consisting of:
(a) the use of enzyme sensitive esters;
(b) the use of dimers;
(c) the use of Schiff bases;
(d) the use of pyridoxal complexes;
(e) the use of caffeine complexes; and
(f) the use of nitric oxide-releasing prodrugs;
(g) the use of prodrugs with fibroblast activation protein α-cleavable oligopeptides;
(h) the use of prodrugs that are products of reaction with an acetylating or carbamylating agent;
(i) the use of prodrugs that are hexanoate conjugates;
(j) the use of prodrugs that are polymer-agent conjugates; and
(k) the use of prodrugs that are subject to redox activation.

As used herein, the term "prodrug" refers to compounds that are transformed *in vivo* to yield a disclosed compound or a pharmaceutically acceptable form of the compound. In some disclosures, a prodrug is a compound that may be converted under physiological conditions or by solvolysis to a biologically active compound as described herein. Thus, the term "prodrug" refers to a precursor of a biologically active compound that is pharmaceutically acceptable. A prodrug can be inactive when administered to a subject, but is then converted *in vivo* to an active compound, for example, by hydrolysis (e.g., hydrolysis in blood or a tissue). In certain cases, a prodrug has improved physical and/or delivery properties over a parent compound from which the prodrug has been derived. The prodrug often offers advantages of solubility, tissue compatibility, or delayed release in a mammalian organism (H. Bundgard, Design of Prodrugs (Elsevier, Amsterdam, 1988), pp. 7-9, 21-24). A discussion of prodrugs is provided in T. Higuchi et al., "Pro-Drugs as Novel Delivery Systems," ACS Symposium Series, Vol. 14 and in E.B. Roche, ed., Bioreversible Carriers in Drug Design (American Pharmaceutical Association & Pergamon Press, 1987). Exemplary advantages of a prodrug can include, but are not limited to, its physical properties, such as enhanced water solubility for parenteral administration at physiological pH compared to the parent compound, enhanced absorption from the digestive tract, or enhanced drug stability for long-term storage.

The term "prodrug" is also meant to include any covalently bonded carriers which release the active compound *in vivo* when the prodrug is administered to a subject. Prodrugs of a therapeutically active compound, as described herein, can be prepared by modifying one or more functional groups present in the therapeutically active compound in such a way that the modifications are cleaved, either in routine manipulation or *in vivo,* to yield the parent therapeutically active compound. Prodrugs include compounds wherein a hydroxy, amino, or mercapto group is covalently bonded to any group that, when the prodrug of the active compound is administered to a subject, cleaves to form a free hydroxy, free amino, or free mercapto group, respectively. Examples of prodrugs described herein include, but are not limited to, formate or benzoate derivatives of an alcohol or acetamide, formamide or benzamide derivatives of a therapeutically active agent possessing an amine functional group available for reaction, and the like.

For example, if a therapeutically active agent or a pharmaceutically acceptable form of a therapeutically active agent contains a carboxylic acid functional group, a prodrug described herein can comprise an ester formed by the replacement of the hydrogen atom of the carboxylic acid group with a group such as C₁₋₈ alkyl, C₂₋₁₂ alkanoyloxymethyl, 1-(alkanoyloxy)ethyl having from 4 to 9 carbon atoms, 1-methyl-1-(alkanoyloxy)ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N(C₁-C₂)alkylamino(C₂-C₃)alkyl (such as (3-dimethylaminoethyl), carbamoyl-(C₁-C₂)alkyl, N,N-di (C₁-C₂)alkylcarbamoyl-(C₁-C₂)alkyl and piperidino-, pyrrolidino-, or morpholino(C₂-C₃)alkyl.

Similarly, if a disclosed compound or a pharmaceutically acceptable form of the compound contains an alcohol functional group, a prodrug described herein can be formed by the replacement of the hydrogen atom of the alcohol group with a group such as (C₁-C₆)alkanoyloxymethyl, 1 -((C₁-C₆))alkanoyloxy)ethyl, 1-methyl-1-((C₁-C₆)alkanoyloxy)ethyl (C₁-C₆)alkoxycarbonyloxymethyl, N(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, P(O)(O(C₁-C₆)alkyl)₂ or glycosyl (the radical resulting from the removal of a hydroxyl group of the hemiacetal form of a carbohydrate).

If a disclosed compound or a pharmaceutically acceptable form of the compound incorporates an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, benzyl, or R-carbonyl is a natural α-aminoacyl or natural α-aminoacyl-natural α-aminoacyl, C(OH)C(O)OY¹ wherein Y¹ is H, (C₁-C₆)alkyl or benzyl, C(OY²)Y³ wherein Y² is (C₁-C₄) alkyl and Y³ is (C₁-C₆)alkyl, carboxy(C₁-C₆)alkyl, amino(C₁-C₄)alkyl or mono-N or di-N,N(C₁-C₆)alkylaminoalkyl,C(Y⁴)Y⁵ wherein Y⁴ is H or methyl and Y⁵ is mono-N or di-N,N(C₁-C₆)alkylamino, morpholino, piperidin-1-yl or pyrrolidin-1-yl.

The use of prodrug systems is described in T. Järvinen et al., "Design and Pharmaceutical Applications of Prodrugs" in Drug Discovery Handbook (S.C. Gad, ed., Wiley-Interscience, Hoboken, NJ, 2005), ch. 17, pp. 733-796. This publication describes the use of enzyme sensitive esters as prodrugs. The use of dimers as prodrugs is described in United States Patent No. 7,879,896 to Allegretti et al.. The use of peptides in prodrugs is described in S. Prasad et al., "Delivering Multiple Anticancer Peptides as a Single Prodrug Using Lysyl-Lysine as a Facile Linker," J. Peptide Sci. 13: 458-467 (2007). The use of Schiff bases as prodrugs is described in United States Patent No. 7,619,005 to Epstein et al.. The use of caffeine complexes as prodrugs is described in United States Patent No. 6,443,898 to Unger et al.. The use of nitric oxide-releasing prodrugs is described in N. Nath et al., "JS-K, a Nitric Oxide-Releasing Prodrug, Modulates β-Catenin/TCF Signaling in Leukemic Jurkat Cells: Evidence of an S-Nitrosylated Mechanism," Biochem. Pharmacol. 80: 1641-1649 (2010). The use of prodrugs with fibroblast activation protein α-cleavable oligopeptides is described in United States Patent Application Publication No. 2002/0155565 by Garin-Chesa et al. . The use of prodrugs that are products of reaction with an acetylating or carbamylating agent is described in J.H. Lin & J.Y.H. Lu, "Role of Pharmacokinetics and Metabolism in Drug Discovery and Development," Pharmacol. Rev. 4: 403-449 (1997). The use of hexanoate conjugates is described in United States Patent No. 8,101,661 to Mickle. The use of polymer-agent conjugates is described in R. Satchi et al., "PDEPT: Polymer-Directed Enzyme Prodrug Therapy," Br. J. Cancer 85: 1070-1076 (2001). The use of prodrugs that are subject to redox activation is described in S.H. van Rijt & P.J. Sadler, "Current Applications and Future Potential for Bioinorganic Chemistry in the Development of Anticancer Drugs," Drug Discov. Today 14: 1089-1097 (2009).

When the improvement described herein is made by use of a multiple drug system, the multiple drug system can be, but is not limited to, a multiple drug system selected from the group consisting of:
(a) inhibitors of multi-drug resistance;
(b) specific drug resistance inhibitors;
(c) specific inhibitors of selective enzymes;
(d) signal transduction inhibitors;
(e) meisoindigo;
(f) imatinib;
(g) hydroxyurea;
(h) dasatinib;
(i) capecitabine;
(j) nilotinib;
(k) repair inhibition agents; and
(l) topoisomerase inhibitors with non-overlapping side effects.

Multi-drug resistance inhibitors are described in United States Patent No. 6,011,069 to Inomata et al..

Specific drug resistance inhibitors are described in T. Hideshima et al., "The Proteasome Inhibitor PS-341 Inhibits Growth, Induces Apoptosis, and Overcomes Drug Resistance in Human Multiple Myeloma Cells," Cancer Res. 61: 3071-3076 (2001).

Selective inhibitors of specific enzymes are described in D. Leung et al.,"Discovering Potent and Selective Reversible Inhibitors of Enzymes in Complex Proteomes," Nature Biotechnol. 21: 687-691 (2003).

Repair inhibition is described in N.M. Martin, "DNA Repair Inhibition and Cancer Therapy," J. Photochem. Photobiol. B 63: 162-170 (2001).

When the improvement described herein is made by biotherapeutic enhancement, the biotherapeutic enhancement can be performed by use in combination as sensitizers/potentiators with a therapeutic agent or technique that can be, but is not limited to, a therapeutic agent or technique selected from the group consisting of:
(a) biological response modifiers;
(b) cytokines;
(c) lymphokines;
(d) therapeutic antibodies;
(e) antisense therapies;
(f) gene therapies;
(g) ribozymes; and
(h) RNA interference.

Biological response modifiers are described in T.E.G.K. Murthy et al., "Biological Response Modifiers," Int. J. Pharmtech Res. 2: 2152-2160 (2010).

Antisense therapies are described, for example, in B. Weiss et al., "Antisense RNA Gene Therapy for Studying and Modulating Biological Processes," Cell. Mol. Life Sci. 55: 334-358 (1999).

Ribozymes are described, for example, in S. Pascolo, "RNA-Based Therapies" in Drug Discovery Handbook (S.C. Gad, ed., Wiley-Interscience, Hoboken, NJ, 2005), ch.27, pp. 1273-1278.

RNA interference is described, for example, in S. Pascolo, "RNA-Based Therapies" in Drug Discovery Handbook (S.C. Gad, ed., Wiley-Interscience, Hoboken, NJ, 2005), ch.27, pp. 1278-1283.

When the biotherapeutic enhancement is use in combination as sensitizers/potentiators with a therapeutic antibody, the therapeutic antibody can be, but is not limited to, a therapeutic antibody selected from the group consisting of bevacizumab (Avastin), rituximab (Rituxan), trastuzumab (Herceptin), and cetuximab (Erbitux).

When the improvement described herein is made by use of biotherapeutic resistance modulation, the biotherapeutic resistance modulation can be, but is not limited to, use against a TKI-resistant malignancy also resistant to a therapeutic agent or technique selected from the group consisting of:
(a) biological response modifiers;
(b) cytokines;
(c) lymphokines;
(d) therapeutic antibodies;
(e) antisense therapies;
(f) gene therapies;
(g) ribozymes; and
(h) RNA interference.

In another disclosure, when the improvement described herein is made by use of biotherapeutic resistance modulation, the biotherapeutic resistance modulation can be, but is not limited to, use against a malignancy associated with a mutation in or dysregulation of the *AHI1* gene also resistant to a therapeutic agent or technique selected from the group consisting of:
(a) biological response modifiers;
(b) cytokines;
(c) lymphokines;
(d) therapeutic antibodies;
(e) antisense therapies;
(f) gene therapies;
(g) ribozymes; and
(h) RNA interference.

When the biotherapeutic resistance modulation described herein is use against tumors resistant to therapeutic antibodies, the therapeutic antibody can be, but is not limited to, a therapeutic antibody selected from the group consisting of bevacizumab (Avastin), rituximab (Rituxan), trastuzumab (Herceptin), and cetuximab (Erbitux).

When the improvement described herein is made by radiation therapy enhancement, the radiation therapy enhancement can be, but is not limited to, a radiation therapy enhancement agent or technique selected from the group consisting of:
(a) use with hypoxic cell sensitizers;
(b) use with radiation sensitizers/protectors;
(c) use with photosensitizers;
(d) use with radiation repair inhibitors;
(e) use with thiol depleting agents;
(f) use with vaso-targeted agents;
(g) use with DNA repair inhibitors;
(h) use with radioactive seeds;
(i) use with radionuclides;
(j) use with radiolabeled antibodies; and
(k) use with brachytherapy.

An alkylating hexitol derivative can be used in combination with radiation for the treatment of a TKI-resistant malignancy.

Hypoxic cell sensitizers are described in C.C. Ling et al., "The Effect of Hypoxic Cell Sensitizers at Different Irradiation Dose Rates," Radiation Res. 109: 396-406 (1987). Radiation sensitizers are described in T.S. Lawrence, "Radiation Sensitizers and Targeted Therapies," Oncology 17 (Suppl. 13) 23-28 (2003). Radiation protectors are described in S.B. Vuyyuri et al., "Evaluation of D-Methionine as a Novel Oral Radiation Protector for Prevention of Mucositis," Clin. Cancer Res. 14: 2161-2170 (2008). Photosensitizers are described in R.R. Allison & C.H. Sibata, "Oncologic Photodynamic Therapy Photosensitizers: A Clinical Review," Photodiagnosis Photodynamic Ther. 7: 61-75 (2010). Radiation repair inhibitors and DNA repair inhibitors are described in M. Hingorani et al., "Evaluation of Repair of Radiation-Induced DNA Damage Enhances Expression from Replication-Defective Adenoviral Vectors," Cancer Res. 68: 9771-9778 (2008). Thiol depleters are described in K.D. Held et al., "Postirradiation Sensitization of Mammalian Cells by the Thiol-Depleting Agent Dimethyl Fumarate," Radiation Res. 127: 75-80 (1991). Vaso-targeted agents are described in A.L. Seynhaeve et al., "Tumor Necrosis Factor α Mediates Homogeneous Distribution of Liposomes in Murine Melanoma that Contributes to a Better Tumor Response," Cancer Res. 67: 9455-9462 (2007).

When the improvement described herein is by use of a novel mechanism of action, the novel mechanism of action can be, but is not limited to, a novel mechanism of action that is a therapeutic interaction with a target or mechanism selected from the group consisting of:
(a) inhibitors of poly-ADP ribose polymerase;
(b) agents that affect vasculature;
(c) agents that promote vasodilation;
(d) oncogenic targeted agents;
(e) signal transduction inhibitors;
(f) agents inducing EGFR inhibition;
(g) agents inducing Protein Kinase C inhibition;
(h) agents inducing Phospholipase C downregulation;
(i) agents including jun downregulation;
(j) agents modulating expression of histone genes;
(k) agents modulating expression of VEGF;
(l) agents modulating expression of ornithine decarboxylase;
(m) agents modulating expression of jun D;
(n) agents modulating expression of v-jun;
(o) agents modulating expression of GPCRs;
(p) agents modulating expression of protein kinase A;
(q) agents modulating expression of protein kinases other than protein kinase A;
(r) agents modulating expression of telomerase;
(s) agents modulating expression of prostate specific genes; and
(t) agents modulating expression of histone deacetylase.

Inhibitors of poly ADP-ribose polymerase include veliparib (ABT-888), AGO14699, iniparib (BSI-201), carboplatin, gemcitabine, INO-1001, MK4827, nicotinamide, olaparib, paclitaxel, temozolomide, and topotecan, and are described in E.A. Comen & M. Robson, "Inhibition of Poly(ADP)-Ribose Polymerase as a Therapeutic Strategy for Breast Cancer," Oncology 24: 55-62 (2010). Agents promoting vasodilation include levosimendan, described in W.G. Toller et al., "Levosimendan, a New Inotropic and Vasodilator Agent," Anesthesiology 104: 556-569 (2006). EGFR inhibition is described in G. Giaccone & J.A. Rodriguez, "EGFR Inhibitors: What Have We Learned from the Treatment of Lung Cancer," Nat. Clin. Pract. Oncol. 11: 554-561 (2005). Protein kinase C inhibition is described in H.C. Swannie & S.B. Kaye, "Protein Kinase C Inhibitors," Curr. Oncol. Rep. 4: 37-46 (2002). Phospholipase C downregulation is described in A.M. Martelli et al., "Phosphoinositide Signaling in Nuclei of Friend Cells: Phospholipase C β Downregulation Is Related to Cell Differentiation," Cancer Res. 54: 2536-2540 (1994). Downregulation of Jun (specifically, c-Jun) is described in A. A. P. Zada et al., "Downregulation of c-Jun Expression and Cell Cycle Regulatory Molecules in Acute Myeloid Leukemia Cells Upon CD44 Ligation," Oncogene 22: 2296-2308 (2003). The role of histone genes as a target for therapeutic intervention is described in B. Calabretta et al., "Altered Expression of G1-Specific Genes in Human Malignant Myeloid Cells," Proc. Natl. Acad. Sci. USA 83: 1495-1498 (1986). The role of VEGF as a target for therapeutic intervention is described in A. Zielke et al., "VEGF-Mediated Angiogenesis of Human Pheochromocytomas Is Associated to Malignancy and Inhibited by anti-VEGF Antibodies in Experimental Tumors," Surgery 132: 1056-1063 (2002). The role of ornithine decarboxylase as a target for therapeutic intervention is described in J.A. Nilsson et al., "Targeting Ornithine Decarboxylase in Myc-Induced Lymphomagenesis Prevents Tumor Formation," Cancer Cell 7: 433-444 (2005). The role of ubiquitin C as a target for therapeutic intervention is described in C. Aghajanian et al., "A Phase I Trial of the Novel Proteasome Inhibitor PS341 in Advanced Solid Tumor Malignancies," Clin. Cancer Res. 8: 2505-2511 (2002). The role of Jun D as a target for therapeutic intervention is described in M.M. Caffarel et al., "JunD Is Involved in the Antiproliferative Effect of Δ9-Tetrahydrocannibinol on Human Breast Cancer Cells," Oncogene 27: 5033-5044 (2008). The role of v-Jun as a target for therapeutic intervention is described in M. Gao et al., "Differential and Antagonistic Effects of v-Jun and c-Jun," Cancer Res. 56: 4229-4235 (1996). The role of protein kinase A as a target for therapeutic intervention is described in P.C. Gordge et al., "Elevation of Protein Kinase A and Protein Kinase C in Malignant as Compared With Normal Breast Tissue," Eur. J. Cancer 12: 2120-2126 (1996). The role of telomerase as a target for therapeutic intervention is described in E.K. Parkinson et al., "Telomerase as a Novel and Potentially Selective Target for Cancer Chemotherapy," Ann. Med. 35: 466-475 (2003). The role of histone deacetylase as a target for therapeutic intervention is described in A. Melnick & J.D. Licht, "Histone Deacetylases as Therapeutic Targets in Hematologic Malignancies," Curr. Opin. Hematol. 9: 322-332 (2002).

When the improvement described herein is made by use of selective target cell population therapeutics, the use of selective target cell population therapeutics can be, but is not limited to, a use selected from the group consisting of:
(a) use against radiation sensitive cells;
(b) use against radiation resistant cells; and
(c) use against energy depleted cells.

The improvement described herein can also be made by use of an alkylating combination with ionizing radiation.

When the improvement described herein is made by use with an agent to enhance the activity of an alkylating hexitol derivative, the agent to enhance the activity of the alkylating hexitol derivative can be, but is not limited to, an agent selected from the group consisting of:
(a) nicotinamide;
(b) caffeine;
(c) tetandrine; and
(d) berberine.

As detailed above, in view of the findings that mutation in, overexpression of or dysregulation of the *AHI1* gene is associated with a number of malignancies, including chronic myelocytic leukemia, an alkylating hexitol derivative can also be used together with an agent that modulates the expression or activity of either the *AHI1* gene or the AHI1 protein. In this alternative disclosure, the method further comprises the administration of a therapeutically effective quantity of an agent that modulates the expression or activity of either the *AHI1* gene or the AHI1 protein.

In one alternative disclosure, the agent that modulates the expression or activity of either the *AHI1* gene or the AHI1 protein is an agent that modulates the expression of the *AHI1* gene. Modulation of the expression of *AHI1* can be carried out at either the level of transcription of the *AHI1* gene or the level of translation of the *AHI1* gene.

In some disclosures, inhibitory nucleotides are used to modulate *AHI1* expression. These include short interfering RNA (siRNA), microRNA (miRNA), and synthetic hairpin RNA (shRNA), antisense nucleic acids, or complementary DNA (cDNA). In some preferred disclosures, a siRNA targeting *AHI1* expression is used. Interference with the function and expression of endogenous genes by double-stranded RNA such as siRNA has been shown in various organisms. See, e.g., A. Fire et al., "Potent and Specific Genetic Interference by Double-Stranded RNA in Caenorhabditis elegans," Nature 391:806-811 (1998); J.R. Kennerdell & R.W. Carthew, "Use of dsDNA-Mediated Genetic Interference to Demonstrate that frizzled and frizzled 2 Act in the Wingless Pathway," Cell 95:1017-1026 (1998); F. Wianni & M. Zernicka-Goetz, "Specific Interference with Gene Function by Double-Stranded RNA in Early Mouse Development," Nat. Cell Biol. 2:70-75 (2000). siRNAs can include hairpin loops comprising self-complementary sequences or double stranded sequences. siRNAs typically have fewer than 100 base pairs and can be, e.g., about 30 bps or shorter, and can be made by approaches known in the art, including the use of complementary DNA strands or synthetic approaches. Such double-stranded RNA can be synthesized by in vitro transcription of single-stranded RNA read from both directions of a template and in vitro annealing of sense and antisense RNA strands. Double-stranded RNA targeting *AHI1* can also be synthesized from a cDNA vector construct in which an *AHI1* gene is cloned in opposing orientations separated by an inverted repeat. Following cell transfection, the RNA is transcribed and the complementary strands reanneal. Double-stranded RNA targeting the *AHI1* gene can be introduced into a cell (e.g., a leukemic cell or other tumor cell) by transfection of an appropriate construct. Typically, RNA interference mediated by siRNA, miRNA, or shRNA is mediated at the level of translation; in other words, these interfering RNA molecules prevent translation of the corresponding mRNA molecules and lead to their degradation. It is also possible that RNA interference may also operate at the level of transcription, blocking transcription of the regions of the genome corresponding to these interfering RNA molecules. The structure and function of these interfering RNA molecules are well known in the art and are described, for example, in R.F. Gesteland et al., eds, "The RNA World" (3rd ed, Cold Spring Harbor Laboratory Press, Cold Spring Harbor, New York, 2006), pp. 535-565.

For these approaches, cloning into vectors and transfection methods are also well known in the art and are described, for example, in J. Sambrook & D.R. Russell, "Molecular Cloning: A Laboratory Manual" (3rd ed., Cold Spring Harbor Laboratory Press, Cold Spring Harbor, 2001).

In addition to double stranded RNAs, other nucleic acid agents targeting *AHI1* can also be employed in the methods described herein, e.g., antisense nucleic acids. Antisense nucleic acids are DNA or RNA molecules that are complementary to at least a portion of a specific target mRNA molecule. In the cell, the single stranded antisense molecule hybridizes to that mRNA, forming a double stranded molecule. The cell does not translate an mRNA in this double-stranded form. Therefore, antisense nucleic acids interfere with the translation of mRNA into protein, and, thus, with the expression of a gene that is transcribed into that mRNA. Antisense methods have been used to inhibit the expression of many genes in vitro. See, e.g., C.J. Marcus-Sekura,"Techniques for Using Antisense Oligodeoxyribonucleotides to Study Gene Expression," Anal. Biochem. 172:289-295 (1988); J.E. Hambor et al., "Use of an Epstein-Barr Virus Episomal Replicon for Anti-Sense RNA-Mediated Gene Inhibition in a Human Cytotoxic T-Cell Clone," Proc. Natl. Acad. Sci. U.S.A. 85:4010-4014 (1988); H Arima et al., "Specific Inhibition of Interleukin-10 Production in Murine Macrophage-Like Cells by Phosphorothioate Antisense Oligonucleotides," Antisense Nucl. Acid Drug Dev. 8:319-327 (1998); and W.-F. Hou et al., "Effect of Antisense Oligodeoxynucleotides Directed to Individual Calmodulin Gene Transcripts on the Proliferation and Differentiation of PC12 Cells," Antisense Nucl. Acid Drug Dev. 8:295-308 (1998). Antisense technology is described further in C. Lichtenstein & W. Nellen, eds., "Antisense Technology: A Practical Approach" (IRL Press, Oxford, 1997).

*AHI1* nucleotide sequences, such as those derived from RNA, are known in the art, and include the gene sequences reported as NM_001134830.1, NM_001134831.1, NM_001134832.1, and NM_017651.4 in GenBank; these sequences represent transcript variants. Based on the known sequences, inhibitory nucleotides (e.g., siRNA, miRNA, or shRNA) targeting PAR1 can be readily synthesized using methods well known in the art. Exemplary siRNAs according to the invention could have up to 29 bps, 25 bps, 22 bps, 21 bps, 20 bps, 15 bps, 10 bps, 5 bps or any integral number of base pairs between these numbers. Tools for designing optimal inhibitory siRNAs include that available from DNAengine Inc. (Seattle, WA) and Ambion, Inc. (Austin, TX).

As another alternative disclosure, the agent that inhibits the expression or activity of the *AHI1* gene or the AHI1 protein can be an agent that inhibits the activity of the AHI1 protein. For example, such an agent can be an antibody that binds either the AHI1 protein or one or more receptors specifically binding to the AHI1 protein. The preparation of such antibodies is well known in the art and need not be described further herein. In general, antibodies according to the present invention can be of any class, such as IgG, IgA, IgD, IgE, IgM, or IgY, although IgG antibodies are typically preferred. Antibodies can be of any mammalian or avian origin, including human, murine (mouse or rat), donkey, sheep, goat, rabbit, camel, horse, or chicken. In some alternatives, the antibodies can be bispecific. The antibodies can be modified by the covalent attachment of any type of molecule to the antibody. For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, or other modifications known in the art. Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., "Antibodies: A Laboratory Manual", (Cold Spring Harbor Laboratory Press, 2nd ed. 1988); Hammerling, et al., in: Monoclonal Antibodies and T-Cell Hybridomas 563-681 (Elsevier, N.Y., 1981), or by other standard methods known in the art. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. For example, suitable antibodies can be produced by phage display or other techniques. Additionally, and not by way of limitation, human antibodies can be made by a variety of techniques, including phage display methods using antibody libraries derived from human immunoglobulin sequences and by the use of transgenic mice that are incapable of expressing functional endogenous immunoglobulins, but which can express human immunoglobulin genes. For example, the human heavy and light chain immunoglobulin gene complexes can be introduced randomly or by homologous recombination into mouse embryonic stem cells. The antibodies can also be produced by expression of polynucleotides encoding these antibodies. Additionally, antibodies according to the present invention can be fused to marker sequences, such as a peptide tag to facilitate purification; a suitable tag is a hexahistidine tag. The antibodies can also be conjugated to a diagnostic or therapeutic agent by methods known in the art. Techniques for preparing such conjugates are well known in the art.

Other methods of preparing these monoclonal antibodies, as well as chimeric antibodies, humanized antibodies, and single-chain antibodies, are known in the art. In general, for administration to human patients described herein, human antibodies, chimeric antibodies, or humanized antibodies are preferred.

In another alternative disclosure, the agent that modulates the activity of the AHI1 protein is an antagonist of a receptor that specifically binds the AHI1 protein, including, but not limited to, serotonin receptor 2C.

In yet another alternative disclosure, the agent that modulates the activity of the AHI1 protein is an antibody that specifically binds a receptor for the AHI1 protein, including, but not limited to, serotonin receptor 2C.

In yet another alternative disclosure, the agent that modulates the activity of the AHI1 protein is an agent that inhibits a transcription factor that has a binding site in the *AHI1* gene promoter, including, but not limited to, RFX1, POU2F1, POU2F1a, Nkx2-5, Evi-1, and RSRFC4.

Another aspect of the present disclosure is a composition to improve the efficacy and/or reduce the side effects of suboptimally administered drug therapy employing an alkylating hexitol derivative for the treatment of a TKI resistant malignancy or a malignancy associated with dysregulation or mutation of the *AHI1* gene comprising an alternative selected from the group consisting of:
(i) a therapeutically effective quantity of a modified alkylating hexitol derivative or a derivative, analog, or prodrug of an alkylating hexitol derivative or a modified alkylating hexitol derivative, wherein the modified alkylating hexitol derivative or the derivative, analog or prodrug of the modified alkylating hexitol derivative possesses increased therapeutic efficacy or reduced side effects for treatment of a TKI-resistant malignancy or a malignancy associated with dysregulation or mutation of the *AHI1* gene as compared with an unmodified alkylating hexitol derivative;
(ii) a composition comprising:
   (a) a therapeutically effective quantity of an alkylating hexitol derivative, a modified alkylating hexitol derivative, or a derivative, analog, or prodrug of an alkylating hexitol derivative or a modified alkylating hexitol derivative; and
   (b) at least one additional therapeutic agent, therapeutic agent subject to chemosensitization, therapeutic agent subject to chemopotentiation, diluent, excipient, solvent system, or drug delivery system, wherein the composition possesses increased therapeutic efficacy or reduced side effects for treatment of a TKI-resistant malignancy or a malignancy associated with dysregulation or mutation of the *AHI1* gene as compared with an unmodified alkylating hexitol derivative;
(iii) a therapeutically effective quantity of an alkylating hexitol derivative, a modified alkylating hexitol derivative, or a derivative, analog, or prodrug of an alkylating hexitol derivative or a modified alkylating hexitol derivative that is incorporated into a dosage form, wherein an alkylating hexitol derivative, a modified alkylating hexitol derivative, or a derivative, analog, or prodrug of an alkylating hexitol derivative or a modified alkylating hexitol derivative incorporated into the dosage form possesses increased therapeutic efficacy or reduced side effects for treatment of a TKI-resistant malignancy or a malignancy associated with dysregulation or mutation of the *AHI1* gene as compared with an unmodified alkylating hexitol derivative;
(iv) a therapeutically effective quantity of an alkylating hexitol derivative, a modified alkylating hexitol derivative, or a derivative, analog, or prodrug of an alkylating hexitol derivative or a modified alkylating hexitol derivative that is incorporated into a dosage kit and packaging, wherein an alkylating hexitol derivative, a modified alkylating hexitol derivative, or a derivative, analog, or prodrug of an alkylating hexitol derivative or a modified alkylating hexitol derivative incorporated into the dosage kit and packaging possesses increased therapeutic efficacy or reduced side effects for treatment of a TKI-resistant malignancy or a malignancy associated with dysregulation or mutation of the *AHI1* gene as compared with an unmodified alkylating hexitol derivative; and
(v) a therapeutically effective quantity of an alkylating hexitol derivative, a modified alkylating hexitol derivative, or a derivative, analog, or prodrug of an alkylating hexitol derivative or a modified alkylating hexitol derivative that is subjected to a bulk drug product improvement, wherein the an alkylating hexitol derivative, a modified alkylating hexitol derivative, or a derivative, analog, or prodrug of an alkylating hexitol derivative or a modified alkylating hexitol derivative subject to the bulk drug product improvement possesses increased therapeutic efficacy or reduced side effects for treatment of a TKI-resistant malignancy or a malignancy associated with dysregulation or mutation of the *AHI1* gene as compared with an unmodified alkylating hexitol derivative.

As described above, the alkylating hexitol derivative described herein can be, but is not limited to, dianhydrogalactitol, a derivative or analog of dianhydrogalactitol, diacetyldianhydrogalactitol, a derivative or analog of diacetyldianhydrogalactitol, dibromodulcitol, or a derivative or analog of dibromodulcitol.

In one alternative disclosure, the composition possesses increased therapeutic efficacy or reduced side effects for treatment of a TKI-resistant malignancy.

In one alternative disclosure, the composition possesses increased therapeutic efficacy or reduced side effects for treatment of a malignancy associated with dysregulation or mutation of the *AHI1* gene.

In one alternative disclosure, the composition comprises a drug combination comprising:
(i) an alkylating hexitol derivative, a modified alkylating hexitol derivative, or a derivative, analog, or prodrug of an alkylating hexitol derivative or a modified alkylating hexitol derivative; and
(ii) an additional therapeutic agent selected from the group consisting of:
   (a) topoisomerase inhibitors;
   (b) fraudulent nucleosides;
   (c) fraudulent nucleotides;
   (d) thymidylate synthetase inhibitors;
   (e) signal transduction inhibitors;
   (f) cisplatin or platinum analogs;
   (g) alkylating agents;
   (h) anti-tubulin agents;
   (i) antimetabolites;
   (j) berberine;
   (k) apigenin;
   (l) amonafide;
   (m) vinca alkaloids;
   (n) 5-fluorouracil;
   (o) curcumin;
   (p) NF-κB inhibitors;
   (q) rosmarinic acid;
   (r) mitoguazone;
   (s) tetrandrine;
   (t) a JAK2 inhibitor;
   (u) a STAT5 inhibitor; and
   (v) a Src inhibitor.

In these alternative disclosures, when the additional therapeutic agent is an alkylating agent, the alkylating agent can be, but is not limited to, an alkylating agent selected from the group consisting of BCNU, BCNU wafers, CCNU, bendamustine (Treanda), and temozolimide (Temodar).

In another alternative disclosure, the composition comprises a drug combination comprising:
(i) an alkylating hexitol derivative, a modified alkylating hexitol derivative, or a derivative, analog, or prodrug of an alkylating hexitol derivative or a modified alkylating hexitol derivative; and
(ii) a BH3 mimetic.

In another alternative, the composition comprises a drug combination comprising:
(i) an alkylating hexitol derivative, a modified alkylating hexitol derivative, or a derivative, analog, or prodrug of an alkylating hexitol derivative or a modified alkylating hexitol derivative; and
(ii) an agent that modulates the expression of the *AHI1* gene or modulates the activity of AHI1 protein.

In still another alternative disclosure, the composition comprises a drug combination comprising:
(i) an alkylating hexitol derivative, a modified alkylating hexitol derivative, or a derivative, analog, or prodrug of an alkylating hexitol derivative or a modified alkylating hexitol derivative;
(ii) an agent that modulates the expression of the *AHI1* gene or the activity of AHI1 protein; and
(iii) a BH3 mimetic.

In another alternative disclosure, the composition comprises:
(i) an alkylating hexitol derivative, a modified alkylating hexitol derivative, or a derivative, analog, or prodrug of an alkylating hexitol derivative or a modified alkylating hexitol derivative; and
(ii) a therapeutic agent subject to chemosensitization selected from the group consisting of:
   (a) topoisomerase inhibitors;
   (b) fraudulent nucleosides;
   (c) fraudulent nucleotides;
   (d) thymidylate synthetase inhibitors;
   (e) signal transduction inhibitors;
   (f) cisplatin or platinum analogs;
   (g) alkylating agents;
   (h) anti-tubulin agents;
   (i) antimetabolites;
   (j) berberine;
   (k) apigenin;
   (l) colchicine or an analog of colchicine;
   (m) genistein;
   (n) etoposide;
   (o) cytarabine;
   (p) camptothecin;
   (q) vinca alkaloids;
   (r) 5-fluorouracil;
   (s) curcumin;
   (t) NF-κB inhibitors;
   (u) rosmarinic acid; and
   (v) mitoguazone;
wherein the alkylating hexitol derivative, a modified alkylating hexitol derivative, or a derivative, analog, or prodrug of an alkylating hexitol derivative or a modified alkylating hexitol derivative acts as a chemosensitizer.

In still another alternative disclosure, the composition comprises:
(i) an alkylating hexitol derivative, a modified alkylating hexitol derivative, or a derivative, analog, or prodrug of an alkylating hexitol derivative or a modified alkylating hexitol derivative; and
(ii) a therapeutic agent subject to chemopotentiation selected from the group consisting of:
   (a) fraudulent nucleosides;
   (b) fraudulent nucleotides;
   (c) thymidylate synthetase inhibitors;
   (d) signal transduction inhibitors;
   (e) cisplatin or platinum analogs;
   (f) alkylating agents;
   (g) anti-tubulin agents;
   (h) antimetabolites;
   (i) berberine;
   (j) apigenin;
   (k) colchicine or analogs of colchicine;
   (l) genistein;
   (m) etoposide;
   (n) cytarabine;
   (o) camptothecins;
   (p) vinca alkaloids;
   (q) topoisomerase inhibitors;
   (r) 5-fluorouracil;
   (s) curcumin;
   (t) NF-κB inhibitors;
   (u) rosmarinic acid;
   (v) mitoguazone; and
   (w) a biotherapeutic.
wherein the alkylating hexitol derivative, a modified alkylating hexitol derivative, or a derivative, analog, or prodrug of an alkylating hexitol derivative or a modified alkylating hexitol derivative acts as a chemopotentiator.

In these alternative disclosures, wherein the additional therapeutic agent is a biotherapeutic, the biotherapeutic can be, but is not limited to, a biotherapeutic selected from the group consisting of Avastin, Herceptin, Rituxan, and Erbitux.

In yet another alternative disclosure, the alkylating hexitol derivative, a modified alkylating hexitol derivative, or a derivative, analog, or prodrug of an alkylating hexitol derivative or a modified alkylating hexitol derivative is subjected to a bulk drug product improvement, wherein the bulk drug product improvement is selected from the group consisting of:
(a) salt formation;
(b) preparation as a homogeneous crystal structure;
(c) preparation as a pure isomer;
(d) increased purity;
(e) preparation with lower residual solvent content; and
(f) preparation with lower residual heavy metal content.

In still another alternative disclosure, the composition comprises an alkylating hexitol derivative, a modified alkylating hexitol derivative, or a derivative, analog, or prodrug of an alkylating hexitol derivative or a modified alkylating hexitol derivative and a diluent, wherein the diluent is selected from the group consisting of:
(a) an emulsion;
(b) dimethylsulfoxide (DMSO);
(c) N-methylformamide (NMF)
(d) dimethylformamide (DMF)
(e) dimethylacetamide (DMA);
(f) ethanol;
(g) benzyl alcohol;
(h) dextrose-containing water for injection;
(i) Cremophor;
(j) cyclodextrins; and
(k) PEG.

In still another alternative disclosure, the composition comprises an alkylating hexitol derivative, a modified alkylating hexitol derivative, or a derivative, analog, or prodrug of an alkylating hexitol derivative or a modified alkylating hexitol derivative and a solvent system, wherein the solvent system is selected from the group consisting of:
(a) an emulsion;
(b) DMSO;
(c) NMF;
(d) DMF;
(e) DMA;
(f) ethanol;
(g) benzyl alcohol;
(h) dextrose-containing water for injection;
(i) Cremophor;
(j) PEG; and
(k) salt systems.

In yet another alternative disclosure, the composition comprises an alkylating hexitol derivative, a modified alkylating hexitol derivative, or a derivative, analog, or prodrug of an alkylating hexitol derivative or a modified alkylating hexitol derivative and an excipient, wherein the excipient is selected from the group consisting of:
(a) mannitol;
(b) albumin;
(c) EDTA;
(d) sodium bisulfite;
(e) benzyl alcohol;
(f) carbonate buffers;
(g) phosphate buffers;
(h) PEG;
(i) vitamin A;
(j) vitamin D;
(k) vitamin E;
(l) esterase inhibitors;
(m) cytochrome P450 inhibitors;
(n) multi-drug resistance (MDR) inhibitors;
(o) organic resins;
(p) detergents;
(q) perillyl alcohol or an analog thereof; and
(r) activators of channel-forming receptors.

In still another alternative disclosure, the alkylating hexitol derivative, modified alkylating hexitol derivative, or derivative, analog, or prodrug of an alkylating hexitol derivative or modified alkylating hexitol derivative is incorporated into a dosage form selected from the group consisting of:
(a) tablets;
(b) capsules;
(c) topical gels;
(d) topical creams;
(e) patches;
(f) suppositories;
(g) lyophilized dosage fills;
(h) immediate-release formulations;
(i) slow-release formulations;
(j) controlled-release formulations; and
(k) liquid in capsules.

In still another alternative disclosure, the alkylating hexitol derivative, modified alkylating hexitol derivative, or derivative, analog, or prodrug of an alkylating hexitol derivative or modified alkylating hexitol derivative is incorporated into a dosage kit and packaging selected from the group consisting of amber vials to protect from light and stoppers with specialized coatings to improve shelf-life stability.

In still another alternative disclosure, the composition comprises: (i) an alkylating hexitol derivative, modified alkylating hexitol derivative, or derivative, analog, or prodrug of an alkylating hexitol derivative or modified alkylating hexitol derivative; and (ii) a drug delivery system, wherein the drug delivery system is selected from the group consisting of:
(a) oral dosage forms;
(b) nanocrystals;
(c) nanoparticles;
(d) cosolvents;
(e) slurries;
(f) syrups;
(g) bioerodible polymers;
(h) liposomes;
(i) slow-release injectable gels;
(j) microspheres; and
(k) targeting compositions with epidermal growth factor receptor-binding peptides.

In still another alternative of a composition described herein the therapeutic agent is a modified alkylating hexitol derivative, and the modification is selected from the group consisting of:
(a) alteration of side chains to increase or decrease lipophilicity;
(b) addition of an additional chemical functionality to alter a property selected from the group consisting of reactivity, electron affinity, and binding capacity; and
(c) alteration of salt form.

In still another alternative of a composition described herein, the therapeutic agent is an alkylating hexitol derivative, modified alkylating hexitol derivative, or derivative or analog of an alkylating hexitol derivative or modified alkylating hexitol derivative and the therapeutic agent is present in the composition in a drug conjugate form, wherein the drug conjugate form is a drug conjugate form selected from the group consisting of:
(a) a polymer system;
(b) polylactides;
(c) polyglycolides;
(d) amino acids;
(e) peptides;
(f) multivalent linkers;
(g) immunoglobulins;
(h) cyclodextrin polymers;
(i) modified transferrin;
(j) hydrophobic or hydrophobic-hydrophilic polymers;
(k) conjugates with a phosphonoformic acid partial ester;
(l) conjugates with a cell-binding agent incorporating a charged cross-linker; and
(m) conjugates with β-glucuronides through a linker.

In still another alternative of a composition described herein, the therapeutic agent is an alkylating hexitol derivative, modified alkylating hexitol derivative, or derivative or analog of an alkylating hexitol derivative or modified alkylating hexitol derivative and the therapeutic agent is in the form of a prodrug system, wherein the prodrug system is selected from the group consisting of:
(a) enzyme sensitive esters;
(b) dimers;
(c) Schiff bases;
(d) pyridoxal complexes;
(e) caffeine complexes;
(f) nitric oxide-releasing prodrugs;
(g) prodrugs with fibroblast activation protein α-cleavable oligopeptides;
(h) products of reaction with an acylating or carbamylating agent;
(i) hexanoate conjugates;
(j) polymer-agent conjugates; and
(k) prodrugs that are subject to redox activation.

In still another alternative of a composition described herein, the therapeutic agent is an alkylating hexitol derivative, modified alkylating hexitol derivative, or derivative, analog, or prodrug of an alkylating hexitol derivative or modified alkylating hexitol derivative and the composition further comprises at least one additional therapeutic agent to form a multiple drug system, wherein the at least one additional therapeutic agent is selected from the group consisting of:
(a) an inhibitor of multi-drug resistance;
(b) a specific drug resistance inhibitor;
(c) a specific inhibitor of a selective enzyme;
(d) a signal transduction inhibitor;
(e) an inhibitor of a repair enzyme; and
(f) a topoisomerase inhibitor with non-overlapping side effects.

In still another alternative of a composition described herein, the composition comprises a therapeutic agent that is an alkylating hexitol derivative, modified alkylating hexitol derivative, or derivative, analog, or prodrug of an alkylating hexitol derivative or modified alkylating hexitol derivative and a BH3 mimetic as described above.

When a pharmaceutical composition described herein includes a prodrug, prodrugs and active metabolites of a compound may be identified using routine techniques known in the art. See, e.g., Bertolini et al., J. Med. Chem., 40, 2011-2016 (1997); Shan et al., J. Pharm. Sci., 86 (7), 765-767; Bagshawe, Drug Dev. Res., 34, 220-230 (1995); Bodor, Advances in Drug Res., 13, 224-331 (1984); Bundgaard, Design of Prodrugs (Elsevier Press 1985); Larsen, Design and Application of Prodrugs, Drug Design and Development (Krogsgaard-Larsen et al., eds., Harwood Academic Publishers, 1991); Dear et al., J. Chromatogr. B, 748, 281-293 (2000); Spraul etal., J. Pharmaceutical & Biomedical Analysis, 10, 601-605 (1992); and Prox et al., Xenobiol., 3, 103-112 (1992).

When the pharmacologically active compound in a pharmaceutical composition described herein possesses a sufficiently acidic, a sufficiently basic, or both a sufficiently acidic and a sufficiently basic functional group, these group or groups can accordingly react with any of a number of inorganic or organic bases, and inorganic and organic acids, to form a pharmaceutically acceptable salt. Exemplary pharmaceutically acceptable salts described herein include those salts prepared by reaction of the pharmacologically active compound with a mineral or organic acid or an inorganic base, such as salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, β-hydroxybutyrates, glycolates, tartrates, methane-sulfonates, propanesulfonates, naphthalene-1-sulfonates, naphthalene-2-sulfonates, and mandelates. If the pharmacologically active compound has one or more basic functional groups, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha-hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like. If the pharmacologically active compound has one or more acidic functional groups, the desired pharmaceutically acceptable salt may be prepared by any suitable method available in the art, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

In the case of agents that are solids, it is understood by those skilled in the art that the inventive compounds and salts may exist in different crystal or polymorphic forms, all of which are intended to be within the scope of the present disclosure and specified formulas.

The amount of a given pharmacologically active agent, such as dianhydrogalactitol or an analog or derivative of dianhydrogalactitol as described above, that is included in a unit dose of a pharmaceutical composition according to the present invention will vary depending upon factors such as the particular compound, disease condition and its severity, the identity (e.g., weight) of the subject in need of treatment, but can nevertheless be routinely determined by one skilled in the art. Typically, such pharmaceutical compositions described herein include a therapeutically effective quantity of the pharmacologically active agent and an inert pharmaceutically acceptable carrier or diluent. Typically, these compositions described herein are prepared in unit dosage form appropriate for the chosen route of administration, such as oral administration or parenteral administration. A pharmacologically active agent as described above can be administered in conventional dosage form prepared by combining a therapeutically effective amount of such a pharmacologically active agent as an active ingredient with appropriate pharmaceutical carriers or diluents according to conventional procedures. These procedures may involve mixing, granulating and compressing or dissolving the ingredients as appropriate to the desired preparation. The pharmaceutical carrier employed may be either a solid or liquid. Exemplary of solid carriers described herein are lactose, sucrose, talc, gelatin, agar, pectin, acacia, magnesium stearate, stearic acid and the like. Exemplary of liquid carriers described herein are syrup, peanut oil, olive oil, water and the like. Similarly, the carrier or diluent may include time-delay or time-release material known in the art, such as glyceryl monostearate or glyceryl distearate alone or with a wax, ethylcellulose, hydroxypropylmethylcellulose, methylmethacrylate and the like.

A variety of pharmaceutical forms can be employed. Thus, if a solid carrier is used, the preparation can be tableted, placed in a hard gelatin capsule in powder or pellet form or in the form of a troche or lozenge. The amount of solid carrier may vary, but generally will be from about 25 mg to about 1 g. If a liquid carrier is used, the preparation will be in the form of syrup, emulsion, soft gelatin capsule, sterile injectable solution or suspension in an ampoule or vial or non-aqueous liquid suspension.

To obtain a stable water-soluble dose form, a pharmaceutically acceptable salt of a pharmacologically active agent as described above is dissolved in an aqueous solution of an organic or inorganic acid, such as 0.3 M solution of succinic acid or citric acid. If a soluble salt form is not available, the agent may be dissolved in a suitable cosolvent or combinations of cosolvents. Examples of suitable cosolvents include, but are not limited to, alcohol, propylene glycol, polyethylene glycol 300, polysorbate 80, glycerin and the like in concentrations ranging from 0-60% of the total volume. In an exemplary embodiment, a compound of Formula I is dissolved in DMSO and diluted with water. The composition described herein may also be in the form of a solution of a salt form of the active ingredient in an appropriate aqueous vehicle such as water or isotonic saline or dextrose solution.

It will be appreciated that the actual dosages of the agents used in the compositions described herein will vary according to the particular complex being used, the particular composition formulated, the mode of administration and the particular site, host and disease and/or condition being treated. Actual dosage levels of the active ingredients in the pharmaceutical compositions described herein can be varied so as to obtain an amount of the active ingredient which is effective to achieve the desired therapeutic response for a particular subject, composition, and mode of administration, without being toxic to the subject. The selected dosage level depends upon a variety of pharmacokinetic factors including the activity of the particular therapeutic agent, the route of administration, the time of administration, the rate of excretion of the particular compound being employed, the severity of the condition, other health considerations affecting the subject, and the status of liver and kidney function of the subject. It also depends on the duration of the treatment, other drugs, compounds and/or materials used in combination with the particular therapeutic agent employed, as well as the age, weight, condition, general health and prior medical history of the subject being treated, and like factors. Methods for determining optimal dosages are described in the art, e.g., Remington: The Science and Practice of Pharmacy, Mack Publishing Co., 20th ed., 2000. Optimal dosages for a given set of conditions can be ascertained by those skilled in the art using conventional dosage-determination tests in view of the experimental data for an agent. For oral administration, an exemplary daily dose generally employed is from about 0.001 to about 3000 mg/kg of body weight, with courses of treatment repeated at appropriate intervals. In some disclosures, the daily dose is from about 1 to 3000 mg/kg of body weight.

Typical daily doses in a patient may be anywhere between about 500 mg to about 3000 mg, given once or twice daily, e.g., 3000 mg can be given twice daily for a total dose of 6000 mg. In one disclosure, the dose is between about 1000 to about 3000 mg. In another disclosure, the dose is between about 1500 to about 2800 mg. In other disclosures, the dose is between about 2000 to about 3000 mg.

Plasma concentrations in the subjects may be between about 100 µM to about 1000 µM. In some disclosures, the plasma concentration may be between about 200 µM to about 800 µM. In other disclosures, the concentration is about 300 µM to about 600 µM. In still other embodiments the plasma concentration may be between about 400 to about 800 µM. Administration of prodrugs described herein is typically dosed at weight levels, which are chemically equivalent to the weight levels of the fully active form.

The compositions described herein may be manufactured using techniques generally known for preparing pharmaceutical compositions, e.g., by conventional techniques such as mixing, dissolving, granulating, dragee-making, levitating, emulsifying, encapsulating, entrapping or lyophilizing. Pharmaceutical compositions may be formulated in a conventional manner using one or more physiologically acceptable carriers, which may be selected from excipients and auxiliaries that facilitate processing of the active compounds into preparations, which can be used pharmaceutically.

Proper formulation is dependent upon the route of administration chosen. For injection, the agents of the invention may be formulated into aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers known in the art. Such carriers enable the compounds of the invention to be formulated as tablets, pills, dragees, capsules, liquids, gels, syrups, slurries, solutions, suspensions and the like, for oral ingestion by a patient to be treated. Pharmaceutical preparations for oral use can be obtained using a solid excipient in admixture with the active ingredient (agent), optionally grinding the resulting mixture, and processing the mixture of granules after adding suitable auxiliaries, if desired, to obtain tablets or dragee cores. Suitable excipients include: fillers such as sugars, including lactose, sucrose, mannitol, or sorbitol; and cellulose preparations, for example, maize starch, wheat starch, rice starch, potato starch, gelatin, gum, methyl cellulose, hydroxypropylmethyl-cellulose, sodium carboxymethylcellulose, or polyvinylpyrrolidone (PVP). If desired, disintegrating agents may be added, such as crosslinked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, polyvinyl pyrrolidone, Carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active agents.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as talc or magnesium stearate, and, optionally, stabilizers. In soft capsules, the active agents may be dissolved or suspended in suitable liquids, such as fatty oils, liquid paraffin, or liquid polyethylene glycols. In addition, stabilizers may be added. All formulations for oral administration should be in dosages suitable for such administration. For buccal administration, the compositions may take the form of tablets or lozenges formulated in conventional manner.

Pharmaceutical formulations for parenteral administration can include aqueous solutions or suspensions. Suitable lipophilic solvents or vehicles include fatty oils such as sesame oil or synthetic fatty acid esters, such as ethyl oleate or triglycerides. Aqueous injection suspensions may contain substances which increase the viscosity of the suspension, such as sodium carboxymethyl cellulose, sorbitol, or dextran. Optionally, the suspension may also contain suitable stabilizers or modulators which increase the solubility or dispersibility of the composition to allow for the preparation of highly concentrated solutions, or can contain suspending or dispersing agents. Pharmaceutical preparations for oral use can be obtained by combining the pharmacologically active agent with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating modulators may be added, such as the cross-linked polyvinyl pyrrolidone, agar, or alginic acid or a salt thereof such as sodium alginate.

Other ingredients such as stabilizers, for example, antioxidants such as sodium citrate, ascorbyl palmitate, propyl gallate, reducing agents, ascorbic acid, vitamin E, sodium bisulfite, butylated hydroxytoluene, BHA, acetylcysteine, monothioglycerol, phenyl-α-naphthylamine, or lecithin can be used. Also, chelators such as EDTA can be used. Other ingredients that are conventional in the area of pharmaceutical compositions and formulations, such as lubricants in tablets or pills, coloring agents, or flavoring agents, can be used. Also, conventional pharmaceutical excipients or carriers can be used. The pharmaceutical excipients can include, but are not necessarily limited to, calcium carbonate, calcium phosphate, various sugars or types of starch, cellulose derivatives, gelatin, vegetable oils, polyethylene glycols and physiologically compatible solvents. Other pharmaceutical excipients are well known in the art. Exemplary pharmaceutically acceptable carriers include, but are not limited to, any and/or all of solvents, including aqueous and non-aqueous solvents, dispersion media, coatings, antibacterial and/or antifungal agents, isotonic and/or absorption delaying agents, and/or the like. The use of such media and/or agents for pharmaceutically active substances is well known in the art. Except insofar as any conventional medium, carrier, or agent is incompatible with the active ingredient or ingredients, its use in a composition according to the present invention is contemplated. Supplementary active ingredients can also be incorporated into the compositions, particularly as described above. For administration of any of the compounds used in the present invention, preparations should meet sterility, pyrogenicity, general safety, and purity standards as required by the FDA Office of Biologics Standards or by other regulatory organizations regulating drugs.

For administration intranasally or by inhalation, the compounds for use described herein are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of gelatin for use in an inhaler or insufflator and the like may be formulated containing a powder mix of the compound and a suitable powder base such as lactose or starch.

The compounds may be formulated for parenteral administration by injection, e.g., by bolus injection or continuous infusion. Formulations for injection may be presented in unit-dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active agents may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents, which increase the solubility of the compounds to allow for the preparation of highly concentrated solutions.

Alternatively, the active ingredient may be in powder form for constitution with a suitable vehicle, e.g., sterile pyrogen-free water, before use. The compounds may also be formulated in rectal compositions such as suppositories or retention enemas, e.g., containing conventional suppository bases such as cocoa butter or other glycerides.

In addition to the formulations described above, the compounds may also be formulated as a depot preparation. Such long-acting formulations may be administered by implantation (for example, subcutaneously or intramuscularly) or by intramuscular injection. Thus, for example, the compounds may be formulated with suitable polymeric or hydrophobic materials (for example, as an emulsion in an acceptable oil) or ion-exchange resins, or as sparingly soluble derivatives, for example, as a sparingly soluble salt.

An exemplary pharmaceutical carrier for hydrophobic compounds is a cosolvent system comprising benzyl alcohol, a nonpolar surfactant, a water-miscible organic polymer, and an aqueous phase. The cosolvent system may be a VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) contains VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may be substituted for dextrose.

Alternatively, other delivery systems for hydrophobic pharmaceutical compounds may be employed. Liposomes and emulsions are known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid- or gel-phase carriers or excipients. Examples of such carriers or excipients include calcium carbonate, calcium phosphate, sugars, starches, cellulose derivatives, gelatin, and polymers such as polyethylene glycols.

A pharmaceutical composition can be administered by a variety of methods known in the art. The routes and/or modes of administration vary depending upon the desired results. Depending on the route of administration, the pharmacologically active agent may be coated in a material to protect the targeting composition or other therapeutic agent from the action of acids and other compounds that may inactivate the agent. Conventional pharmaceutical practice can be employed to provide suitable formulations or compositions for the administration of such pharmaceutical compositions to subjects. Any appropriate route of administration can be employed, for example, but not limited to, intravenous, parenteral, intraperitoneal, intravenous, transcutaneous, subcutaneous, intramuscular, intraurethral, or oral administration. Depending on the severity of the malignancy or other disease, disorder, or condition to be treated, as well as other conditions affecting the subject to be treated, either systemic or localized delivery of the pharmaceutical composition can be used in the course of treatment. The pharmaceutical composition as described above can be administered together with additional therapeutic agents intended to treat a particular disease or condition, which may be the same disease or condition that the pharmaceutical composition is intended to treat, which may be a related disease or condition, or which even may be an unrelated disease or condition.

Pharmaceutical compositions described herein can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J.R. Robinson, ed., Marcel Dekker, Inc., New York, 1978. Pharmaceutical compositions are preferably manufactured under GMP conditions. Formulations for parenteral administration may, for example, contain excipients, sterile water, or saline, polyalkylene glycols such as polyethylene glycol, oils of vegetable origin, or hydrogenated naphthalenes. Biocompatible, biodegradable lactide polymers, lactide/glycolide copolymers, or polyoxyethylene-polyoxypropylene copolymers may be used to control the release of the compounds. Other potentially useful parenteral delivery systems for molecules of the invention include ethylene-vinyl acetate copolymer particles, osmotic pumps, and implantable infusion systems. Formulations for inhalation may contain excipients, for example, lactose, or may be aqueous solutions containing, e.g., polyoxyethylene-9-lauryl ether, glycocholate and deoxycholate, or can be oily solutions for administration or gels.

Pharmaceutical compositions described herein are usually administered to the subjects on multiple occasions. Intervals between single dosages can be weekly, monthly or yearly. Intervals can also be irregular as indicated by therapeutic response or other parameters well known in the art. Alternatively, the pharmaceutical composition can be administered as a sustained release formulation, in which case less frequent administration is required. Dosage and frequency vary depending on the half-life in the subject of the pharmacologically active agent included in a pharmaceutical composition. The dosage and frequency of administration can vary depending on whether the treatment is prophylactic or therapeutic. In prophylactic applications, a relatively low dosage is administered at relatively infrequent intervals over a long period of time. Some subjects may continue to receive treatment for the rest of their lives. In therapeutic applications, a relatively high dosage at relatively short intervals is sometimes required until progression of the disease is reduced or terminated, and preferably until the subject shows partial or complete amelioration of symptoms of disease. Thereafter, the subject can be administered a prophylactic regime.

For the purposes of the present application, treatment can be monitored by observing one or more of the improving symptoms associated with the disease, disorder, or condition being treated, or by observing one or more of the improving clinical parameters associated with the disease, disorder, or condition being treated, as described above.

Sustained-release formulations or controlled-release formulations are well-known in the art. For example, the sustained-release or controlled-release formulation can be (1) an oral matrix sustained-release or controlled-release formulation; (2) an oral multilayered sustained-release or controlled-release tablet formulation; (3) an oral multiparticulate sustained-release or controlled-release formulation; (4) an oral osmotic sustained-release or controlled-release formulation; (5) an oral chewable sustained-release or controlled-release formulation; or (6) a dermal sustained-release or controlled-release patch formulation.

The pharmacokinetic principles of controlled drug delivery are described, for example, in B.M. Silber et al., "Pharmacokinetic/Pharmacodynamic Basis of Controlled Drug Delivery" in Controlled Drug Delivery: Fundamentals and Applications (J.R. Robinson & V.H.L. Lee, eds, 2d ed., Marcel Dekker, New York, 1987), ch. 5, pp. 213-251.

One of ordinary skill in the art can readily prepare formulations for controlled release or sustained release comprising a pharmacologically active agent according to the present invention by modifying the formulations described above, such as according to principles disclosed in V.H.K. Li et al, "Influence of Drug Properties and Routes of Drug Administration on the Design of Sustained and Controlled Release Systems" in Controlled Drug Delivery: Fundamentals and Applications (J.R. Robinson & V.H.L. Lee, eds, 2d ed., Marcel Dekker, New York, 1987), ch. 1, pp. 3-94

This process of preparation typically takes into account physicochemical properties of the pharmacologically active agent, such as aqueous solubility, partition coefficient, molecular size, stability, and nonspecific binding to proteins and other biological macromolecules. This process of preparation also takes into account biological factors, such as absorption, distribution, metabolism, duration of action, the possible existence of side effects, and margin of safety, for the pharmacologically active agent. Accordingly, one of ordinary skill in the art could modify the formulations into a formulation having the desirable properties described above for a particular application.

United States Patent No. 6,573,292 by Nardella, United States Patent No. 6,921,722 by Nardella, United States Patent No. 7,314,886 to Chao et al., and United States Patent No. 7,446,122 by Chao et al., which disclose methods of use of various pharmacologically active agents and pharmaceutical compositions in treating a number of diseases and conditions, including cancer, and methods of determining the therapeutic effectiveness of such pharmacologically active agents and pharmaceutical compositions.

Typically, the therapeutically effective quantity of dianhydrogalactitol is about 40 mg/m². The therapeutically effective quantity of diacetyldianhydrogalactitol or dibromodulcitol is similar taking into account differences in molecular weight.

Typically, the dianhydrogalactitol is administered by a route selected from the group consisting of intravenous and oral. Preferably, the dianhydrogalactitol is administered intravenously. Similar routes can be used for diacetyldianhydrogalactitol or dibromodulcitol

The method can further comprise the step of administering a therapeutically effective dose of ionizing radiation.

The invention is illustrated by the following Example. This Example is included for illustrative purposes only.

### Example

### Use of Dianhydrogalactitol to Inhibit Growth of Tumor Cells

### Materials and Methods:

Cell Lines and Culture Conditions: All cells were cultured in DMEM (Dulbecco's Modified Eagle's medium; Invitrogen/Gibco) with 10% FBS (fetal bovine serum; Invitrogen/Gibco) at 37° C with 5% CO₂, and subcultured twice weekly during the experimental period.

Drugs: A stock solution of 100 mM was kept at -20° C before use. Dianhydrogalactitol (DAG; results with DAG are shown as "VAL" in the figures) was provided by Del Mar Pharmaceuticals Ltd. A stock solution of 100 mM was prepared by dissolving the lyophilized powder in the injection vial in sterile phosphate buffered saline (PBS) and kept at 20° C before use.

Growth Assays: Each cell line used was seeded at 3000 cells/well in 100 µL medium in a 96-well plate (BD Falcon) and incubated overnight. Cells were then treated with DAG at concentrations of 0.1-100 µM in fresh medium for 72 hours. The cells were fixed in 2% paraformaldehyde (Sigma-Aldrich) with nuclear dye Hoechst 33342 (1 µg/mL) (Sigma-Aldrich). After gentle washing with PBS, the cells were kept in fresh PBS and the plates were kept at 4° C in the dark before HCS (high content screening) (ThermoFisher Scientific) analysis. Twenty view fields per well were scanned and analyzed. Growth inhibition was calculated as a percentage of the control without the solvent and the drug; the samples treated with solvent alone served as a reference. There were three replicates for each treatment and the experiments were repeated once.

### Results

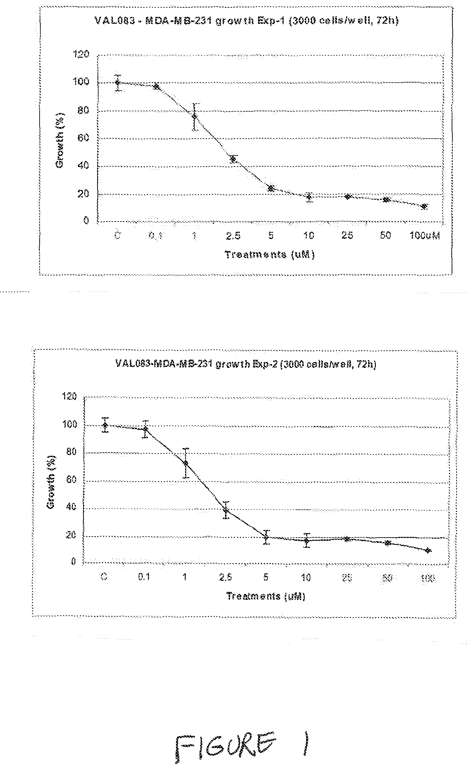

Figure 1 shows the effect of dianhydrogalactitol in concentration ranges from 0.1 µM to 100 µM on the cell line MDA-MB-231. This is an estrogen-independent human breast cancer cell line (T. Hiraga et al., "The Bisphosphonate Ibandronate Promotes Apoptosis in MDA-MB-231 Human Breast Cancer Cells in Bone Metastases," Cancer Res. 61: 4418-4424 (2001), incorporated herein by this reference).

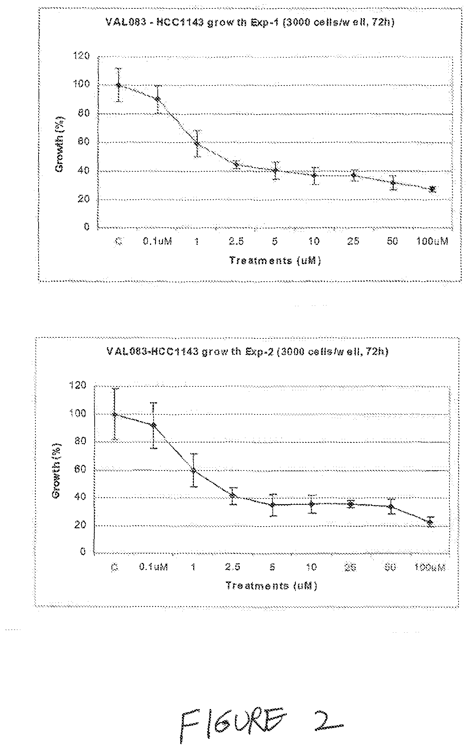

Figure 2 shows the effect of dianhydrogalactitol in concentration ranges from 0.1 µM to 100 µM on the cell line HCC1143. HCC1143 is a triple-negative breast cancer cell line that is insensitive to PARP or TKIs and is known to be an aggressive metastasizer (D. Tryfonopoulos et al., "Src: A Potential Target for the Treatment of Triple-Negative Breast Cancer," Ann. Oncol. 22: 2234-2240 (2011)).

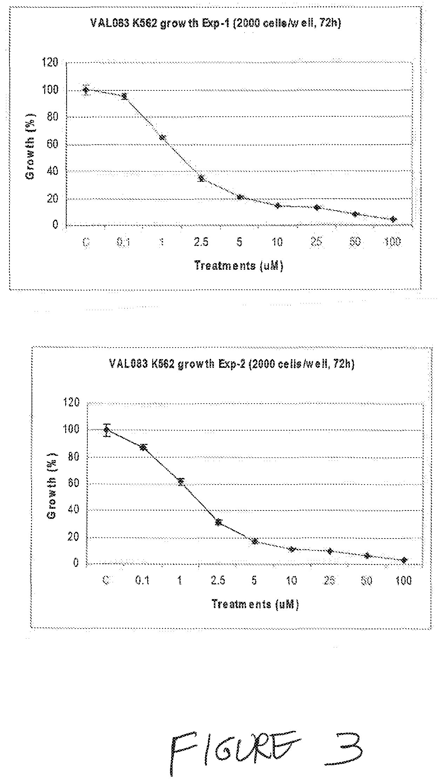

Figure 3 shows the effect of dianhydrogalactitol in concentration ranges from 0.1 µM to 100 µM on the cell line K562. K562 is a myelogenous leukemia cell line (C.B. Lozzio & B.B. Lozzio, "Human Chronic Myelogenous Leukemia Cell-Line with Positive Philadelphia Chromosome," Blood 45: 321-224 (1975)); wild-type K562 itself lacks the *BIM* deletion polymorphism and retains sensitivity to TKIs (K.P. Ng et al., "A Common BIM Deletion Polymorphism Mediates Intrinsic Resistance and Inferior Responses to Tyrosine Kinase Inhibitors in Cancer," Nat. Med. 18: 521-528 (2012).

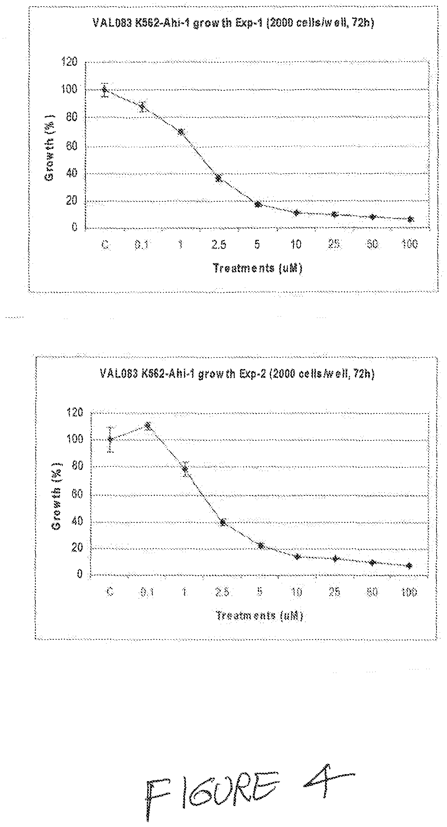

Figure 4 shows the effect of dianhydrogalactitol in concentration ranges from 0.1 µM to 100 µM on the cell line K562-AHI-1. The cell line K562-AHI-1 bears a mutation in which expression of *AHI1* is dysregulated. The AHI-1 mutation confers resistance to the TKI imatinib to K562 cells.

### Results

Dianhydrogalactitol showed a high degree of cytotoxicity against all cell lines tested. Notably, the cytotoxic effect of dianhydrogalactitol against K562 was not appreciably diminished by the introduction of a mutation in *AHI1* in K562-AHI-1. Additionally, dianhydrogalactitol showed a high degree of cytotoxicity on the triple-negative breast cancer cell line HCC1143 that is insensitive to PARP or TKIs and is known to be an aggressive metastasizer.

### ADVANTAGES OF THE INVENTION

The present invention is defined by the claims. This disclosure provides effective methods and compositions for treating TKI-resistant malignancies, especially in subjects possessing a germline polymorphism that causes an alternative splicing in the *BIM* gene that results in the generation of alternatively spliced isoforms of BIM that lack the crucial BH3 domain that is involved in the promotion of apoptosis. The present disclosure also describes effective methods and compositions for the treatment of malignancies associated with cells with a dysregulation of or a mutation in the *AHI1* gene. These methods and compositions described herein can treat such malignancies without relying on the inhibition of thymidine kinase. They are well tolerated and do not cause significant side effects.

Compositions and methods described herein possess industrial applicability for the preparation of a medicament for the treatment of a number of diseases and conditions in subjects possessing germline polymorphisms that block the effectiveness of tyrosine kinase inhibitors or in subjects with malignancies associated with cells with a dysregulation of or a mutation in the *AHI1* gene, especially hyperproliferative diseases, and possess industrial applicability as pharmaceutical compositions.

## Claims

1. A medicament comprising a therapeutically effective quantity of an alkylating hexitol derivative for use in the treatment of a malignancy in a patient resistant to tyrosine kinase inhibitor (TKI) chemotherapy wherein the malignancy is **characterized by** resistance to at least one tyrosine kinase inhibitor (TKI) due to:
(1) at least one mutation in a gene encoding a protein that is a target of at least one TKI; or
(2) the presence of at least one additional gene in either a wild-type or mutated state encoding a protein that confers resistance to the therapeutic effects of at least one TKI,
wherein the additional gene in either a wild-type or mutated state encoding the protein that confers resistance to the therapeutic effects of at least one TKI is *AHI-1*, or
wherein the resistance to at least one TKI is due to a mutation in the kinase domain of ABL1 protein that is part of a BCR-ABL fusion protein that is a target of TKIs,
wherein the alkylating hexitol derivative is dianhydrogalactitol, and
wherein the malignancy is selected from the group consisting of chronic myelogenous leukemia (CML), non-small cell lung carcinoma (NSCLC) and triple-negative breast cancer.

2. A medicament comprising a therapeutically effective quantity of dianhydrogalactitol for use in the treatment of a malignancy in a subject resistant to TKI chemotherapy suffering from a malignancy who has a germline deletion polymorphism conferring resistance to tyrosine kinase inhibitors (TKIs), wherein the malignancy is selected from the group consisting of chronic myelogenous leukemia (CML), non-small cell lung carcinoma (NSCLC) and triple-negative breast cancer.

3. The medicament for the use according to claim 2 wherein the germline DNA deletion polymorphism is a germline DNA deletion polymorphism of 2903 bp located in the *BIM* gene, and wherein the germline DNA deletion polymorphism causes a splicing variation that leads to expression of an isoform of BIM protein that lacks a BH3 domain and thus inhibits the induction of apoptosis.

4. The medicament for the use according to any one of claims 1 or 2, wherein the medicament further comprises a therapeutically effective quantity of a BH3 mimetic, wherein the BH3 mimetic is selected from the group consisting of:
(a) a peptide;
(b) a modified peptide;
(c) a terpyridine-based peptidomimetic;
(d) a terephthalamide-based peptidomimetic;
(e) a benzoylurea-based peptidomimetic;
(f) obatoclax;
(g) TW37;
(h) an analog of TW37 selected from (1) an analog of TW37 wherein one or more of the hydrogens of the benzene rings are replaced with an alkyl having from 1 to 6 carbon atoms; and (2) an analog of TW37 wherein one or more of the hydrogens in the hydroxyl groups of the trihydroxyphenyl moiety is replaced with an alkyl having from 1 to 6 carbon atoms;
(i) (-) gossypol;
(j) apogossypolone;
(k) A-385358;
(l) an analog of A-385358 selected from (1) an analog of A-385358 wherein one or more of the hydrogens of the benzene rings are replaced with alkyl having from 1 to 6 carbon atoms; and (2) an analog of A-385358 where the dimethylamino moiety is replaced with another moiety including one or two alkyl groups having from 1 to 6 carbon atoms bound to the amino group of the diethylamino moiety;
(m) ABT-737;
(n) an analog of ABT-737 selected from (1) an analogs of ABT-737 wherein the chlorine bound to the benzene ring is replaced with fluorine, bromine, or iodine; (2) an analog of ABT-737 wherein one or more of the hydrogens of the benzene rings are replaced with alkyl having from 1 to 6 carbon atoms; (3) an analog of ABT-737 wherein one or more of the hydrogens of the piperazinyl moiety are replaced with alkyl having from 1 to 6 carbon atoms; and (4) an analog of ABT-737 where the dimethylamino moiety is replaced with another moiety including one or two alkyl groups having 1 to 6 carbon atoms bound to the amino group of the diethylamino moiety;
(o) ABT-263;
(p) an analog of ABT-263 selected from (1) an analog of ABT-263 wherein the chlorine bound to the benzene ring is replaced with fluorine, bromine, or iodine; (2) an analog of ABT-263 wherein one or more of the hydrogens of the benzene rings are replaced with alkyl having 1 to 6 carbon atoms; (3) an analog of ABT-263 wherein one or more of the hydrogens of the piperazinyl moiety are replaced with alkyl having 1 to 6 carbon atoms; and (4) an analog of ABT-263 where the dimethylamino moiety is replaced with another moiety including one or two alkyl groups having 1 to 6 carbon atoms bound to the amino group of the diethylamino moiety;
(q) TM-1206; and
(r) an analog of TM-1206 selected from (1) an analog of TM-1206 wherein one or more of the hydrogens of the benzene rings are replaced with alkyl having 1 to 6 carbon atoms; and (2) an analog of TM-1206 wherein one or more of the hydrogens in the hydroxyl groups of the trihydroxyphenyl moiety is replaced with alkyl having from 1 to 6 carbon atoms.

5. The medicament for the use according to claim 4, wherein the medicament further comprises an additional therapeutic agent selected from the group consisting of:
(a) a therapeutically effective quantity of a tyrosine kinase inhibitor selected from the group consisting of erlotinib, afatinib, dacomitinib, imatinib, bosutinib, nilotinib, and dasatinib;
(b) a therapeutically effective quantity of a JAK2 inhibitor selected from the group consisting of (E)-N-benzyl-2-cyano-3-(3,4-dihydroxyphenyl)acrylamide (AG490), ruxolitinib, tofacitinib, tofacitinib citrate, N-tert-butyl-3-(5-methyl-2-(4-(2-(pyrrolidin-1-yl)ethoxy)phenyl amino)pyrimidin-4-ylamino)benzenesulfonamide (TG-101348), (S)-5-chloro-N2-(1-(5-fluoropyrimidin-2-yl)ethyl)-N4-(5-methyl-1H-pyrazol-3-yl) pyrimidine-2,4-diamine (AZD1480), N-(cyanomethyl)-4-(2-(4-morpholinophenylamino)pyrimidin-4-yl)benzamide (CYT387), baricitinib, (S,E)-3-(6-bromopyridin-2-yl)-2-cyano-N-(1-phenylethyl) acrylamide (WP1066), S-ruxolitinib, N-tert-butyl-3-(5-methyl-2-(4-(4-methylpiperazin-1-yl)phenylamino)pyrimidin-4-ylamino)benzene sulfonamide (TG101209), N-[3-(4-methyl-1-piperazinyl)phenyl]-8-[4-(methylsulfonyl)phenyl]-[1,2,4]triazolo[1,5-a]pyridin-2-amine (CEP33779), 8-(3,5-difluoro-4-(morpholinomethyl)phenyl)-2-(1-(piperidin-4-yl)-1H-pyrazol-4-yl)quinoxaline (NVP-BSK805), (S)-5-fluoro-2-(1-(4-fluorophenyl)ethylamino)-6-(5-methyl-1H-pyrazol-3-ylamino)nicotinonitrile (AZ 960), 3-(4-chloro-2-fluorobenzyl)-2-methyl-N-(3-methyl-1H-pyrazol-5-yl)-8-(morpholinomethyl)imidazo[1,2-b]pyridazin-6-amine (LY2784544), 1-cyclopropyl-3-(3-(5-(morpholinomethyl)-1H-benzo[d]imidazol-2-yl)-1H-pyrazol-4-yl)urea (AT9283), pacritinib (SB1518), (S)-N-(4-(2-((4-morpholinophenyl) amino)pyrimidin-4-yl)phenyl)pyrrolidine-2-carboxamide (XL019), and N-tert-butyl-3-(5-methyl-2-(4-(2-(pyrrolidin-1-yl)ethoxy)phenylamino) pyrimidin-4-ylamino)benzenesulfonamide (TG101348);
(c) a therapeutically effective quantity of a STAT5 inhibitor selected from the group consisting of N'-((4-oxo-4H-chromen-3-yl)methylene)nicotinohydrazide and pimozide; and
(d) a therapeutically effective quantity of a Src inhibitor selected from the group consisting of dasatinib, saracatinib, bosutinib, N-benzyl-2-(5-(4-(2-morpholinoethoxy)phenyl)pyridin-2-yl)acetamide (KX2-391), CGP76030, and 4-methyl-3-(1-methyl-6-(pyridin-3-yl)-1H-pyrazolo[3,4-d]pyrimidin-4-ylamino)-N-(3-(trifluoromethyl)phenyl)benzamide (NVP-BHG712).

6. The medicament for the use according to any one of claim 1 or 3, wherein the medicament is a drug combination further comprising a therapeutic agent selected from the group consisting of:
(a) a thymidylate synthetase inhibitor;
(b) a signal transduction inhibitor;
(c) a cisplatin or platinum analog;
(d) an alkylating agent;
(e) an anti-tubulin agent;
(f) an antimetabolite;
(g) berberine;
(h) apigenin;
(i) colchicine or an analog thereof selected from cholchiceinamide, N-desacetylthiocolchicine, demecolcine, N-acetyliodocolchinol, trimethylcolchicinie acid (TMCA) methyl ether, N-acetylcolchinol, TMCA ethyl ether, isocolchicine, isocolchiceinamide, iso-TMCA methyl ether, colchiceine, TMCA, N-benzoyl TMCA, colchicosamide, colchicoside, colchinol and colchinoic acid;
(j) genistein;
(k) etoposide;
(1) cytarabine;
(m) a camptothecin;
(n) a vinca alkaloid;
(o) a topoisomerase inhibitor;
(p) 5-fluorouracil;
(q) curcumin;
(r) a NF-κB inhibitor;
(s) rosmarinic acid;
(t) mitoguazone;
(u) meisoindigo;
(v) imatinib;
(w) dasatinib;
(x) nilotinib;
(y) an epigenetic modulator;
(z) a transcription factor inhibitor;
(aa) taxol;
(ab) homoharringtonine;
(ac) pyridoxal;
(ad) spiro germanium;
(ae) caffeine;
(af) nicotinamide;
(ag) methylglyoxalbisguanylhydrazone;
(ah) a Rho kinase inhibitor;
(ai) a 1,2,4-benzotriazine oxide;
(aj) an alkylglycerol;
(ak) an inhibitor of a Mer, Ax1, or Tyro-3 receptor kinase;
(al) an inhibitor of ATR kinase;
(am) a modulator of Fms kinase, Kit kinase, MAP4K4 kinase, TrkA kinase, or TrkB kinase;
(an) endoxifen;
(ao) a mTOR inhibitor;
(ap) an inhibitor of Mnkla kinase, Mknlb kinase, Mnk2a kinase, or Mnk2b kinase;
(aq) a modulator of pyruvate kinase M2;
(ar) a modulator of a phosphoinositide 3-kinase;
(as) a cysteine protease inhibitor;
(at) phenformin;
(au) a Sindbis virus-based vector;
(av) a peptidomimetics that act as mimetic of Smac and inhibits a IAP to promote apoptosis;
(aw) a Raf kinase inhibitor;
(ax) a nuclear transport modulator;
(ay) an acid ceramidase inhibitor and a choline kinase inhibitor;
(az) a tyrosine kinase inhibitor;
(ba) an anti-CSl antibody;
(bb) an inhibitor of protein kinase CK2;
(bc) an anti-guanylyl cyclase C (GCC) antibody;
(bd) a histone deacetylase inhibitor;
(be) a cannabinoid;
(bf) a glucagon-like peptide-1 (GLP-1) receptor agonist;
(bg) an inhibitor of Bc1-2 or Bc1-xL;
(bh) a Stat3 pathway inhibitor;
(bi) an inhibitor of polo-like kinase 1 (Plk1);
(bj) a GBPAR1 activator;
(bk) a modulator of serine-threonine protein kinase and poly(ADP-ribose) polymerase (PARP) activity;
(bl) a taxane;
(bm) an inhibitor of dihydrofolate reductase;
(bn) an inhibitor of aromatase;
(bo) a benzimidazole-based anti-neoplastic agent;
(bp) an O6-methylguanine-DNA-methyltransferase (MGMT) inhibitor;
(bq) a CCR9 inhibitor;
(br) an acid sphingomyelinase inhibitor;
(bs) a peptidomimetic macrocycle;
(bt) a cholanic acid amide;
(bu) a substituted oxazaphosphorine;
(bv) an anti-TWEAK receptor antibody;
(bw) an ErbB3 binding protein;
(bx) a glutathione S-transferase-activated anti-neoplastic compound;
(by) a substituted phosphorodiamidate;
(bz) an inhibitor of MEKK protein kinase;
(ca) a COX-2 inhibitor;
(cb) an anti-IL-6 receptor antibody;
(cc) an antioxidant;
(cd) an isoxazole inhibitor of tubulin polymerization;
(ce) a PARP inhibitor;
(cf) an Aurora protein kinase inhibitor;
(cg) a peptide that binds to prostate-specific membrane antigen;
(ch) a CD 19 binding agent;
(ci) a benzodiazepine;
(cj) a Toll-like receptor (TLR) agonist;
(ck) a bridged bicyclic sulfamide;
(cl) an inhibitor of epidermal growth factor receptor kinase;
(cm) a ribonuclease of the T2 family having actin-binding activity;
(cn) myrsinoic acid A;
(co) an inhibitor of a cyclin-dependent kinase;
(cp) an inhibitor of the interaction between p53 and MDM2;
(cq) an inhibitor of the receptor tyrosine kinase MET;
(cr) largazole;
(cs) an inhibitor of AKT protein kinase;
(ct) 2'-fluoro-5-methyl-β-L-arabinofuranosyluridine or L-deoxythymidine;
(cu) a HSP90 modulator;
(cv) an inhibitor of a JAK kinase;
(cw) an inhibitor of a PDK1 protein kinase;
(cx) a PDE4 inhibitor;
(cy) an inhibitor of proto-oncogene c-Met tyrosine kinase;
(da) an inhibitor of indoleamine 2,3-dioxygenase;
(db) an agent that inhibits expression of ATDC (TRIM29);
(dc) a proteomimetic inhibitor of the interaction of nuclear receptor with a coactivator peptide;
(dd) an antagonist of a XIAP family protein;
(de) a tumor-targeted superantigen;
(df) an inhibitor of a Pim kinase;
(dg) an inhibitor of a CHK1 or a CHK2 kinase;
(dh) an inhibitor of an angiopoietin-like 4 protein;
(di) a Smo antagonist;
(dj) a nicotinic acetylcholine receptor antagonist;
(dk) a farnesyl protein transferase inhibitor;
(dl) an adenosine A3 receptor antagonist;
(dm) a cancer vaccine;
(dn) a JAK2 inhibitor; and
(do) a Src inhibitor.

## Patentansprüche

1. Ein Medikament, das eine therapeutisch wirksame Menge eines alkylierenden Hexitolderivats beinhaltet, zur Verwendung bei der Behandlung einer malignen Erkrankung bei einem gegen Tyrosinkinasehemmer(TKI)-Chemotherapie resistenten Patienten, wobei die maligne Erkrankung durch Resistenz gegen mindestens einen Tyrosinkinasehemmer (TKI) gekennzeichnet ist, bedingt durch:
(1) mindestens eine Mutation in einem Gen, das für ein Protein kodiert, das ein Ziel von mindestens einen TKI ist; oder
(2) das Vorliegen von mindestens einem zusätzlichen Gen entweder in einem Wildtyp- oder mutierten Zustand, das für ein Protein kodiert, das Resistenz gegen die therapeutischen Wirkungen von mindestens einem TKI verleiht,
wobei das zusätzliche Gen entweder im Wildtyp- oder mutierten Zustand, das für das Protein kodiert, das Resistenz gegen die therapeutischen Wirkungen von mindestens einem TKI verleiht, *AHI-1* ist oder
wobei die Resistenz gegen mindestens einen TKI durch eine Mutation in der Kinasedomäne von ABL1-Protein bedingt ist, die Teil eines BCR-ABL-Fusionsprotein ist, das ein Ziel von TKIs ist,
wobei das alkylierende Hexitolderivat Dianhydrogalactitol ist und
wobei die maligne Erkrankung aus der Gruppe ausgewählt ist, die aus chronischer myeloischer Leukämie (CML), nichtkleinzelligem Lungenkarzinom (NSCLC) und tripelnegativem Brustkrebs besteht.

2. Ein Medikament, das eine therapeutisch wirksame Menge von Dianhydrogalactitol beinhaltet, zur Verwendung bei der Behandlung einer malignen Erkrankung bei einem gegen TKI-Chemotherapie resistenten Individuum, das an einer malignen Erkrankung leidet, der einen Keimbahn-Deletionspolymorphismus aufweist, der Resistenz gegen Tyrosinkinasehemmer (TKIs) verleiht, wobei die maligne Erkrankung aus der Gruppe ausgewählt ist, die aus chronischer myeloischer Leukämie (CML), nichtkleinzelligem Lungenkarzinom (NSCLC) und tripelnegativem Brustkrebs besteht.

3. Medikament zur Verwendung nach Anspruch 2, wobei der Keimbahn-DNA-Deletionspolymorphismus ein Keimbahn-DNA-Deletionspolymorphismus von 2903 bp, befindlich in dem *BIM-*Gen, ist und wobei der Keimbahn-DNA-Deletionspolymorphismus eine Spleißvariation verursacht, die zur Expression einer Isoform von BIM-Protein führt, der eine BH3-Domäne fehlt und die daher die Induktion der Apoptose hemmt.

4. Medikament zur Verwendung nach einem der Ansprüche 1 oder 2, wobei das Medikament ferner eine therapeutisch wirksame Menge eines BH3-Mimetikums beinhaltet, wobei das BH3-Mimetikum aus der Gruppe ausgewählt ist, die aus Folgenden besteht:
(a) einem Peptid;
(b) einem modifizierten Peptid;
(c) einem terpyridinbasierten Peptidomimetikum;
(d) einem terephthalamidbasierten Peptidomimetikum;
(e) einem benzoylharnstoffbasierten Peptidomimetikum;
(f) Obatoclax;
(g) TW37;
(h) einem Analogon von TW37, ausgewählt aus (1) einem Analogon von TW37, in dem einer oder mehrere der Wasserstoffe der Benzolringe durch ein Alkyl mit 1 bis 6 Kohlenstoffatomen ersetzt werden; und (2) einem Analogon von TW37, in dem einer oder mehrere der Wasserstoffe in den Hydroxylgruppen des Trihydroxyphenylteils durch ein Alkyl mit 1 bis 6 Kohlenstoffatomen ersetzt werden;
(i) (-)Gossypol;
(j) Apogossypolon;
(k) A-385358;
(l) einem Analogon von A-385358, ausgewählt aus (1) einem Analogon von A-385358, in dem einer oder mehrere der Wasserstoffe der Benzolringe durch ein Alkyl mit 1 bis 6 Kohlenstoffatomen ersetzt werden; und (2) einem Analogon von A-385358, in dem der Dimethylaminoteil durch einen anderen Teil ersetzt wird, der ein oder zwei Alkylgruppen mit 1 bis 6 Kohlenstoffatomen umfasst, die an die Aminogruppe des Diethylaminoteils gebunden sind;
(m) ABT-737;
(n) einem Analogon von ABT-737, ausgewählt aus (1) einem Analogon von ABT-737, in dem das Chlor, das an den Benzolring gebunden ist, durch Fluor, Brom oder Iod ersetzt wird; (2) einem Analogon von ABT-737, in dem einer oder mehrere der Wasserstoffe der Benzolringe durch Alkyl mit 1 bis 6 Kohlenstoffatomen ersetzt werden; (3) einem Analogon von ABT-737, in dem einer oder mehrere der Wasserstoffe des Piperazinylteils durch Alkyl mit 1 bis 6 Kohlenstoffatomen ersetzt werden; und (4) einem Analogon von ABT-737, in dem der Dimethylaminoteil durch einen anderen Teil ersetzt wird, der ein oder zwei Alkylgruppen mit 1 bis 6 Kohlenstoffatomen umfasst, die an die Aminogruppe des Diethylaminoteils gebunden sind;
(o) ABT-263;
(p) einem Analogon von ABT-263, ausgewählt aus (1) einem Analogon von ABT-263, in dem das Chlor, das an den Benzolring gebunden ist, durch Fluor, Brom oder Iod ersetzt wird; (2) einem Analogon von ABT-263, in dem einer oder mehrere der Wasserstoffe der Benzolringe durch Alkyl mit 1 bis 6 Kohlenstoffatomen ersetzt werden; (3) einem Analogon von ABT-263, in dem einer oder mehrere der Wasserstoffe des Piperazinylteils durch Alkyl mit 1 bis 6 Kohlenstoffatomen ersetzt werden; und (4) einem Analogon von ABT-263, in dem der Dimethylaminoteil durch einen anderen Teil ersetzt wird, der eine oder zwei Alkylgruppen mit 1 bis 6 Kohlenstoffatomen umfasst, die an die Aminogruppe des Diethylaminoteils gebunden sind;
(q) TM-1206; und
(r) einem Analogon von TM-1206, ausgewählt aus (1) einem Analogon von TM-1206, in dem einer oder mehrere der Wasserstoffe der Benzolringe durch Alkyl mit 1 bis 6 Kohlenstoffatomen ersetzt werden; und (2) einem Analogon von TM-1206, in dem einer oder mehrere der Wasserstoffe in den Hydroxylgruppen des Trihydroxyphenylteils durch Alkyl mit 1 bis 6 Kohlenstoffatomen ersetzt werden.

5. Medikament zur Verwendung nach Anspruch 4, wobei das Medikament ferner ein zusätzliches Therapeutikum beinhaltet, das aus der Gruppe ausgewählt ist, die aus Folgenden besteht:
(a) einer therapeutisch wirksamen Menge eines Tyrosinkinasehemmers, der aus der Gruppe ausgewählt ist, die aus Erlotinib, Afatinib, Dacomitinib, Imatinib, Bosutinib, Nilotinib und Dasatinib besteht;
(b) einer therapeutisch wirksamen Menge eines JAK2-Hemmers, der aus der Gruppe ausgewählt ist, die aus den Folgenden besteht: (E)-N-Benzyl-2-cyano-3-(3,4-dihydroxyphenyl)acrylamid (AG490), Ruxolitinib, Tofacitinib, Tofacitinibcitrat, N-tert-Butyl-3-(5-methyl-2-(4-(2-(pyrrolidin-1-yl)ethoxy)phenylamino)pyrimidin-4-ylamino)benzolsulfonamid (TG-101348), (S)-5-Chlor-N2-(1-(5-fluorpyrimidin-2-yl)ethyl)-N4-(5-methyl-1H-pyrazol-3-yl) pyrimidin-2,4-diamin (AZD1480), N-(Cyanomethyl)-4-(2-(4-morpholinophenylamino)pyrimidin-4-yl)benzamid (CYT387), Baricitinib, (S,E)-3-(6-Brompyridin-2-yl)-2-cyano-N-(1-phenylethyl)acrylamid (WP1066), S-Ruxolitinib, N-tert-Butyl-3-(5-methyl-2-(4-(4-methylpiperazin-1-yl)phenylamino)pyrimidin-4-ylamino)benzolsulfonamid (TG 101209), N-[3-(4-Methyl-1-piperazinyl)phenyl]-8-[4-(methylsulfonyl)phenyl]-[1,2,4]triazolo[1,5-a]pyridin-2-amin (CEP33779), 8-(3,5-Difluor-4-morpholinomethyl)phenyl)-2-(1(piperidin-4-yl)-1H-pyrazol-4-yl)chinoxalin (NVP-BSK805), (S)-5-Fluor-2-(1-(4-fluorphenyl)ethylamino)-6-(5-methyl-1H-pyrazol-3-ylamino)nicotinonitril (AZ 960), 3-(4-Chlor-2-fluorbenzyl)-2-methyl-N-(3-methyl-1H-pyrazol-5-yl)-8-(morpholinomethyl)imidazo[1,2-b]pyridazin-6-amin (LY2784544), 1-Cyclopropyl-3-(3-(5-(morpholinomethyl)-1H-benzo[d]imidazol-2-yl)-1H-pyrazol-4-yl)harnstoff (AT9283), Pacritinib (SB1518), (S)-N-(4-(2-((4-Morpholinophenyl)amino)pyrimidin-4-yl)phenyl)pyrrolidin-2-carboxamid (XL019) und N-tert-Butyl-3-(5-methyl-2-(4-(2-(pyrrolidin-1-yl)ethoxy)phenylamino)pyrimidin-4-ylamino)benzolsulfonamid (TG 101348);
(c) einer therapeutisch wirksamen Menge eines STAT5-Hemmers, der aus der Gruppe ausgewählt ist, die aus N'-((4-Oxo-4H-chromen-3-yl)methylen)nicotinohydrazid und Pimozid besteht; und
(d) einer therapeutisch wirksamen Menge eines Src-Hemmers, der aus der Gruppe ausgewählt ist, die aus Dasatinib, Saracatinib, Bosutinib, N-Benzyl-2-(5-(4-(2-morpholinoethoxy)phenyl)pyridin-2-yl)acetamid (KX2-391), CGP76030 und 4-Methyl-3-(1-methyl-6-(pyridin-3-yl)-1H-pyrazolo[3,4-d]pyrimidin-4-ylamino)-N-(3-(trifluormethyl)phenyl)benzamid (NVP-BHG712) besteht.

6. Medikament zur Verwendung nach einem der Ansprüche 1 oder 3, wobei das Medikament eine Wirkstoffkombination ist, die ferner ein Therapeutikum beinhaltet, das aus der Gruppe ausgewählt ist, die aus den Folgenden besteht:
(a) einem Thymidylatsynthetasehemmer;
(b) einem Signaltransduktionshemmer;
(c) einem Cisplatin- oder Platin-Analogon;
(d) einem Alkylierungsmittel;
(e) einem Antitubulinmittel;
(f) einem Antimetaboliten;
(g) Berberin;
(h) Apigenin;
(i) Colchicin oder einem Analogon davon, ausgewählt aus Cholchiceinamid, N-Desacetylthiocolchicin, Demecolcin, N-Acetyliodocolchinol, Trimethylcolchicinsäure(TMCA)-Methylether, N-Acetylcolchinol, TMCA-Ethylether, Isocolchicin, Isocolchiceinamid, Iso-TMCA-Methylether, Colchicein, TMCA, N-Benzoyl-TMCA, Colchicosamid, Colchicosid, Colchinol und Colchinoesäure;
(j) Genistein;
(k) Etoposid;
(l) Cytarabin;
(m) einem Camptothecin;
(n) einem Vincaalkaloid;
(o) einem Topoisomerasehemmer;
(p) 5-Fluorouracil;
(q) Curcumin;
(r) einem NF-KB-Hemmer;
(s) Rosmarinsäure;
(t) Mitoguazon;
(u) Meisoindigo;
(v) Imatinib;
(w) Dasatinib;
(x) Nilotinib;
(y) einem epigenetischen Modulator;
(z) einem Transkriptionsfaktorhemmer;
(aa) Taxol;
(ab) Homoharringtonin;
(ac) Pyridoxal;
(ad) Spirogermanium;
(ae) Coffein;
(af) Nicotinamid;
(ag) Methylglyoxalbisguanylhydrazon;
(ah) einem Rho-Kinasehemmer;
(ai) einem 1,2,4-Benzotriazinoxid;
(aj) einem Alkylglycerol;
(ak) einem Hemmer einer Mer-, Ax1- oder Tyro-3-Rezeptorkinase;
(al) einem Hemmer von ATR-Kinase;
(am) einem Modulator von Fms-Kinase, Kit-Kinase, MAP4K4-Kinase, TrkA-Kinase oder TrkB-Kinase;
(an) Endoxifen;
(ao) einem mTOR-Hemmer;
(ap) einem Hemmer von Mnk1a-Kinase, Mkn1b-Kinase, Mnk2a-Kinase oder Mnk2b-Kinase;
(aq) einem Modulator von Pyruvatkinase M2;
(ar) einem Modulator einer Phosphoinositid-3-kinase;
(as) einem Cysteinproteasehemmer;
(at) Phenformin;
(au) einem sindbisvirusbasierten Vector;
(av) einem Peptidomimetikum, das als Mimetikum von Smac wirkt und ein IAP hemmt, um Apoptose zu fördern;
(aw) einem Raf-Kinasehemmer;
(ax) einem Kerntransport-Modulator;
(ay) einem Säureceramidasehemmer und einem Cholinkinasehemmer;
(az) einem Tyrosinkinasehemmer;
(ba) einem Anti-CS1-Antikörper;
(bb) einem Hemmer von Proteinkinase CK2;
(bc) einem Anti-Guanylylcyclase-C(GCC)-Antikörper;
(bd) einem Histondeacetylasehemmer;
(be) einem Cannabinoid;
(bf) einem Glucagon-like-Peptid-1(GLP-1)-Rezeptoragonisten;
(bg) einem Hemmer von Bc1-2 oder Bc1-xL;
(bh) einem Stat3-Pathway-Hemmer;
(bi) einem Hemmer von Polo-like-Kinase 1 (Plk1);
(bj) einem GBPAR1-Aktivator;
(bk) einem Modulator von Serin-Threonin-Proteinkinase-und Poly(ADP-ribose)polymerase(PARP)-Aktivität;
(bl) einem Taxan;
(bm) einem Hemmer von Dihydrofolatreductase;
(bn) einem Hemmer von Aromatase;
(bo) einem benzimidazolbasierten antineoplastischen Mittel;
(bp) einem O6-Methylguanin-DNA-methyltransferase(MGMT)-Hemmer;
(bq) einem CCR9-Hemmer;
(br) einem Säuresphingomyelinasehemmer;
(bs) einem Peptidomimetikum-Makrozyklus;
(bt) einem Cholansäureamid;
(bu) einem substituierten Oxazaphosphorin;
(bv) einem Anti-TWEAK-Rezeptor-Antikörper;
(bw) einem ErbB3-Bindungsprotein;
(bx) einer Glutathion-S-transferase-aktivierten antineoplastischen Verbindung;
(by) einem substituierten Phosphorodiamidat;
(bz) einem Hemmer von MEKK-Proteinkinase;
(ca) einem COX-2-Hemmer;
(cb) einem Anti-IL-6-Rezeptor-Antikörper;
(cc) einem Antioxidans;
(cd) einem Isoxazolhemmer der Tubulinpolymerisation;
(ce) einem PARP-Hemmer;
(cf) einem Aurora-Proteinkinasehemmer;
(cg) einem Peptid, das an prostataspezifisches Membranantigen bindet;
(ch) einem CD19-Bindungsmittel;
(ci) einem Benzodiazepin;
(cj) einem Toll-like-Rezeptor(TLR)-Agonisten;
(ck) einem verbrückten bicyclischen Sulfamid;
(cl) einem Hemmer von epidermaler Wachstumsfaktor-Rezeptorkinase;
(cm) einer Ribonuclease der T2-Familie mit Actin-Bindungsaktivität;
(cn) Myrsinoesäure A;
(co) einem Hemmer einer cyclinabhängigen Kinase;
(cp) einem Hemmer der Wechselwirkung zwischen p53 und MDM2;
(cq) einem Hemmer der Rezeptortyrosinkinase MET;
(cr) Largazol;
(cs) einem Hemmer von AKT-Proteinkinase;
(ct) 2'-Fluor-5-methyl-β-L-arabinofuranosyluridin oder L-Deoxythymidin;
(cu) einem HSP90-Modulator;
(cv) einem Hemmer einer JAK-Kinase;
(cw) einem Hemmer einer PDK1-Proteinkinase;
(cx) einem PDE4-Hemmer;
(cy) einem Hemmer von Protoonkogen-c-Met-Tyrosinkinase;
(da) einem Hemmer von Indolamin-2,3-dioxygenase;
(db) einem Mittel, das die Expression von ATDC (TRIM29) hemmt;
(dc) einem Proteomimetikumhemmer der Wechselwirkung von Kernrezeptor mit einem Coaktivatorpeptid;
(dd) einem Antagonisten eines Proteins der XIAP-Familie;
(de) einem auf einen Tumor abzielenden Superantigen;
(df) einem Hemmer einer Pim-Kinase;
(dg) einem Hemmer einer CHK1- oder CHK2-Kinase;
(dh) einem Hemmer eines Angiopoetin-like-4-Proteins;
(di) einem Smo-Antagonisten;
(dj) einem Nicotinacetylcholin-Rezeptorantagonisten;
(dk) einem Farnesyl-Proteintransferasehemmer;
(dl) einem Adenosin-A3-Rezeptorantagonisten;
(dm) einem Krebsimpfstoff;
(dn) einem JAK2-Hemmer; und
(do) einem Src-Hemmer.

## Revendications

1. Médicament comprenant une quantité thérapeutiquement efficace d'un dérivé d'hexitol alkylant, destiné à une utilisation dans le traitement d'une malignité chez un patient résistant à une chimiothérapie par inhibiteur de la tyrosine kinase (ITK), la malignité étant **caractérisée par** une résistance à au moins un inhibiteur de la tyrosine kinase (ITK) due à :
(1) au moins une mutation dans un gène codant une protéine qui est une cible d'au moins un ITK ; ou
(2) la présence d'au moins un gène additionnel, soit à l'état sauvage, soit muté, codant une protéine qui apporte une résistance aux effets thérapeutiques d'au moins un ITK,
le gène additionnel soit à l'état sauvage, soit muté codant la protéine qui apporte une résistance aux effets thérapeutiques d'au moins un ITK étant *AHI-1*, ou
la résistance à au moins un ITK étant due à une mutation dans le domaine kinase de la protéine ABL1 qui fait partie d'une protéine de fusion BCR-ABL qui est une cible des IKT,
le dérivé d'hexitol alkylant étant le dianhydrogalactitol, et
la malignité étant sélectionnée dans le groupe constitué d'une leucémie myéloïde chronique (LMC), d'un carcinome pulmonaire non à petites cellules (CPNPC), et d'un cancer du sein triple-négatif.

2. Médicament comprenant une quantité thérapeutiquement efficace de dianhydrogalactitol, destiné à une utilisation dans le traitement d'une malignité chez un sujet résistant à une chimiothérapie par ITK souffrant d'une malignité présentant un polymorphisme de délétion au niveau de la lignée germinale qui apporte une résistance aux inhibiteurs de la tyrosine kinase (ITK), la malignité étant sélectionnée dans le groupe constitué d'une leucémie myéloïde chronique (LMC), d'un carcinome pulmonaire non à petites cellules (CPNPC), et d'un cancer du sein triple-négatif.

3. Médicament destiné à l'utilisation selon la revendication 2, le polymorphisme de délétion dans l'ADN de la lignée germinale étant un polymorphisme de délétion dans l'ADN de la lignée germinale au niveau du site 2903 bp situé dans le gène *BIM,* et le polymorphisme de délétion dans l'ADN de la lignée germinale provoquant une variation d'épissage qui conduit à l'expression d'un isoforme de la protéine BIM qui ne comporte pas de domaine BH3 et inhibe ainsi l'induction de l'apoptose.

4. Médicament destiné à l'utilisation selon l'une quelconque des revendications 1 ou 2, le médicament comprenant en outre une quantité thérapeutiquement efficace d'un mimétique de BH3, le mimétique de BH3 étant sélectionné dans le groupe constitué de :
(a) un peptide ;
(b) un peptide modifié ;
(c) un peptidomimétique à base de terpyridine ;
(d) un peptidomimétique à base de téréphtalamide ;
(e) un peptidomimétique à base de benzoylurée ;
(f) l'obatoclax ;
(g) le TW37 ;
(h) un analogue du TW37 sélectionné parmi (1) un analogue du TW37 dans lequel un ou plusieurs des atomes d'hydrogène des cycles benzène sont remplacés par un groupe alkyle comportant de 1 à 6 atomes de carbone ; et (2) un analogue du TW37 dans lequel un ou plusieurs des atomes d'hydrogène dans les groupes hydroxyle de la fraction trihydroxyphényle sont remplacés par un groupe alkyle comportant de 1 à 6 atomes de carbone ;
(i) le (-)-gossypol ;
(j) l'apogossypolone ;
(k) l'A-385358 ;
(l) un analogue de l'A-385358 sélectionné parmi (1) un analogue de l'A-385358 dans lequel un ou plusieurs des atomes d'hydrogène des cycles benzène sont remplacés par un groupe alkyle comportant de 1 à 6 atomes de carbone ; et (2) un analogue de l'A-385358 dans lequel la fraction diméthylamino est remplacée par une autre fraction comprenant un ou deux groupes alkyle comportant de 1 à 6 atomes de carbone liés au groupe amino de la fraction diéthylamino ;
(m) l'ABT-737 ;
(n) un analogue de l'ABT-737 sélectionné parmi (1) un analogue de l'ABT-737 dans lequel l'atome de chlore lié au cycle benzène est remplacé par un atome de fluor, de brome, ou d'iode ; (2) un analogue de l'ABT-737 dans lequel un ou plusieurs des atomes d'hydrogène des cycles benzène sont remplacés par un groupe alkyle comportant de 1 à 6 atomes de carbone ; (3) un analogue de l'ABT-737 dans lequel un ou plusieurs des atomes d'hydrogène de la fraction pipérazinyle sont remplacés par un groupe alkyle comportant de 1 à 6 atomes de carbone ; et (4) un analogue de l'ABT-737 dans lequel la fraction diméthylamino est remplacée par une autre fraction comprenant un ou deux groupes alkyle comportant de 1 à 6 atomes de carbone liés au groupe amino de la fraction diéthylamino ;
(o) l'ABT-263 ;
(p) un analogue de l'ABT-263 sélectionné parmi (1) un analogue de l'ABT-263 dans lequel l'atome de chlore lié au cycle benzène est remplacé par un atome de fluor, de brome, ou d'iode ; (2) un analogue de l'ABT-263 dans lequel un ou plusieurs des atomes d'hydrogène des cycles benzène sont remplacés par un groupe alkyle comportant de 1 à 6 atomes de carbone ; (3) un analogue de l'ABT-263 dans lequel un ou plusieurs des atomes d'hydrogène de la fraction pipérazinyle sont remplacés par un groupe alkyle comportant de 1 à 6 atomes de carbone ; et (4) un analogue de l'ABT-263 dans lequel la fraction diméthylamino est remplacée par une autre fraction comprenant un ou deux groupes alkyle comportant de 1 à 6 atomes de carbone liés au groupe amino de la fraction diéthylamino ;
(q) le TM-1206 ; et
(r) un analogue du TM-1206 sélectionné parmi (1) un analogue du TM-1206 dans lequel un ou plusieurs des atomes d'hydrogène des cycles benzène sont remplacés par un groupe alkyle comportant de 1 à 6 atomes de carbone ; et (2) un analogue du TM-1206 dans lequel un ou plusieurs des atomes d'hydrogène dans les groupes hydroxyle de la fraction trihydroxyphényle sont remplacés par un groupe alkyle comportant de 1 à 6 atomes de carbone.

5. Médicament destiné à l'utilisation selon la revendication 4, le médicament comprenant en outre un agent thérapeutique additionnel sélectionné dans le groupe constitué de :
(a) une quantité thérapeutiquement efficace d'un inhibiteur de la tyrosine kinase sélectionné dans le groupe constitué de l'erlotinib, de l'afatinib, du dacomitinib, de l'imatinib, du bosutinib, du nilotinib et du dasatinib ;
(b) une quantité thérapeutiquement efficace d'un inhibiteur de JAK2 sélectionné dans le groupe constitué du (E)-N-benzyl-2-cyano-3-(3,4-dihydroxyphényl)acrylamide (AG490), du ruxolitinib, du tofacitinib, du citrate de tofacitinib, du N-tert-butyl-3-(5-méthyl-2-(4-(2-(pyrrolidin-1-yl)éthoxy)phénylamino)pyrimidin-4-ylamino)benzènesulfonamide (TG-101348), de la (S)-5-chloro-N2-(1-(5-fluoropyrimidin-2-yl)éthyl)-N4-(5-méthyl-1H-pyrazol-3-yl)pyrimidine-2,4-diamine (AZD1480), du N-(cyanométhyl)-4-(2-(4-morpholinophénylamino)pyrimidin-4-yl)benzamide (CYT387), du baricitinib, du (S,E)-3-(6-bromopyridin-2-yl)-2-cyano-N-(1-phényléthyl)acrylamide (WP1066), du S-ruxolitinib, du N-tert-butyl-3-(5-méthyl-2-(4-(4-méthylpipérazin-1-yl)phénylamino)pyrimidin-4-ylamino)benzènesulfonamide (TG101209), de la N-[3-(4-méthyl-1-pipérazinyl)phényl]-8-[4-(méthylsulfonyl)phényl]-[1,2,4]triazolo[1,5-a]pyridin-2-amine (CEP33779), de la 8-(3,5-difluoro-4-(morpholinométhyl)phényl)-2-(1(pipéridin-4-yl)-1H-pyrazol-4-yl)quinoxaline (NVP-BSK805), du (S)-5-fluoro-2-(1-(4-fluorophényl)éthylamino)-6-(5-méthyl-1H-pyrazol-3-ylamino)nicotinonitrile (AZ 960), de la 3-(4-chloro-2-fluorobenzyl)-2-méthyl-N-(3-méthyl-1H-pyrazol-5-yl)-8-(morpholinométhyl)imidazo[1,2-b]pyridazin-6-amine (LY2784544), de la 1-cyclopropyl-3-(3-(5-(morpholinométhyl)-1H-benzo[d]imidazol-2-yl)-1H-pyrazol-4-yl)urée (AT9283), du pacritinib (SB1518), du (S)-N-(4-(2-((4-morpholinophényl)amino)pyrimidin-4-yl)phényl)pyrrolidine-2-carboxamide (XL019), et du N-tert-butyl-3-(5-méthyl-2-(4-(2-(pyrrolidin-1-yl)éthoxy)phénylamino)pyrimidin-4-ylamino)benzènesulfonamide (TG101348) ;
(c) une quantité thérapeutiquement efficace d'un inhibiteur de STAT5 sélectionné dans le groupe constitué du N'-((4-oxo-4H-chromén-3-yl)méthylène)nicotinohydrazide et du pimozide ; et
(d) une quantité thérapeutiquement efficace d'un inhibiteur de Src sélectionné dans le groupe constitué du dasatinib, du saracatinib, du bosutinib, du N-benzyl-2-(5-(4-(2-morpholinoéthoxy)phényl)pyridin-2-yl)acétamide (KX2-391), du CGP76030, et du 4-méthyl-3-(1-méthyl-6-(pyridin-3-yl)-1H-pyrazolo[3,4-d]pyrimidin-4-ylamino)-N-(3-(trifluorométhyl)phényl)benzamide (NVP-BHG712).

6. Médicament destiné à l'utilisation selon l'une quelconque des revendications 1 ou 3, le médicament étant une association médicamenteuse comprenant en outre un agent thérapeutique sélectionné dans le groupe constitué de :
(a) un inhibiteur de la thymidylate synthétase ;
(b) un inhibiteur de la transduction des signaux ;
(c) un analogue du cisplatine ou du platine ;
(d) un agent alkylant ;
(e) un agent anti-tubuline ;
(f) un antimétabolite ;
(g) la berbérine ;
(h) l'apigénine ;
(i) la colchicine ou un analogue de celle-ci sélectionné parmi le cholchicéinamide, la N-désacétylthiocolchicine, la démécolcine, le N-acétyliodocolchinol, l'éther méthylique de l'acide triméthylcolchicinique (TMCA), le N-acétylcolchinol, l'éther éthylique du TMCA, l'isocolchicine, l'isocolchicéinamide, l'éther méthylique de l'iso-TMCA, la colchicéine, le TMCA, l'acide N-benzoyltriméthylcolchicinique, le colchicosamide, le colchicoside, le colchinol et l'acide colchinoïque ;
(j) la génistéine ;
(k) l'étoposide ;
(l) la cytarabine ;
(m) une camptothécine ;
(n) un alcaloïde de la pervenche ;
(o) un inhibiteur de la topoisomérase ;
(p) le 5-fluoro-uracile ;
(q) la curcumine ;
(r) un inhibiteur de NF-kB ;
(s) l'acide rosmarinique ;
(t) la mitoguazone ;
(u) le méisoindigo ;
(v) l'imatinib ;
(w) le dasatinib ;
(x) le nilotinib ;
(y) un modulateur épigénétique ;
(z) un inhibiteur de facteur de transcription ;
(aa) le taxol ;
(ab) l'homoharringtonine ;
(ac) le pyridoxal ;
(ad) le spirogermanium ;
(ae) la caféine ;
(af) le nicotinamide ;
(ag) la méthylglyoxalbisguanylhydrazone ;
(ah) un inhibiteur de la Rho kinase ;
(ai) un oxyde de 1,2,4-benzotriazine ;
(aj) un alkylglycérol ;
(ak) un inhibiteur d'un récepteur à activité kinase Mer, Ax1, ou Tyro-3 ;
(al) un inhibiteur de l'ATR kinase ;
(am) un modulateur de la Fms kinase, de la Kit kinase, de la MAP4K4 kinase, de la TrkA kinase, ou de la TrkB kinase ;
(an) l'endoxifène ;
(ao) un inhibiteur de mTOR ;
(ap) un inhibiteur de la Mnk1a kinase, de la Mkn1b kinase, de la Mnk2a kinase, ou de la Mnk2b kinase ;
(aq) un modulateur de la pyruvate kinase M2 ;
(ar) un modulateur d'une phosphoinositide 3-kinase ;
(as) un inhibiteur de la cystéine protéase ;
(at) la phenformine ;
(au) un vecteur dérivé du virus Sindbis ;
(av) un peptidomimétique qui agit comme un mimétique de Smac et inhibe une protéine inhibitrice de l'apoptose (PIA) pour favoriser l'apoptose ;
(aw) un inhibiteur de la Raf kinase ;
(ax) un modulateur du transport nucléaire ;
(ay) un inhibiteur de la céramidase acide et un inhibiteur de la choline kinase ;
(az) un inhibiteur de la tyrosine kinase ;
(ba) un anticorps anti-CS1 ;
(bb) un inhibiteur de la protéine kinase CK2 ;
(bc) un anticorps anti-guanylyl cyclase C (GCC) ;
(bd) un inhibiteur d'histone désacétylase ;
(be) un cannabinoïde ;
(bf) un agoniste du récepteur du glucagon-like peptide-1 (GLP-1) ;
(bg) un inhibiteur de Bcl-2 ou Bcl-xL ;
(bh) un inhibiteur de la voie Stat3 ;
(bi) un inhibiteur de la polo-like kinase 1 (Plk1) ;
(bj) un activateur de GBPAR1 ;
(bk) un modulateur de l'activité des protéines sérine/thréonine kinases et de la poly(ADP-ribose) polymérase (PARP) ;
(bl) un taxane ;
(bm) un inhibiteur de la dihydrofolate réductase ;
(bn) un inhibiteur de l'aromatase ;
(bo) un agent antinéoplasique à base de benzimidazole ;
(bp) un inhibiteur de l'O6-méthylguanine-ADN-méthyltransférase (MGMT) ;
(bq) un inhibiteur de CCR9 ;
(br) un inhibiteur de la sphingomyélinase acide ;
(bs) un macrocycle peptidomimétique ;
(bt) un amide d'acide cholanique ;
(bu) une oxazaphosphorine substituée ;
(bv) un anticorps anti-récepteur de TWEAK ;
(bw) une protéine se liant à ErbB3 ;
(bx) un composé antinéoplasique activé par la glutathione S-transférase ;
(by) un phosphorodiamidate substitué ;
(bz) un inhibiteur de la protéine kinase MEKK ;
(ca) un inhibiteur de COX-2 ;
(cb) un anticorps anti-récepteur d'IL-6 ;
(cc) un antioxydant ;
(cd) un inhibiteur isoxazole de la polymérisation de la tubuline ;
(ce) un inhibiteur de PARP ;
(cf) un inhibiteur de la protéine kinase Aurora ;
(cg) un peptide qui se lie à l'antigène membranaire spécifique de la prostate ;
(ch) un agent qui se lie à CD19 ;
(ci) une benzodiazépine ;
(cj) un agoniste de récepteur Toll-like (TLR) ;
(ck) un sulfamide bicyclique ponté ;
(cl) un inhibiteur de kinase ciblant les récepteurs du facteur de croissance épidermique ;
(cm) une ribonucléase de la famille T2 ayant une activité de liaison à l'actine ;
(cn) l'acide myrsinoïque A ;
(co) un inhibiteur d'une kinase dépendante de la cycline ;
(cp) un inhibiteur de l'interaction entre p53 et MDM2 ;
(cq) un inhibiteur du récepteur à activité tyrosine kinase MET ;
(cr) le largazole ;
(cs) un inhibiteur de la protéine kinase AKT ;
(ct) la 2'-fluoro-5-méthyl-β-L-arabinofuranosyluridine ou la L-désoxythymidine ;
(cu) un modulateur de HSP90 ;
(cv) un inhibiteur d'une kinase JAK ;
(cw) un inhibiteur d'une protéine kinase PDK1 ;
(cx) un inhibiteur de PDE4 ;
(cy) un inhibiteur de la tyrosine kinase proto-oncogène c-Met ;
(da) un inhibiteur de l'indoléamine 2,3-dioxygénase ;
(db) un agent qui inhibe l'expression d'ATDC (TRIM29) ;
(dc) un inhibiteur protéomimétique de l'interaction d'un récepteur nucléaire avec un peptide co-activateur ;
(dd) un antagoniste d'une protéine de la famille XIAP ;
(de) un superantigène ciblé anti-tumoral ;
(df) un inhibiteur d'une Pim kinase ;
(dg) un inhibiteur d'une kinase CHK1 ou CHK2 ;
(dh) un inhibiteur d'une protéine 4 similaire à l'angiopoïétine ;
(di) un antagoniste de Smo ;
(dj) un antagoniste des récepteurs nicotiniques de l'acétylcholine ;
(dk) un inhibiteur de la farnésyl-protéine transférase ;
(dl) un antagoniste du récepteur A3 à l'adénosine ;
(dm) un vaccin contre le cancer ;
(dn) un inhibiteur de JAK2 ; et
(do) un inhibiteur de Src.
